(19) Europäisches Patentamt
European Patent Office
Office européen des brevets

(11) Publication number: **0 021 678**
**B1**

(12) # EUROPEAN PATENT SPECIFICATION

(45) Date of publication of patent specification: **07.11.84**

(21) Application number: **80301900.9**

(22) Date of filing: **06.06.80**

(51) Int. Cl.³: **C 07 D 205/08,**
C 07 D 403/12,
C 07 D 405/14,
C 07 D 409/14,
C 07 D 417/12,
C 07 D 417/14, A 61 K 31/395

(54) **1-Sulpho-2-oxoazetidine derivatives, their production and pharmaceutical compositions thereof.**

(30) Priority: **08.06.79 JP 72813/79**
**22.03.80 JP 36366/80**

(43) Date of publication of application:
**07.01.81 Bulletin 81/01**

(45) Publication of the grant of the patent:
**07.11.84 Bulletin 84/45**

(84) Designated Contracting States:
**AT BE CH DE FR GB IT LI LU NL SE**

(56) References cited:
**DE-A-2 529 941**
**DE-A-2 645 085**
**DE-A-2 735 854**

(73) Proprietor: **Takeda Chemical Industries, Ltd.**
**27, Doshomachi 2-chome Higashi-ku**
**Osaka-shi Osaka, 541 (JP)**

(72) Inventor: **Matsuo, Taisuke**
*
**deceased (JP)**
Inventor: **Sugawara, Tohru**
**5-10 Kofudai 3-chome**
**Toyono-cho Toyono-gun Osaka 563-01 (JP)**
Inventor: **Masuya, Hirotomo**
**1-19 Matsuodai 4-chome**
**Inagawa-cho Kawabe-gun Hyogo 666-02 (JP)**
Inventor: **Kawano, Yasuhiko**
**D-402, 21 Kashikiriyama**
**Suita, Osaka 565 (JP)**
Inventor: **Ochiai, Michihico**
**23-13, Senriyamahigashi, 1-chome**
**Suita Osaka 565 (JP)**

(74) Representative: **Lewin, John Harvey et al**
**Elkington and Fife High Holborn House 52/54**
**High Holborn**
**London WC1V 6SH (GB)**

**EP 0 021 678 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

This invention relates to novel azetidine derivatives and methods for producing these compounds.

Azetidine derivatives are disclosed in DE—A1—2529941, DE—A1—2645085 and DE—A1—2735854. These documents do not disclose azetidine compounds having a sulpho group at the 1-position.

In our search for new and useful azetidine derivatives, we have discovered azetidine derivatives having a sulpho group in the 1-position. Our finding was followed by further studies which have culminated in the completion of this invention.

Thus, the present invention provides a 1-sulpho-2-oxoazetidine derivative of the general formula:

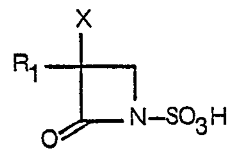

(I)

wherein $R_1$ is amino, acylated amino or protected amino and X is hydrogen or methoxy, or a salt thereof, but excluding the compound wherein X is methoxy and $R_1$ is amino substituted with an acyl group $R_7$—NH—CH($R_9$)—CO— in which $R_7$ is gammaglutamyl and $R_9$ is methyl and also provides a method for the production of a compound of the formula:

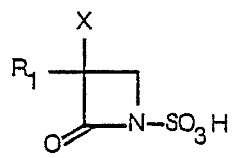

(I′)

wherein $R_1$ is amino, acylated amino or protected amino and X is hydrogen or methoxy, or a salt thereof.

The objective compounds of the formula (I′) may be produced by sulphonating a compound of the formula:

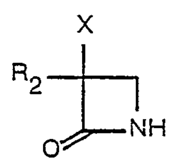

(II)

wherein $R_2$ is acylated amino or protected amino and X is as defined above, and optionally removing the amino-protecting group, or acylating the compound of the formula (IV) thus obtained:

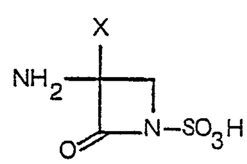

(IV)

wherein X is as defined above.

Referred to the above general formulae, the acyl groups on the acylated amino groups $R_1$ and $R_2$ may, for example, be the acyl groups which are found as substituents on the 6-amino group of known penicillin derivatives and the 7-amino group of known cephalosporin derivatives.

Specific examples of such acyl groups include the following:

(a) groups of the formula $R_6$—CO—

wherein $R_6$ is a lower alkyl group or a substituted or unsubstituted heterocyclic group;

(b) groups of the formula:

$R_7$—NH—CH—CO—
|
$R_9$

wherein $R_7$ is hydrogen, an optionally substituted amino acid residue, an amino-protecting group, a group of the formula $R_8$—$(CH_2)_n$—CO— [$R_8$ is an optionally substituted heterocyclic group, optionally substituted phenyl, optionally substituted lower alkyl, optionally substituted phenylthio or lower alkylthio; n is an integer of 0 to 4, the group —$(CH_2)_n$— optionally being substituted], a group of the formula

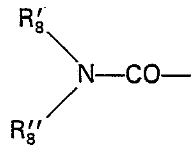

[$R_8'$ and $R_8''$ may be same or different, and are hydrogen, lower alkyl, lower alkyl carbamoyl optionally substituted phenylcarbonyl or sulpho] or a group of the formula $R_8'''$—$SO_2$—[$R_8'''$ is an optionally substituted lower alkyl];

$R_9$ is hydrogen, an optionally substituted lower alkyl, optionally substituted phenyl, optionally substituted heterocyclic group, cycloalkenylene or optionally substituted heterocyclecarbonylamino in which an alkylene chain may stand between the heterocyclic and carbonylamino moieties;

(c) groups of the formula:

$$R_{10}—R_{11}—CO—$$

wherein $R_{10}$ is a group of the formula

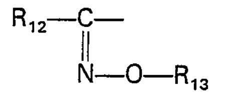

[$R_{12}$ is an optionally substituted heterocyclic group or optionally substituted phenyl, $R_{13}$ is hydrogen, optionally substituted lower acyl, lower alkyl or a group of the formula —$R_{14}R_{15}$($R_{14}$ is a lower alkylene or lower alkenylene group, $R_{15}$ is carboxyl, eser thereof or heterocyclic group)], $R_{121}$ is a bond or a group of the formula

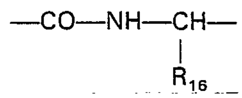

($R_{16}$ is a lower alkyl, optionally substituted phenyl or optionally substituted hetercyclic group);

(d) groups of the formula:

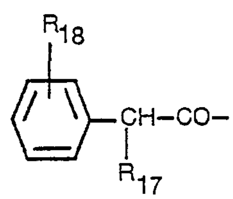

wherein $R_{17}$ is hydroxyl, sulphoxy, carboxyl, optionally substituted sulphamoyl, sulpho, optionally substituted phenoxycarbonyl, benzyloxycarbonyl or formyloxy; $R_{18}$ is hydrogen, a lower alkyl group, a lower alkoxy group, halogen, nitro or hydroxyl; and

(e) groups of the formula:

$$R_{19}—R_{20}—CH_2—CO—$$

wherein $R_{19}$ is a cyano, optionally substituted phenyl, optionally substituted phenoxy, optionally substituted lower alkyl, optionally substituted alkenyl or optionally substituted heterocyclic group; $R_{20}$ is a bond or —S—.

The lower alkyl group $R_6$ is preferably a group of 1 to 6 carbon atoms. The heterocyclic moiety of the optionally substituted heterocyclic group $R_6$ is a 5- or 6-membered heterocyclic group including 1 or 2 nitrogen atoms and may optionally include a single oxygen atom. Examples of this heterocyclic group include isoxazolyl, piperazinyl, imidazolinyl etc. The substituents on such heterocyclic groups may, for example, be lower alkyl of 1 to 3 carbon atoms, lower alkoxy of 1 to 3 carbon atoms, halogen, nitro, amino, oxo, thioxo and optionally substituted phenyl. The substituent on the aforementioned optionally substituted phenyl group may include, for example, lower alkyl of 1 to 3 carbon atoms, lower alkoxy of 1 to 3 carbon atoms, halogen, nitro and amino.

As examples of the amino acid residue of the optionally substituted amino acid residue $R_7$, there may be mentioned glycyl, alanyl, valyl, leucyl, isoleucyl, seryl, threonyl, cysteinyl, cystyl, methionyl, $\alpha$- or $\beta$-aspartyl, $\alpha$- or $\gamma$-glutamyl, lysyl, arginyl, phenylalanyl, phenylglycyl, tyrosyl, histidyl, tryptophyl, prolyl, etc. However, in the case of X=methoxy, an example ($R_7$=glutamyl, $R_9$=methyl) is eliminated. The substituent on the aforementioned optionally substituted amino acid residue may include, for example, amino, lower alkyl amino, amino-protecting group, carbamoyl, methylcarbamoyl, sulphamoyl, benzyl, 4-ethyl-2,3-dioxo-1-piperazinocarbonyl and 4-ethyl-2,3-dioxo-1-piperazinocarbonylamino.

The lower alkyl moiety of the lower alkylamino is preferably alkyl of 1 to 3 carbon atoms.

The amino-protecting group may, for example, by one of the protective groups mentioned hereinafter for amino groups. The amino-protecting group $R_7$ may, for example, be one of the protective groups mentioned hereinafter for amino groups.

The optionally substituted heterocyclic group $R_8$ in the group represented by the formula $R_8—(CH_2)_n—CO—$ includes, for example, 5- or 6-membered heterocyclic groups including one sulphur, nitrogen or oxygen atom, 5- or 6-membered heterocyclic groups including 2 to 4 nitrogen atoms, and 5- or 6-membered heterocyclic groups including one or two nitrogen atoms and one sulphur or oxygen atom. These heterocyclic groups may each be fused to a 6-membered ring including 1 or 2 nitrogen atoms, a benzene ring or a 5-membered ring including one sulphur atom. As examples of this heterocyclic group $R_8$, there may be mentioned 2-pyridyl, 3-pyridyl, 4-pyridyl, pyrimidyl, pyrazinyl, pyridazinyl, piperazinyl, pyrazolinyl, imidazolidinyl, thiazolyl, isothiazolyl, oxazolyl, isoxazolyl, pyrido [2,3-d]-pyrimidinyl, benzopyranyl, 1,8-naphthyridinyl, 1,5-naphthridinyl, 1,6-naphthyridinyl, 1,7-naphthyridinyl, 2,7-naphthyridinyl, 2,6-naphthyridinyl, quinolyl, thieno[2,3-b]pyridinyl, tetrazolyl, thiadiazolyl, oxadiazolyl, triazinyl, thienyl, pyrrolyl, furyl, etc. The substituents on such optionally substituted heterocyclic groups $R_8$ include, for example optionally substituted alkyl of 1 to 12 carbon atoms, lower alkoxy of 1 to 3 carbon atoms, hydroxyl, oxo, thioxo, formyl, trifluoromethyl, amino, halogen, lower alkylsulphonyl of 1 to 3 carbon atoms, coumarin-3-carbonyl, 4-formyl-1-piperazinyl, pyrrolaldoimino, furanaldoimino, thiophenaldoimino, mesyl, amino-protecting group, acylamino of 2 to 4 carbon atoms which may be substituted by halogen, etc. The amino-protecting group may, for example, be one of the protective groups mentioned hereinafter for amino groups. The substituents on the optionally substituted alkyl of 1 to 12 carbon atoms include, for example, phenyl, halogen, hydroxy, dialkylamino, etc. The alkyl moiety of the dialkylamino is preferably alkyl of 1 to 3 carbon atoms.

The substituents on the optionally substituted phenyl group $R_8$ include, for example, lower alkyl of 1 to 3 carbon atoms, lower alkoxy of 1 to 3 carbon atoms, halogen, hydroxyl and amino. The lower alkyl moiety of the lower alkylthio group $R_8$ is preferably alkyl of 1 to 3 carbon atoms.

The substituents on the optionally substituted phenylthio group $R_8$ include, for example, lower alkyl of 1 to 3 carbon atoms, lower alkoxy of 1 to 3 carbon atoms halogen, hydroxy, amino, etc.

The substituents which may optionally be substituted on the group represented by the formula $—(CH_2)_n—$ include, for example, amino and a group of the formula $—NH—COR_8''''$ [$R_8''''$ is amino or optionally substituted piperazinyl]. As examples of the substituent on the optionally substituted piperazinyl group $R_8''''$, there may be mentioned lower alkyl of 1 to 3 carbon atoms, lower alkoxy of 1 to 3 carbon atoms, hydroxy, oxo, thioxo and halogen.

Referring to the above formula, the lower alkyl represented by $R_8'$ and/or $R_8''$ is preferably a group of 1 to 3 carbon atoms. The lower alkyl moiety of the lower alkylcarbamoyl is preferably a group of 1 to 3 carbon atoms.

As examples of the substituents on the optionally substituted phenylcarbonyl group, there may be mentioned lower alkyl of 1 to 3 carbon atoms, lower alkoxy of 1 to 3 carbon atoms, halogen, hydroxy, sulphoxy, benzyloxy, etc. The lower alkyl moiety of the optionally substituted lower alkyl group $R_8'''$ in the formula $R_8'''—SO_2—$ is preferably a moiety of 1 to 6 carbon atoms, which may be substituted by one or two of substituents such as amino, carboxyl, benzyloxycarbonyl or protected amino. The protective group in the protected amino may, for example, be one of the protective groups mentioned hereinafter for amino groups.

The lower alkyl moiety of the optionally substituted lower alkyl group $R_9$ is preferably a moiety of 1 to 3 carbon atoms. As examples of the substituent on the optionally substituted lower alkyl, they may be mentioned phenyl, carbamoyl, methylcarbamoyl, methylthio, thienylacetamide, ethoxycarbonylmethylcarbamoyl, N-methyltetrazolylthio, halogen and sulphamoyl. The substituents on optionally substituted phenyl groups $R_9$ include, for example, lower alkyl of 1 to 3 carbon atoms, lower alkoxy of 1 to 3 carbon atoms, halogen, hydroxy, sulphoxy, benzyloxy, benzoyloxy, trimethylsilyl, acyloxy of 2 to 10 carbon atoms, etc.

The heterocyclic ring on the optionally substituted heterocyclic group $R_9$ may, for example, be a five-membered heterocyclic group with one sulphur, nitrogen or oxygen atom, a five-membered heterocyclic group with one or two nitrogen atoms and one sulphur or oxygen atom or a five- or six-membered heterocyclic group with 2 to 4 nitrogen atoms. Examples of such heterocyclic groups are thiazolyl, isothiazolyl, oxazolyl, isoxazolyl, thienyl, furyl, pyrrolyl, imidazolyl, pyrazinyl, pyrimidinyl, pyridazinyl, piperazinyl, triazinyl, tetrazolyl, thiadiazolyl, oxadiazolyl, etc. The substituents in these cases are lower alkyl of 1 to 3 carbon atoms, lower alkoxy of 1 to 3 carbon atoms, halogen, hydroxy, nitro, sulphoxy, amino and acylamino of 2 to 4 carbon atoms which may optionally be substituted by halogen, to name but a few.

The cycloalkenylene $R_9$ is preferably five- to six-membered cycloalkenylene, such as cyclohexenyl or cyclohexadienyl. The heterocyclic moiety of the optionally substituted heterocyclic-carbonylamino which may optionally have an alkylene chain between the heterocyclic and carbonylamino group represented by $R_9$ includes, for example, a six-membered heterocyclic group with two nitrogen atoms. Among such heterocyclic groups is piperazinyl. The substituents may, for example, be alkyl of 1 to 12

carbon atoms, lower alkoxy of 1 to 3 carbon atoms, oxo, thioxo, amino and so forth. The alkylene chain is preferably an alkylene chain of 1 to 3 carbon atoms and as examples of the chain they may be mentioned methylene, ethylene and n-propylene.

Referring, further to the above formula, the heterocyclic ring of the optionally substituted heterocyclic group $R_{12}$ in the group $R_{10}$ represented by the formula:

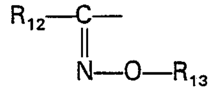

includes, for example, five-membered heterocyclic groups including one nitrogen, sulphur or oxygen atom, which five-membered heterocyclic groups may optionally include one nitrogen atom or no nitrogen atom. Among examples of the heterocyclic group are 2-thiazolyl, 4-thiazolyl, 5-thiazolyl, 2-thienyl, 3-thienyl, 2-furyl, 3-furyl, 2-pyrrolyl, 3-pyrrolyl, etc. The substituents on such heterocyclic group inlcude, for example, lower alkyl of 1 to 3 carbon atoms, lower alkoxy of 1 to 3 carbon atoms, hydroxy, halogen, amino, and acylamino group of 2 to 4 carbon atoms which may optionally be substituted by halogen.

The substituents on the optionally substituted phenyl group $R_{12}$ inlcude, for example, lower alkyl of 1 to 3 carbon atoms, lower alkoxy of 1 to 3 carbon atoms, halogen, nitro, amino, hydroxy and substituted hydroxy. The substituents of the substituted hydroxy may, for example, be benzyl, benzoyl, acyl of 2 to 10 carbon atoms, $\gamma$-D-glutamyl or 3-amino-3-carboxypropyl.

The lower alkyl group $R_{13}$ is preferably a group of 1 to 3 carbon atoms. The optionally substituted lower acyl group $R_{13}$ is preferably a group of 2 to 4 carbon atoms and the substituents of this acyl group may, for example, by halogen. The lower alkylene $R_{14}$ in the group $R_{14}$—$R_{15}$ of the group $R_{13}$ is preferably a group of 1 to 3 carbon atoms, such as methylene, ethylene, propylene, isopropylene, etc. The lower alkenylene $R_{14}$ is preferably a group of 2 to 3 carbon atoms, such as vinylene, propenylene, etc. The carboxyl ester $R_{15}$ may, for example, be the methyl ester, ethyl ester, propyl ester, etc. The heterocyclic group $R_{15}$ may, for example, be six-membered heterocyclic groups with one nitrogen and oxygen atom, such as morpholino, etc.

The lower alkyl group $R_{16}$ in the group $R_{11}$ as represented by the formula:

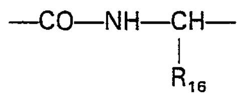

is preferably a group of 1 to 3 carbon atoms. As examples of substituents on optionally substituted phenyl groups $R_{16}$, there may be mentioned lower alkyl of 1 to 3 carbon atoms, lower alkoxy of 1 to 3 carbon atoms, halogen, nitro, amino, acyloxy of 2 to 10 carbon atoms, etc. The optionally substituted heterocyclic group $R_{16}$ may, for example, be five-membered heterocyclic groups with one sulphur, nitrogen or oxygen atom, five-membered heterocyclic groups with one or two nitrogen atoms and one sulphur or oxygen atom and five-membered heterocyclic groups with two to four nitrogen atoms, such as thiazolyl, isothiazolyl, oxazolyl, isoxazolyl, thienyl, furyl, pyrrolyl, thiadiazolyl, oxadiazolyl, triazinyl, tetrazolyl, thiazolyl, oxazolyl, isoxazolyl, thienyl, furyl, pyrrolyl, thiadiazolyl, oxadiazolyl, triazinyl, tetrazolyl, imidazolyl, pyrazinyl, pyrimidinyl, pyridazinyl, piperazinyl, etc. Substituents on the optionally substituted heterocyclic group include, for example, lower alkyl of 1 to 3 carbon atoms, lower alkoxy of 1 to 3 carbon atoms, halogen, hydroxy, amino and acylamino group of 2 to 4 carbon atoms which may optionally be substituted by halogen.

Substituents on optionally substituted sulphamoyl groups $R_{17}$ include, for example, lower alkyl of 1 to 3 carbon atoms, amidino, etc. Substituents on the optionally substituted phenoxycarbonyl group $R_{17}$ include, for example, lower alkyl of 1 to 3 carbon atoms and lower alkoxy of 1 to 3 carbon atoms.

The lower alkyl or lower alkoxy $R_{18}$ is preferably a group of 1 to 3 carbon atoms, respectively.

Substituents on optionally substituted phenyl groups $R_{19}$ include, for example, lower alkyl of 1 to 3 carbon atoms, lower alkoxy of 1 to 3 carbon atoms, halogen, nitro, amino, hydroxy, optionally substituted aminomethyl, etc. Substituents on the optionally substituted aminomethyl may, for example, by carbamoyl, (2-oxo-3-benzylideneamino-imidazolidin-1-yl)carbonyl, (2-oxoimidazolidin-1-yl)carbamoyl, etc. Substituents on the optionally substituted phenoxy group $R_{19}$, for example, include lower alkyl of 1 to 3 carbon atoms, lower alkoxy of 1 to 3 carbon atoms, halogen, nitro, amino, hydroxy and aminomethyl. The optionally substituted lower alkyl group $R_{19}$ is preferably a group of 1 to 6 carbon atoms, the substituents being exemplified by halogen, hydroxy, cyano, trifluoromethyl, etc.

The alkenyl of the optionally substituted alkenyl group $R_{19}$ may, for example, be vinyl propenyl, etc., and the substituents may, for example, be carboxyl, cyano, etc. Examples of the heterocyclic ring of the optionally substituted heterocyclic group $R_{19}$ include five- to six-membered heterocyclic groups including one sulphur atom or one to four nitrogen atoms and five- or six-membered heterocyclic groups including one sulphur atoms and one nitrogen or oxygen atom. Thus, 2-thienyl, 3-thienyl, 2-pyridyl, 3-pyridyl, 4-pyridyl, 2-thiazolyl, 4-thiazolyl, 5-thiazolyl, isothiazolyl, 1-tetrazolyl, 5-tetrazolyl,

pyrrolidinyl, imidazolyl, 1,4-oxathiinyl, etc., may be mentioned by way of example. Substituents on such optionally substituted heterocyclic group $R_{19}$ include, for example, lower alkyl of 1 to 3 carbon atoms, lower alkoxy of 1 to 3 carbon atoms, halogen, nitro, hydroxy, amino carboxy, oxo, acylamino of 2 to 4 carbon atoms which may optionally be substituted by halogen, acyl of 2 to 4 carbon atoms and so forth.

The alkyl group of 1 to 12 carbon atoms, mentioned hereinbefore, may, for example, be methyl, trifluoromethyl, ethyl, n-propyl, isopropyl, n-butyl, isobutyl, sec-butyl, tertbutyl, pentyl, isopentyl, hexyl, isohexyl, heptyl, octyl, nonyl, decyl, undecyl, dodecyl, or the like.

The lower alkyl group of 1 to 6 carbon atoms, mentioned hereinbefore, may, for example, be methyl trifluoromethyl, ethyl, n-propyl, isopropyl, n-butyl, isobutyl, sec-butyl, tert-butyl, pentyl, isopentyl, hexyl, isohexyl, etc.

The lower alkyl group of 1 to 3 carbon atoms, also mentioned hereinbefore, may, for example, be methyl, trifluoromethyl, ethyl, n-propyl, isopropyl or the like.

The lower alkoxy group of 1 to 3 carbon atoms, mentioned hereinbefore, may, for example, be methoxy, ethoxy, n-propoxy, isopropoxy or the like.

The halogen includes chlorine, bromine, iodine and fluorine.

The lower alkylsulphonyl group containing 1 to 3 carbon atoms includes, for example, methylsulphonyl, ethylsulphonyl, n-propylsulphonyl, isopropylsulphonyl, etc.

The acylamino group of 2 to 4 carbon atoms includes, for example, acetylamino, propionylamino, n-butyrylamino, isobutyrylamino, etc.

The acyloxy group of 2 to 10 carbon atoms includes, for example, acetoxy, n-propionyloxy, n-butyryloxy, isobutyryloxy, n-pentanoxyloxy, n-hexanoyloxy, n-heptanoyloxy, n-octanoyloxy, n-nonanoyloxy, n-decanoyloxy, etc.

Referred to the aforementioned acyl group, the acyl group represented by the formula $R_6$—CO—(wherein $R_6$ has the same meaning as defined hereinbefore) includes, for example, 3-(2,6-dichlorophenyl)-5-methylisoxazol-4-yl-carbonyl, 4-ethyl-2,3-dioxo-1-piperazinocarbonyl, (2-oxoimidazolidin-1-yl)carbonyl, etc.

The acyl group represented by the formula:

$$R_7\text{—NH—}\underset{\displaystyle R_9}{\underset{|}{\text{CH}}}\text{—CO—}$$

(wherein $R_7$ and $R_9$ have the same meanings as defined hereinbefore) includes, for example,

D-alanyl,
D-phenylalanyl,
α-benzyl N-carbobenzoxy-γ-D-glutamyl-D-alanyl,
D-phenylglycyl-D-alanyl,
N-carbobenzoxy-D-phenylglycyl,
D-alanyl-D-phenylglycyl,
γ-D-glutamyl-D-alanyl,
N-carbobenzoxy-D-alanyl-D-phenylglycyl,
D-carbamoyltryptophyl-D-phenylglycyl,
methylamidoasparaginyl-D-phenylglycyl,
N-carbobenzoxymethylamidoasparaginyl-D-phenylglycyl,
N-carbobenzoxy-D-phenylglycyl-D-phenylglycyl,
2-(2,3-diaminopropionamido)-2-phenylacetyl,
D-alanyl-D-alanyl, 2-[2-amino-3-(N-methylcarbamoyl)propionamido]acetyl,
2-(2-amino-3-sulphamoylpropionamido)-2-phenylacetyl,
2-[2-amino-3-(4-ethyl-2,3-dioxo-1-piperazinocarboxamido)-propionamido]-2-phenylacetyl,
D-2-(4-ethyl-2,3-dioxo-1-piperazinocarboxamido)-2-(4-methoxyphenyl)acetyl,
4-ethyl-2,3-dioxo-1-piperazinocarbonyl,
D-2-[2,(4-ethyl-2,3-dioxo-1-piperazinocarboxamido)-3-(N-methylcarbamoyl)propionamido]-2-phenylacetyl,
D-2-[2-(4-ethyl-2,3-dioxo-1-piperazinocarboxamido)acetamido]-2-phenylacetyl,
D-2-(3-sulphamoyl-2-benzyloxycarboxamidopropionamido)-2-phenylacetyl,
D-2-[2-benzyloxycarboxamido-3-(4-methoxyphenyloxycarboxamido)-propionamido]-2-phenylacetyl,
2-[2-benzyloxycarboxamido-3-(N-methylcarbamoyl)propionamido]acetyl,
2-(N-carbobenzoxy-D-phenylglycylamino)-3-(N-methylcarbamoyl)propionyl,
N-carbobenzoxy-D-alanyl,
2-(benzyloxycarboxamido)-2-phenylacetyl,
2-benzyloxycarboxamido-3-N-methylcarbamoylpropionyl,
N-(4-ethyl-2,3-dithioxo-1-piperazinocarbonyl)-D-phenylglycyl,
2-(2-amino-4-thiazoyl)-2-(4-ethyl-2,3-dioxo-1-piperazinocarboxamido)acetyl,
2-(2-phenylacetamido)propionyl,

2-(4-ethyl-2,3-dioxo-1-piperazinocarboxamido)-2-thienylacetyl,
2-(3-furfurylideneamino-2-oxoimidazolidine-1-carboxamido)-2-(4-hydroxyphenyl)acetyl,
2-(8-hydroxy-1,5-naphthyridine-7-carboxamido)-2-phenylacetyl,
2-(4-ethyl-2,3-dioxo-1-piperazinocarboxamido)-2-phenylacetyl,
2-(4-n-octyl-2,3-dioxo-1-piperazinocarboxamido)-2-phenylacetyl,
2-(4-ethyl-2,3-dioxo-1-piperazinocarboxamido)-2-(4-sulphoxyphenyl)acetyl,
2-(4-ethyl-2,3-dioxo-1-piperazinocarboxamido)-2-(4-chlorophenyl)acetyl,
2-(4-n-octyl-2,3-dioxo-1-piperazinocarboxamido)-2-(4-hydroxysulphonyloxyphenyl)acetyl,
2-(4-ethyl-2,3-dioxo-1-piperazinocarboxamido)-2-(4-methoxyphenyl)acetyl,
2-(4-ethyl-2,3-dioxo-1-piperazinocarboxamido)-2-(4-trimethylsilylphenylacetyl),
2-(4-ethyl-2,3-dioxo-1-piperazinocarboxamido)-2-(3-chloro-4-methoxyphenyl)acetyl,
2-(4-ethyl-2,3-dioxo-1-piperazinocarboxamido)-2-(3-chloro-4-hydroxysulphonyloxyphenyl)acetyl,
2-(4-ethyl-2,3-dioxo-1-piperazinocarboxamido)-2-(3-chloro-4-hydroxyphenyl)acetyl,
2-(4-ethyl-2,3-dioxo-1-piperazinocarbonyl)-2-(4-benzyloxyphenyl)acetyl,
2-(4-n-octyl-2,3-dioxo-1-piperazinocarboxamido)-2-(4-hydroxyphenyl)acetyl,
N-(4-ethyl-2,3-dioxo-1-piperazinocarbonyl)glutaminyl,
N-(4-ethyl-2,3-dioxo-1-piperazinocarbonyl)phenylalanyl,
N-(4-ethyl-2,3-dioxo-1-piperazinocarbonyl)-D-alanyl,
2-(4-ethyl-2,3-dioxo-1-piperazinocarboxamido)-2-(4-hydroxyphenyl)acetyl,
2,2-bis(4-ethyl-2,3-dioxo-1-piperazinocarboxamido)acetyl,
2-(4-ethyl-2,3-dioxo-1-piperazinocarboxamido)-2-(1-cyclohexen-1-yl)acetyl,
2-(4-n-octyl-2,3-dioxo-1-piperazinocarboxamido)-2-thienylacetyl,
2-(4-ethyl-2,3-dioxo-1-piperazinocarboxamido)-2-(2-amino-4-thiazolyl)acetyl,
2-(4-ethyl-2,3-dioxo-1-piperazinocarboxamido)-2-(2-chloroacetamido-4-thiazolyl)acetyl,
2-(4-ethyl-2,3-dioxo-1-piperazinocarboxamido)-2-(2-methyl-4-thiazolyl)acetyl,
2-(4-ethyl-2,3-dioxo-1-piperazinocarboxamido)-2-(2-acetamido-4-thiazolyl)acetyl,
2-(4-n-octyl-2,3-dioxo-1-piperazinocarboxamido)-2-(2-amino-4-thiazolyl)acetyl,
2-(4-ethyl-2,3-dioxo-1-piperazinocarboxamido)-2-furylacetyl,
2-(4-ethyl-2,3-dioxo-1-piperazinocarboxamido)-2-(2-pyrrolyl)acetyl,
2-(4-ethyl-2,3-dithioxo-1-piperazinocarboxamido)-2-(4-hydroxyphenyl)acetyl,
2-(4-n-octyl-2,3-dioxo-1-piperazinocarboxamido)-2-(2-chloroacetamido-4-thiazolyl)acetyl,
N-(4-ethyl-2,3-dioxo-1-piperazinocarbonyl)-D-methionyl,
D-2-[4-(2-phenylethyl)-2,3-dioxo-1-piperazinocarboxamido]phenylacetyl,
D-2-(4-ethyl-2,3-dioxo-1-piperazinocarboxamido)-2-(4-benzoyloxyphenyl)acetyl,
2,5-bis(4-ethyl-2,3-dioxo-1-piperazinocarboxamido)pentanoyl,
2-(4-ethyl-2,3-dioxo-1-piperazinocarboxamido)-3-N-methylcarbamoyl)propionyl,
2,3-bis(4-ethyl-2,3-dioxo-1-piperazinocarboxamido)propionyl,
2-(4-ethyl-2,3-dioxo-1-piperazinocarboxamido)-3-chloro-propionyl,
2-(4-ethyl-2,3-dioxo-1-piperazinocarboxamido)-2-(4-n-octanoyloxyphenyl)acetyl,
2-(4-ethyl-2,3-dioxo-1-piperazinocarboxamido)-3-sulphamoylpropionyl,
2-(4-ethyl-2,3-dioxo-1-piperazinocarboxamido)-3-[(1-methyl-1H-tetrazol-5-yl)thio]propionyl,
2-(4-ethyl-2,3-dioxo-1-piperazinocarboxamido)acetyl,
D-2-[4-(2-hydroxyethyl)-2,3-dioxo-1-piperazinocarboxamido]-2-phenylacetyl,
D-2-[4-(2-chloroethyl)-2,3-dioxo-1-piperazinocarboxamido]-2-phenylacetyl,
2-(4-ethyl-2,3-dioxo-1-piperazinocarboxamido)-3-(ethoxycarbonylmethylcarbamoyl)-propionyl,
2-(4-ethyl-2,3-dioxo-1-piperazinocarboxamido)-3-(thienylacetamido)propionyl,
2-(4-ethyl-2,3-dioxo-1-piperazinocarboxamido)-3-[2-(1H-tetrazol-1-yl)acetamido]propionyl,
2-(4-ethyl-2,3-dioxo-1-piperazinocarboxamido)-2-(1H-tetrazol-1-yl)acetyl,
2-[(2-oxo-3-furfurylideneaminoimidazolidin-1-yl)carboxamido]-2-phenylacetyl,
2-[(2-oxo-3-furfurylideneaminoimidazolidin-1-yl)carboxamido]-2-(4-hydroxyphenyl)acetyl,
2-[(2-oxo-3-furfurylideneaminoimidazolidin-1-yl)carboxamido]-2-(4-hydroxysulphonyloxyphenyl)-acetyl,
2-[[2-oxo-3-(thiophen-2-aldoimino)imidazolidin-1-yl]carboxamido]-2-phenylacetyl,
2-[(2-oxo-3-furfurylideneaminoimidazolidin-1-yl]carboxamido]-2-thienylacetyl,
2-(3-methylsulphonyl-2-oxoimidazolidine-1-carboxamido)-2-phenylacetyl,
2-[(2-oxo-3-furfurylideneaminoimidazolin-1-yl)carboxamido]-2-(2-amino-4-thiazoyl)acetyl,
2-[(2-oxo-3-furfurylideneaminoimidazolidin-1-yl)carboxamido]-2-(2-chloroacetamido-4-thiazolyl)-acetyl,
2-[(3-mesyl-2-oxoimidazolidin-1-yl)-carboxamido]-2-phenylacetyl,
2-[[2-oxo-3-(thiophen-2-aldoimino)imidazolidin-1-yl]carboxamido]-2-thienylacetyl,
2-[(3-mesyl-2-oxoimidazolidin-1-yl)carboxamido]-2-thienylacetyl,
D-2-[(2-oxo-3-furfurylideneaminoimidazolidin-1-yl)carboxamido]propionyl,
2-(4-hydroxy-6-methylnicotinamido)-2-phenylacetyl,
2-(4-hydroxy-6-methylnicotinamido)-2-(4-hydroxyphenyl)acetyl,

2-[5,8-dihydro-2-(4-formyl-1-piperazinyl)-5-oxopyrido[2,3-d]pyrimidine-6-carboxamido]-2-phenylacetyl,
2-(3,5-dioxo-1,2,4-triazine-6-carboxamido)-2-(4-hydroxyphenyl)-acetyl,
2-(3-furfurylideneamino-2-oxomidazolidine-1-carboxamido)-2-phenylacetyl,
2-(courmarine-3-carboxamido)-2-phenylacetyl,
2-(4-hydroxy-7-methyl-1,8-naphthyridine-3-carboxamido)-2-phenylacetyl,
2-(4-hydroxy-7-trifluoromethylquinoline-3-carboxamido)-2-phenylacetyl,
N-[2-(2-amino-4-thiazolyl)acetyl]-D-phenylglycyl,
2-(6-bromo-1-ethyl-1,4-dihydro-4-oxothieno[2,3-b]pyridine-3-carboxamido)-2-phenylacetyl,
2-[2-(2-amino-4-thiazolyl)acetamido]-2-phenylacetyl,
2-[2-(2-chloroacetamido-4-thiazolyl)acetamido]-2-phenylacetyl,
2-(2,5-dioxo-1,2,4-triazino-6-carboxamido)-2-thienylacetyl,
2-(2,4-dioxopyrimidino-5-carboxamido)-2-thienylacetyl,
2-(6-hydroxy-1,5-naphthyridinylcarboxamido)-2-phenylacetyl,
2-[2-(4-ethyl-2,3-dioxo-1-piperazinocarboxamido)-2-thienylacetamido]-2-phenylacetyl,
2-(2-ureido-2-thienylacetamido)-2-phenylacetyl,
2-(2-ureido-2-thienylacetamido)-2-(4-hydroxysulphonyloxyphenyl)acetyl,
2-(2-ureido-2-thienylacetamido)-2-(4-hydroxyphenyl)acetyl,
2-(N-carbobenzoxypropylamino)-2-furylacetyl,
α-(thienylmethylcarbonyl)alanyl,
2-(4-chlorobenzoylureido)-2-thienylacetyl,
2-(2-(thienylacetamido)acetyl,
N-benzylcarboxamido-D-alanyl,
N-(4-hydroxybenzoyl)-D-alanyl,
2-(4-chlorobenzamido)propionyl,
2-(4-aminobenzamido)acetyl,
N-(4-ethyl-2,3-dioxo-1-piperazinocarbonyl)methionyl-D-phenylglycyl,
D-2-[2-(2,6-dichlorophenylthio)acetamido]-2-phenylacetyl,
2-(carbamoyl)amino-2-thienylacetyl,
N-carbamoyl-D-phenylglycyl,
2-(3-methylcarbamoyl-3-methyl-1-ureido)-2-phenylacetyl,
2-(3-methylcarbamoyl-3-methyl-1-ureido)-2-(4-hydroxyphenyl)acetyl,
2-(3-methylcarbamoyl-3-methyl-1-ureido)-2-thienylacetyl,
2-[3-(2-hydroxybenzoyl)-1-ureido]-2-phenylacetyl,
2-[3-(2-benzoyloxybenzoyl)-1-ureido]-2-(4-hydroxysulphonyloxyphenyl)acetyl,
2-[3-(2-hydroxybenzoyl)-1-ureido]-2-(4-hydroxyphenyl)acetyl,
2-[3-(2-benzyloxybenzoyl)-1-ureido]-2-phenylacetyl,
2-[3-(2-benzyloxybenzoyl)-1-ureido]-2-(4-hydroxyphenyl)acetyl,
D-2-[2-(benzyloxycarboxamido)-2-(benzyloxycarbonyl)ethanesulphonamido]-2-phenylacetyl,
N-mesyl-D-phenylglycyl, etc.

The acyl group represented by the formula $R_{10}$—$R_{11}$—CO— (wherein $R_{10}$ and $R_{11}$ have the same meanings as defined hereinbefore) inludes, for example,
N-[2-(2-amino-4-thiazolyl)-2-methoxyiminoacetyl]-D-alanyl,
N-[2-(2-amino-4-thiazoyl)-2-methoxyiminoacetyl]-D-phenylglycyl,
2-(2-amino-4-thiazolyl)-2-[2-(2-amino-4-thiazolyl)-2-methoxyiminoacetamido]acetyl,
2-(2-chloroacetamido-4-thiazolyl)-2-[2-(2-chloroacetamido-4-thiazolyl)-2-methoxyimino-acetamido]acetyl,
2-(2-chloroacetamido-4-thiazolyl)-2-methoxyiminoacetyl,
2-(2-amino-4-thiazolyl)-2-methoxyiminoacetyl,
2-(2-amino-4-thiazolyl)-2-oxyiminoacetyl,
2-thienyl-2-methoxyiminoacetyl,
2-furyl-2-methoxyiminoacetyl,
2-(4-hydroxyphenyl)-2-methoxyiminoacetyl,
2-phenyl-2-methoxyiminoacetyl,
2-phenyl-2-oxoiminoacetyl,
2-thienyl-2-oxyiminoacetyl,
2-thienyl-2-dichloroacetyloxyiminoacetyl,
2-[4-(γ-D-glutamyloxy)phenyl]-2-oxyiminoacetyl,
2-[4-(3-amino-3-carboxypropoxy)phenyl]-2-oxyiminoacetyl,
2-thienyl-2-(3-morpholinopropoxyimino)acetyl,
2-[2-(2-amino-4-thiazolyl)-2-methoxyiminoacetamido]-2-phenylacetyl,
2-[2-(2-chloroacetamido-4-thiazolyl)-2-methoxyiminoacetamido]-2-phenylacetyl,
2-[2-(2-amino-4-thiazolyl)-2-methoxyiminoacetamido]acetyl, etc.

The acyl group represented by the formula:

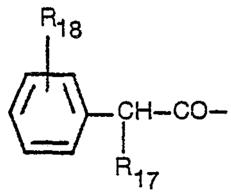

(wherein $R_{17}$ and $R_{18}$ have the same meanings as defined hereinbefore) includes for example,
α-sulphophenylacetyl,
α-sulphoxyphenylacetyl,
α-hydroxyphenylacetyl,
α-sulphamoylphenylacetyl,
α-phenoxycarbonylphenylacetyl,
α-(p-tolyoxycarbonyl)phenylacetyl,
α-formyloxyphenylacetyl,
α-carboxyphenylacetyl,
α-benzyloxycarbonylphenylacetyl,
2-(N,N-dimethylsulphamoyl)-2-phenylacetyl, etc.

The acyl group of the formula:

$$R_{19}—R_{20}—CH_2—CO—$$

(wherein $R_{19}$ and $R_{20}$ have the same meanings as defined hereinbefore) includes, for example,
cyanoacetyl,
phenylacetyl,
phenoxyacetyl,
trifluoromethylthioacetyl,
cyanomethylthioacetyl,
1H-tetrazoyl-1-acetyl,
2-thienylacetyl,
2-(2-amino-4-thiazolyl)acetyl,
2-(2-chloroacetamido-4-thiazolyl)acetyl,
4-pyridylthioacetyl,
2-thienylthioacetyl,
3,5-dichloro-1,4-dihydro-4-oxopyridine-1-acetyl,
β-carboxyvinylthioacetyl,
2-(2-aminomethylphenyl)acetyl,
2-(2-N-carbobenzoxyaminomethylphenyl)acetyl,
2-(2-ureidomethylphenyl)acetyl,
2-[2-(2-oxoimidazolidin-1-yl)carbonylaminomethylphenyl]acetyl,
2-[2-(2-oxo-3-benzylideneaminoimidazolidin-1-yl)carbonylaminomethylphenyl]acetyl,
2-(5,6-dihydro-1,4-oxathiin-2-yl)acetyl,
2-(2,5-dioxopyrrolidin-3-yl)acetyl,
2-succinimidoacetyl,
2-(1-acetyl-2,4-dioxoimidazolidin-3-yl)acetyl, etc.

The amino and/or carboxyl group in the acyl group described above may optionally carry a protective group.

Such amino-protecting groups include those groups which will be mentioned hereinafter as "amino-protecting groups". The carboxyl-protecting groups include any and all groups, such as ester and silyl residues, which are usually employed for the protection of carboxyl in the field of β-lactam chemistry and in organic chemistry in general, such as ester and silyl residues. Thus, for example, the ester and silyl residues may be methyl, ethyl, propyl, isopropyl, tert-butyl, tert-amyl, benzyl, p-nitrobenzyl, p-methoxybenzyl, benzhydryl, phenacyl, phenyl, p-nitrophenyl, methoxymethyl, ethoxymethyl, benzyloxymethyl, acetoxymethyl, pivaloxymethyl, β-methylsulphonylethyl, methylthiomethyl, trityl, β,β,β-trichloroethyl, β-iodoethyl, trimethylsilyl, dimethylsilyl, acetylmethyl, p-nitrobenzoylmethyl, p-mesylbenzoylmethyl, phthalimidomethyl, propionyloxymethyl, 1,1-dimethylpropyl, 3-methyl-3-butenyl, succinimidomethyl, 3,5-di-tert-butyl-4-hydroxybenzyl, mesylmethyl, benzenesulphonylmethyl, phenylthiomethyl, dimethylaminoethyl, pyridine-1-oxide-2-methyl, methylsulphinylmethyl, bis(p-methoxyphenyl)methyl, 2-cyano-1,1-dimethylethyl, etc. This invention provides new monocyclic compounds and the selection of such protective groups is only marginal to the gist of this invention. Especially, benzyl, β,β,β-trichloroethyl, p-nitrobenzyl or p-methoxybenzyl is preferable.

The "amino-protecting groups" which is used to protect the amino group in the practice of this invention may expediently be one of those groups used in the field of β-lactam chemistry or in the field of peptide synthesis. Thus, use may be made, for example, of aromatic acyl groups such as phthaloyl, p-nitrobenzoyl, p-tert-butylbenzoyl, p-tertbutylbenzenesulphonyl, benzenesulphonyl, toluenesulphonyl,

etc,; aliphatic acyl groups such as formyl, acetyl, propionyl, monochloroacetyl, dichloroacetyl, trichloroacetyl, methanesulphonyl, ethanesulphonyl, trifluoroacetyl, maloyl, succinyl, etc.; esterified carboxyl gorups such as methoxycarbonyl, ethoxycarbonyl, t-butoxycarbonyl, isopropoxycarbonyl, 2-cyanoethoxycarbonyl, β,β,β-trichloroethoxycarbonyl, benzyloxycarbonyl, p-nitrobenzyloxycarbonyl, p-methoxybenzyloxycarbonyl, diphenylmethyloxycarbonyl, methoxymethyloxycarbonyl, acetylmethyloxycarbonyl, isobornyloxycarbonyl, phenyloxycarbonyl; methylene groups such as (hexahydro-1H-azepin-1-yl)methylene; sulphonyl groups such as 2-amino-2-carboxyethylsulphonyl, etc.; and amino-protecting groups other than acyl groups, such as trityl, 2-nitrophenylthio, benzylidene, 4-nitrobenzylidene, di- or trialkylsilyl, benzyl, p-nitrobenzyl, etc. The present invention is not particularly concerned with limitations on the selection of amino-protecting groups as it is not regarding the carboxyl-protecting groups. Especially, monochloroacetyl, benzyloxycarbonyl, p-methoxybenzyloxycarbonyl or p-nitrobenzyloxycarbonyl is preferable.

The starting compound (II) according to this invention can be produced, for example, by the following procedures.

PROCEDURE 1)

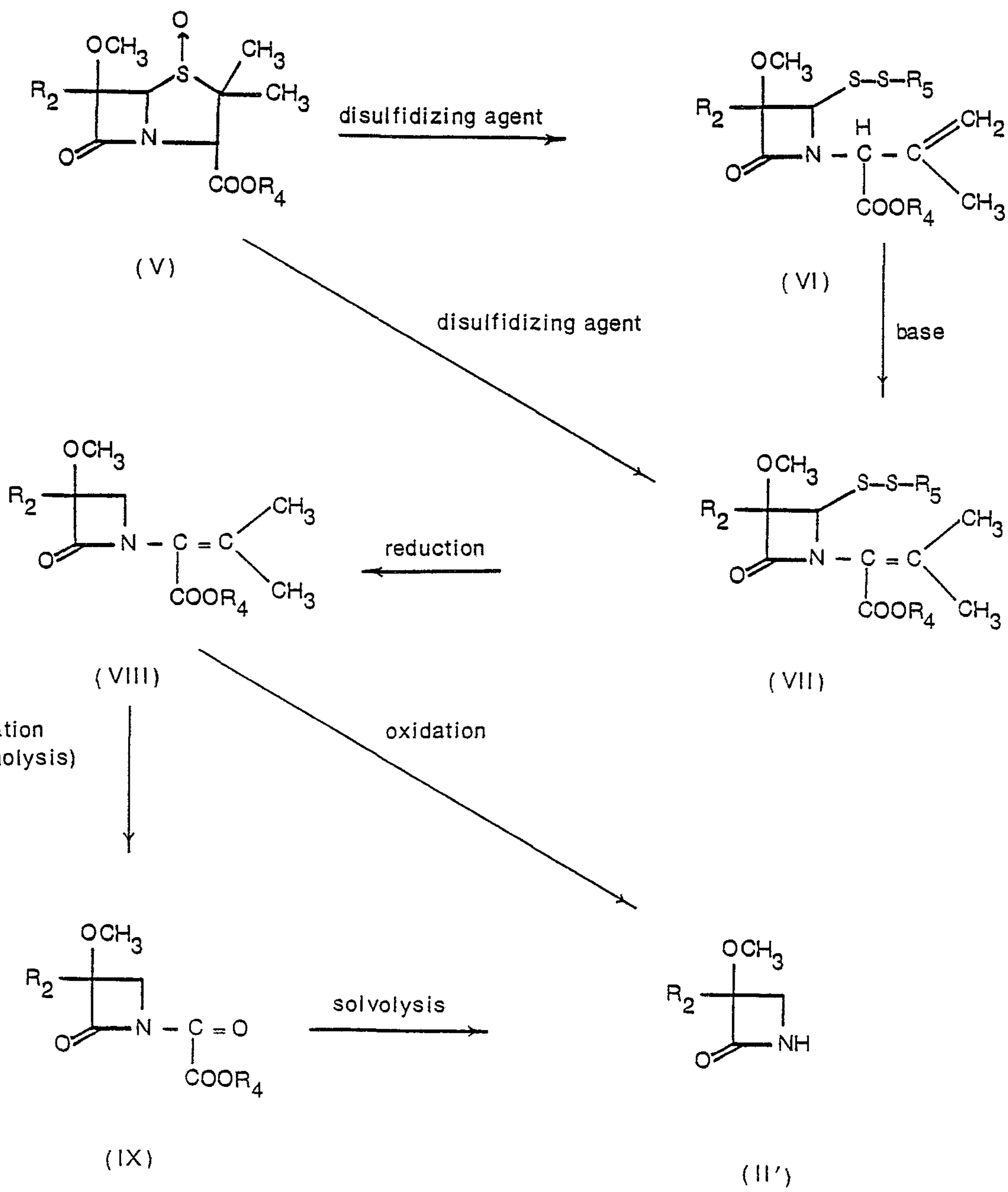

(V)
(VI)
(VII)
(VIII)
(IX)
(II')

PROCEDURE 2)

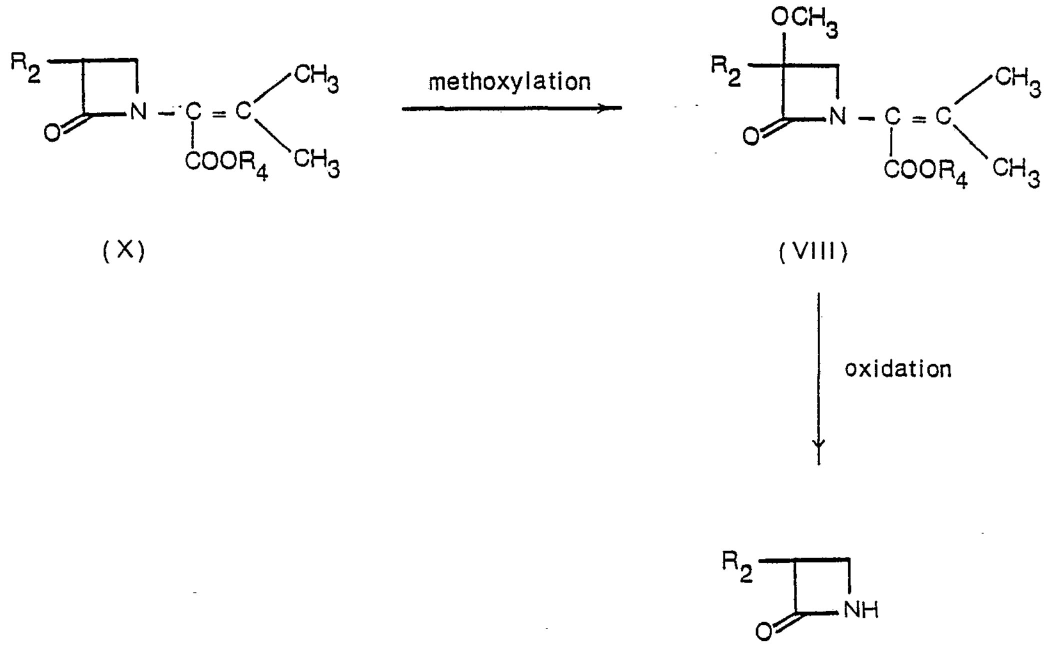


(X)

(VIII)

(II'')

PROCEDURE 3)

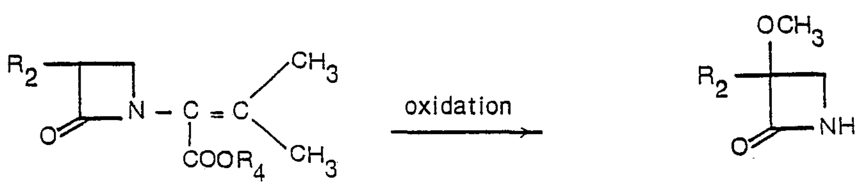


(X)

(II')

Regarding the symbols used in the above reaction formulae, $R_2$ has the same meaning as defined hereinbefore, $R_4$ is an ester residue and $R_5$ is a thiol residue.

Exemplary species of the members defined in the above definitions are as follows.

The ester residue $R_4$ includes, for example, methyl, ethyl, propyl, isopropyl, n-butyl, t-butyl, pentyl, cyclopentyl, cyclohexyl, bezyl, p-nitrobenzyl, benzhydryl, alkoxyalkyl, alkanoyloxymethyl, alkenyl, trichloroethyl, methylsulphonylethyl, benzoylmethyl, methoxybenzyl, trityl, methylthiomethyl, pivaloyloxymethyl, $\alpha$-acetoxybutyl, etc.

The thiol residue $R_5$ includes, for example, alkyl groups (e.g. methyl, ethyl, propyl, isopropyl, n-butyl, t-butyl, isobutyl, n-amyl, vinyl, 1-isopropenyl, etc.), substituted alkyl groups (e.g. methoxymethyl, ethoxymethyl, benzyl, phenethyl, xylylmethyl, p-chlorobenzyl, p-nitrobenzyl, p-methoxybenzyl, etc.), unsubstituted and substituted aryl groups (e.g. phenyl, xylyl, tolyl, naphthyl, chlorophenyl, nitrophenyl, methoxyphenyl, etc.), heterocyclic groups (e.g. benzothiazolyl, benzoxazolyl, thiazolyl, thiadiazolyl, oxazolyl, thienyl, pyridyl, oxadiazolyl, oxatriazolyl, imidazolyl, benzimidazolyl, triazolyl, tetrazolyl, etc.), acyl groups (e.g. acetyl, propionyl, benzoyl, thioacetyl, thiopropionyl, thiobenzoyl, etc.), carbamoyl groups (e.g. methylcarbamoyl, dimethylcarbamoyl, phenylcarbamoyl, etc.), the corresponding and other thiocarbamoyl groups.

The above-mentioned procedures for producing the azetidine derivative (I) of this invention will be described in detail.

*Procedure 1)*

This method is related to a fundamental synthetic method for optically active azetidine derivatives (II').

The compound (V) used as a starting material can be easily prepared, for example by the method described in Journal of the American Chemical Society *95*, 2401 (1973) or a method analogous thereto. In the first stage, compound (V) is reacted with a disulphidizing agent. The term "disulphidizing agent" is used herein to include all the reactants that are capable of disulphidizing the sulphur in the 1-position of compound (V) and, in particular, thiol compounds of the formula $R_5$—SH and disulphides of the formula $R_5$—S—S—$R_5$ ($R_5$ has the same meaning as defined hereinbefore).

This reaction is carried out in the absence of a solvent or in an appropriate solvent. The solvent includes, for example, dioxane, N,N-dimethylacetamide, N,N-dimethylformamide, bezene, toluene, tert-butanol, isopropanol, methyl ethyl ketone, etc., mixtures of such solvents, and other solvents which will not interfere with the reaction. While the reaction temperature is not particularly critical, it is normally advantageous to carry out the reaction at a temperature between 70°C and 150°C.

When a disulphide compound of $R_5$—S—S—$R_5$ is employed as the disulphidizing agent, the reaction is catalytically accelerated by the presence of an acid or a base. This acid includes, for example, sulphuric acid, phosphoric acid, hydrochloric acid and other mineral acids, p-toluenesulphonic acid, methanesulphonic acid, phenylphosphonic acid, acetic acid, formic acid and other organic acids, and Lewis acids such as ferric chloride, zinc chloride, boron trifluoride, etc. When such an acid is employed, there is predominantly obtained a 1-($\alpha$-isopropenyl)-azetidine (VI) which contains a double bond in the exo-position. The base mentioned above includes, for example, pyridine, quinoline, N,N-dimethylaniline, triethylamine, etc. In this case, depending on the reaction solvent, time, temperature etc., there is obtained a 1-($\alpha$-isopropylidene)-azetidine (VII) having a double bond in the endo-position in addition to the 1-($\alpha$-isopropenyl)-azetidine (VI). This 1-($\alpha$-isopropyliden)-azetidine (VII) can also be easily obtained by treating 1-($\alpha$-isopropenyl)-azetidine (VI) with a base. The reaction according to this procedure is preferably carried out in streams of an inert gas such as nitrogen, helium or the like. The useful molar ratio of disulphidizing agent to starting compound (V) depends on the S-nucleophilicity of the disulphidizing agent used but, generally speaking, about 1 to about 10 equivalents of the agent are employed. After completion of the reaction, the product compound (VI) can be isolated in optional purity by purification procedures known *per se*, e.g. extraction with solvents, recrystallization, chromatography, etc.

The compound (VI), on treatment with a base, yields the compound (VII). The base used for this purpose may be the above-mentioned base which can be used as a catalyst in the reaction between compound (VI) and disulphidizing agent. In carrying out this reaction, the base need not be employed in a large amount. Thus, relative to compound (VI), about 0.01 to about 0.2 mol equivalent is sufficient. The reaction is generally carried out in a solvent such as dichloromethane, chloroform, benzene, toluene, tert-butanol, methanol, ethanol, tetrahydrofuran, dioxane, methyl ethyl ketone, N,N-dimethylacetamide, N,N-dimethylformamide, etc., or a mixture of such solvents. Any other solvent that will not interfere with the reaction may also be employed. While the reaction temperature is not particularly critical, the reaction proceeds at room temperature in many instances. The product derivative (VII) in which $R_5$ is a group of the formula:

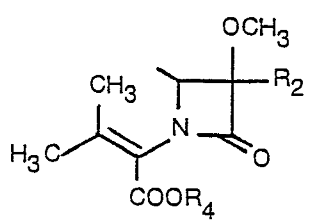

is a compound which is obtained simultaneously or partially in this reaction step and can be converted to compound (VIII) by treatment with a reducing agent.

The compound (VII) is then subjected to a reductive desulphurization reaction. The reductive desulphurizing agent used for this purpose may, for example, be Raney nickel or Raney cobalt. This reaction is usually carried out in a solvent. The solvent includes, for example, methanol, ethanol, propanol, tetrahydrofuran, dioxane, ethyl acetate, water, etc., although other common organic solvents which do not intefere with the reaction may also be employed. This reaction proceeds rapidly under mild conditions, e.g. at room temperature to about 80°C.

The compound (VIII) is then oxidized in order to remove the N-side chain. This oxidation reaction includes an oxidation reaction with an oxidizing agent and a subsequent solvolysis with a solvent or a basic or acidic catalyst.

The oxidizing agent used in the above oxidation reaction includes, for example, ozone, alkali metal permanganate (e.g. potassium permanganate, sodium permanganate, etc.), alkaline earth metal permanganate (e.g. barium permanganate, etc.), osmium tetraoxide, lead tetraacetate, etc. This oxidation reaction is usually carried out in a solvent. This solvent includes, for example, tetrahydrofuran, dioxane, N,N-dimethylformamide, N,N-dimethylacetamide, benzene, acetone, pyridine, methanol, ethanol, propanol, butanol, water, chloroform, dichloromethane, carbon tetrachloride, etc. It may be a mixture of such solvents. The proportion of the oxidizing agent relative to compound (VIII) may be about 1.0 to 4.0 molar equivalents, preferably about 1.0 to 1.2 molar equivalents, although excess ozone if it is used may be employed. While the reaction temperature is not particularly critical, the reaction usually proceeds under cooling or at room temperature. The reaction normally goes to completion with a short time. When a permanganate, for instance, is employed as the oxidizing agent, it is preferable to employ a buffer solution such as a phosphate buffer and carry out the reaction in the neutral pH region so as to minimize the decomposition of the starting compound (VI) or/and product compound (I). When ozone is used as the oxidizing agent, the conversion of compound (VIII) to compound (IX) can be effected by ozonolysis e.g. by carrying out the reaction in a solvent such as chloroform, dichloromethane or carbon tetrachloride, followed by removing the excess ozone and decomposiing the ozonide of compound (VIII) with dimethyl sulphide.

The conversion of compound (IX) to compound (II') is effected by subjecting compound (IX) to solvolysis. This reaction is carried out in a suitable solvent and may be optionally conducted with the aid of a basic or acidic catalyst. The base used in such a procedure includes, for instance, inorganic bases such as the hydroxides, carbonates, etc., or alkali metals such as lithium, potassium, sodium, etc., or alkaline earth metals such as calcium, magnesium, etc.; organic bases such as metal alkoxides, organic amines, quaternary ammonium salts, etc.; basic ion exchange resins and so forth. The acid used in a similar manner includes inorganic acids and their salts such as hydrochloric acid, sulphuric acid, phosphoric acid, zinc chloride, zinc sulphate, ferric chloride, ferric sulphate, etc., organic acids such as formic acid, acetic acid, p-toluenesulphonic acid, trifluoroacetic acid, etc., silica gel, acidic ion exchange resins and so forth. The solvent used for this reaction includes, for example, water, methanol, ethanol, propanol, tetrahydrofuran, dioxane, ethyl acetate, etc., as well as mixtures thereof. Any other solvent that will not interfere with the reaction may also be employed likewise. This reaction usually proceeds easily under mild conditions, e.g. under cooling to a slightly elevated temperature.

The reaction product in each step can be separated in optional purity by a purification procedure known *per se*, e.g. extraction with solvents, recrystallization, chromatography, etc.

*Procedure 2)*

This procedure relates to a fundamental route of synthesis for the production of optically inactive azetidine derivative (II').

The starting compound (X) can be easily prepared by the method described in Molecular Modification in Drug Design *45*, 15 (1964) or a method analogous thereto.

The methoxylation reaction of compound (X) to compound (VIII) is carried out by reaction an alkali metal salt of methanol, which is of the formula $MOCH_3$ (wherein M is an alkali metal), and a halogenating agent with the compound (X) in the presence of methanol. As examples of the alkali metal salt of methanol, there may be mentioned lithium methoxide, sodium methoxide, potassium methoxide, etc. The halogenating agent is a halogen compound capable of acting as a positive-halogen donor, e.g. halogen (chlorine, bromine, etc.), N-haloimides (N-chlorosuccinimide, N-bromosuccinimide, etc.), N-halo-sulphanamides (N-chlorobenzenesulphonamide, N-chloro-p-toluenesulphamide, etc), 1-halobenzotriazoles, organic hypochlorite, etc.). This reaction is carried out in a solvent. Examples of the solvent include tetrahydrofuran, dioxane, dichloromethane, chloroform, acetonitrile, methanol, N,N-dimethylformamide, N,N-dimethylacetamide, etc. as well as various mixtures thereof. Any other solvent that will not interfere with the contemplated reaction may likewise be employed. To carry out the reaction, the starting compound (X) is dissolved or suspended in the above-mentioned solvent and, then, the alkali metal salt of methanol, methanol and halogenating agent are added. The desirable proportions of these agents, relative to each mol of starting compound (X), are not less than 1 mol of methanol, about 1 to 3.5 mols of the alkali metal salt of methanol and about 1 to 2 mols of halogenating agent. The reaction proceeds readily under coling or at room temperature to about 30°C. The reaction can be quenched by making the reaction system acidic. A suitable acid to quench the reaction may, for example, be formic acid, acetic aid or trichloroacetic acid. After the reaction has thus been quenched, any excess halogenating agent can be removed by treatment with a reducing agent such as sodium thiosulphate or a trialkyl phosphite, for instance.

After completion of the above reaction, the product compound (VIII) can be isolated in an optional purity by conventional separation-purification procedures, for example, by extraction with a solvent, recrystallization, chromatography, etc.

The compound (VIII) is then subjected to procedures similar to the oxidation procedures described hereinbefore in connection with the conversion of compound (VIII) to compound (II'), whereby an optically inactive form of compound (II') is obtained.

*Procedure 3)*

In this procedure, the compound (X) obtained, for example, by the method described in Molecular Modification in Drug Design *45*, 15 (1964) or a method analogous thereto is oxidized to compound (II"). This oxidation reaction can be effected by procedures similar to those used in the converion of

compound (VIII) to compound II″) according to the above procedure 1).

The sulphonation reaction according to this invention is a reaction by which a sulpho group is introduced into the substrate compound. Thus, for example, it can be carried out be reacting the compound (II) with sulphur trioxide or a reactive derivative of sulphur trioxide.

The above-mentioned reactive derivative of sulphur trioxide includes, for example, sulphur trioxide-pyridine, sulphur trioxide-dioxane, sulphur trioxide-trimethylamine, sulphur trioxide-chlorosulphonic acid and other addition compounds of sulphur trioxide.

To conduct this reaction, about 1 to 5 mols, preferably about 1 to 2 mols, of sulphur trioxide or the reactive sulphur trioxide derivative is added to one mole of compound (II). The reaction mixture is about 0° to 80°C and preferably about 10° to 40°C. The above reaction may be carried out in a solvent. The solvent includes, for example, water, ethers (e.g. dioxane, tetrahydrofuran, diethyl ether, etc.), esters (e.g. ethyl acetate, ethyl formate, etc.), halogenated hydrocarbons (e.g. chloroform, dichloromethane, etc.), hydrocarbons (e.g. benzene, toluene, n-hexane, etc.), amides (e.g. dimethylformamide, dimethylacetamide, etc.) and other common organic solvents. These solvents may be used alone or in combination. After completion of the reaction, the compound (I) can be isolated in an optional purity by conventional separation-purification procedures, for example, by extraction with a solvent, recrystallization, chromatography, etc.

The compound (I) wherein $R_1$ is amino, i.e. compound (IV), is useful as an intermediate for the production of a useful medicine. The compound (I) wherein $R_1$ is acylated amino, i.e. compound (III), can be produced by acylating the compound (IV).

The compound (III) is represented by the formula:

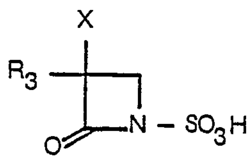

(III)

wherein $R_3$ is an acylated amino group and X is hydrogen or methoxy. The acyl groups on the acylated amino group $R_3$ may be exemplified as the same as those of $R_1$ and $R_2$.

The acylation according to this invention is accomplished by reacting the compound (IV) with an acylating agent containing the acyl group corresponding to the one contained in the acylated amino group which is represented by $R_1$, $R_2$ or $R_3$.

The acylating agent used in this reaction may, for example, be an organic carboxylic acid containing such an acyl group $R_3$ or a reactive derivative of such acid. The reactive derivative of the organic acid includes, for example, the acid anhydride, activated amide, activated ester or the like. More specifically, the following reactive derivatives of organic acids may be mentioned.

1) Acid anhydrides

The acid anhydrides include, for example, hydrogen halides (e.g. hydrochloric acid, hydrobromic acid, etc.) mixed acid anhydrides, monoalkyl carbonic acid mixed acid anhydrides, aliphatic carboxylic acid mixed acid anhydrides (mixed acid anhydrides with e.g. acetic acid, pivalic acid, valeric acid, isopentanoic acid, trichloroacetic acid, etc.), aromatic carboxylic acid mixed acid anhydrides (mixed acid anhydrides with e.g. benzoic acid, etc.), symmetric acid anhydrides, etc.

2) Activated amides

The activated amides include, for example, the amides with pyrazole, imidazole, 4-substituted-imidazole, dimethylpyrazole, benzotriazole, etc.

3) Activated esters

The activated esters include, for example, methyl ester, ethyl ester, methoxymethyl ester, propargyl ester, 4-nitrophenyl ester, 2.4-dinitrophenyl ester, trichlorophenyl ester, pentachlorophenyl ester, mesylphenyl ester, ester of said carboxylic or other acids with 1-hydroxy-1H-2-pyridone, N-hydroxysuccinimide, N-hydroxyphthalimide, etc.

The reactive derivative of the organic acid is selected according to the type of acid chosen and, when a free acid is used as the acylating agent, the reaction is desirably carried out in the presence of a condensing agent. The condensing agent includes, for example, N,N′-dicyclohexylcarbodiimide, N-cyclohexyl-N′-morpholinoethylcarbodiimide, N-cyclohexyl-N′-(4-diethylaminocyclohexyl)carbodiimide, N-ethyl-N′-(3-dimethylaminopropyl)carbodiimide, etc.

This acylation reaction is generally carried out in a solvent. The solvent includes, for example, water, acetone, dioxane, acetonitrile, dichloromethane, chloroform, dichloroethane, tetrahydrofuran, ethyl acetate, dimethylformamide, pyridine, etc., as well as the common organic solvents which do not interfere with the reaction. These solvents, if they were hydrophilic, may be used in a mixture with water.

Furthermore, the acylation reaction can be conducted in the presence of a base, for example, alkali metal carbonates, trialkylamines (e.g. trimethylamine, triethylamine, tributylamine, N-methylmorpholine, N-methylpiperidine, etc.), N,N-dialkylaniline, N,N-dialkylbenzylamine, pyridine, picoline, lutidine, 1,5-diazabicyclo[4, 3, 0]non-5-ene, 1,4-diazabicyclo[2, 2, 2]-octane, 1,8-diazabicyclo[5, 4, 4]undecene-7, etc. The base, as well as the condensing agent, may be used as the solvent as well, only if it is liquid. The reaction temperature is not particularly critical and, in many cases, the reaction is carried out under cooling to room temperature.

Referring to the acylation reaction, when the reactive derivative of the starting compound (IV) with respect to its amino group or a salt thereof and the acylating agent respectively contain an asymmetric carbon atom, the corresponding stereoisomers can be employed respectively or as a mixture. Moreover, when the reaction yields such isomers in admixture, they may be fractionally isolated by procedures known *per se*, for example, by column chromatography, recrystallization, etc.

The compound (I) which has a protective group is useful as an intermediate for the production of a useful medicine. For example, the compound (I) which does not have a protective group can be obtained by the removal of the protective group.

The removal of the protective group from the azetidine derivative (I) can be effected by a choice of the hitherto known procedures, the choice depending on the type of protective group. Thus, for example, the method may comprise the use of an acid, a base or hydrazine, or may be a reductive method or a method comprising permitting an iminohalogenating agent to act on the substrate compound and, then, an iminoetherifying agent to act thereon and, finally and if necessary, hydrolyzing the same. In the method employing an acid, while the choice depends on the type of protective group and other conditions, the acid may, for example, be an inorganic acid (e.g. hydrochloric acid, sulphuric acid, phosphoric acid, etc.), an organic acid (e.g. formic acid, acetic acid, trifluoroacetic acid, propionic acid, benzenesulphonic acid, p-toluenesulphonic acid, etc.) or an acidic ion exchange resin. In the method involving the use of a base, while the choice depends on the type of protective group and other conditions, the base may, for example, be an inorganic base suh as the hydroxide or carbonate or an alkali metal (e.g. sodium, potassium, etc.) or alkaline earth metal (e.g. calcium, magnesium etc.), an alkali metal alkoxide, an organic base (e.g. organic amines, quaternary ammonium salts, etc.) or a basic ion exchange resin.

When, in the above methods involving the use of a base or an acid, a solvent is employed, it is generally desirable, in many cases, to use a hydrophilic organic solvent, water or a mixture thereof.

The reductive method, while the choice depends on the type of protective group and other conditions, may be a method employing a metal (e.g. tin, zinc, etc.) or a metal compound (e.g. chromous dichloride, chromous acetate, etc.) and an organic, inorganic or other acid (e.g. acetic acid, propionic acid, hydrochloric acid, etc.), or a method involving the presence of a metal catalyst for catalytic reduction. As examples of the catalyst used for such catalytic reduction, there may be mentioned platinum catalysts such as platinum wire, platinum sponge, platinum black, platinum oxide, colloidal platinum, etc., palladium catalysts such as palladium sponge, palladium black, palladium oxide, palladium-barium sulphate, palladium-barium carbonate, palladium-carbon, palladium-silica gel, colloidal palladium, etc., and nickel catalysts such as reduced nickel, nickel oxide, Raney nickel, Urushibara nickel, etc.

In the reductive method employing a metal and an acid, the combination of a metal compound, e.g. a compound of iron, chromium or the like, with an inorganic acid, e.g. hydrochloric acid, or an organic acid, e.g. formic acid, acetic acid, propionic acid or the like, may be employed. The reductive procedure is normally carried out in a solvent. In the case of catalytic reduction, alcohols such as methanol, ethanol, propanol, isopropyl alcohol, etc., and ethyl acetate, etc., are commonly employed. In the procedure involving the use of a metal and an acid, the solvent is usually water, acetone or the like, but when the acid is liquid, it may be utilized as the solvent as well.

The reaction is usually carried out under cooling to under warming, preferably in a temperature range of about 0°C to about 30°C.

Referring to the procedure comprising the use of an iminohalogenating agent and, then, an iminoetherifying agent, followed by hydrolysis to remove the protective group, the iminohalogenating agent may, for example, be phosphorus trichloride, phosphorus pentachloride, phosphorus tribromide, phosphorus pentabromide, phosphorus oxychloride, thionyl chloride or phosgene. The reaction temperature is not critical and, in many cases, the reaction is carried out under room temperature to under cooling. The iminoetherifying agent which is then permitted to act on the resulting reaction product may, for example, be an alcohol or a metal alkoxide. Thus, the alcohol includes, for example, alkanols such as methanol, ethanol, propanol, isopropyl alcohol, n-butanol, tert-butanol, etc.; and compounds wherein the alkyl moieties of such alkanols as mentioned above have been substituted by alkoxy groups such as methoxy, ethoxy, propoxy, isopropoxy, butoxy, etc. The metal alkoxide includes, for example, alkali metal alkoxides (sodium alkoxides, potassium alkoxides, etc.) and alkaline earth metal alkoxides (calcium alkoxides, barium alkoxides, etc.) as may be derived from the above-mentioned and other alcohols.

When, for example, the protective group is an organic carboxylic acid residue and the carbon atom adjacent to its carbonyl group carries a certain substituent such as a free amino, hydroxyl,

mercapto, carboxyl or sulpho group, it is advantageous first to carry out a treatment for enhancing the adjacent group effect of such group so as to increase the reactivity of the carbonyl group before carrying out the removal of the protective group. In this connection, the case in which the substituent group on the carbon atom adjacent to the carbonyl group is a free amino group will be described by way of illustration. Thus, the free amino group may be converted to a thioureido group and, then, the necessary deacylation reaction is carried out. This and other procedures known in the art of cleavage of peptide bonds an be utilized to remove the protective group.

The temperature for this reaction is not espectially critical but may be suitably selected according to the type of protective group and the method then applied for removing the protective group. It is preferable, after all, that the reaction be carried out under cooling, or up to a slightly elevated temperature.

There are cases in which the derivative in the carboxyl function of the compound wherein $R_1$ is a group containing such a carboxyl group is transformed into a carboxyl group in the course of this reaction and such cases are also included in the concept and ambit of this invention.

The compound (I) thus obtained by removal of the protective group can be converted, if desired, to a desired salt thereof in a conventional manner.

The compound (I), which contains a sulpho group, is generally capable of forming a salt with a base. Therefore, the compound (I) may then be isolated as a salt, which, in turn, may be converted to the free form or to a different salt. The free compound (I) may, of course, be converted to a salt. The base mentioned above may be an inorganic base, e.g. lithium, potassium, sodium, calcium, ammonium, etc., or an organic base, e.g. pyridine, collidine, triethylamine, triethanolamine, etc.

The salt form of compound (I) is also included in the scope of this invention.

To convert the salt form of compound (I) to the free compound (I), a method using an acid, for example, can be employed. The type of acid varies with different protective groups and other conditions. However, such inorganic acids as hydrochloric acid, sulphuric acid, phosphoric acid, etc., and such organic acids as formic acid, acetic acid, p-toluenesulphonic acid, etc., are generally employed. Apart from the types of acids mentioned above, acidic ion exchange resins are also useful. The solvent may, for example, be acetone, tetrahydrofuran, methanol, ethanol, dioxane or the like, water, or a mixture of water and such a solvent.

The compound (IV), as the starting compound for the acylation reaction, may be used in the form of a salt. The salt may be any of the salts mentioned in connection with salts of compound (I).

The acylation reaction, where the starting material is a salt as mentioned above, may give rise to a salt of compound (III). In such cases, the salt of compound (III) can be converted to a different salt and isolated as such, just as mentioned for compound (I).

Such salts may be converted to free compound (III) and isolated as such. This conversion of a salt to the free compound (III) can be effected in the same manner as described hereinbefore in connection with compound (I).

The compound (I) may exist as diastereoisomers or optical-isomers. In such cases, the respective isomers and their mixtures are also included in the scope of this invention. These isomers separately or as mixtures, can be used as medicines.

When such mixtures of isomers are recovered as products, each mixture may be resolved into the component isomers by a conventional optical resolution method or other purification methods, e.g. extraction with solvent, recrystallization, chromatography, etc.

The compound (I) thus obtained is useful as a drug, being active against certain gram-positive and gram-negative bacteria. By way of example, the compounds are active against the following micro-organisms.

The media and compounds employed in the anti-bacterial test are as follows:

Media:

TSA = Trypticase soy agar [Baltimore Biologicals (U.S.A.)]

B—TSA = Blood trypticase soy agar

Compounds:

Compound A = Sodium 3-[2-(2-amino-4-thiazolyl)-2-methoxyiminoacetamido]-2-oxo-azetidine-1-sulphonate

Compound B = Sodium 3-[D(-)-N-(4-ethyl-2,3-dioxo-1-piperazinocarbonyl)-phenyl-glycin-amido]-2-oxoazetidine-1-sulphonate

Compound C = Sodium 3-[D(-)-N-(4-ethyl-2,3-dioxo-1-piperazinocarbonyl)phenylglycinamido]-3-methoxy-2-oxoazetidine-1-sulphonate

Compound D = Sodium 3-[2-(4-ethyl-2,3-dioxo-1-piperazinocarboxamido)-2-(2-amino-4-thiazolyl)-acetamido]-2-oxoazetidine-1-sulphonate

Compound E = Sodium 3-[DL-2-(4-ethyl-2,3-dioxo-1-piperazinocarboxamido)-2-thienyl-acetamido]-2-oxoazetidine-1-sulphonate

Compound F = Sodium 3-[D-2-(4-ethyl-2,3-dioxo-1-piperazinocarboxamido)-2-thienylacetamido]-2-oxoazetidine-1-sulphonate

Compound G = Sodium 3-[D-2-(4-n-octyl-2,3-dioxo-1-piperazinocarboxamido)-2-phenylacetamido]-2-oxoazetidine-1-sulphonate

Compound H = Sodium 3-[2-(4-n-octyl-2,3-dioxo-1-piperazinocarboxamido)-2-(2-amino-4-thiazolyl)-acetamido]-2-oxoazetidine-1-sulphonate

Compound I = Sodium 3-[D-2-[(2-oxo-3-furfurylideneaminoimidazolidin-1-yl)-carboxamido]-2-phenylacetamido]-2-oxoazetidine-1-sulphonate

Compound J = Sodium 3-[2-(4-n-octyl-2,3-dioxo-1-piperazinocarboxamido)-2-thienylacetamido]-2-oxoazetidine-1-sulphonate

Compound K = Sodium 3-[D-2-[[2-oxo-3-(thiophen-2-aldoimino)-imidazolidin-1-yl]-carboxamido]-2-phenylacetamido]-2-oxoazetidine-1-sulphonate

Compound L = Sodium 3-[2-[(2-oxo-3-furfurylideneaminoimidazolidin-1-yl)-carboxamido]-2-thienylacetamido]-2-oxoazetidine-1-sulphonate

TABLE 1

| Test organism | Medium | Minimum inhibitory concentration (μg/ml) Compound A | Compound B |
|---|---|---|---|
| Staphylococcus aureus FDA209P | TSA | 50 | 6.25 |
| Staphylococcus aureus 308A—1 | TSA | 25 | 3.13 |
| Staphylococcus aureus 1840 | TSA | 100 | 25 |
| Staphylococcus aureus FDA209P | B—TSA | 50 | 6.25 |
| Escherichia coli NIHJ JC—2 | TSA | 0.78 | 1.56 |
| Escherichia coil O—111 | TSA | 0.39 | 0.2 |
| Escherichia coil T—7 | TSA | >100 | >100 |
| Citrobacter freundii IFO 12681 | TSA | 1.56 | 6.25 |
| Klebsiella pheumoniae DT | TSA | 0.78 | 0.2 |
| Enterobacter cloacae IFO 12937 | TSA | >100 | 50 |
| Serratia marcescens IFO 12648 | TSA | 12.5 | 3.13 |
| Proteus vulgaris IFO 3988 | TSA | 1.56 | 0.1 |
| Proteus mirabilis IFO 3849 | TSA | 6.25 | 0.78 |
| Proteus morganii IFO 3168 | TSA | 25 | 25 |
| Pseudomonas aeruginosa IFO 3455 | TSA | 6.25 | 1.56 |
| Pseudomonas aeruginosa U 31 | TSA | >100 | >100 |
| Acinetobacter calcoaceticus IFO 13006 | TSA | 6.25 | 25 |
| Candida albicans TA | TSA | >100 | >100 |
| Streptococcus pyogenes E—14 | B—TSA | 3.13 | 3.13 |
| Streptococcus pyogenes S—8 | B—TSA | 3.13 | 6.25 |
| Streptococcus mitis America | B—TSA | 25 | 25 |
| Streptococcus faecium IFO 3128 | B—TSA | >100 | >100 |

TABLE 1 (continued)

| Test organism | Medium | Minimum inhibitory concentration (μg/ml) Compound A | Compound B |
|---|---|---|---|
| Streptococcus pneumoniae Type I | B—TSA | 12.5 | 6.25 |
| Corynebacterium diphtheriae Toront | B—TSA | 1.56 | 3.13 |
| Bordetella bronchiseptica Sagami | B—TSA | 100 | >100 |

TABLE 2

| Test organism | Medium | Minimum inhibitory concentration (μg/ml) of Compound C |
|---|---|---|
| Staphylococcus FDA209P | TSA | 100 |
| Escherichia coli NIHJ JC—2 | TSA | 50 |
| Escherichia coli O—111 | TSA | 12.5 |
| Klebsiella pheumoniae DT | TSA | 12.5 |
| Enterobacter cloacae IFO 12937 | TSA | >100 |
| Serratia marcescens IFO 12648 | TSA | 50 |
| Proteus vulgaris IFO 3988 | TSA | 12.5 |
| Proteus mirabilis IFO 3849 | TSA | 50 |
| Pseudomonas morganii IFO 3168 | TSA | >100 |
| Pseudomonas aeruginosa U 31 | TSA | >100 |
| Candida albicans TA | TSA | >100 |
| Streptococcus pyogenes E—14 | B—TSA | 50 |
| Streptococcus pyogenes S—8 | B—TSA | 50 |
| Corynebacterium diphtheriae Toronto | B—TSA | 50 |

TABLE 3

Minimum inhibitory concentration (MIC) ($\mu$g/ml)

| Test Organism | Medium | Compound D | Compound E | Compound F | Compound G | Compound H |
|---|---|---|---|---|---|---|
| Staphylococcus aureus FDA 209P | TSA | 12.5 | 6.25 | 6.25 | 1.56 | 6.25 |
| Staphylococcus aureus 308A–1 | TSA | 6.25 | 6.25 | 6.25 | 0.78 | 3.13 |
| Staphylococcus aureus 1840 | TSA | 25 | 25 | 25 | 25 | 12.5 |
| Escherichia coli NIHJ JC–2 | TSA | 0.39 | 1.56 | 0.78 | 3.13 | 12.5 |
| Escherichia coli 0–111 | TSA | 0.1 | 0.2 | 0.2 | 0.78 | 3.13 |
| Escherichia coli T–7 | TSA | >100 | >100 | >100 | >100 | >100 |
| Citrobacter freundii IFO 12681 | TSA | 1.56 | 3.13 | 1.56 | 3.13 | 12.5 |
| Klebsiella pneumoniae DT | TSA | 0.2 | 0.78 | 0.2 | 0.78 | 12.5 |
| Enterobacter cloacae IFO 12937 | TSA | 25 | 3.13 | 50 | 6.25 | 50 |
| Serratia marcescens IFO 12648 | TSA | 0.78 | 3.13 | 1.56 | 1.56 | 12.5 |
| Proteus vulgaris IFO 3988 | TSA | 0.1 | <0.1 | <0.1 | 0.78 | 6.25 |
| Proteus mirabilis IFO 3849 | TSA | 25 | 0.78 | 0.78 | 3.13 | 25 |
| Proteus morganii IFO 3168 | TSA | 12.5 | 12.5 | 12.5 | 1.56 | 6.25 |
| Pseudomonas aeruginosa IFO 3455 | TSA | 3.13 | 3.13 | 6.25 | 1.56 | 12.5 |
| Pseudomonas aeruginosa U 31 | TSA | >100 | >100 | >100 | 25 | 50 |
| Acinetobacter calcoaceticus IFO 13006 | TSA | 50 | 50 | 25 | 3.13 | 50 |
| Candida albicans TA | TSA | >100 | >100 | >100 | >100 | >100 |
| Staphylococcus aureus FDA 209P | B–TSA | 12.5 | 6.25 | 6.25 | 3.13 | 12.5 |

TABLE 3 (Continued)

| Test Organism \ Medium \ Compound | | Compound D | Compound E | Compound F | Compound G | Compound H |
|---|---|---|---|---|---|---|
| Streptococcus pyogenes E–14 | B–TSA | 3.13 | 1.56 | 3.13 | 1.56 | 3.13 |
| Streptococcus pyogenes S–8 | B–TSA | 3.13 | 3.13 | 3.13 | 1.56 | 1.56 |
| Streptococcus mitis America | B–TSA | 25 | 25 | 25 | 25 | 25 |
| Streptococcus faecium IFO3128 | B–TSA | >100 | >100 | >100 | >100 | >100 |
| Streptococcus pneumoniae Type I | B–TSA | 6.25 | 3.13 | 6.25 | 3.13 | 3.13 |
| Corynebacterium diphtheriae Tronto | B–TSA | 3.13 | 0.39 | 0.78 | 25 | 0.78 |
| Bordetella bronchiseptica Sagami | B–TSA | >100 | >100 | >100 | >100 | >100 |

TABLE 4

Minimum inhibitory concentration (MIC) ($\mu g/ml$)

| Test organism \ Medium \ Compound | | Compound I | Compound J | Compound K | Compound L |
|---|---|---|---|---|---|
| Staphylococcus aureus FDA 209P | TSA | 6.25 | 1.56 | 6.25 | 6.25 |
| Staphylococcus aureus 308 A–1 | TSA | 3.13 | 1.56 | 3.13 | 3.13 |
| Staphylococcus aureus 1840 | TSA | 50 | 6.25 | 50 | 25 |
| Escherichia coli NIHJ JC–2 | TSA | 1.56 | 3.13 | 1.56 | 1.56 |
| Escherichia coli O–111. | TSA | 3.13 | 1.56 | 0.39 | <0.1 |
| Escherichia coli T–7 | TSA | >100 | >100 | >100 | >100 |
| Citrobacter freundii IFO 12681 | TSA | 6.25 | 3.13 | 1.56 | 1.56 |
| Klebsiella pneumoniae DT | TSA | 0.2 | 1.56 | 0.39 | <0.1 |
| Enterobacter cloacae IFO 12937 | TSA | 25 | 6.25 | 12.5 | 25 |

TABLE 4 (Continued)

| Test organism | Medium | Compound I | Compound J | Compound K | Compound L |
|---|---|---|---|---|---|
| Serratia marcescens IFO 12648 | TSA | 1.56 | 1.56 | 1.56 | 1.56 |
| Proteus vulgaris IFO 3988 | TSA | 0.2 | 1.56 | 0.39 | 0.2 |
| Proteus mirabilis IFO 3849 | TSA | 1.56 | 3.13 | 1.56 | 0.78 |
| Proteus morganii IFO 3168 | TSA | 12.5 | 1.56 | 12.5 | 12.5 |
| Pseudomonas aeruginosa IFO 3455 | TSA | 3.13 | 6.25 | 3.13 | 3.13 |
| Pseudomonas aeruginosa U 31 | TSA | >100 | 25 | >100 | >100 |
| Acinetobacter calcoaceticus IFO 13006 | TSA | 25 | 6.25 | 50 | 50 |
| Candida albicans TA | TSA | >100 | >100 | >100 | >100 |
| Staphylococcus aureus FDA 209P | B–TSA | 6.25 | 3.13 | 6.25 | 6.25 |
| Streptococcus pyogenes E–14 | B–TSA | 0.39 | 1.56 | 0.78 | 0.78 |
| Streptococcus pyogenes S–8 | B–TSA | 0.78 | 1.56 | 1.56 | 0.78 |
| Streptococcus mitis America | B–TSA | 3.13 | 25 | 1.56 | 6.25 |
| Streptococcus faecium IFO 3128 | –TSA | >100 | >100 | >100 | >100 |
| Streptococcus pneumoniae Type I | –TSA | 1.56 | 1.56 | 1.56 | 1.56 |
| Corynebacterium diphtheriae Tronto | –TSA | 0.78 | 0.78 | 3.13 | 1.56 |
| Bordetella bronchiseptica Sagami | –TSA | >100 | >100 | >100 | >100 |

The acute toxicity ($LD_{50}$) of compound (I) in mice, by intravenous administration, is generally not less than 500 mg/kg.

The compound (I) is of value in the treatment of mammalian animals (e.g. mouse, rat, human being, etc.) infected by the above-mentioned and other micro-organisms.

As a bacterial infection remedy, the compound (I) can be applied, for example, to the treatment of respiratory organ infections, urinary tract infections, suppurative diseases, bile duct infections, intestinal infections, gynecological and obstetric infections, surgical infections, etc., in the above-mentioned mammals. The daily dose is about 20 to about 200 mg/kg body weight as compound (I) and is preferably administered in 2 to 4 portions daily, i.e. about 5 to about 100 mg/kg body weight per dose. The compound (I), or a physiologically acceptable salt thereof, can be orally administered in dosage

forms such as tablets, capsules, drops, etc., which can be prepared by established pharmaceutical procedures. The compound and salt each can also be processed into injectable preparations by routine pharmaceutical procedures, for instance, and after mixing with a sterile vehicle which is obtainable by conventional procedures, be administered parenterally.

This invention will be further described by way of the following reference and working Examples.

### Reference Example 1

A mixture of 4.1 g of methyl 6β-benzyloxycarboxamido-6β-methoxypenicillanate-1-oxide and 10 ml of n-amylmercaptan is stirred at 110°C for 24 hours. The excess n-amylmercaptan is distilled off and the residue is chromatographed on a column of silica gel [eluted with n-hexane-ethyl acetate (2:1)] to give 2.5 g of methyl 4β-n-amyldithio-3β-benzyloxycarboxamido-3α-methoxy-2-oxoazetidine-1-(α-isopropenyl)acetate.

$IR\nu^{KBr}_{max}cm^{-1}$; 3300, 1767, 1736

NMR ($CDCl_3$, ppm); 0.93 (t, $—CH_3$), 1.2—1.7 (m, $—CH_2—$), 1.92 (s, $—CH_3$), 2.76 (t, $—S—CH_2—$), 3.60 (s, $—CH_3$), 3.83 (s, $—CH_3$),

4.92 (s, —CH—), 5.07 (s, —CH—),

5.20 (m, $—CH_2$), 5.23 (s, $—CH_2—$), 5.66 (s, —NH—), 7.42 (s, aromatic H).

### Reference Example 2

0.15 g of triethylamine is added to a solution of 2.3 g of methyl 4β-n-amyldithio-3β-benzyloxycarboxamido-3α-methoxy-2-oxoazetidine-1-(α-isopropenyl) acetate in 60 ml of methylene chloride and the mixture is stirred at room temperature for 1.5 hours. The solvent is distilled off and the residue is chromatographed on a column of silica gel [eluted with n-hexane-ethyl acetate (4:1)] to give 2.2 g of methyl 4β-n-amyldithio-3β-benzyloxycarboxamido-3α-methoxy-2-oxoazetidine-1-(α-isopropylidene) acetate.

$IR\nu^{KBr}_{max}cm^{-1}$; 3300, 1768, 1735

NMR ($CDCl_3$, ppm); 0.92 (t, $—CH_3$), 1.15—1.98 (m, $—CH_2—$), 2.08 (s, $—CH_3$), 2.32 (s, $—CH_3$), 2.65 (t, $—S—CH_2—$), 3.64 (s, $—CH_3$), 3.83 (s, $—CH_3—$), 5.23 (s, $—CH_2—$),

5.32 (s, —CH—), 5.70 (s, NH), 7.42 (s, aromatic H).

### Reference Example 3

18 ml of Raney nickel are added to a solution of 2.1 g of methyl 4β-n-amyldithio-3β-benzyloxycarboxamido-3α-methoxy-2-oxoazetidine-1-(α-isopropylidene)acetate in 40 ml of ethanol, and the mixture is stirred at room temperature for one hour. After removal of Raney nickel by filtration, the solvent is removed under reduced pressure and the residue is chromatographed on a column of silica gel [eluted with n-hexane-ethyl acetate (3:1)] to give 0.62 g of methyl 3-benzyloxycarboxamido-3-methoxy-2-oxoazetidine-1-(α-isopropylidene)acetate.

$IR\nu^{KBr}_{max}cm^{-1}$; 1760, 1718, 1510.

NMR ($CDCl_3$, ppm); 1.93 (s, $—CH_3$), 2.20 (s, $—CH_3$), 3.50 (s, $—CH_3$), 3.70 (s, $—CH_3$), 3.91 (dd, J=4, 6Hz, $C_4—H$), 5.13 (s, $—CH_2—$), 6.03 (s, NH), 7.26 (aromatic H).

### Reference Example 4

6.0 g of methyl 3-benzyloxycarboxamido-3-methoxy-2-oxoazetidine-1-(α-isopropylidene)-acetate are dissolved in 150 ml of methylene chloride, and ozone gas is introduced into the solution at —50°C to —30°C. The reaction mixture is blue after one hour. Then, the excess ozone is removed by the introduction of nitrogen gas, followed by addition of dimethyl sulphide. After stirring at room temperature for an hour, the reaction mixture is washed with water and the solvent is evaporated to give 6.1 g of methyl 3-benzyloxycarboxamido-3-methoxy-2-oxoazetidine-1-α-ketoacetate. This product is dissolved in 75 ml of methanol, and to the solution is added 19 ml of 0.002% sodium methoxide in methanol. After stirring at room temperature for 15 minutes, 0.3 g of acetic acid is added, and the solvent is distilled off. The residue is dissolved in ethyl acetate and the solution is washed with water. After removal of the solvent, the residue is chromatographed on a column of silica gel [eluted with ethyl acetate-n-hexane (1:1)] to give 2.7 g of 3-benzyloxycarboxamido-3-methoxy-2-oxoazetidine as crystals

Optical rotation: $[\alpha]_D^{25}$ + 68.2° (c=1, MeOH)
IR$\nu_{max}^{CHCl_3}$ cm$^{-1}$, 3420, 1774, 1723,
NMR ($CDCl_3$, ppm); 3.45 (s, $CH_3$), 3.60 (d, J=6Hz, $C_4$—H), 3.80 (d, J=6Hz, $C_4$—H), 5.14 (s, —$CH_2$—), 6.74 (broad s, NH), 7.34 (s, aromatic H).

Reference Example 5

5.7 ml of t-butyl hypochlorite are added to a solution of 14 g of methyl 3-benzyloxycarboxyamido-2-oxoazetidine-1-($\alpha$-isopropylidene)acetate in 400 ml of dry tetrahydrofuran (=THF) and, then, a solution of 0.348 g lithium in 32 ml methanol is added with stirring at −30 to −20°C. The mixture is maintained at −15°C for 30 minutes, and 1 ml of acetic acid is added, and the solvent is distilled off. The residue is dissolved in ethyl acetate, and after washing with water, the solvent is distilled off. The residue is chromatographed on a column of silica gel [eluted with n-hexane-ethyl acetate (1:1)] to give 11.1 g of methyl 3-benzyloxycarboxamido-3-methoxy-2-oxoazetidine-1-($\alpha$-isopropylidene)acetate as crystals.

m.p. 77°C
IR$\nu_{max}^{KBr}$cm$^{-1}$; 1761, 1723.
NMR ($CDCl_3$, ppm); 1.91 (s, $CH_3$), 2.22 (s, $CH_3$) 3.53 (s, $CH_3$), 3.73 (s, $CH_3$), 4.1 (ABq, J=6Hz, $C_4$—H), 5.20 (s, —$CH_2$—), 6.58 (s, NH), 7.36 (s, aromatic H).

Reference Example 6

7.2 g of the methyl 3-benzyloxycarboxamido-3-methoxy-2-oxoazetidine-1-($\alpha$-isopropylidene)-acetate obtained in Reference Example 5 are dissolved in 150 ml of methylene chloride and ozone gas is introduced into the solution at −50°C to −30°C. The reaction mixture is blue after 55 minutes. Then, nitrogen gas is introduced until the solution becomes colourless. Then, 6 ml of dimethyl sulphide are added, followed by stirring at room temperature for 30 minutes. The reaction mixture is washed with water and the solvent is evaporated to give 8.1 g of methyl 3-benzyloxycarboxamido-3-methoxy-2-oxoacetidine-1-$\alpha$-ketoacetate. This is dissolved in 100 ml of methanol, followed by the addition of 25 ml of 0.002% sodium methoxide in methanol. After stirring at room temperature for 15 minutes, the solvent is distilled off, and the residue is dissolved in ethyl acetate. The solution is washed with water, and the solvent is evaporated to give 3.3 g of 3-benzyloxycarboxamido-3-methoxy-2-oxoazetidine as crystals. In IR and NMR, this product is in agreement with the optically active compound obtained in Reference Example 4.

Optical rotation: $[\alpha]_D^{25}$ 0° (c=1, MeOH).

Reference Example 7

A solution of 47.5 g of methyl 3-phenylacetamido-2-oxoazetidine-1-($\alpha$-isopropylidene)acetate in 750 ml of methylene chloride is cooled to a temperature below −70°C, followed by the addition of 93.7 g of finely divided phosphorus pentachloride and 71.2 g of pyridine. The mixture is stirred in ice-water for 70 minutes. The reaction mixture is cooled to −70°C and after addition of 150 ml of n-butanol, the temperature is returned gradually to 0°C. After an hour, 300 ml of cold water is added and the water layer is adjusted to pH 6.2 with sodium hydrogen carbonate. It is extracted with chloroform and the solvent is distilled off. By the above procedure 26 g of methyl 3-amino-2-oxoazetidine-1-($\alpha$-isopropylidene)acetate are obtained.

IR$\nu_{max}^{CHCl_3}$ cm$^{-1}$; 3400, 3330, 1750, 1720.
NMR ($CDCl_3$, ppm); 1.90 (s, —$CH_3$), 2.04 (br, s, —$NH_2$), 2.16 (s, —$CH_3$), 3.2—3.9 (m, —$CH_2$—), 3.73 (s, —$CH_3$),

4.28 (m, —CH—).

Reference Example 8

While a solution of 58 g of methyl 3-amino-2-oxoazetidine-1-($\alpha$-isopropylidene)acetate in 240 ml of methylene chloride is stirred under ice-cooling, 120 ml of propylene oxide and, then, 56.3 g of carbobenzoxy chloride are added. The reaction mixture is returned to room temperature and, then, stirred for 30 minutes. The solvent is distilled off and diethyl ether is added to the residue, whereupon crystals separate out. By the above procedure 82.6 g of methyl 3-benzyloxycarboxamido-2-oxoazetidine-1-($\alpha$-isopropylidene)acetate are obtained.

IR$\nu_{max}^{KBr}$cm$^{-1}$; 3280, 1738, 1710.
NMR ($CDCl_3$, ppm); 1.95 (s, —$CH_3$), 2.19 (s, —$CH_3$), 3.4—3.9 (m, —$CH_2$—), 3.74 (s, —$OCH_3$),

4.89 (m, —CH—), 5.11 (s, $—CH_2—$), 5.66 (d, —NH—), 7.34 (s, aromatic H).

Reference Example 9

13.3 g of methyl 3-benzyloxycarboxamido-2-oxoazetidine-1-(α-isopropylidene)acetate are dissolved in 400 ml of methylene chloride, and ozone gas is introduced under cooling at −30°C to −20°C. The reaction mixture is blue after 2 hours. Nitrogen gas is introduced to remove the excess ozone and, after addition of 20 ml of dimethyl sulphide, the mixture is stirred at room temperature for an hour. The reaction mixture is washed with water and the solvent is distilled off to give 19.9 g of methyl 3-benzyloxycarboxamido-2-oxoazetidine-1-α-ketoacetate. This product is dissolved in 200 ml of methanol, then 30 ml of 0.002% sodium methoxide in methanol are added, and the mixture is stirred at room temperature for 15 minutes. To this reaction mixture is added 0.5 g of acetic acid, the solvent is distilled off and cold water-methanol (3:1) are added, whereby 7.62 g of 3-benzyloxycarboxamido-2-oxoazetidine are obtained.

IR$\nu_{max}^{KBr}$ $cm^{-1}$; 3350, 1740, 1725, 1700.
NMR ($DMSO-d_6$, ppm); 3.08—3.40 (m, $—CH_2—$),

4.63 (m, —CH), 5.00 (s, $—CH_2—$), 7.28 (s, aromatic H), 7.80 (s, —NH), 7.85 (d, —NH).

Reference Example 10

220 mg of 3-benzyloxycarboxamido-2-oxoazetidine and 400 mg of 10% palladium-carbon are suspended in 12 ml of alcohol and the suspension is stirred intensely in a hydrogen gas stream. After 30 minutes, the catalyst is filtered off and the filtrate is concentrated to give 76 mg of 3-amino-2-oxoazetidine as crystals.

IR$\nu_{max}^{CHCl_3}$ $cm^{-1}$; 3425, 3300, 1760.
NMR ($DMSO-d_6$, ppm); 2.63 (br. s, $—NH_2$), 2.80—3.33 (m, $—CH_2—$),

4.00 (m, —CH—), 7.63 (s, —NH).

Reference Example 11

A mixture of 0.20 g of 3-benzyloxycarboxamido-3-methoxy-2-oxoazetidine, 0.50 g of palladium black and 5 ml of THF is stirred in a hydrogen gas stream for 90 minutes. The catalyst is filtered off and the filtrate is concentrated to give 0.09 g of 3-amino-3-methoxy-2-oxoazetidine.

IR$\nu_{max}^{Nujol}$$cm^{-1}$; 3250, 1740.
NMR ($CDCl_3$, ppm); 2.35 (broad s, $NH_2$), 3.40 (dd, J=6Hz, $C_4$—H), 3.45 (s, $CH_3$), 6.7 (broad S, NH).

Reference Example 12

0.20 g of 3-amino-3-methoxy-2-oxoazetidine is dissolved in 20 ml of methylene chloride and under cooling at −15°C, 10 ml of propylene oxide are added, followed by addition of a solution of the acid chloride prepared from 0.76 g of D-N-(4-ethyl-2,3-dioxo-1-piperazinocarbonyl)phenylglycine in 10 ml of methylene chloride. The mixture is stirred at the same temperature for 30 minutes, after which 0.475 g of pyridine is added, followed by stirring for an additional hour. The reaction mixture is concentrated under reduced pressure and after cold-water is added to the residue, it is extracted with THF-ethyl acetate. The extract is washed with water and concentrated under reduced pressure, and the residue is purified by silica gel column chromatography. The above procedure provides 0.43 g of 3-[N-(4-ethyl-2,3-dioxo-1-piperazinocarbonyl)-D-phenylglycinamido]-3-methoxy-2-oxoazetidine.

IR$\nu_{max}^{KBr}$$cm^{-1}$; 3270, 1760, 1710, 1670, 1505, 1190.
NMR ($DMSO-d_6$ ppm); 1.10 (t, J=7Hz, $CH_3$), 3.02 (dd, J=3, 6Hz, $C_4$—βH), 3.39 (t, J=6Hz, $C_4$—αH), 3.41 (q, J=7Hz, $—CH_2—$), 3.45—3.65 (m, $—CH_2—$), 3.30—4.00 (m, $—CH_2—$), 4.86 (ddd, J=3, 6, 8Hz, $C_3$—H),

5.46 (d, J=8Hz, —CH—),

7.2—7.5 (m, aromatic H), 8.00 (s, NH), 9.12 (d, J=8Hz, NH), 9.81 (d, J=8Hz, NH).

Reference Example 13

0.5 g of 10% palladium-carbon is added to a solution of 0.331 g of 3-benzyloxycarboxamido-2-oxoazetidine in 25 ml of ethanol, and the mixture is stirred in a hydrogen gas stream for an hour. The catalyst is filtered off and the filtrate is concentrated. The resulting 3-amino-2-oxoazetidine is dissolved in 5 ml of DMF, followed by addition of 0.535 g of D-N-(3-furfurylideneamino-2-oxo-1-imidazolidino-carbonyl)phenylglycine and 0.341 g of dicyclohexylcarbodiimide. The mixture is stirred for 2 hours and the crystals separated are filtered off. The filtrate is concentrated to provide 0.514 g of 3-[D-2[(3-furfurylideneamino-2-oxoimidazolidin-1-yl)carboxamido]-2-phenylacetamido]-2-oxoazetidine.

$IR\nu_{max}^{KBr}cm^{-1}$; 3300, 1750, 1720, 1660.

Reference Example 14

A solution of 0.693 g of 1-hexamethyleneiminecarboxaldehyde dimethyl acetal in 6 ml of methylene chloride is added to a solution of 0.344 g of 3-amino-2-oxoazetidine in a mixture of DMF (6 ml) and methylene chloride (6 ml). The mixture is stirred at room temperature for an hour, after which methylene chloride is added. It is then washed with water and concentrated to obtain 0.20 g of 3-[(hexahydro-1H-azepin-1-yl)methyleneamine]-2-oxoazetidine.

$IR\nu_{max}^{KBr}cm^{-1}$; 3180, 2430, 1740, 1700, 1620.

NMR (DMSO-$d_6$, ppm); 1.64—3.40 (m, —$CH_2$—), 3.36 (t, J=6Hz, $C_4$—$\beta$H), 3.90 (dd, J=2, 6Hz, $C_4$—$\alpha$H), 4.40 (dd, J=2, 6Hz, $C_3$—H), 7.46 (s, —CH=N—).

Reference Example 15

2.5 g of the 3-benzyloxycarboxamido-3-methoxy-2-oxoazetidine obtained in Reference Example 4 are dissolved in 25 ml of THF, and after 0.5 g of palladium black is added, the mixture is stirred in a hydrogen gas stream for an hour, at the end of which time the catalyst is filtered off. On the other hand, a solution of 4.46 g of N-carbobenzoxy-D-alanine in 35 ml of THF is cooled to −40°C and 1.89 g of diphosgene and 4.2 g of triethylamine are added. To this solution is added the above filtrate at −40°C and the mixture is stirred at room temperature for 2 hours. After filtration, the filtrate is concentrated under reduced pressure and the residue is purified by silica gel column chromatography. The above procedure provides 0.905 g of 3-(N-carbobenzoxy-D-alaninamido)-3-methoxy-2-oxoazetidine.

$[\alpha]_D^{22°}$ + 79.5° (c=1, MeOH).

$IR\nu_{max}^{KBr}cm^{-1}$; 1755, 1680 1515.

NMR (DMSO-$d_6$, ppm); 1.22 (d, J=7Hz, $CH_3$), 3.32 (s, —$OCH_3$), 3.40, 3.48 (each d, J=7Hz, $C_4$—H),

4.12 (m, $-\overset{|}{C}H-$), 5.04 (s, $CH_2$), 7.36 (s, aromatic H), 8.26 (s, NH), 8.98 (d, J=7Hz, NH).

Reference Example 16

A mixture of 0.482 g of 3-(N-carbobenzoxy-D-alanylamino)-3-methoxy-2-oxoazetidine, 0.5 g of palladium black, 10 ml of THF and 5 ml of methanol is stirred in a hydrogen stream for 30 minutes, the catalyst is filtered off and the filtrate is concentrated to dryness under reduced pressure. The residue is dissolved in 2 ml of dimethylacetamide, followed by addition of 0.2 g of triethylamine. While the mixture is stirred under ice-cooling, a solution of 0.337 g of 4-ethyl-2,3-dioxo-1-piperazinocarbonyl chloride is added. The mixture is stirred at room temperature for an hour, after which it is filtered and the filtrate is concentrated under reduced pressure. The residue is purified by silica gel chromatography.

By the above procedure 0.432 g of 3 - [D - N - (4 - ethyl - 2,3 - dioxo - 1 - piperazinocarbonyl)alanylamino] - 3 - methoxy - 2 - oxoazetidine is obtained.

$IR\nu_{max}^{KBr}cm^{-1}$; 1760, 1710, 1670, 1510.

NMR (DMSO-$d_6$, ppm); 1.10 (t, J = 7Hz, $CH_3$), 1.34, 1.44 (each d, J=7Hz, —$CH_3$), 3.36 (s, $CH_3$), 3.90 (m, —$CH_2$—),

4.48 (m, $-\overset{|}{C}H-$), 8.31 (broad s, NH), 9.74 (d, J=7Hz, NH), 9.82 (s, NH).

Reference Example 17

In the same manner as Reference Example 12=A, Reference Example 13=B, Reference Example 14=C, Reference Example 15=D and Reference Example 16=E, 3-amino-2-oxoazetidine or 3-amino-3-methoxy-2-oxoazetidine is reacted with acylating agents to obtain the compounds described below. In

the following description, (a) is the product, (b) the starting material, (c) the method used and (d) the physicochemical constants of the product.

(1) (a) 3-Phenylacetamido-2-oxoazetidine
(b) 3-Amino-2-oxoazetidine
(c) A
(d) IR$\nu_{max}^{KBr}$cm$^{-1}$; 3330, 3270, 1780, 1655.
NMR (DMSO-$d_6$, ppm); 3.05 (dd, J=3, 5Hz, $C_4$—βH), 3.40 (t, J=5Hz, $C_4$—αH), 3.46 (s, —$CH_2$—), 4.38 (ddd, J=3, 5, 8Hz, $C_3$—H), 7.28 (s, aromatic H), 7.93 (broad s, NH), 8.67 (d, J=8Hz, NH).

(2) (a) 3-Thienylacetamido-2-oxoazetidine
(b) 3-Amino-2-oxoazetidine
(c) A
(d) IR$\nu_{max}^{KBr}$cm$^{-1}$; 1775, 1715, 1650, 1530.
NMR (DMSO-$d_6$, ppm); 3.07 (dd, J=3, 5Hz, $C_4$—βH), 3.42 (t, J=5Hz, $C_4$—αH), 3.69 (s, —$CH_2$—), 4.85 (ddd, J=3, 5, 8Hz, $C_3$—H), 6.8—7.4 (m, thienyl H), 7.96 (br. s, NH), 8.77 (d, J=8Hz, NH).

(3) (a) 3-[2-(2-Chloroacetamido-4-thiazolyl)-2-methoxyimino-acetamido]-2-oxoazetidine(*syn*-isomer)
(b) 3-Amino-2-oxoazetidine
(c) A
(d) IR$\nu_{max}^{KBr}$cm$^{-1}$; 1740, 1690, 1660.
NMR (DMSO-$d_6$, ppm); 3.14 (dd, J=3, 5Hz, $C_4$—βH), 3.49 (t, J=5Hz, $C_4$—αH), 3.90 (s, $CH_3$), 4.37 (s, $ClCH_2$—), 4.99 (ddd, J=3, 5, 8Hz, $C_3$—H), 7.43

(s, 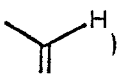), 8.02 (s, NH), 9.22 (d, J=8Hz, NH), 12.86 (br.s, NH).

(4) (a) 3-[2-(2-Chloroacetamido-4-thiazolyl)acetamido]-2-oxoazetidine
(b) 3-Amino-2-oxoazetidine
(c) A
(d) IR$\nu_{max}^{KBr}$cm$^{-1}$; 1740, 1825, 1703, 1655.
NMR (DMSO-$d_6$, ppm); 3.09 (dd, J=3, 5Hz, $C_4$—βH), 3.42 (t, J=5Hz, $C_4$—αH), 3.54 (s, —$CH_2$—), 4.36 (s, $ClCH_2$—), 4.86 (ddd, J=3, 5, 8Hz, $C_3$—H), 6.97

(s, 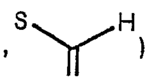), 7.96 (s, NH), 8.65 (d, J=8Hz, NH).

(5) (a) 3-(α-Sulphophenylacetamido)-2-oxoazetidine sodium salt
(b) 3-Amino-2-oxoazetidine
(c) A
(d) IR$\nu_{max}^{KBr}$cm$^{-1}$; 1750, 1655, 1510, 1210, 1190, 1040.
NMR (DMSO-$d_6$, ppm); 3.03 (dd, J=2.5, 5Hz, $C_4$—βH), 3.39 (t, J=5Hz, $C_4$—αH),

4.54 (s, —CH—),

4.82 (ddd, J=2.5, 5, 8Hz, $C_3$—H), 7.1—7.6 (m, aromatic H), 7.92 (broad s, NH), 8.77 (d, J=8Hz, NH).

(6) (a) 3-[D-2-(Benzyloxycarboxamido)-2-phenylacetamido]-2-oxoazetidine
(b) 3-Amino-2-oxoazetidine
(c) B
(d) IR$\nu_{max}^{KBr}$cm$^{-1}$; 3330, 1760, 1740, 1690, 1670.
NMR (DMSO-$d_6$, ppm); 2.92 (dd, J=3, 6Hz, $C_4$—βH), 3.33 (t, J=6Hz, $C_4$—αH), 4.82 (ddd, J=3, 6, 8Hz, $C_3$—H), 5.03 (s, —$CH_2$—),

5.23 (d, J=8Hz, —CH—),

7.31 (s, aromatic H), 7.81 (d, J=8Hz, NH), 7.93 (s, NH), 8.88 (d, J=8Hz, NH).

(7) (a) 3-[2-(2-Chloroacetamido-4-thiazolyl)-2-methoxy-iminoacetamido]-3-methoxy-2-oxoazetidine(*syn*-isomer)
(b) 3-Amino-3-methoxy-2-oxoazetidine
(c) A
(d) IR$\nu^{KBr}_{max}$cm$^{-1}$; 3280, 1760, 1675, 1540.
NMR ($d_6$-DMSO, ppm); 3.44 (s, —$CH_3$), 3.60 (ABq, J=6, 20Hz, $C_4$—$H_2$), 3.92 (s, —$CH_3$), 4.38 (s, —$CH_2$—), 7.42 (s, aromatic H), 8.33 (s, —NH—), 9.78 (s, NH), 12.75 (s, NH).

(8) (a) 3-Cyanoacetamido-2-oxoazetidine
(b) 3-Amino-2-oxoazetidine
(c) A
(d) IR$\nu^{KBr}_{max}$cm$^{-1}$; 2270, 1770, 1715, 1662, 1511.
NMR (DMSO-$d_6$, ppm); 3.10 (dd, J=2, 6Hz, $C_4$—βH), 3.35 (s, —$CH_2$—), 3.40 (t, J=6Hz, $C_4$—αH), 3.72 (s, —$CH_2$—), 4.80 (ddd, J=2, 6, 8Hz, $C_3$—H), 7.93 (s, NH), 8.84 (d, J=8Hz, NH).

(9) (a) 3-(1H-Tetrazole-1-acetamido)-2-oxoazetidine
(b) 3-Amino-2-oxoazetidine
(c) A
(d) IR$\nu^{KBr}_{max}$cm$^{-1}$; 3280, 1735, 1670.
NMR (DMSO-$d_6$, ppm); 3.14 (dd, J=2, 5Hz, $C_4$—βH), 3.46 (t, J=5Hz, $C_4$—αH), 4.90 (q, J=2, 5Hz, $C_3$—H), 5.35 (s, —$CH_2$—), 8.03 (broad s, NH), 9.15 (broad s, NH) 9.35

(s, 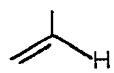).

(10) (a) 3-[3-(2,6-Dichlorophenyl)-5-methylisoxazol-4-yl]-carboxamido-2-oxoazetidine
(b) 3-Amino-2-oxoazetidine
(c) A
(d) IR$\nu^{KBr}_{max}$cm$^{-1}$; 1760, 1660.
NMR (DMSO-$d_6$, ppm); 2.68 (s, $CH_3$), 3.07 (dd, J=2, 5Hz, $C_4$—βH), 3.39 (t, J=5Hz, $C_4$—αH), 4.87 (ddd, J=2, 5, 8Hz, $C_3$—H), 7.53 (s, aromatic H), 8.60 (d, J=8Hz, NH).

(11) (a) 3-(N-Carbobenzoxy-D-alaninamido)-2-oxo-azetidine
(b) 3-Amino-2-oxoazetidine
(c) B
(d) IR$\nu^{KBr}_{max}$cm$^{-1}$; 1770, 1720, 1675, 1645.
NMR (DMSO-$d_6$, ppm); 1.22 (d, J=7Hz, —$CH_3$), 3.06 (dd, J=2, 6Hz, $C_4$—βH), 3.40 (t, J=6Hz, $C_4$—αH),

4.06 (dd, J=7, 8Hz, —CH—),

4.85 (ddd, J=2, 6, 8Hz, $C_3$—H), 5.05 (s, —$CH_2$—), 7.38 (s, aromatic H), 7.94 (broad s, NH), 8.52 (d, J=8Hz, NH).

(12) (a) 3-(α-Benzyl N-carbobenzoxy-γ-D-glutamyl-D-alaninamido)-2-oxoazetidine
(b) 3-Amino-2-oxoazetidine
(c) B
(d) IR$\nu^{KBr}_{max}$cm$^{-1}$; 3260, 1740, 1700, 1655, 1640, 1550, 1520.
NMR (DMSO-$d_6$, ppm); 1.17 (d, J=7Hz, $CH_3$—), 1.93 (m, —$CH_2$—), 2.22 (dd, J=7Hz, —$CH_2CO$—), 3.03 (dd, J=2, 6Hz, $C_4$—βH), 3.38 (t, J=6Hz, $C_4$—αH), 4.84 (ddd, J=2, 6, 8Hz, $C_3$—αH), 5.05 (s, —$CH_2$—), 5.12 (s, —$CH_2$—), 7.37 (s, aromatic H), 7.73 (d, J=8Hz, NH), 7.92 (s, NH), 7.95 (d, J=8Hz, NH), 8.48 (d, J=8Hz, NH).

(13) (a) 3-(α-Ureidophenylacetamido)-2-oxoazetidine
(b) 3-Amino-2-oxoazetidine
(c) B
(d) IR$\nu^{KBr}_{max}$cm$^{-1}$; 3440, 3340, 3280, 3250, 1760, 1740, 1650, 1540.
NMR (DMSO-$d_6$, ppm); 2.97 (dd, J=3, 6Hz, $C_4$—βH), 3.38 (t, J=6Hz, $C_4$—αH), 4.86 (ddd, J=3, 6, 8Hz, $C_3$—H),

5.32 (d, J=8Hz, —CH—),

5.70 (s, $NH_2$), 6.82 (d, J=8Hz, NH), 7.35 (broad s, aromatic H), 7.99 (s, NH), 9.02 (d, J=8Hz, NH).

(14) (a) 3-[D-2-(4-Ethyl-2,3-dioxo-1-piperazinocarboxamido)-2-phenylacetamido]-3-methoxy-2-oxoazetidine
(b) 3-Amino-3-methoxy-2-oxoazetidine
(c) A
(d) IR$\nu^{KBr}_{max}$cm$^{-1}$; 3270, 1760, 1710, 1670, 1505, 1190.
NMR (DMSO-$d_6$, ppm); 1.09 (t, J=7Hz, $CH_3$), 3.36 (s, —$CH_3$),

5.60 (d, J=7Hz, —CH—),

8.25 (s, NH), 9.60 (s, NH), 9.78 (d, J=7Hz, NH).

(15) (a) 3-[D-2-(4-Ethyl-2,3-dioxo-1-piperazinocarboxamido)-2-(4-hydroxyphenyl)acetamido]-2-oxoazetidine
(b) 3-Amino-2-oxoazetidine
(c) A
(d) IR$\nu^{KBr}_{max}$cm$^{-1}$; 1750, 1710, 1670, 1505.
NMR (DMSO-$d_6$, ppm); 1.09 (t, J=8Hz, —$CH_3$), 4.86 (m, $C_3$—H),

5.32 (d, J=7Hz, —CH—),

6.98 (ABq, J=9, 46Hz, phenyl H), 7.96 (s, NH), 8.99 (d, J = 8Hz, NH), 9.70 (d, J=7Hz, NH).

(16) (a) 3-(4-Ethyl-2,3-dioxo-1-piperazinocarboxamido)-2-oxoazetidine
(b) 3-Amino-2-oxoazetidine
(c) A
(d) IR$\nu^{KBr}_{max}$cm$^{-1}$; 3280, 1745, 1710, 1675, 1520.
NMR (DMSO-$d_6$, ppm); 1.11 (t, J=7Hz, $CH_3$), 3.27 (dd, J=3, 6Hz, $C_4$—βH), 3.42 (t, J=6Hz, $C_4$—αH), 3.42 (q, J=7Hz, —$CH_2$—), 3.5—4.1 (m, —$CH_2$—), 4.87 (ddd, J=3, 6, 8Hz, $C_3$—H), 7.98 (s, NH), 9.18 (d, J=8Hz, NH).

(17) (a) 3-[N-(4-Ethyl-2,3-dioxo-1-piperazinocarbonyl)-D-alaninamido]-2-oxoazetidine
(b) 3-Amino-2-oxoazetidine
(c) A
(d) IR$\nu^{KBr}_{max}$cm$^{-1}$; 3290, 1730, 1700, 1665.
NMR (DMSO-$d_6$, ppm); 1.10 (t, J=7Hz, —$CH_3$), 1.30 (d, J=7Hz, —$CH_3$), 3.06 (dd, J=3, 6Hz, $C_4$—βH), 3.4—4.1 (m, —$CH_2$—), 3.43 (q, J=7Hz, —$CH_2$—), 3.46 (t, J=6Hz, $C_4$—αH),

4.34 (quintet, J=7Hz, —CH—),

4.88 (ddd, J=3, 6, 8Hz, $C_3$—H), 7.99 (s, NH), 8.78 (d, J=8Hz, NH), 9.25 (d, J=7Hz, NH).

(18) (a) 3-(2-Methoxyimino-2-phenylacetamido)-2-oxoazetidine (*syn*-isomer)
(b) 3-Amino-2-oxoazetidine
(c) B
(d) NMR ($CDCl_3$, ppm); 3.26 (dd, J=2, 5Hz, $C_4$—βH), 3.52 (t, J=5Hz, $C_4$—αH), 3.92 (s, $CH_3$), 4.96 (ddd, J=2, 5, 8Hz, $C_3$—H), 6.47 (br.s, NH), 7.2—7.7 (m, aromatic H).

(19) (a) 3-[D-2-(4-Ethyl-2,3-dioxo-1-piperazinocarboxamido)-2-(4-methoxyphenyl)acetamido]-2-oxoazetidine
(b) 3-Amino-2-oxoazetidine
(c) A
(d) IR$\nu^{KBr}_{max}$cm$^{-1}$; 3280, 1750, 1710, 1670, 1505.
NMR (DMSO-$d_6$, ppm); 1.09 (t, J=7Hz, —$CH_3$), 2.94 (dd, J=3, 6Hz, $C_4$—βH), 3.38 (t, J=6Hz, $C_4$—αH), 3.41 (q, J=7Hz, —$CH_2$—), 3.4—4.1 (m, —$CH_2$—), 3.76 (s, —$CH_3$), 4.86 (ddd, J=3, 6, 8Hz, $C_3$—H),

5.38 (d, J=7Hz, —CH—),

6.93, 7.33 (ABq, J=9Hz, aromatic H), 7.98 (s, NH), 9.04 (d, J=8Hz, NH), 9.74 (d, J=7Hz, NH).

(20) (a) 3-[D-2-[2-(2-Chloroacetamido-4-thiazolyl)-2-methoxyiminoacetamido]-2-phenylacetamido]-2-oxoazetidine
(a mixture of *syn*- and *anti*- isomers)
(b) 3-Amino-2-oxoazetidine
(c) E
(d) $IR\nu_{max}^{KBr}cm^{-1}$; 3270, 1740, 1660, 1540.
NMR ($DMSO-d_6$, ppm); 2.97 (dd, J=3, 6Hz, $C_4$—$\beta$H), 3.42 (t, J=6Hz, $C_4$—$\alpha$H), 3.88 (s, —$CH_3$), 4.39 (s, $ClCH_2$—), 4.93 (ddd, J=3, 6, 8Hz, $C_3$—H),

5.62 (d, J=8Hz, —CH—),

7.2—7.6 (m, aromatic H) 7.47 (s, 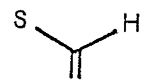 ),

8.03 (s, NH), 8.93 (d, J=8Hz, NH), 9.34 (d, J=8Hz, NH), 12.7 (broad s, NH).

(21) (a) 3-[D-2-(6-Bromo-1,4-dihydro-1-ethyl-4-oxothieno[2,3-b]pyridine-3-carboxamido)-2-phenylacetamido]-2-oxoazetidine
(b) 3-Amino-2-oxoazetidine
(c) E
(d) $IR\nu_{max}^{KBr}cm^{-1}$; 3290, 1770, 1660, 1600, 1530, 1500.
NMR ($DMSO-d_6$, ppm); 1.43 (t, J=7Hz, $CH_3$—), 2.97 (dd, J=3, 6Hz, $C_4$—$\beta$H), 3.40 (t, J=6Hz, $C_4$—$\alpha$H), 4.27 (q, J=7Hz, —$CH_2$—), 4.90 (ddd, J=3, 6, 8Hz, $C_3$—H),

5.70 (d, J=7Hz, —CH—),

7.2, 7.6 (m, phenyl H), 7.63 (s, aromatic H), 7.98 (s, NH), 8.70 (s, aromatic H), 9.11 (d, J=8Hz, NH), 10.95 (d, J=7Hz, NH).

(22) (a) 3-[2-(4-Ethyl-2,3-dioxo-1-piperazinocarboxamido)-2-(2-chloroacetamido-4-thiazolyl)-acetamido]-2-oxoazetidine
(b) 3-Amino-2-oxoazetidine
(c) B
(d) $IR\nu_{max}^{KBr}cm^{-1}$; 3270, 1740, 1702, 1670.
NMR ($DMSO-d_6$, ppm); 1.10 (t, J=7Hz, $CH_3$), 3.40 (q, J=7Hz, —$CH_2$—), 3.4—4.1 (m, —$CH_2$—), 4.87 (broad s, $C_3$—H), 4.37 (s, $ClCH_2$—),

5.55 (d, J=7Hz, —CH—),

7.22 (s, aromatic H), 7.98 (s, NH), 8.91 (d, J=8Hz, NH), 9.76 (d, J=7Hz, NH), 12.6 (broad s, NH).

(23) (a) 3-[2-(4-Ethyl-2,3-dioxo-1-piperazinocarboxamido)-2-(2-acetamido-4-thiazolyl)-acetamido]-2-oxoazetidine
(b) 3-Amino-2-oxoazetidine
(c) B
(d) $IR\nu_{max}^{KBr}cm^{-1}$; 3270, 1740, 1710, 1670.
NMR ($DMSO-d_6$, ppm); 1.10 (t, J=7Hz, —$CH_3$), 2.14 (s, $CH_3$), 3.0—3.7 (m, $C_4$—H), 4.83 (m, $C_3$—H),

5.56 (d, J=7Hz, —CH—),

7.18 (s, 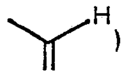),

7.97 (s, NH), 9.13 (d, J=8Hz, NH), 9.73 (d, J=7Hz, NH), 12.2 (broad s, NH).

(24) (a) 3-[2-[2-(2-Chloroacetamido-4-thiazolyl)-2-methoxyiminoacetamido]-2-(2-chloroacetamido-4-thiazolyl)acetamido]-2 -oxoazetidine(*syn*-isomer)
(b) 3-Amino-2-oxoazetidine
(c) B
(d) $IR\nu^{KBr}_{max}cm^{-1}$; 3250, 1745, 1655, 1550.
NMR (DMSO-$d_6$, ppm); 3.0—3.2 (m, $C_4$—βH), 3.44 (t, J=6Hz, $C_4$—αH), 3.88 (s, —$CH_3$), 4.32 (s, $ClCH_2$—), 4.86 (m $C_3$—H),

5.62 (d, J=8Hz, —CH—),

7.13 (s, 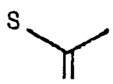), 7.51 (s, 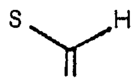),

8.00 (s, NH), 8.76 (d, J=8Hz, NH), 9.21 (d, J=8Hz, NH), 12.2 (broad s, NH).

(25) (a) 3-[D-2-(2,3-Dioxo-4-n-octyl-1-piperazinocarboxamido)-2-phenylacetamido]-2-oxoazetidine
(b) 3-Amino-2-oxoazetidine
(c) B
(d) $IR\nu^{KBr}_{max}cm^{-1}$; 1755, 1710, 1670, 1500, 1085.
NMR (DMSO-$d_6$, ppm); 0.7—1.7 (m, —$CH_2$—, $CH_3$), 2.93 (dd, J=3, 6Hz, $C_4$—βH), 3.36 (t, J=6Hz, $C_4$—αH), 3.2—4.1 (m, —$CH_2$—), 4.85 (ddd, J=3, 6, 8Hz, $C_3$—H)

5.42 (d, J=7Hz, —CH—),

7.2—7.5 (m, aromatic H), 7.98 (s, NH), 9.12 (D, J=8Hz, NH), 9.83 (d, J=7Hz, NH).

(26) (a) 3-[D-2-(Courmarin-3-carboxamido)-2-phenylacetamido]-2-oxoazetidine
(b) 3-Amino-2-oxoazetidine
(c) E
(d) $IR\nu^{KBr}_{max}cm^{-1}$; 3280, 1795, 1740, 1710, 1660, 1610, 1560, 1180.
NMR (DMSO-$d_6$, ppm); 3.01 (dd, J=3, 6Hz, $C_4$—H), 3.43 (t, J=6Hz, $C_4$—H), 4.95 (ddd, J=3, 6, 8Hz, $C_3$—H),

5.72 (d, J=7Hz, —CH—),

7.3—9.0 (m, aromatic H), 9.23 (d, J=8Hz, NH), 9.68 (d, J=7Hz, NH).

(27) (a) 3-[2-(2,3-Dioxo-4-n-octyl-1-piperazinocarboxamido)-2-(2-chloroacetamido-4-thiazolyl)-acetamido]-2-oxoazetidine
(b) 3-Amino-2-oxoazetidine
(c) B
(d) $IR\nu^{KBr}_{max}cm^{-1}$; 3270, 2925, 1750, 1710, 1670.
NMR (DMSO-$d_6$, ppm); 4.31 (s, $ClCH_2$—), 4.81 (m, $C_3$—H),

5.50 (d, J=7Hz, —CH—),

7.17 (s, 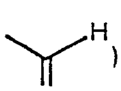),

7.96 (s, NH), 8,88 (d, J=9Hz, NH), 9.75 (d, J=7Hz, NH), 12.63 (s, NH).

(28) (a) 3-[D-2-(4-Hydroxy-7-trifluoromethylquinoline-3-carboxamido)-2-phenylacetamido]-2-oxoazetidine
(b) 3-Amino-2-oxoazetidine
(c) B
(d) $IR\nu_{max}^{KBr}cm^{-1}$; 3240, 1740, 1645, 1520.
NMR(DMSO-$d_6$, ppm); 3.12 (dd, J=3, 5Hz, $C_4$—βH), 3.45 (t, J=5Hz, $C_4$—αH), 4.87 (m, $C_3$—H),

5.72 (d, J=8Hz, —CH—),
|

7.99 (s, NH), 9.07 (d, J=8Hz, NH), 10.86 (d, J=8Hz, NH).

(29) (a) 3-[2-(2,3-Dioxo-4-n-octyl-1-piperazinocarboxamido)-2-thienylacetamido]-2-oxoazetidine
(b) 3-Amino-2-oxoazetidine
(c) B
(d) $IR\nu_{max}^{KBr}cm^{-1}$; 3270, 2920, 1755, 1710, 1670.
NMR (DMSO-$d_6$, ppm); 0.86 (t, J=7Hz, $CH_3$), 3.01, 3.09 (dd, J=3, 6Hz, $C_4$—βH), 3.37 (t, J=7Hz, —$CH_2$—), 3.4—4.1 (m, —$CH_2$—), 4.86 (m, $C_3$—H),

5.73 (d, J=7Hz, —CH—),
|

6.9—7.6 (m, aromatic H), 8.01 (s, NH), 9.14, 9.17 (d, J=8Hz, NH), 9.76 (d, J=7Hz, NH).

(30) (a) 3-[D-2-(2,3-Dioxo-4-n-octyl-1-piperazinocarboxamido)-2-(4-hydroxyphenyl)acetamido]-2-oxoazetidine
(b) 3-Amino-2-oxoazetidine
(c) B
(d) $IR\nu_{max}^{KBr}cm^{-1}$; 3260, 2910, 1740, 1705, 1670.
NMR (DMSO-$d_6$, ppm); 0.86 (t, J=7Hz, $CH_3$), 2.95 (dd, J=3, 6Hz, $C_4$—βH), 4.86 (ddd, J=3, 6, 8Hz, $C_3$—H),

5.32 (d, J=7Hz, —CH—),
|

6.97 (ABq, J=8, 46Hz, aromatic H), 7.98 (s, NH), 8.99 (d, J=8Hz, NH), 9.42 (s, OH), 9.70 (d, J=7Hz, NH).

(31) (a) 3-[D-2-[(3-Furfurylideneamino-2-oxoimidazolidin-1-yl)-carboxamido]-2-(4-hydroxyphenyl)-acetamido]-2-oxoazetidine
(b) 3-Amino-2-oxoazetidine
(c) B
(d) $IR\nu_{max}^{KBr}cm^{-1}$; 1750, 1720, 1660, 1420.
NMR (DMSO-$d_6$, ppm); 2.97 (dd, J=3, 6Hz, $C_4$—βH), 3.35 (t, J=6Hz, $C_4$—αH), 3.80 (broad s, —$CH_2$—), 4.88 (ddd, J=3, 6, 8Hz, $C_3$—H),

5.33 (d, J=7Hz, —CH—),
|

6.6—7.9 (m, furyl H), 6.74, 7.22 (ABq, J=46, 9Hz, phenyl H), 7.74 (s, —CH=), 7.98 (s, NH), 8.92 (d, J=7Hz, NH), 9.00 (d, J=7Hz, NH), 9.44 (s, OH).

(32) (a) 3-[D-2-[(3-Furfurylideneamino-2-oxoimidazolidin-1-yl)-carboxamido]-2-thienylacetamido]-2-oxoazetidine
(b) 3-Amino-2-oxoazetidine
(c) B
(d) $IR\nu_{max}^{KBr}cm^{-1}$; 3300, 1750, 1720, 1660, 1415.

(33) (a) 3-[D-2-[[3-(Thiophen-2-aldoimino)-2-oxoimidazolidin-1-yl]-carboxamido]-2-phenylacetamido]-2-oxoazetidine
(b) 3-Amino-2-oxoazetidine
(c) B
(d) $IR\nu_{max}^{KBr}cm^{-1}$; 1755, 1720, 1600.

(34) (a) 3-[2-(4-Ethyl-2,3-dioxo-1-piperazinocarboxamido)-2-(2-pyrrolyl)acetamido]-2-oxo-azetidine
(b) 3-Amino-2-oxoazetidine
(c) B
(d) $IR\nu_{max}^{KBr}cm^{-1}$; 3280, 1745, 1705, 1670, 1500, 1190.
NMR ($DMSO-d_6$, ppm); 1.10 (t, J=7Hz, $—CH_3$), 3.03 3.10 (dd, J=3, 6Hz, $C_4—\beta H$), 3.40 (q, J=7Hz, $—CH_2—$), 3.4—4.1 (m, $—CH_2—$), 4.86 (m, $C_3—H$),

5.46 (d, J=7Hz, —CH—),
|

5.9—6.8 (m, pyrrolyl H), 7.98 (s, NH), 8.89, 8.91 (d, J=8Hz, NH), 9.48 (d, J=7Hz, NH), 10.72 (broad s, NH).

(35) (a) 3-[2-(2,3-Dioxo-4-n-octyl-1-piperazinocarboxamido)-2-thienylacetamido]-3(S)-methoxy-2-oxoazetidine
(b) 3-Amino-3-methoxy-2-oxoazetidine
(c) A
(d) $IR\nu_{max}^{KBr}cm^{-1}$; 3270, 2920, 1760, 1705 1675.
NMR ($d_6$-DMSO, ppm); 0.86 (t, $CH_3$), 3.20 (s, $OCH_3$), 3.4—4.1 (m, ring $CH_2$), 3.44, 3.57 (ABq, J=6, 13Hz, $C_4—H$),

5.90 (d, J=7Hz, —CH—),
|

6.9—7.6 (m, thienyl H), 8.36 (s, NH), 9.74 (d, J=7Hz, NH).

(36) (a) 3-[2-(2,3-Dioxo-4-n-octyl-1-piperazinocarboxamido)-2-thienylacetamido]-3(R)-methoxy-2-oxoazetidine
(b) 3-Amino-3-methoxy-2-oxoazetidine
(c) A
(d) $IR\nu_{max}^{KBr}cm^{-1}$; 3270, 2920, 1760, 1705, 1675.
NMR ($d_6$-DMSO, ppm); 0.86 (t, $CH_3$), 3.36 (s, $OCH_3$), 3.39, 3.48 (ABq, J=9, 6Hz, $C_4—H$), 3.4—4.1 (m, ring $CH_2$),

5.89 (d, J=7Hz, —CH—),
|

6.9—7.6 (m, thienyl H), 8.31 (s, NH), 9.67 (s, NH), 9.70 (d, J=7Hz, NH).

(37) (a) 3-(D-$\alpha$-Sulphophenylacetamido)-3-methoxy-2-oxoazetidine sodium salt
(b) 3-Amino-3-methoxy-2-oxoazetidine
(c) A
(d) $IR\nu_{max}^{KBr}cm^{-1}$; 3270, 1745, 1670, 1200, 1040.
NMR ($DMSO-d_6$, ppm); 3.31, 3.41 (s, $CH_3$), 3.47 (ABq, J=6, 12Hz, $C_4—H$), 5.65,

5.70 (s, —CH—),
|

7.2—7.5 (m, aromatic H), 8.29 (s, NH), 9.2, 9.29 (s, NH).

(38) (a) 3-(N-Carbobenzoxy-D-alanyl-D-phenylglycinamido)-2-oxoazetidine
(b) 3-Amino-2-oxoazetidine
(c) B
(d) $IR\nu_{max}^{KBr}cm^{-1}$; 3290, 1750, 1690, 1640, 1520.
NMR ($DMSO-d_6$, ppm); 1.20 (d, J=6Hz, $CH_3—$), 2.91 (dd, J=2, 6Hz, $C_4—\beta H$), 3.35 (t, J=6Hz, $C_4—\alpha H$),

4.17 (m, —CH—),
|

4.83 (ddd, J=2, 6, 8Hz, $C_3—H$), 4.99 (s, $—CH_2—$),

5.42 (d, J=9Hz, —CH—),

7.30 (s, aromatic H), 7.94 (br. s, NH), 8.97 (d, J=9Hz, NH).

(39) (a) 3-[D-2-(2-Ureido-2-thienylacetamido)-2-phenylacetamido]-2-oxoazetidine
(b) 3-Amino-2-oxoazetidine
(c) B
(d) $IR\nu_{max}^{KBr}cm^{-1}$; 1758, 1640, 1520.

(40) (a) 3-Cyanomethylacetamido-2-oxoazetidine
(b) 3-Amino-2-oxoazetidine
(c) B
(d) $IR\nu_{max}^{KBr}cm^{-1}$; 2270, 1770, 1715, 1662, 1511.
NMR (DMSO-$d_6$, ppm); 3.10 (dd, J=2, 6Hz, $C_4$—$\beta$H), 3.35 (s, —$CH_2$—), 3.72 (s, —$CH_2$—), 3.40 (t, J=6Hz, $C_4$—$\alpha$H), 4.80 (ddd, J=2, 6, 8Hz, $C_3$—H), 7.93 (s, NH), 8.84 (d, J=8Hz, NH).

(41) (a) 3-[D-2-[2-(2-Chloracetamido-4-thiazolyl)acetamido]-2-phenylacetamido]-2-oxoazetidine
(b) 3-Amino-2-oxoazetidine
(c) E
(d) $IR\nu_{max}^{KBr}cm^{-1}$; 1741, 1680, 1650, 1634, 1530.

(42) (a) 3-[2-(4-Ethyl-2,3-dioxo-1-piperazinocarboxamido)-2-thienylacetamido]-2-oxoazetidine
(b) 3-Amino-2-oxoazetidine
(c) A
(d) $IR\nu_{max}^{KBr}cm^{-1}$; 1752, 1707, 1670, 1497, 1160, 1182.
NMR (DMSO-$d_6$, ppm); 1.08 (t, J=7Hz, $CH_3$), 3.02 (dd, J=3, 6Hz, $C_4$—$\beta$H), 3.3 4.1 (m, —$CH_2$—, $C_4$—$\alpha$H), 4.86 (ddd, J=3, 6, 8Hz, $C_3$—H),

5.71 (d, J=7Hz, —CH—),

6.9—7.5 (m, thienyl H), 8.01 (s, NH), 9.20, 9.18 (each d, J=8Hz, NH), 9.77 (d, J=7Hz, NH).

(43) (a) 3-(2-Methoxyimino-2-thienylacetamido)-2-oxoazetidine
(b) 3-Amino-2-oxoazetidine
(c) B
(d) $IR\nu_{max}^{KBr}cm^{-1}$; 1760, 1652, 1628.

(44) (a) 3-[2-Thienyl-2-(3-morpholinopropoxyimino)acetamido]-2-oxoazetidine
(b) 3-Amino-2-oxoazetidine
(c) B
(d) $IR\nu_{max}^{KBr}cm^{-1}$; 3240, 1750, 1658, 1540—1520.
NMR (DMSO-$d_6$, ppm); 3.18 (dd, J=3, 6Hz, $C_4$—H), 4.21 (t, J=6Hz, $C_4$—H), 4.93 (ddd, J=2, 6, 8Hz, $C_3$—H) 8.01 (s, NH), 9.25 (d, J=8Hz, NH).

(45) (a) 3-[D-2-[2-(4-Ethyl-2,3-dioxo-1-piperazinocarboxamido)-2-thienylacetamido]-2-phenylacetamido]-2-oxoazetidine
(b) 3-Amino-2-oxoazetidine
(c) B
(d) $IR\nu_{max}^{KBr}cm^{-1}$; 3290, 1760, 1708, 1672, 1648, 1510, 1190.

(46) (a) 3-[2-(2,5-Dioxo-1,2,4-triazino-6-carboxamido]-2-oxoazetidine
(b) 3-Amino-2-oxoazetidine
(c) B
(d) $IR\nu_{max}^{KBr}cm^{-1}$; 3250, 1760, 1720, 1680, 1504, 1420, 1210.
NMR (DMSO-$d_6$, ppm); 3.10 (dd, J=2, 6Hz, $C_4$—H), 3.40 (t, J=6Hz, $C_4$—H), 4.85 (ddd, J=2, 6, 8Hz, $C_3$—H),

5.85 (d, J=7Hz, —CH8),

8.00 (s, NH), 9.16, 9.19 (each d, J=8Hz, NH), 9.60 (d, J=7Hz, NH).

(47) (a) 3-[2-(4-Ethyl-2,3-dioxo-1-piperazinocarboxamido)-2-(2-methyl-4-thiazolyl)acetamido]-2-oxoazetidine
(b) 3-Amino-2-oxoazetidine
(c) B
(d) $IR\nu^{KBr}_{max}cm^{-1}$; 3280, 1708, 1670, 1500, 1187.
NMR ($DMSO-d_6$, ppm); 1.08 (t, J=7Hz, $CH_3$), 2.63 (s, $CH_3$), 3.34 (dd, J=3, 6Hz, $C_4$—H), 4.83 (ddd, J=2, 6, 8Hz, $C_3$—H),

5.52 (d, J=7Hz, —CH—), 7.40 ( s, 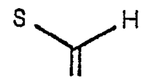 ),

7.96 (s, NH), 8.95 (d, J=8Hz, NH), 9.78 (d, J=7Hz, NH).

(48) (a) 3-[2-[3-(4-Chlorobenzoyl)ureido]-2-thienylacetamido]-2-oxoazetidine
(b) 3-Amino-2-oxoazetidine
(c) B
(d) $IR\nu^{KBr}_{max}cm^{-1}$; 3270, 1760, 1685, 1664, 1525, 1463, 1282.
NMR ($DMSO-d_6$, ppm); 3.25 (dd, J=3, 6Hz, $C_4$—H), 3.57 (t, J=6Hz, $C_4$—H), 4.94 (ddd, J=2, 6.8Hz, $C_3$—H), 5.82 (s, —CH—).

(49) (a) 3-Cyanomethylthioacetamido-3-methoxy-2-oxoazetidine
(b) 3-Amino-3-methoxy-2-oxoazetidine
(c) A
(d) $IR\nu^{KBr}_{max}cm^{-1}$; 1758, 1670, 1520.
NMR ($DMSO-d_6$, ppm); 3.33 (s, $CH_3$), 3.33, 3.70 (each s, —$CH_2$—), 3.2—3.8 (m, $C_4$—H), 8.27 (s, NH), 9.28 (s, NH).

(50) (a) 3-(2-Benzyloxycarbonyl-2-phenylacetamido)-3-methoxy-2-oxoazetidine
(b) 3-Amino-3-methoxy-2-oxoazetidine
(c) A
(d) $IR\nu^{KBr}_{max}cm^{-1}$; 1775, 1722, 1685, 1160.

(51) (a) 3-[2-(5,6-Dihydro-1,4-oxathiin-2-yl)acetamido)-2-oxoazetidine
(b) 3-Amino-2-oxoazetidine
(c) B
(d) $IR\nu^{KBr}_{max}cm^{-1}$; 1745, 1720, 1650, 1640.
NMR ($DMSO-d_6$, ppm); 3.05 (dd, J=3, 6Hz, $C_4$—H), 3.40 (t, J=6Hz, $C_4$—H), 4.20 (t, J=5Hz, —$CH_2$—), 4.83 (ddd, J=2, 6, 8Hz, $C_3$—H),

5.10 (s, 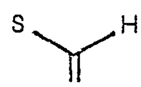 ), 7.91 (br. S, NH), 8.42 (d, J=8Hz, NH).

(52) (a) 3-(N-Carbamoyl-D-tryptophyl-D-phenylglycinamido)-2-oxoazetidine
(b) 3-Amino-2-oxoazetidine
(c) B
(d) $IR\nu^{KBr}_{max}cm^{-1}$; 1755, 1640, 1540—1520.
NMR ($DMSO-d_6$, ppm); 2.97 (dd, J=2, 6Hz, $C_4$—H), 3.48 (t, J=6Hz, $C_4$—H),

4.52 (m, —CH—),

4.86 (ddd, J=2, 6, 8Hz, $C_3$—H), 5.51 (s, —$CH_2$—), 5.52 (d, J=8Hz, —CH—),

5.55 (br. s, NH), 7.21 ( s, 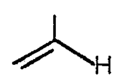 ),

7.45 (s, aromatic H).

(53) (a) 3-[D-N-(4-Ethyl-2,3-dioxo-1-piperazinocarbonyl)phenylalaninamido]-2-oxoazetidine
(b) 3-Amino-2-oxoazetidine
(c) B
(d) $IR\nu^{KBr}_{max}cm^{-1}$; 1750, 1710, 1670, 1518.

NMR ($DMSO-d_6$, ppm); 1.08 (t, J=7Hz, $CH_3$), 2.90 (dd, J=2, 6Hz, $C_4$—βH), 3.38 (m, —$CH_2$—), 3.42 (t, J=6Hz, $C_4$—αH),

4.56 (dd, J=6, 8Hz, —CH—),

4.84 (ddd, J=2, 6, 8Hz, $C_3$—H), 7.21 (s, aromatic H), 7.95 (s, NH), 8.79 (d, J=8Hz, NH), 9.15 (d, J=8Hz, NH).

(54) (a) 3-[2-(2,4-Dioxopyrimidino-5-carboxamido)-2-thienylacetamido]-2-oxoazetidine
(b) 3-Amino-2-oxoazetidine
(c) B
(d) $IR\nu_{max}^{KBr}cm^{-1}$; 1740, 1700, 1650—1680 (broad), 1508.
NMR ($DMSO-d_6$, ppm); 3.04 (dd, J=2, 6Hz, $C_4$—βH), 3.42 (t, J=6Hz, $C_4$—αH), 4.84 (ddd, J=2, 6, 8Hz, $C_3$—H),

5.85 (d, J=8Hz, —CH—),

7.99 (s, NH), 9.16, 9.19 (each d, J=8Hz, NH), 9.60 (d, J=8Hz, NH).

(55) (a) 3-[D-2-(2-Ureido-2-thienylacetamido)-2-(4-hydroxyphenyl)-acetamido]-2-oxoazetidine
(b) 3-Amino-2-oxoazetidine
(c) B
(d) $IR\nu_{max}^{KBr}cm^{-1}$; 1760, 1650, 1530, 1510.

(56) (a) 3-[D-N-(4-Ethyl-2,3-dioxo-1-piperazinocarbonyl)glutaminylamino]-2-oxoazetidine
(b) 3-Amino-2-oxoazetidine
(c) B
(d) $IR\nu_{max}^{KBr}cm^{-1}$; 1757, 1700, 1670.

(57) (a) 3-[2-(4-Ethyl-2,3-dioxo-1-piperazinocarboxamido)-2-(1-cyclohexen-1-yl)acetamido]-2-oxoazetidine
(b) 3-Amino-2-oxoazetidine
(c) B
(d) $IR\nu_{max}^{KBr}cm^{-1}$; 3300, 2930, 1755, 1710, 1670.
NMR ($DMSO-d_6$, ppm); 1.10 (t, J=8Hz, $CH_3$), 1.33—1.70 (m, —$CH_2$—), 1.70—2.13 (m, —$CH_2$—), 3.40 (q, —$CH_2$—),

4.76 (d, J=8Hz, —CH—), 5.73 ( broad s, 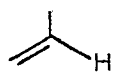 ),

7.93 (s, NH), 8.73, 8.76 (d, J=8Hz, NH), 9.40 (d, J=8Hz, NH).

(58) (a) 3-[2-(4-Ethyl-2,3-dioxo-1-piperazinocarboxamido)-2-(4-chlorophenyl)acetamido]-2-oxoazetidine
(b) 3-Amino-2-oxoazetidine
(c) $IR\nu_{max}^{KBr}cm^{-1}$; 1755 1710, 1670, 1520.
NMR ($DMSO-d_6$, ppm); 1.10 (t, J=7Hz, $CH_3$), 3.0 (m, $C_4$—H), 3.42 (q, J=7Hz, —$CH_2$—), 4.85 (m, $C_3$—H), 5.44,

5.46 (d, J=7Hz, —CH—),

7.46 (s, aromatic H), 8.02 (broad s, NH), 9.16 (d, J=8Hz, NH), 9.84 (d, J=7Hz, NH).

(59) (a) 3-[2-(4-Ethyl-2,3-dioxo-1-piperazinocarboxamido)-2-(4-trimethylsilylphenyl)acetamido]-2-oxazetidine
(b) 3-Amino-2-oxoazetidine
(c) $IR\nu_{max}^{KBr}cm^{-1}$; 1755, 1710, 1570, 1500.
NMR ($DMSO-d_6$, ppm); 0.26 (s, $CH_3$—), 3.0 (m, $C_4$—H), 4.85 (m, $C_3$—H), 5.42,

5.44 (d, J=8Hz, —CH—),

7.40 (d, J=8Hz, phenyl H), 7.54 (d, J=8Hz, phenyl H), 8.02 (broad s, NH), 9.10 (s, NH), 9.84 (d, J=8Hz, NH).

(60) (a) 3-[D-N-(4-Ethyl-2,3-dioxo-1-piperazinocarbonyl)methionyl-D-phenylglycinamido]-2-oxoazetidine
(b) 3-Amino-2-oxoazetidine
(c) B
(d) $IR\nu^{KBr}_{max}cm^{-1}$; 3300, 1760, 1710, 1675, 1640, 1520.
NMR ($DMSO-d_6$, ppm); 1.08 (t, J=7Hz, $CH_3$), 2.06 (s, $CH_3$), 2.96 (dd, J=2, 6Hz, $C_4$—βH),

5.54 (d, J=8Hz, —CH—),

4.86 (m, $C_3$—H), 7.96 (broad s, NH), 9.26 (d, J=8Hz, NH).

(61) (a) 3-[2-(4-Ethyl-2,3-dioxo-1-piperazinocarboxamido)-2-(1-cyclohexen-1-yl)acetamido]-3-methoxy-2-oxoazetidine
(b) 3-Amino-2-oxoazetidine
(c) B
(d) $IR\nu^{KBr}_{max}cm^{-1}$; 3300, 1760, 1710, 1675.
NMR ($DMSO-d_6$, ppm); 1.10 (t, J=7Hz, $CH_3$), 1.52 (m, —$CH_2$—), 1.96 (m, —$CH_2$—), 3.30 (s, $CH_3$), 3.41 (q, J=7Hz, —$CH_2$—), 3.54 (m, —$CH_2$—), 3.90 (m, —$CH_2$—), 4.70,

4.97 (each d, J=7Hz, —CH—), 5.84 ( broad s, 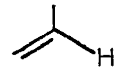 ),

8.31 (broad s, NH), 9.44 (broad s, NH), 9.38 (d, J=7Hz, NH).

(62) (a) 3-[D-2-(3-Methylcarbamoyl-3-methyl-1-ureido)-2-phenylacetamido]-2-oxoacetidine
(b) 3-Amino-2-oxoazetidine
(c) B
(d) $IR\nu^{KBr}_{max}cm^{-1}$; 3270, 1756, 1687m 1662, 1626.
NMR ($DMSO-d_6$, ppm); 2.72 (d, J=4Hz, —$CH_3$), 3.11 (s, $CH_3$), 3.42 (t, J=5Hz, $C_4$—αH), 4.93 (m, $C_3$—H),

5.42 (d, J=7Hz, —CH—),

7.43 (s, aromatic H, NH), 8.03 (s, NH), 9.15 (d, J=9Hz, NH), 10.08 (d, J=7Hz, NH).

(63) (a) 3-[2-(3-Methylcarbamoyl-3-Methyl-1-ureido)-2-thienylacetamido]-2-oxoazetidine
(b) 3-Amino-2-oxoazetidine
(c) B
(d) $IR\nu^{KBr}_{max}cm^{-1}$; 3275, 1755, 1680.

(64) (a) 3-[D-2-(3-Methylcarbamoyl-3-methyl-1-ureido)-2-(4-benzyloxy)phenylacetamido]-2-oxoazetidine
(b) 3-Amino-2-oxoazetidine
(c) B
(d) $IR\nu^{KBr}_{max}$3330, 3270, 1752, 1688, 1662, 1625.

(65) (a) 3-[D-2-[3-(2-Benzyloxybenzoyl)-1-ureido]-2-phenylacetamido]-2-oxoazetidine
(b) 3-Amino-2-oxoazetidine
(c) B
(d) $IR\nu^{KBr}_{max}cm^{-1}$; 3330, 1763, 1692, 1668.
NMR ($DMSO-d_6$, ppm); 3.03 (dd, J=3, 6Hz, $C_4$—H), 3.47 (t, J=6Hz, $C_4$—H), 4.93 (m, $C_3$—H), 5.37 (s, —$CH_2$—),

5.56 (d, J=7Hz, —CH—)

7.47 (s, aromatic H), 7.25—8.10 (m, aromatic H), 8.17 (s, NH), 9.27 (d, J=7Hz, NH), 9.63 (d, J=7Hz, NH), 10.43 (s, NH).

(66) (a) 3-[D-2-[3-(2-Benzyloxybenzoyl)-1-ureido]-2-(4-hydroxyphenyl)acetamido]-2-oxoazetidine
(b) 3-Amino-2-oxoazetidine
(c) B
(d) $IR\nu_{max}^{KBr}cm^{-1}$; 3300, 1743, 1688, 1662.

(67) (a) 3-[D-2-(3-Chloro-4-hydroxyphenyl)-2-(4-ethyl-2,3-dioxo-1-piperazinocarboxamido)-acetamide]-2-oxoazetidine
(b) 3-Amino-2-oxoazetidine
(c) B
(d) $IR\nu_{max}^{KBr}cm^{-1}$; 3300, 1750, 1713, 1685.
NMR (DMSO-$d_6$, ppm); 1.13 (t, J=7Hz, —$CH_3$), 3.05 (dd, J=2, 5Hz, $C_4$—H), 3.30—3.53 (m, —$CH_2$—, $C_4$—H), 3.53—4.2 (m, —$CH_2$—, 4.97 (m, $C_3$—H),

5.47 (d, J=7Hz, —CH—),
|

7.00—7.73 (m, aromatic H), 8.13 (s, NH), 9.25 (d, J=9Hz, NH), 9.97 (d, J=7Hz, NH).

(68) (a) 3-[D-2-(3-Chloro-4-methoxyphenyl)-2-(4-ethyl-2,3-dioxo-1-piperazinocarboxamido)-acetamido]-2-oxoazetidine
(b) 3-Amino-2-oxoazetidine
(c) B
(d) $IR\nu_{max}^{KBr}cm^{-1}$; 3270, 1755, 1708, 1668.

(69) (a) 3-[D-2-(2-Benzyloxycarboxamido-3-N-methylcarbamoyl-propionamido)-2-phenyl-acetamido]-2-oxoazetidine (a mixture of diastereoisomers)
(b) 3-Amino-2-oxoazetidine
(c) B
(d) $IR\nu_{max}^{KBr}cm^{-1}$; 3290, 1766, 1690, 1640.

(70) (a) 3-[D-2-(3-Benzyloxycarboxamido-3-N-methylcarbamoyl-propionamido)-2-phenyl-acetamido]-2-oxoazetidine
(b) 3-Amino-2-oxoazetidine
(c) B
(d) $IR\nu_{max}^{KBr}cm^{-1}$; 3300, 1750, 1690, 1640, 1620.

(71) (a) 3-[2-(2,5-Dioxopyrrolilin-3-yl)acetamido]-2-oxoazetidine
(b) 3-Amino-2-oxoazetidine
(c) B
(d) $IR\nu_{max}^{KBr}cm^{-1}$; 3240, 1750, 1710, 1680.
NMR (DMSO-$d_6$, ppm); 2.3—3.6 (m, —$CH_2$—, $C_4$—H), 4.6—5.0 (m, $C_3$—H), 7.95 (broad s, NH), 8.64 (d, J=8Hz, NH).

(72) (a) 3-(2-Succinimidoacetamido)-2-oxoazetidine
(b) 3-Amino-2-oxoazetidine
(c) B
(d) $IR\nu_{max}^{KBr}cm^{-1}$; 3240, 1750, 1710, 1680.
NMR (DMSO-$d_6$, ppm); 2.68 (s, —$CH_2$—$CH_2$—), 3.02 (dd, J=3, 5, 6Hz, $C_4$—βH), 3.41 (t, J=6Hz, $C_4$—αH), 3.98 (s, —$CH_2$—), 4.6—5.0 (m, $C_3$—H), 7.95 (broad s, NH), 8.72 (d, J=8Hz, NH).

(73) (a) 3-[2-(2-Carbobenzoxyaminomethylphenyl)acetamido]-2-oxoazetidine
(b) 3-Amino-2-oxoazetidine
(c) B
(d) $IR\nu_{max}^{KBr}cm^{-1}$; 3280, 1760, 1700, 1665.
NMR (DMSO-$d_6$, ppm); 3.04, (dd, J=3, 6Hz, $C_4$—βH), 3.37 (t, J=6Hz, $C_4$—αH), 3.54 (s, —$CH_2$—), 4.25 (d, J=6Hz, —$CH_2$—), 4.65—5.0 (m, $C_3$—H), 5.02 (s, —$CH_2$—), 7.20 (s, aromatic H,) 7.33 (s, aromatic H), 7.98 (broad s, NH), 7.98 (d, J=6Hz, NH), 8.72 (d, J=8Hz, NH).

(74) (a) 3-(2-Methoxyimino-2-furylacetamido)-2-oxoazetidine
(b) 3-Amino-2-oxoazetidine
(c) B
(d) $IR\nu_{max}^{KBr}cm^{-1}$; 3240, 1760, 1670.

NMR (DMSO-$d_6$, ppm); 3.19 (dd, J=3.5, 6Hz, $C_4$—H), 3.28 (s, —$CH_3$), 3.48 (t, J=6Hz, $C_4$—H), 4.7—5.1 (m, $C_3$—H), 6.63 (m, 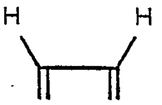), 7.78 (m, 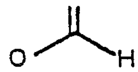), 7.98 (broad s, NH), 9.27 (d, J=8Hz, NH).

(75) (a) 3-[2-[2-(3-Trichloroacetylureidomethyl)phenyl]acetamido]-2-oxoazetidine
(b) 3-Amino-2-oxoazetidine
(c) B
(d) IR$\nu_{max}^{KBr}$cm$^{-1}$; 3300, 1760, 1725, 1700, 1670.
NMR (DMSO-$d_6$, ppm); 2.8—3.6 (m, $C_4$—H), 3.58 (s, —$CH_2$—), 4.42 (d, J=6Hz, —$CH_2$—), 4.6—5.1 (m, $C_3$—H), 7.27 (s, aromatic H), 7.97 (broad s, NH), 8.23 (d, J=6Hz, NH), 8.26 (broad, S, NH), 8.79 (d, J=8.5Hz, NH).

(76) (a) 3-[2-(3,5-Dichloro-4-pyrridon-1-yl)acetamido]-2-oxoazetidine
(b) 3-Amino-2-oxoazetidine
(c) A
(d) IR$\nu_{max}^{KBr}$cm$^{-1}$; 3260, 3200, 1760, 1670, 1630, 1230.
NMR (DMSO-$d_6$, ppm); 3.10 (dd, J=3, 5.5Hz, $C_4$—$\beta$H), 3.33 (t, J=5.5Hz, $C_4$—$\alpha$H), 4.74 (s, —$CH_2$—), 4.5—5.05 (m, $C_3$—H), 7.99 (broad s, NH), 8.17 (s, 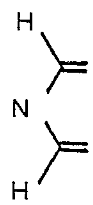), 8.93 (d, J=8.5Hz, NH).

(77) (a) 3-(2-Benzyloxycarbonyl-2-phenylacetamido)-2-oxoazetidine
(b) 3-Amino-2-oxoazetidine
(c) B
(d) IR$\nu_{max}^{KBr}$cm$^{-1}$; 3380, 3320, 1780, 1735, 1665.

(78) (a) 3-[2-(N-Carbobenzoxyprolinamido)-2-furylacetamido]-2-oxoazetidine
(b) 3-Amino-2-oxoazetidine
(c) B
(d) IR$\nu_{max}^{KBr}$cm$^{-1}$; 3270, 1765, 1700, 1665.

(79) (a) 3-[2-(1-Acetyl-2,4-dioxoimidazolidin-3-yl)acetamido]-2-oxoazetidine
(b) 3-Amino-2-oxoazetidine
(c) B
(d) IR$\nu_{max}^{KBr}$cm$^{-1}$; 3290, 1810, 1763, 1735, 1690.
NMR (DMSO-$d_6$, ppm); 2.45 (s, $CH_3$), 3.01 (dd, J=6, 3.5Hz, $C_4$—$\beta$H), 3.44 (t, J=6Hz, $C_4$—$\alpha$H), 4.07 (s, —$CH_2$—), 4.28 (s, —$CH_2$—), 4.87 (ddd, J=3.5, 6Hz, $C_3$—H), 7.99 (broad s, NH), 8.83 (d, J=8Hz, NH).

(80) (a) 3-[2-(2-Oxoimidazolidin-1-yl)acetamido]-2-oxoazetidine
(b) 3-Amino-2-oxoazetidine
(c) A
(d) IR$\nu_{max}^{KBr}$cm$^{-1}$; 3260, 1730, 1670.
NMR (DMSO-$d_6$, ppm); 3.1—4.0 (m, —$CH_2$—, $C_4$—H), 4.6—5.0 (m, $C_3$—H), 7.54 (broad s, NH), 7.88 (broad s, NH), 8.56 (d, J=8.5Hz, NH).

(81) (a) 3-[2-(4-Ethyl-2,3-dioxo-1-piperazinocarboxamido)-2-furylacetamido]-2-oxoazetidine
(b) 3-Amino-2-oxoazetidine
(c) B
(d) IR$\nu_{max}^{KBr}$cm$^{-1}$; 3290, 1770, 1725, 1700, 1680.
NMR (DMSO-$d_6$, ppm); 1.10 (t, J=7Hz, $CH_3$—), 3.1—4.1 (m, —$CH_2$—, $C_4$—H),

5.69 (d, J=6.5Hz, —CH—),

7.20 (m, 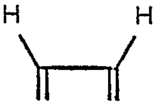 ), 7.45 (m, 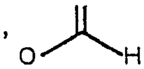 ).

(82) (a) 3[D-α-(Thienylmethylcarbonyl)alaninamido]-2-oxoazetidine
(b) 3-Amino-2-oxoazetidine
(c) E
(d) IR$\nu_{max}^{KBr}$cm$^{-1}$; 3260, 1740, 1645.
NMR (DMSO-$d_6$, ppm); 1.19 (d, J=6.5Hz, —$CH_3$), 3.01 (dd, J=3, 6Hz, $C_4$—H), 3.28 (s, —$CH_2$—), 3.37 (t, J=6Hz, $C_4$—H),

3.8—4.6 (m, —CH—),

4.65 (m, $C_3$—H), 6.87 (m, 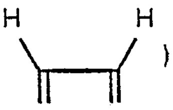 ), 7.25 (m, 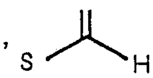 ).

7.81 (broad s, NH), 8.17 (d, J=8Hz, NH), 8.53 (d, J=6.5Hz, NH).

(83) (a) 3-(N-Carbobenzoxy-D-alaninamido)-3-methoxy-2-oxoazetidine
(b) 3-Amino-3-methoxy-2-oxoazetidine
(c) D
(d) IR$\nu_{max}^{KBr}$cm$^{-1}$; 1755, 1680, 1515
NMR (DMSO-$d_6$, ppm); 1.22 (d, J=7Hz, $CH_3$), 3.32 (s, $CH_3$), 3.40, 3.48 (each m, $C_4$—H),

4.12 (m, —CH—),

5.04 (s, $CH_2$), 7.36 (s, aromatic H), 8.26 (s, NH), 8.98 (d, J=7Hz, NH).

(84) (a) 3-[N-(4-Ethyl-2,3-dioxo-1-piperazinocarbonyl)-D-alaninamido]-3-methoxy-2-oxoazetidine
(b) 3-Amino-3-methoxy-2-oxoazetidine
(c) E
(d) IR$\nu_{max}^{KBr}$cm$^{-1}$; 1760, 1710, 1670, 1510.
NMR (DMSO-$d_6$, ppm); 1.10 (t, J=7Hz, $CH_3$), 1.34, 1.44 (d, J=7Hz, $CH_3$), 3.36 (s, $CH_3$), 3.90 (m, —$CH_2$—),

4.48 (m, —CH—),

8.31 (broad s, NH), 9.74 (d, J=7Hz, NH), 9.82 (s, NH).

(85) (a) 3-(N-Carbobenzoxy-D-phenylglycol-D-phenylglycinamido)-3-methoxy-2-oxoazetidine
(b) 3-Amino-3-methoxy-2-oxoazetidine
(c) E
(d) IR$\nu_{max}^{KBr}$cm$^{-1}$; 1765, 1680, 1640, 1510.
NMR (DMSO-$d_6$, ppm); 3.06, 3.26 (s, $CH_3$), 3.40 (m, $C_4$—H), 5.05 (s, —$CH_2$—),

5.46 (d, J=8Hz, —CH—), 5.60 (d, J=Hz, —CH—),

7.84 (broad s, NH), 8.73 (d, J=8Hz, NH), 9.40 (s, NH).

(86) (a) 3-[N-(4-Ethyl-2,3-dioxo-1-piperazinocarbonyl)-D-methioninamido]-2-oxoazetidine
(b) 3-Amino-2-oxoazetidine
(c) B
(d) IR$\nu_{max}^{KBr}$cm$^{-1}$; 1750, 1705, 1670, 1520, 1190.
NMR (DMSO-$d_6$, ppm); 1.10 (t, J=7Hz, $CH_3$), 1.96 (m, —$CH_2$—), 2.05 (s, $CH_3$), 2.44 (t, J=7Hz, —$CH_2$—), 3.08 (q, J=2, 6Hz, $C_4$—βH), 3.41 (q, J=7Hz, —$CH_2$—), 3.58 (m, —$CH_2$—), 3.90 (m, —$CH_2$—), 4.43 (q, J=7Hz, —CH—),

4.84 (m, $C_3$—H), 7.97 (broad s, NH), 8.82 (d, J=7Hz, NH), 9.43 (d, J=7Hz, NH).

(87) (a) 3-[D-2-[2,3-Dioxo-4-(2-phenethyl)-1-piperazinocarboxamido]-2-phenylacetamido]-2-oxoazetidine
(b) 3-Amino-2-oxoazetidine
(c) B
(d) $IR\nu^{KBr}_{max}cm^{-1}$; 3280, 1755, 1710, 1670, 1500, 1190.
NMR (DMSO-$d_6$, ppm); 2.84 (t, J=7Hz, —$CH_2$—), 2.95 (dd, J=3, 6Hz, $C_4$—βH), 4.88 (ddd, J=3, 6, 8Hz, $C_3$—H),

5.46 (d, J=7Hz, —CH—),

7.25—7.5 (m, aromatic H), 8.00 (s, NH), 9,.12 (d, J=8Hz, NH), 9.80 (d, J=7Hz, NH).

(88) (a) 3-[D-2-(4-Ethyl-2,3-dioxo-1-piperazinocarboxamido)-2-(4-benzoyloxyphenyl)acetamido]-2-oxoazetidine
(b) 3-Amino-2-oxoazetidine
(c) B
(d) $IR\nu^{KBr}_{max}cm^{-1}$; 3280, 1755, 1730, 1710, 1670, 1500, 1265, 1205.
NMR ($d_6$-DMSO ppm); 1.10 (t, —$CH_3$), 2.99 (dd, J=3, 6Hz, $C_4$—H), 3.41 (q, —$CH_2$—), 3.42 (t, J=6Hz, $C_4$—H), 3.4—4.1 (m, ring $CH_2$), 4.91 (ddd, J=3, 6, 8Hz, $C_3$—H),

5.53 (d, J=7Hz, —CH—),

7.2—8.2 (m, phenyl H), 8.02 (s, NH), 9.19 (d, J=8Hz, NH), 9.88 (d, J=7Hz, NH).

(89) (a) 3-(2-Benzyloxycarboxamido-3-N-methylcarbamoylpropionamido)-3-methoxy-2-oxo-azetidine
(b) 3-Amino-3-methoxy-2-oxoazetidine
(c) B
(d) $IR\nu^{KBr}_{max}cm^{-1}$; 3350, 1760, 1700, 1650.
NMR ($CDCl_3$, ppm); 2.73 (m, $CH_3$, —$CH_2$—), 3.43 (s, $CH_3$), 3.66 (m, $C_4$—H),

4.63 (m, —CH—),

5.12 (s, —$CH_2$—), 6.70 (d, J=7Hz, NH), 6.77 (m, NH), 7.12 (m, NH), 8.53 (s, NH).

(90) (a) 3-[2-(2-Chloroacetamido-4-thiazolyl)-2-[(3-furfurylideneamino-2-oxoimidazolidin-1-yl)-carboxamido]acetamido]-2-oxoazetidine
(b) 3-Amino-2-oxoazetidine
(c) B
(d) $IR\nu^{KBr}_{max}cm^{-1}$; 3310, 1750, 1725, 1660.

(91) (a) 3-[D-2-(2-Phenylacetamido)propionamido-3-methoxy-2-oxoazetidine
(b) 3-Amino-3-methoxy-2-oxoazetidine
(c) E
(d) $IR\nu^{KBr}_{max}cm^{-1}$; 1758, 1645, 1520.
NMR (DMSO-$d_6$, ppm); 1.23, 1.24 (each d, J=7Hz, $CH_3$), 2.79, 2.95 (each s, —$CH_2$—), 3.31, 3.47 (each s, $CH_3$),

4.46 (m, —CH—),

7.27 (s, aromatic H), 8.1—8.35 (m, NH), 8.98 (d, J=6Hz, NH).

(92) (a) 3-[2-[[2-Oxo-3-(thiophen-2-aldoimino)imidazolidin-1-yl]-carboxamido]-2-thienyl-acetamido]-2-oxoazetidine
(b) 3-Amino-2-oxoazetidine
(c) B

(d) $IR\nu_{max}^{KBr}cm^{-1}$; 3300, 1750, 1715, 1660.

(93) (a) 3-[D-2-[(3-Mesyl-2-oxoimidazolidin-1-yl)-carboxamido]-2-phenylacetamido]-2-oxoazetidine
(b) 3-Amino-2-oxoazetidine
(c) B
(d) $IR\nu_{max}^{KBr}cm^{-1}$; 3320, 1750, 1730, 1665, 1165.

(94) (a) 3-[2-[(3-Mesyl-2-oxoimidazolidin-1-yl)-carboxamido]-2-thienylacetamido]-2-oxoazetidine
(b) 3-Amino-2-oxoazetidine
(c) B
(d) $IR\nu_{max}^{KBr}cm^{-1}$; 3310, 1750, 1730, 1665, 1520.

(95) (a) 3-[D-2-(2,6-Dichlorophenylthioglycolamido)-2-phenylacetamido]-2-oxoazetidine
(b) 3-Amino-2-oxoazetidine
(c) B
(d) $IR\nu_{max}^{KBr}cm^{-1}$; 1753, 1657, 1639.
NMR ($DMSO-d_6$, ppm); 2.97 (dd, J=3, 6Hz, $C_4$—H), 3.36 (t, J=6Hz, $C_4$—H), 3.93 (s, —$CH_2$—), 4.86 (ddd, J=2, 6, 8Hz, $C_3$—H),

5.47 (d, J=7Hz, —CH—),
|

7.25, 8.0 (m, aromatic H), 8.98 (d, J=7Hz, NH), 9.09 (d, J=8Hz, NH).

(96) (a) 3-[D-2-(4-Ethyl-2,3-dioxo-1-piperazinocarboxamido)-3-phenylpropionamido]-3-methoxy-2-oxoazetidine
(b) 3-Amino-3-methoxy-2-oxoazetidine
(c) A
(d) $IR\nu_{max}^{KBr}cm^{-1}$; 1760, 1700, 1660, 1518.
NMR ($DMSO-d_6$, ppm); 1.09 (t, J=7Hz, $CH_3$—),

4.72 (m, —CH—),
|

5.32 (s, NH), 9.15 (d, J–7Hz, NH), 9.43 (s, NH).

(97) (a) 3-(2-Dichloroacetoxyimino-2-thienylacetamido)-2-oxoazetidine
(b) 3-Amino-2-oxoazetidine
(c) A
(d) $IR\nu_{max}^{KBr}cm^{-1}$; 1745, 1720, 1670, 1650.
NMR ($DMSO-d_6$, ppm); 3.18 (dd, J=2, 6Hz, $C_4$—$\beta$H), 3.45 (t, J=6Hz, $C_4$—$\alpha$H), 4.92 (ddd, J=2, 6, 8Hz, $C_3$—H), 6.44 (s, —CH<), 7.06, 7.71 (m, thienyl H), 7.91 (s, NH), 9.14 (d, J=8Hz, NH).

(98) (a) 3-(2-Phenyl-2-sulphamoylacetamido)-2-oxoazetidine
(b) 3-Amino-2-oxoazetidine
(c) B
(d) $IR\nu_{max}^{KBr}cm^{-1}$; 3320, 1730, 1662, 1540.
NMR ($DMSO-d_6$, ppm); 3.20 (dd, J=3, 6Hz, $C_4$—H), 3.50 (t, J=6Hz, $C_4$—H), 4.87, 4.98 (each dd, J=3, 6Hz, $C_3$—H),

5.17 (s, —CH—),
|

7.3—7.8 (m, aromatic H).

(99) (a) 3-[2-(N,N-Dimethylsulphamoyl)-2-phenyl-acetamido]-2-oxoazetidine
(b) 3-Amino-2-oxoazetidine
(c) B
(d) $IR\nu_{max}^{KBr}cm^{-1}$; 3320, 1723, 1672, 1636, 1140.
NMR ($DMSO-d_6$, ppm); 2.63, 2.67 (s, $CH_3$), 3.02 (m, $C_4$—H), 3.40, 3.46 (t, J=6Hz, $C_4$—H), 4.82 (m, $C_3$—H),

5.28 (s, —CH—),

8.01 (s, NH), 9.06 (d, J=8Hz, NH).

(100) (a) 3-[2,5-Bis(4-ethyl-2,3-dioxo-1-piperazinocarboxamido)pentanamido]-2-oxoazetidine
(b) 3-Amino-2-oxoazetidine
(c) B
(d) $IR\nu^{KBr}_{max}cm^{-1}$; 3300, 2930, 1760, 1720, 1670, 1520, 1188.
NMR (DMSO-$d_6$, ppm); 1.09 (t, J=7Hz, —$CH_3$),

4.37 (m, —CH—),

4.80 (m, $C_3$—H), 7.93 (s, NH), 8.78 (d, J=7Hz, NH), 8.82 (t, J=6Hz, NH), 9.22 (d, J=8Hz, NH).

(101) (a) 3-[2,5-Bis(4-ethyl-2,3-dioxo-1-piperazinocarboxamido)pentanamido]-3-methoxy-2-oxo-azetidine
(b) 3-Amino-3-methoxy-2-oxoazetidine
(c) B
(d) $IR\nu^{KBr}_{max}cm^{-1}$; 3300, 1768, 1712, 1679, 1520, 1190.
NMR (DMSO-$d_6$, ppm); 1.09 (t, J=7Hz, $CH_3$), 3.33 (s, $CH_3$),

4.51 (m,—CH—),

8.29 (s, NH), 8.83 (t, J=6Hz, NH), 9.20 (d, J=7Hz, NH), 9.37 (s, NH).

(102) (a) 3-[D-2-[4-(2-Chloroethyl)-2,3-dioxo-1-piperazinocarboxamido]-2-phenylacetamido]-2-oxoazetidine
(b) 3-Amino-2-oxoazetidine
(c) B
(d) $IR\nu^{KBr}_{max}cm^{-1}$; 3250, 1760, 1705, 1670.

(103) (a) 3-[D-3-Chloro-2-(4-ethyl-2,3-dioxo-1-piperazinocarboxamido)propionamido]-2-oxoazetidine
(b) 3-Amino-2-oxoazetidine
(c) B
(d) $IR\nu^{KBr}_{max}cm^{-1}$; 1750, 1710, 1670, 1510, 1185.
NMR (DMSO-$d_6$, ppm); 1.10 (t, J=7Hz, $CH_3$), 3.05 (dd, J=2, 6Hz, $C_4$—$\beta$H), 3.42 (q, J=7Hz, —$CH_2$—), 3.56 (m, —$CH_2$—), 3.92 (m, —$CH_2$—),

4.72 (m, —CH—),

4.90 (m, $C_3$—H), 8.00 (broad s, NH), 8.92 (d, J=7Hz, NH), 9.48 (d, J=7Hz, NH).

(104) (a) 3-[2-Benzyloxycarboxamido-2-benzyloxycarbonylethane-sulphonamido]-2-oxoazetidine
(b) 3-Amino-2-oxoazetidine
(c) A
(d) $IR\nu^{KBr}_{max}cm^{-1}$; 1745, 1720, 1700, 1520, 1340, 1270, 1200, 1145.
NMR (DMSO-$d_6$, ppm); 3.03 (dd, J=2, 6Hz, $C_4$—$\beta$H), 3.40 (d, J=6Hz, $C_4$—$\alpha$H), 3.58 (dd, J=6, 14Hz, —$CH_2$—),

4.60 (m, —CH—, $C_3$—H),

5.07 (s, —$CH_2$—), 5.16 (s, —$CH_2$—), 7.36, 7.38 (each s, aromatic H), 7.88 (d, J=7Hz, NH), 8.02 (broad s, NH), 8.34 (d, J=7Hz, NH).

(105) (a) 3-[2-(4-Ethyl-2,3-dioxo-1-piperazinocarboxamido)-3-[(1-methyl-5H-tetrazol-5-yl)thio]-propionamido]-2-oxoazetidine
(b) 3-Amino-2-oxoazetidine
(c) B

(d) $IR\nu_{max}^{KBr}cm^{-1}$; 1750, 1710, 1675, 1510, 1190
NMR (DMSO-$d_6$, ppm); 1.10 (t, J=7Hz, $CH_3$), 3.04 (dd, J=2, 6Hz, $C_4$—βH), 3.41 (q, J=7Hz, —$CH_2$—), 3.92 (s, $CH_3$),

4.74 (m, —CH—),
|

4.82 (m, $C_3$—H), 8.01 (broad s, NH), 8.97 (d, J=7Hz, NH), 9.38 (d, J=7Hz, NH).

(106) (a) 3-[D-2-(2-Benzyloxycarboxamido-2-benzyloxycarbonylethanesulphonamido)-2-phenylacetamido]-2-oxoazetidine
(b) 3-Amino-2-oxoazetidine
(c) E
(d) $IR\nu_{max}^{KBr}cm^{-1}$; 1750, 1710, 1680, 1520.
NMR (DMSO-$d_6$, ppm); 2.96 (dd, J=2, 6Hz, $C_4$—βH),

4.40 (m, —CH—),
|

4.78 (m, $C_3$—H), 5.04 (s, —$CH_2$—), 5.11 (s, —$CH_2$—), 7.2—7.6 (m, aromatic H), 7.80 (d, J=7Hz, NH), 7.96 (broad s, NH), 8.20 (d, J=7Hz, NH), 8.97 (d, J=7Hz, NH).

(107) (a) 3-[D-2-(2-Benzyloxycarboxamido-3-sulphamoylpropionamido)-2-phenylacetamido]-2-oxoazetidine
(b) 3-Amino-2-oxoazetidine
(c) B
(d) $IR\nu_{max}^{KBr}cm^{-1}$; 1760, 1715, 1670, 1530.

(108) (a) 3-[D-2-[2-Benzyloxycarboxamido-3-(4-methoxyphenyloxycarboxamido)propionamido]-2-phenylacetamido]-2-oxoazetidine
(b) 3-Amino-2-oxoazetidine
(c) B
(d) $IR\nu_{max}^{KBr}cm^{-1}$; 3300, 1762, 1675, 1640.

(109) (a) 3-[D-2-[3-Benzyloxycarboxamido-2-(4-ethyl-2,3-dioxo-1-piperazinocarboxamido)propionamido]-2-phenylacetamido]-2-oxoazetidine
(b) 3-Amino-2-oxoazetidine
(c) B
(d) $IR\nu_{max}^{KBr}cm^{-1}$; 3295, 1758, 1705, 1670, 1640.

(110) (a) 3-[2-[2-Benzyloxycarboxamido-3-(N-methylcarbamoyl)propionamido]acetamido-3-methoxy-2-oxoazetidine
(b) 3-Amino-3-methoxy-2-oxoazetidine
(c) E
(d) $IR\nu_{max}^{KBr}cm^{-1}$; 3390—3270, 1762, 1695—1650.
NMR (DMSO-$d_6$, ppm); 2.55 (d, J=5Hz, $CH_3$), 2.40—2.60 (m, —$CH_2$—), 3.30 (s, $CH_3$), 3.40 (ABq, J=6, 10Hz, $C_4$—H), 3.75 (d, J=6Hz, $CH_2$),

4.33 (m, —CH—),
|

5.01 (s, $CH_2$), 7.33 (s, aromatic H), 7.70 (m, NH), 8.04 (d, J=5Hz, NH), 8.25 (s, NH), 8.88 (s, NH), 9.10 (m, NH).

(111) (a) 3-[2-(4-Ethyl-2,3-dioxo-1-piperazinocarboxamido)acetamido]-3-methoxy-2-oxoacetidine
(b) 3-Amino-3-methoxy-2-oxoazetidine
(c) E
(d) $IR\nu_{max}^{KBr}cm^{-1}$; 3275, 1760, 1708, 1670.
NMR (DMSO-$d_6$, ppm); 1.18 (t, J=7Hz, $CH_3$), 3.40 (s, $CH_3$), 3.47 (q, J=7Hz, —$CH_2$—), 3.57—3.80 (m, —$CH_2$—), 3.65 (ABq, J=5, 11Hz, $C_4$—H), 3.93—4.20 (m, —$CH_2$—), 4.07 (d, J=6Hz, —$CH_2$—), 7.58 (s, NH), 8.70 (s, NH), 9.23 (t, J=6Hz, NH).

(112) (a) 3-[D-2-[2-(4-Ethyl-2,3-dioxo-1-piperazinocarboxamido)-3-(N-methylcarbamoyl)propionamido]-2-phenylacetamido]-2-oxoazetidine
(b) 3-Amino-2-oxoazetidine

(c) B
(d) $IR\nu_{max}^{KBr}cm^{-1}$; 3300, 1748, 1708, 1662.

(113) (a) 3-[2-(4-Ethyl-2,3-dioxo-1-piperazinocarboxamido-3-(N-methylcarbamoyl)propionamido]-2-oxoazetidine
(b) 3-Amino-2-oxoazetidine
(c) B
(d) $IR\nu_{max}^{KBr}cm^{-1}$; 3300, 1750, 1712, 1672.

(114) (a) 3-[2-(D-2-Benzyloxycarboxamido-2-phenylacetamido)-3-(N-methylcarbamoyl)propionamido]-2-oxoazetidine
(b) 3-Amino-2-oxoazetidine
(c) B
(d) $IR\nu_{max}^{KBr}cm^{-1}$; 3300, 1752, 1690, 1645.

(115) (a) 3-[D-2-(3-Furfurylideneamino-2-oxoimidazolidin-1-yl)carboxamido]-2-phenylacetamido]-3-(S)-methoxy-2-oxoazetidine
(b) 3-Amino-3-methoxy-2-oxoazetidine
(c) A
(d) $IR\nu_{max}^{KBr}cm^{-1}$; 3280, 1760, 1670, 1475, 1410, 1230.
NMR (DMSO-$d_6$, ppm); 3.08 (s, $CH_3$), 3,42, 3.56 (d, J=6Hz, $C_4$—H), 3.79 (s, —$CH_2$—),

5.62 (d, J=7Hz, —CH—),

6.5—7.9 (m, aromatic H), 7.73 (s, —CH=N—), 8.35 (s, NH), 9.04 (d, J=7Hz, NH), 9.59 (s, NH).

(116) (a) 3-[D-2-[(3-Furfurylideneamino-2-oxo-imidazolidin-1-yl)carboxamido]-2-phenylacetamido]-3(R)-methoxy-2-oxoazetidine
(b) 3-Amino-3-methoxy-2-oxoazetidine
(c) A
(d) $IR\nu_{max}^{KBr}cm^{-1}$; 3280, 1760, 1720, 1670, 1475, 1410, 1230.
NMR (DMSO-$d_6$, ppm); 3.26, 3.42 (d, J=6Hz, $C_4$—H), 3.34 (s, $CH_3$), 3.78 (s, —$CH_2$—),

5.61 (d, J=7Hz, —CH—),

6.5—7.9 (m, aromatic H), 7.73 (s, —CH=N—), 8.23 (s, NH), 8.98 (d, J=7Hz, NH), 9.54 (s, NH).

(117) (a) 3-[D-2-(4-Ethyl-2,3-dioxo-1-piperazinocarboxamido)-2-(4-octanoyloxyphenyl)acetamido]-2-oxoazetidine
(b) 3-Amino-2-oxoazetidine
(c) B
(d) $IR\nu_{max}^{KBr}cm^{-1}$; 3280, 2925, 2850, 1750, 1710, 1670, 1500, 1190.

(118) (a) 3-[D-3-(N-Ethoxycarbonylmethyl)carbamoyl-2-(4-ethyl-2,3-dioxo-1-piperazinocarboxamido)propionamido]-2-oxoazetidine
(b) 3-Amino-2-oxoazetidine
(c) B
(d) $IR\nu_{max}^{KBr}cm^{-1}$; 3300, 1740, 1705, 1668.

(119) (a) 3-[D-2-(4-Ethyl-2,3-dioxo-1-piperazinocarboxamido)-3-(2-thienylacetamido)propionamido]-2-oxoazetidine
(b) 3-Amino-2-oxoazetidine
(c) B
(d) $IR\nu_{max}^{KBr}cm^{-1}$; 3300, 1750, 1710, 1670.

(120) (a) 3-(N-mesly-D-phenylglycinamido)-2-oxoazetidine
(b) 3-Amino-2-oxoazetidine
(c) B
(d) $IR\nu_{max}^{KBr}cm^{-1}$; 1750, 1705, 1670, 1520.

(121) (a) 3-[D-2-[2-(4-Ethyl-2,3-dioxo-1-piperazinocarboxamido)acetamido]-2-phenylacetamido]-2-oxoazetidine
(b) 3-Amino-2-oxoazetidine
(c) B
(d) $IR\nu_{max}^{KBr}cm^{-1}$; 3275, 1760, 1712, 1673, 1665, 1650.
NMR (DMSO-$d_6$, ppm); 1.10 (t, J=7Hz, $CH_3$), 3.00 (dd, J=3, 5Hz, $C_4$—H), 3.33—3.77 (m, —$CH_2$—), 3.77—4.27 (m, —$CH_2$—), 4.05 (d, J=6Hz, —$CH_2$—), 4.90 (m, $C_3$—H),

5.53 (d, J=9Hz, —CH—),

7.37 (s, aromatic H), 7.97 (s, NH), 8.88 (d, J=9Hz, NH) 9.13 (d, J=9Hz, NH), 9.32 (t, J=6Hz, NH).

## Example 1

1.23 g of 3-phenylacetamido-2-oxoazetidine is dissolved in 15 ml of N,N-dimethylformamide (DMF), followed by addition of 1.15 g of pyridine-sulphur trioxide complex. The mixture is stirred for 6 hours. After 50 ml of diethyl ether is added, the powdery precipitate is collected by filtration and washed with ether and, then, with ethanol. By the above procedure 1.47 g of pyridinium 3-phenylacetamido-2-oxoazetidine-1-sulphonate is obtained.

$IR\nu_{max}^{KBr}cm^{-1}$; 3300, 1775, 1665, 1300—1190, 1045.
NMR($d_6$-DMSO, ppm); 3.25 (dd, J=3, 6Hz, $C_4$—H), 3.46 (s, —$CH_2$), 3.62 (t, j=6Hz, $C_4$—H), 4.84 (ddd, J=3, 6, 8Hz, $C_3$—H), 7.29 (s, aromatic H), 7.9—9.0 (m, aromatic H), 8.83 (d, J=8Hz, NH).

## Example 2

0.21 g of 3-thienylacetamido-2-oxoazetidine is dissolved in 2 ml of DMF, followed by addition of 0.318 g of pyridine-sulphur trioxide complex. The mixture is stirred for one day. To the reaction mixture is added 20 ml of diethyl ether and the oily precipitate is purified by Amberlite XAD—II [Rohm and Haas Co., (U.S.A.)] chromatography. 0.175 g of 3-(thienylacetamido)-2-oxoazetidine-1-sulphonic acid is obtained. (Amberlite is a Registered Trade Mark).
$IR\nu_{max}^{KBr}cm^{-1}$; 1760, 1660, 1250, 1050.
NMR($d_6$-DMSO, ppm); 3.32 (dd, J=3, 6Hz, $C_4$—H), 3.61 (t, J=6Hz, $C_4$—H), 3.69 (s, —$CH_2$—), 4.84 (ddd, J=3, 6, 8Hz, $C_3$—H), 6.8—7.4 (m, aromatic H), 8.88 (d, J=8Hz, NH).

## Example 3

0.631 g of 3-[2-(2-chloroacetamido-4-thiazolyl)-2-methoxyiminoacetamido]-2-oxoazetidine (*syn*-isomer) is dissolved in 10 ml of DMF, followed by addition of 0.637 g of pyridine-sulphur trioxide complex. The mixture is stirred for 3 days. To this reaction mixture is added 30 ml of diethyl ether, and the oily precipitate is passed through Dowex 50W resin (Na-form) [Dow Chemical (U.S.A.)]. The eluate is freeze-dried to obtain 0.89 g of crude sodium 3 - [2 - (2 - chloroacetamido - 4 - thiazolyl) - 2 - methoxyiminoacetamido] - 2 - oxoacetidine - 1 - sulphonate (*syn*-isomer).

$IR\nu_{max}^{KBr}cm^{-1}$; 3430, 1760, 1690, 1650, 1140.
NMR($D_2O$, ppm); 3.95 (dd, J=3, 6Hz, $C_4$—H), 4.12 (s, —$CH_3$), 4.15 (t, J=6Hz, $C_4$—H), 4.93 (s, —$CH_2Cl$), 5.22 (dd, J=3, 6Hz, $C_3$—H),

7.52 (s, 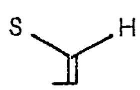 ),

The crude sodium 3 - [2 - (2 - chloroacetamido - 4 - thiazolyl) - 2 - methoxyiminoacetamido] - 2 - oxoazetidine - 1 - sulphonate (*syn*-isomer) obtained above (0.556 g) is dissolved in 8 ml of water, and to the solution is added 0.172 g of sodium N-methyl-dithiocarbamate under ice-cooling and stirring. The mixture is stirred for 3 hours, after which any insoluble matter is filtered off. The filtrate is purified by XAD—II chromatography to yield 0.174 g of sodium 3 - [2 - (2 - amino - 4 - thiazolyl) - 2 - methoxyiminoacetamido] - 2 - oxoazetidine - 1 - sulphonate (*syn*-isomer).

$IR\nu_{max}^{KBr}cm^{-1}$; 3450, 1770, 1670, 1610, 1260, 1050.
NMR($D_2O$, ppm); 3.89 (dd, J=4, 6Hz, $C_4$—H), 4.03 (s, —$CH_3$), 4.09 (t, J=6Hz, $C_4$—H), 5.16 (dd, J=4, 6Hz, $C_3$—H),

7.01 (s, 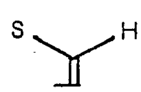 ).

Example 4

0.515 g of 3-[2-(2-chloroacetamido-4-thiazolyl) acetamido-2-oxoazetidine is suspended in 20 ml of DMF, followed by addition of 0.325 g of pyridine-sulphur trioxide complex. The mixture is worked up as described in Example 3 to obtain 0.569 g of sodium 3 - [2 - (2 - chloroacetamido - 4 - thiazolyl)-acetamido] - 2 - oxoazetidine - 1 - sulphonate.

IR$\nu_{max}^{KBr}$cm$^{-1}$; 3430, 1760, 1660, 1550, 1260, 1150, 1050.

NMR($d_6$-DMSO, ppm); 3.30 (dd, J=3, 6Hz, $C_4$—H), 3.52 (s, —$CH_2$—), 3.60 (t, J=6Hz, $C_4$—H, 4.34 (s, —$CH_2$Cl), 4.85 (ddd, J=3, 6, 8Hz, $C_3$—H),

6.97 ( s, 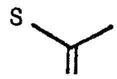 ),

8.74 (d, J=8Hz, NH).

0.486 g of sodium 3-[2-(2-chloroacetamido-4-thiazolyl)acetamido-2-oxoazetidine-1-sulphonate is dissolved in 6 ml of water, and to the solution is added 0.154 g of sodium N-methyldithiocarbamate under ice-cooling and stirring. The mixture is treated as described in Example 3 to obtain 0.144 g of sodium 3 - [2 - (2 - amino - 4 - thiazolyl)acetamido] - 2 - oxoazetidine - 1 - sulphonate.

IR$\nu_{max}^{KBr}$cm$^{-1}$; 3400, 3270, 1760, 1650, 1610, 1270, 1235, 1200, 1050.

NMR($d_6$-DMSO, ppm); 3.25 (dd, J=3, 6Hz, $C_4$—H), 3.30 (s, —$CH_2$—), 3.62 (t, J=6Hz, $C_4$—H), 4.85 (ddd, J=3, 6, 8Hz, $C_3$—H),

6.25 ( s, 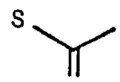 ),

6.82 (s, $NH_2$), 8.66 (d, J=8Hz, NH).

Example 5

0.46 g of 3-($\alpha$-sulphophenyl-acetamido)-2-oxoazetidine sodium salt is dissolved in 5 ml of DMF, followed by addition of 0.478 g of pyridine-sulphur trioxide complex. The mixture is stirred for 3 days. To the reaction mixture 30 ml of diethyl ether are added and the oily precipitate is passed through Dowex 50 W resin (Na-form). The eluate is purified by Amberlite XAD—II chromatography. By the above procedure 0.61 g of disodium 3-($\alpha$-sulphophenylacetamido)-2-oxoazetidine-1-sulphonate is obtained.

IR$\nu_{max}^{KBr}$cm$^{-1}$; 3450, 1760, 1660, 1630, 1200—1100, 1045.

NMR($d_6$-DMSO, ppm); 3.17 (dd, J=3, 6Hz, $C_4$—H), 3.58 (t, J=6Hz, $C_4$—H),

4.56 (s, —CH—),

4.76 (m, $C_3$—H), 7.2—7.6 (m, aromatic H), 8.55 (d, J=8Hz, NH).

Example 6

0.285 g of 3-[2-(4-ethyl-2,3-dioxo-1-piperazino-carboxamido)-2-phenylacetamido]-2-oxoazetidine is dissolved in 3 ml of DMF, followed by addition of 0.234 g of pyridine-sulphur trioxide complex. The mixture is stirred for 4 days, after which it is worked up as described in Example 5. The above procedure yields 0.219 g of sodium 3 - [2 - (4 - ethyl - 2,3 - dioxo - 1 - piperazinocarboxyamido) - 2-phenylacetamido] - 2 - oxoazetidine - 1 - sulphonate.

IR$\nu_{max}^{KBr}$cm$^{-1}$; 3470, 3280, 1760, 1705, 1670, 1510, 1250, 1190, 1050.

NMR($d_6$-DMSO, ppm); 1.10 (t, J=7Hz, —$CH_3$), 3.13 (dd, J=3, 6Hz, $C_4$—H), 3.41 (q, J=7Hz, —$CH_2$—), 3.45—3.65 (m, —$CH_2$—), 3.59 (t, J=6Hz, $C_4$—H), 3.80—4.00 (m, —$CH_2$), 4.85 (ddd, J=3, 6, 8Hz, $C_3$—H),

5.45 (d, J=8Hz, —CH—),

7.2—7.5 (m, aromatic H), 9.20 (d, J=8Hz, NH), 9.81 (d, J=8Hz, NH).

Example 7

0.177 g of 3-(2-benzoyloxycarboxamido-2-phenylacetamido)-2-oxoazetidine is dissolved in 1 ml of DMF, followed by addition of 0.16 g of pyridine-sulphur trioxide complex. The mixture is stirred for 4

days. To this reaction mixture 10 ml of diethyl ether are added and the oily precipitate is treated with ethanol to obtain 0.092 g of pyridinium 3-(2-benzyloxycarboxamido-2-phenylacetamido)-2-oxo-azetidine-1-sulphonate.

IR$\nu_{max}^{KBr}$cm$^{-1}$; 3280, 1765, 1685, 1660, 1300—2100, 1040.

NMR($d_6$-DMSO, ppm); 3.16 (dd, J=4, 6Hz), $C_4$—H), 3.57 (t, J=6Hz, $C_4$—H), 4.83 (ddd, J=4, 6, 8Hz, $C_3$—H), 5.07 (s, —$CH_2$—),

5.26 (d, J=8Hz, —CH—),

7.36 (s, aromatic H), 7.85 (broad d, NH), 7.9—90 (m, aromatic H).

Example 8

0.25 g of 3-benzyloxycarboxamido-3-methoxy-2-oxoazetidine is dissolved in 2 ml of DMF, followed by addition of 0.318 g of pyridine-sulphur trioxide complex. The mixture is stirred for 5 days. By working up as described in Example 3, 0.35 g of sodium 3-benzyloxycarboxamido-3-methoxy-2-oxoazetidine-1-sulphonate is obtained.

IR$\nu_{max}^{KBr}$cm$^{-1}$; 3450, 1760, 1715, 1250, 1140, 1050.

Example 9

0.825 g of 3-[2-(2-chloroacetamido-4-thiazolyl)-2-methoxyiminoacetamido]-3-methoxy-2-oxoazetidine (*syn*-isomer) is dissolved in 7 ml of DMF, followed by addition of 0.796 g of pyridine-sulphur trioxide complex. The mixture is reacted for 3 days. By working up as described in Example 5, 0.568 g of sodium 3 - [2 - (2 - chloroacetamido - 4 - thiazolyl) - 2 - methoxyiminoacetamido] - 3 - methoxy - 2 - oxoazetidine - 1 - sulphonate (*syn*-isomer) is obtained.

IR$\nu_{max}^{KBr}$cm$^{-1}$; 3470, 3250, 1770, 1670, 1540, 1280, 1230, 1170, 1050.

NMR($d_6$-DMSO, ppm); 3.43 (s, —$CH_3$), 3.61, 3.84 (ABq, J=6Hz, $C_4$—H), 3.92 (s, —$CH_3$), 4.39 (s, —$CH_2$—),

7.44 ( s, 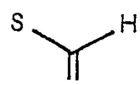 ),

9.89 (s, NH), 12.7 (broad, NH).

0.478 g of the above sodium 3 - [2 - (2 - chloroacetamido - 4 - thiazolyl) - 2 - methoxy-iminoacetamido] - 3 - methoxy - 2 - oxoazetidine -1 - sulphonate (*syn*-isomer) is dissolved in 7 ml of water, followed by addition of 0.13 g of sodium N-methyl dithiocarbamate. The mixture is stirred for one hour. The insoluble matter is filtered off and the filtrate is purified by Amberlite XAD—II chromatography, whereupon 0.267 g of sodium 3 - [2 - (2 - amino - 4 - thiazolyl) -2 - methoxyimino-acetamido] - 3 - methoxy - 2 - oxoazetidine - 1 - sulphonate (*syn*-isomer) is obtained.

IR$\nu_{max}^{KBr}$cm$^{-1}$; 3425, 3325, 1765, 1670, 1615, 1520, 1290—20, 1170, 1045.

NMR($d_6$-DMSO, ppm); 3.41 (s, —$CH_3$), 3.59, 3.79 (ABq, J=6Hz, $C_4$—H), 3.87 (s, —$CH_3$),

6.72 ( s, 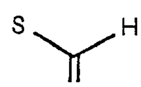 ),

7.12 (broad s, NH), 9.79 (s, NH).

Example 10

0.15 g of 3-cyanoacetamido-2-oxoazetidine is dissolved in 0.5 ml of DMF, followed by addition of 0.30 g of pyridine-sulphur trioxide complex. The mixture is stirred for 3 days, and the reaction mixture is treated as described in Example 5, whereupon 0.183 g of sodium 3-cyanoacetamido-2-oxoazetidine-1-sulphonate is obtained.

IR$\nu_{max}^{KBr}$cm$^{-1}$; 2270, 1760, 1660, 1250.

NMR($D_2O$, ppm); 3.91 (dd, J=2, 6Hz, $C_4$—H), 3.96 (s, —$CH_2$—), 4.05 (t, J=6Hz, $C_4$—H), 5.08 (dd, J=2, 6Hz, $C_3$—H).

Example 11

0.16 g of 3-[2-(1H-tetrazol-1-yl)acetamido]-2-oxoazetidine is dissolved in 1 ml of DMF, followed by addition of 0.26 g of pyridine-sulphur trioxide complex. The mixture is stirred at room temperature for 3 days. By working up as described in Example 5, 0.154 g of sodium 3-[2-(1H-tetrazol-1-yl)-acetamido]-2-oxoazetidine-1-sulphonate is obtained.

IR$\nu_{max}^{KBr}$cm$^{-1}$; 1762, 1665, 1250.

NMR($d_6$-DMSO, ppm); 3.21 (dd, J=2, 6Hz, $C_4$—H), 3.61 (t, J=6Hz, $C_4$—H), 4.85 (ddd, J=2, 6, 8Hz, $C_3$—H), 5.26 (s, —$CH_2$—), 9.22 (d, J=8Hz, NH),

9.30 (s, 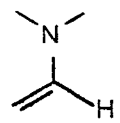 ).

Example 12

0.214 g of 3-[3-(2,6-dichlorophenyl)-5-methylisoxazol-4-yl]carboxamido-2-oxoazetidine is dissolved in 1 ml of DMF, followed by addition of 0.20 g of pyridine-sulphur trioxide complex. The mixture is stirred for 3 days. By working up as described in Example 5, 0.212 g of sodium 3-[3-(2,6-dichlorophenyl)-5-methylisoxazol-4-yl]carboxamido-2-oxoazetidine-1-sulphonate is obtained.

IR$\nu_{max}^{KBr}$cm$^{-1}$; 1762, 1665, 1250.

NMR($d_6$-DMSO, ppm); 2.77 (s, —$CH_3$), 3.25 (dd, J=2, 6Hz, $C_4$—H), 3.57 (t, J=6Hz, $C_4$—H), 4.86 (ddd, J=2, 6, 8Hz, $C_3$—H), 7.53 (s, aromatic H), 8.74 (d, J=8Hz, NH).

Example 13

A solution of 0.44 g of 3-benzyloxycarboxamido-2-oxoazetidine and 0.32 g of pyridine-sulphur trioxide complex in 2 ml of DMF is allowed to stand at room temperature for 2 days. By working up as described in Example 1, 0.613 g of pyridine 3-benzyloxycarboxamido-2-oxoazetidine-1-sulphonate is obtained as light-yellow needles.

m.p. 134—138°C (decomp.)

Elemental analysis, $C_{16}H_{17}N_3O_6S$

| | | | |
|---|---|---|---|
| Calcd. | C, 50.65; | H, 4.52; | N, 11.08 |
| Found: | C, 50.57; | H, 4.51; | N, 11.04 |

IR$\nu_{max}^{KBr}$cm$^{-1}$; 3320, 1760, 1695, 1530, 1270, 1240, 1055.

NMR($d_6$-DMSO, ppm); 3.30 (dd, J=2, 6Hz, $C_4$—H), 3.62 (dd, J=6Hz, $C_4$—H), 4.64 (ddd, J=8, 6, 2Hz, $C_3$—H), 5.06 (s, —$CH_2$), 6.60—7.40 (broad s, NH), 8.0 (d, J=8Hz, NH),

8.10 (dd, J = 6Hz, 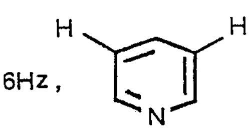 ), 8.62 (dd, J = 6Hz, 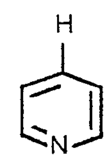 ),

8.94 (dd, J = 6Hz, 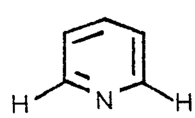 ).

Example 14

A mixture of 2.185 g of 3-(N-carbobenzoxy-D-alaninamido)-2-oxoazetidine, 2.39 g of pyridine-sulphur trioxide complex and 10 ml of dry DMF is stirred at room temperature for 4 days. Diethyl ether is added to this reaction mixture, whereby 2.7 g of pyridinium 3-(N-carbobenzoxy-D-alaninamido)-2-oxoazetidine-1-sulphonate is obtained as colourless needles.

m.p. 120—125°C (decomp.)

IR$\nu_{max}^{KBr}$cm$^{-1}$; 1762, 1678, 1660, 1525, 1250, 1040.

NMR($d_6$-DMSO, ppm); 1.22 (d, J=7Hz, $CH_3$), 3.27 (dd, J=6, 2Hz, $C_4$—H), 3.63 (t, J=6Hz, $C_4$—H),

4.03 (q, J=7Hz, —CH—),

4.90 (ddd, J=8, 6, 2Hz, $C_3$—H), 5.03 (s, —$CH_2$—), 7.33 (s, aromatic H), 8.0—9.1 (m, aromatic H).

The above pyridium 3-(N-carbobenzoxy-D-alaninamido)-2-oxoazetidine-1-sulphonate (0.15 g) is treated with Dowex 50 W (Na-form) to obtain the sodium salt. To the solution 20 mg of acetic acid and 50 mg of palladium black are added, and the mixture is stirred in a hydrogen gas stream for 20 minutes. The catalyst is filtered off and the filtrate is freeze-dried to obtain 84 mg of sodium 3-D-alaninamido-2-oxoazetidine-1-sulphonate acetate.

IR$\nu_{max}^{KBr}$cm$^{-1}$; 1760, 1670, 1550, 1260, 1240, 1045.

NMR($D_2O$, ppm); 1.62 (d, J=7Hz, —$CH_3$), 1.98 (s, —$CH_3$), 3.80 (dd, J=2, 6Hz, $C_4$—H), 3.99 (t, J=6Hz, $C_4$—H),

4.20 (q, J=7Hz, —CH—),

5.00 (dd, J=2, 6Hz, $C_3$—H).

Example 15

A mixture of 0.30 g of 3-($\alpha$-benzyl N-carbobenzoxy-$\gamma$-D-glutamyl-D-alaninamido)-2-oxozetidine, 0.187 g of pyridine-sulphur trioxide complex and 1 ml of DMF is stirred until a homogenous solution is obtained and the solution is allowed to stand at room temperature for 12 hours. By working up as described in Example 7, 0.26 g of pyridinium 3 - ($\alpha$ - benzyl N - carbobenzoxy - $\gamma$ - D - glutamyl - D - alaninamido) - 2 - oxoazetidine - 1 - sulphonate is obtained as crystals.

IR$\nu_{max}^{KBr}$cm$^{-1}$; 3300, 1760, 1740, 1690, 1640, 1570, 1270, 1210, 1045.

The above pyridinium 3-($\alpha$-benzyl N-carbobenzoxy-$\gamma$-D-glutamyl-D-alaninamido)-2-oxoazetidine-1-sulphonate (0.26 g) is passed through Dowex 50 W (Na-form), and the eluate is freeze-dried and dissolved in 20 ml of water. To the solution is added 0.20 g of palladium black and the mixture is stirred in a hydrogen gas steam for an hour. The catalyst is filtered off and the filtrate is freeze-dried to yield 0.1 g of sodium 3-($\gamma$-D-glutamyl-D-alaninamido)-2-oxoazetidine-1-sulphonate.

IR$\nu_{max}^{KBr}$cm$^{-1}$; 1760, 1650, 1630, 1530, 1260, 1240, 1050.

NMR($D_2O$, ppm); 1.43 (d, J=7Hz, —$CH_3$), 2.16 (q, J=7Hz, —$CH_2$—), 2.54 (t, J=7Hz, —$CH_2$—), 3.74 (dd, J=2, 6Hz, $C_4$—H),

3.80 (t, J=6Hz, —CH—),

3.97 (t, J=6Hz, $C_4$—H),

4.34 (q, J=7Hz, —CH—),

4.96 (dd, J=2, 6Hz, $C_3$—H).

Example 16

0.13 g of pyridinium 3-benzyloxycarboxamido-2-oxoazetidine-1-sulphonate is dissolved in 4.5 ml of 60% ethanol, followed by addition of 0.13 g of 10% palladium-carbon (hydrous: Nippon Engelhard Industries Ltd.). The mixture is stirred in a hydrogen gas stream at room temperature for 1.5 hours. The catalyst is filtered off and the filtrate is concentrated to give 60 mg of 3-amino-2-oxoazetidine-1-sulphonic acid.

IR$\nu_{max}^{KBr}$cm$^{-1}$; 3400, 1750, 1240, 1050.

NMR($D_2O$, ppm); 3.56 (dd, J=3, 6Hz, $C_4$—H), 4.05 (t, J=6Hz, $C_4$—H), 4.45 (dd, J=3, 6Hz, $C_3$—H).

50 mg of the above 3-amino 2-oxoazetidine-1-sulphonate are dissolved in 1 ml of water, followed by addition of a solution of 69 mg phenylacetyl chloride in 1 ml tetrahydrofuran and 101 mg of sodium hydrogen carbonate in alternate portions with ice-cooling and stirring. After stirring at room temperature for 30 minutes, the mixture is adjusted to pH 5.8 with phosphoric acid. The tetrahydrofuran is then distilled off under reduced pressure. The water layer is washed with ethyl acetate and purified by XAD—II chromatography, whereby 42 mg of sodium 3-phenylacetamido-2-oxoazetidine-1-sulphonate is obtained.

IR$\nu_{max}^{KBr}$cm$^{-1}$; 3310, 1780, 1670, 1300—1200, 1080, 1065.

NMR($d_6$-DMSO, ppm); 3.27 (dd, J=3, 6Hz), $C_4$—H), 3.46 (s, —$CH_2$—), 3.60 (t, J=6Hz, $C_4$—H), 4.84 (ddd, J=3, 6, 8Hz, $C_3$—H), 7.29 (s, aromatic H), 8.83 (d, J=8Hz, NH).

Example 17

0.349 g of 3-($\alpha$-ureidophenyl-acetamido)-2-oxoazetidine is dissolved in 5 ml of DMF, followed by addition of 0.955 g of pyridine-sulphur trioxide complex. The mixture is stirred for 4 days. By working up as described in Example 5, the following two products are obtained. Sodium 3-($\alpha$-ureidophenylacetamido)-2-oxoazetidine-1-sulphonate, 86 mg.

$IR\nu_{max}^{KBr}cm^{-1}$; 3430, 3340, 1755, 1650, 1510, 1240, 1190, 1040.

NMR($d_6$-DMSO, ppm); 3.14 (dd, J=3,6Hz, $C_4$—H), 3.54 (t, J=6Hz, $C_4$—H), 4.83 (ddd, J=3, 6, 8Hz), $C_3$—H),

5.28 (d, J=8Hz, —CH—),

5.68 (s, $NH_2$), 6.80 (d, J=8Hz, NH), 7.35 (broad s, aromatic H), 9.12 (d, J=8Hz, NH).

Disodium 3-(α-sulphonatoureidophenylacetamido)-2-oxoazetidine-1-sulphonate, 0.455 g.

$IR\nu_{max}^{KBr}cm^{-1}$; 3440, 1755, 1660, 1520, 1230, 1140, 1040.

NMR($d_6$-DMSO, ppm); 3.14 (dd, J=3, 6Hz, $C_4$—H), 3.57 (t, J=6Hz, $C_4$—H), 4.83 (ddd, J=3, 6, 8Hz, $C_3$—H),

5.33 (d, J=8Hz, CH—),

7.2—7.5 (m, aromatic H), 7.6 (d, J=8Hz, NH), 8.36 (broad s, NH), 9.18 (d, J=8Hz, NH).

## Example 18

0.313 g of 3 - [2 - (4 - ethyl - 2,3 - dioxo - 1 - piperazino - carboxamido) - 2 - phenylacetamido] - 3 - methoxy - 2 - oxoazetidine is dissolved in 3 ml of DMF, followed by addition of 0.359 g of pyridine-sulphur trioxide complex. The mixture is stirred for 5 days, and the reaction mixture is treated in the manner as described in Example 5, 0.202 g of sodium 3 - [2 - (4 - ethyl - 2,3 - dioxo - 1 - piperazinocarboxamido) - 2 - phenylacetamido] - 3 - methoxy - 2 - oxoazetidine - 1 - sulphonate is obtained.

$IR\nu_{max}^{KBr}cm^{-1}$; 3460, 1770, 1710, 1675, 1510, 1250, 1190, 1050.

## Example 19

0.404 g of 3 - [2 - (4 - ethyl - 2,3 - dioxo - 1 - piperazino-carboxyamido) - 4 - hydroxyphenylacetamido] - 2 - oxoazetidine is dissolved in 4 ml of DMF, followed by addition of 0.637 g of pyridine-sulphur trioxide complex. The mixture is stirred for 4 days, and the reaction mixture is treated in the manner as described in Example 5, whereby 0.203 g of disodium 3 - [2 - (4 - ethyl - 2,3 - dioxo - 1 - piperazinocarboxamido) - 2 - (4 - sulphonatoxyphenyl)acetamido] - 2 - oxoazetidine - 1 - sulphonate is obtained.

$IR\nu_{max}^{KBr}cm^{-1}$; 3470, 1760, 1710, 1675, 1500, 1250, 1190, 1050.

NMR($d_6$-DMSO, ppm); 1.09 (t, J=7Hz, $CH_3$), 3.19 (dd, J=3, 5Hz, $C_4$—H), 3.42 (q, J=7Hz, —$CH_2$—), 4.86 (m, $C_3$—H),

5.40 (d, J=7Hz, —CH—),

7.24 (ABq, J=9, 17Hz, aromatic H), 9.20 (d, J=8Hz, NH), 9.76 (d, J=7Hz, NH).

## Example 20

0.404 g of 3 - [2 - (4 - ethyl - 2,3 - dioxo - 1 - piperazinocarboxamido) - 2 - (4 - hydroxyphenyl)acetamido] - 2 - oxoazetidine is dissolved in 2 ml of DMF, followed by addition of 0.191 g of pyridine-sulphur trioxide complex. The mixture is stirred for 4 days. By working up as described in Example 5, 0.096 g of disodium 3 - [2 - (4 - ethyl - 2,3 - dioxo - 1 - piperazinocarboxamido) - 2 - (4 - sulphonatoxyphenyl)acetamido] - 2 - oxoazetidine - 1 - sulphonate and 0.08 g of sodium 3 - [2 - (4 - ethyl - 2,3 - dioxo - 1 - piperazinocarboxamido) - 2 - (4 - hydroxyphenyl)acetamido] - 2 - oxoazetidine - 1 - sulphonate are obtained. Properties of the latter compound:

$IR\nu_{max}^{KBr}cm^{-1}$; 3470, 3300, 1755, 1710, 1675, 1500, 1265, 1240, 1190, 1050.

NMR($d_6$-DMSO, ppm); 1.09 (t, J=7Hz, —$CH_3$), 2.97 (dd, J=3, 5Hz, $C_4$—H), 3.34 (q, J=7Hz, —$CH_2$—), 3.35 (t, J=5Hz, $C_4$—H), 4.84 (m, $C_3$—H),

5.40 (d, J=7Hz, —CH—),

7.24 (ABq, J=8, 16Hz, aromatic H), 7.99 (s, OH), 9.10 (d, J=8Hz, NH), 9.77 (d, J=7Hz, NH).

Example 21

0.128 g of 3-(4-ethyl-2,3-dioxo-1-piperazinocarboxamido)-2-oxoazetidine is dissolved in 3 ml of DMF, followed by addition of 0.16 g of pyridine-sulphur trioxide complex. The mixture is stirred for 3 days. By working up as described in Example 5, 0.095 g of sodium 3-(4-ethyl-2,3-dioxo-1-piperazinocarboxamido)-2-oxoazetidine-1-sulphonate is obtained.

$IR\nu_{max}^{KBr}cm^{-1}$; 3300, 1760, 1710, 1675, 1520, 1260, 1180, 1050.

Example 22

0.326 g of 3-[N-(4-ethyl-2,3-dioxo-1-piperazinocarbonyl)-D-alaninamido]-2-oxoazetidine is dissolved in 3 ml of DMF, followed by addition of 0.319 g of pyridine-sulphur trioxide complex. The mixture is stirred for 3 days, and the reaction mixture is treated in the manner as described in Example 5, whereby 0.253 g of sodium 3 - [N - (4 - ethyl - 2,3 - dioxo - 1 - piperazinocarbonyl) - D - alaninamido] - 2 - oxoazetidine -1 - sulphonate is obtained.

$IR\nu_{max}^{KBr}cm^{-1}$; 3300, 1760, 1710, 1675, 1520, 1250, 1190, 1050.

Example 23

0.152 g of 3-amino-2-oxoazetidine is added to 2 ml of DMF, followed by addition of 0.315 g 2,4-dioxo-5-phenyl-1,3-dioxolan in small portions. After 10 minutes, 0.563 g of pyridine-sulphur trioxide complex is added. The mixture is allowed to stand at room temperature for 3 days, and the reaction mixture is treated in the manner as described in Example 5. The above procedure yields 0.790 g of disodium 3-(α-sulphonatoxyphenylacetamido)-2-oxoazetidine-1-sulphonate.

$IR\nu_{max}^{KBr}cm^{-1}$; 1762, 1663, 1240.

NMR($d_6$-DMSO, ppm); 3.32 (dd, J=2, 5.5Hz, $C_4$—H), 3.52 (t, J=5.5Hz, $C_4$—H), 4.77 (ddd, J=2, 8, 5.5Hz, $C_3$—H),

5.39 (s, —C*H*—),

7.33 (s, aromatic H), 8.63 (d, J=8Hz, NH).

Example 24

0.157 g of 3-(2-*syn*-methoxyimino-2-phenylacetamido)-2-oxoazetidine is dissolved in 1 ml of DMF, followed by addition of 0.202 g of pyridine-sulphur trioxide complex. The mixture is allowed to stand at room temperature for 3 days, and the reaction mixture is treated in the manner as described in Example 5. The above procedure yields 0.183 g of sodium 3-(2-*syn*-methoxyimino-2-phenylacetamido)-2-oxoazetidine-1-sulphonate.

$IR\nu_{max}^{KBr}cm^{-1}$; 1760, 1660, 1530, 1250.

NMR(DMSO-$d_6$, ppm); 3.67 (t, J=5.5Hz, $C_4$—H), 3.90 (s, —$OCH_3$), 4.95 (ddd, J=2, 8 5.5Hz, $C_3$—H), 7.3—7.7 (m, aromatic H), 9.38 (d, J=9Hz, NH).

Example 25

0.18 g of pyridinium 3-methoxy-3-benzyloxycarboxamido-2-oxoazetidine-1-sulphonate is dissolved in 3 ml of 60% ethanol, followed by addition of 0.15 g of 10% palladium-carbon. The mixture is stirred in a hydrogen gas stream at room temperature for an hour, and the catalyst is filtered off. The filtrate is concentrated to give 35 mg of 3-amino-3-methoxy-2-oxoazetidine-1-sulphonic acid.

$IR\nu_{max}^{KBr}cm^{-1}$; 3400, 1758, 1240, 1050.

NMR($D_2O$, ppm); 3.76 (4.25 (ABq, J=6Hz, $C_4$—H), 3.52 (s, $OCH_3$).

30 mg of the above 3-amino-3-methoxy-2-oxoazetidine-1-sulphonic acid are dissolved in 1 ml of water, and to the solution are added a solution of 89 mg of the acid chloride prepared from 2-(2-chloroacetamido-4-thiazolyl)-2-methoxyiminoacetic acid (*syn*-isomer) in 1 ml of tetrahydrofuran and 36 mg of sodium hydrogen carbonate in alternate portions with ice-cooling and stirring. The mixture is further stirred at room temperature for 20 minutes and adjusted to pH 5.8 with phosphoric acid. After removal of tetrahydrofuran under reduced pressure, the water layer is washed with ethyl acetate and purified by Amberlite XAD—II chromatography. The above procedure yields 28 mg of sodium 3 - [2 - (2 - chloroacetamido - 4 - thiazolyl) - 2 - methoxyiminoacetamido] - 3 - methoxy - 2 - oxoazetidine - 1 - sulphonate (*syn*-isomer) which is the same compound as the one described in Example 9.

Example 26

0.126 g of 3 - [D - 2 - (4 - ethyl - 2,3 - dioxo - 1 - piperazinocarboxamido) - 2 - (4 - methoxyphenyl)acetamido] - 2 - oxoazetidine is dissolved in 2 ml of DMF, followed by addition of 0.096 g of pyridine-sulphur trioxide complex. The mixture is stirred for 3 days, after which it is treated

as described in Example 5. The above procedure provides 0.071 g of sodium 3 - [D - 2 - (4 - ethyl - 2,3 - dioxo - 1 - piperazinocarboxamido) - 2 - (4 - methoxyphenyl)acetamido] - 2 - oxoazetidine - 1 - sulphonate.

$IR\nu^{KBr}_{max}cm^{-1}$; 1760, 1670, 1245.

NMR($d_6$-DMSO, ppm); 1.10 (t, J=7Hz, —$CH_3$), 3.12 (dd, J=3, 6Hz, $C_4$—H), 3.40 (q, J=7Hz, —$CH_2$—), 3.57 (t, J=6Hz, $C_4$—H), 3.4—4.0 (m, —$CH_2$—), 3.76 (s, $OCH_3$, 4.84 (ddd, J=3, 6, 8Hz, $C_3$—H),

5.36 (d, J=7Hz, —CH—),

6.92, 7.33 (ABq, J=8Hz, aromatic H), 9.16 (d, J=8Hz, NH), 9.73 (d, J=7Hz, NH).

## Example 27

0.446 g of 3 - [D - 2 - [2 - (2 - chloroacetamido - 4 - thiazolyl) - 2 - methoxyiminoacetamido] - 2 - phenylacetamido] - 2 - oxoazetidine (a mixture of *syn-* and *anti*-isomers, is dissolved in 4 ml of DMF, followed by addition of 0.297 g of pyridine-sulphur trioxide complex. The mixture is stirred for 3 days and worked up in the manner as described in Example 5. The above procedure provides 0.327 g of sodium 3 - [D - 2 - [2 - (2 - chloroacetamido - 4 - thiazolyl) - 2 - methoxyiminoacetamido] - 2 - phenylacetamido] - 2 - oxoazetidine - 1 - sulphonate (a mixture of *syn-* and *anti*-isomers).

$IR\nu^{KBr}_{max}cm^{-1}$; 1765, 1670, 1550, 1270, 1200.

NMR($d_6$-DMSO, ppm); 3.17 (dd, J=3, 6Hz, $C_4$—H), 3.62 (t, J=6Hz, $C_4$—H), 3.89, 4.04 (s, $CH_3$), 4.40 (s, $ClCH_2$—), 4.90 (ddd, J=3, 6, 8Hz, $C_3$—H), 5.61,

5.63 (d, J=8Hz, —CH—),

7.2—7.6 (m, aromatic H), 7.46, 8.01 ( s, 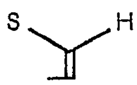 ),

9.01, 9.14 (d, J=8Hz, NH), 8.84, 9.32 (d, J=8Hz, NH), 12.6 (broad s, NH).

0.233 g of the above sodium 3 - [D - 2 - [2 - (2 - chloroacetamido - 4 - thiazolyl) - 2 - methoxyiminoacetamido] - 2 - phenylacetamido] - 2 - oxoazetidine - 1 - sulphonate (a mixture of *syn-* and *anti*-isomers) is dissolved in 3 ml of water, and under ice-cooling and stirring, 0.057 g of sodium N-methyldithiocarbamate is added, followed by stirring for 45 minutes. The insoluble matter is filtered off and the filtrate is purified by Amberlite XAD—II chromatography. The procedure provides 0.053 g of sodium 3 - [D - 2 - [2 - (2 - amino - 4 - thiazolyl) - 2 - methoxyiminoacetamido] - 2 - phenylacetamido] -2 - oxoazetidine - 1 - sulphonate (*syn*-isomer) and 0.087 g of the *anti*-isomer.

*Syn*-isomer:

$IR\nu^{KBr}_{max}cm^{-1}$; 3430, 3300, 1760, 1655, 1525, 1260, 1240, 1195, 1050.

NMR($d_6$-DMSO, ppm); 3.17 (dd, J=3, 6Hz, $C_4$—H), 3.61 (t, J=6Hz, $C_4$—H), 3.84 (s, $CH_3$), 4.87 (ddd, J=3, 6, 8Hz, $C_3$—H),

5.56 (d, J=7Hz, —CH—), 6.80 ( s, 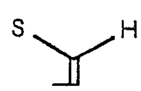 ),

7.13 (broad s, $NH_2$), 7.2—7.6 (m, aromatic H), 8.93 (d, J=8Hz, NH), 9.19 (d, J=7Hz, NH).

*Anti*-isomer:

$IR\nu^{KBr}_{max}cm^{-1}$; 3430, 3310, 1760, 1660, 1525, 1260, 1240, 1195, 1050, 1025.

NMR($d_6$-DMSO, ppm); 3.17 (dd, J=3, 6Hz, $C_4$—H), 3.61 (t, J=6Hz, $C_4$—H), 3.98 (s, $CH_3$), 4.88 (ddd, J=3, 6, 8Hz, $C_3$—H),

5.56 (d, J=8Hz, —CH—), 7.02 (broad s, NH), 7.44 ( s, 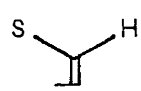 ),

7.2—7.6 (m, aromatic H), 8.90 (d, J=8Hz, NH), 9.05 (d, J=8Hz, NH).

Example 28

0.302 g of 3 - [D - 2 - (6 - bromo - 1,4 - dihydro - 1 - ethyl - 4 - oxothieno[2,3 - b]-pyridine - 3 - carboxamido) - 2 - phenylacetamido] - 2 - oxoazetidine is dissolved in 4 ml of DMF, followed by addition of 0.191 g of pyridine-sulphur trioxide complex. The reaction mixture is stirred for one day and worked up in the manner as described in Example 5. The above procedure provides 0.17 g of sodium 3 - [D - 2 - (6 - bromo - 1,4 - dihydro - 1 - ethyl - 4 - oxothieno[2,3 - b]pyridine - 3 - carboxamido) - 2 - phenylacetamido] - 2 - oxoazetidine - 1 - sulphonate.

IR$\nu_{max}^{KBr}$cm$^{-1}$; 3440, 3270, 1755, 1650, 1590, 1525, 1495, 1260, 1235, 1050.

NMR($d_6$-DMSO, ppm); 1.43 (t, J=7Hz, $CH_3$), 3.15 (dd, J=3, 6Hz, $C_4$—H), 3.59 (t, J=6Hz, $C_4$—H), 4.27 (q, J=7Hz, —$CH_2$—), 4.86 (ddd, J=3, 6, 8Hz, $C_3$—H),

5.78 (d, J=8Hz, —CH—),

7.2—7.5 (m, aromatic H), 7.62 (s, aromatic H), 8.69 (s, aromatic H), 9.22 (d, J=8Hz, NH), 10.95 (d, J=8Hz, NH).

Example 29

0.729 g of 3 - [2 - (4 - ethyl - 2,3 - dioxo - 1 - piperazinocarboxamido) - 2 - (2 - chloroacetamido - 4 - thiazolyl) acetamido] - 2 - oxoazetidine is dissolved in 6 ml of DMF, followed by addition of 0.478 g of pyridine-sulphur trioxide complex. The mixture is stirred for 2 days and, then, worked up as described in Example 5. The above procedure provides 0.434 g of sodium 3 - [2 - (4 - ethyl - 2,3 - dioxo - 1 - piperazinocarboxamido) - 2 - (2 - chloroacetamido - 4 - thiazolyl)-acetamido] - 2 - oxoazetidine - 1 - sulphonate.

IR$\nu_{max}^{KBr}$cm$^{-1}$; 3450, 1760, 1675, 1270.

NMR($d_6$-DMSO, ppm); 1.10 (t, J=7Hz, —$CH_3$), 3.19 (dd, J=3, 6Hz, $C_4$—H), 3.43 (q, J=7Hz, —$CH_2$—), 3.56 (t, J=6Hz, $C_4$—H), 4.36 (s, —$CH_2Cl$), 4.80 and 4.86 (m, $C_3$—H),

5.54 (d, J=7Hz, —CH—), 7.21 (s, 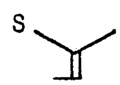 ),

9.01, 9.07 (d, J=8Hz, NH), 9.74 (d, J=7Hz, NH), 12.7 (broad, s, NH).

0.235 g of the above sodium 3 - [2 - (4 - ethyl - 2,3 - dioxo - 1 - piperazinocarboxamido) - 2 - (2 - chloroacetamido - 4 - thiazolyl)acetamido] - 2 - oxoazetidine - 1 - sulphonate is dissolved in 3 ml of water and, under ice-cooling and stirring, 0.057 g of sodium N-methyldithiocarbamate is added. The mixture is stirred for 30 minutes, after which the insolubles are filtered off. The filtrate is purified by Amberlite XAD—II chromatography to provide 0.13 g of sodium 3 - [2 - (4 - ethyl - 2,3 - dioxo - 1 - piperazinocarboxamido) - 2 - (2 - amino - 4 - thiazolyl)acetamido] - 2 - oxo-azetidine - 1 - sulphonate.

IR$\nu_{max}^{KBr}$cm$^{-1}$; 3430, 1760, 1675, 1515, 1260.

NMR($d_6$-DMSO, ppm); 1.09 (6, J=7Hz, —$CH_3$), 3.19 (dd, J=3, 6Hz, $C_4$—H), 3.41 (q, J=7Hz, —$CH_2$—), 3.63 (t, J=6Hz, $C_4$—H), 4.80, 4.87 (m, $C_3$—H),

5.27 (d, J=7Hz, —CH—), 6.48 (s, 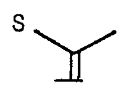 ),

7.01 (s, $NH_2$), 8.90, 8.94 (d, J=8Hz, NH), 9.68 (d, J=7Hz, NH).

Example 30

0.303 g of 3 - [2 - (4 - ethyl - 2,3 - dioxo - 1 - piperazinocarboxamido) - 2 - (2 - acetamido - 4 - thiazolyl) - acetamido] - 2 - oxoazetidine is dissolved in 3 ml of DMF, followed by addition of 0.213 g of pyridine-sulphur trioxide complex. The mixture is stirred for 3 days and, then, worked up in the manner as described in Example 5. The above procedure provides 0.101 g of sodium 3 - [2 - (4 - ethyl - 2,3 - dioxo - 1 - piperazinocarboxamido) - 2 - (2 - acetamido - 4 - thiazolyl)acetamido] - 2 - oxoazetidine - 1 - sulphonate.

IR$\nu_{max}^{KBr}$cm$^{-1}$; 3430, 1760, 1705, 1670, 1275.

NMR($d_6$-DMSO, ppm); 1.10 (t, J=7Hz, —$CH_3$), 2.13 (s, —$COCH_3$), 3.18 (dd, J=3, 6Hz, $C_4$—H), 3.42 (q, J=7Hz, —$CH_2$—), 3.57 (t, J=6Hz, $C_4$—H), 4.78, 4.85 (m, $C_3$—H),

5.52 (d, J=7Hz, —CH—), 7.11 ( s, 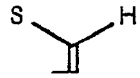 ),

8.99, 9.05 (d, J=8Hz, NH), 9.71 (d, J=7Hz, NH), 12.25 (broad s, NH).

Example 31

0.635 g of 3 - [2 - [2 - (2 - chloroacetamido - 4 - thiazolyl) - 2 - methoxyiminoacetamido] - 2 - (2 - chloroacetamido - 4 - thiazolyl)acetamido] - 2 - oxoazetidine (*syn*-isomer) is dissolved in 5 ml of DMF, followed by addition of 0.35 g of pyridine-sulphur trioxide complex. The mixture is stirred for one day and, then, worked up in the manner as described in Example 5. The above procedure provides 0.271 g of sodium 3 - [2 - [2 - (2 - chloroacetamido - 4 - thiazolyl) - 2 - methoxyimino-acetamido] - 2 - (2 - chloroacetamido - 4 - thiazolyl)acetamido] - 2 - oxoazetidine - 1 - sulphonate (*syn*-isomer).

IR$\nu_{max}^{KBr}$cm$^{-1}$; 3430, 1760, 1660, 1540, 1265, 1190, 1050.
NMR($d_6$-DMSO, ppm); 3.27 (m, $C_4$—H), 3.67 (t, J=6Hz, $C_4$—H), 3.91 (s, $CH_3$), 4.38 (s, $ClCH_2$—), 4.90 (m, $C_3$—H),

5.66 (d, J=8Hz, —CH—), 7.18 ( s, 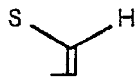 ), 7.55 ( s, 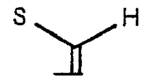 ),

8.84, 8.88 (d, J=8Hz, NH), 9.25, 9.30 (d, J=8Hz, NH), 12.6 (broad, s, NH), 12.75 (broad s, NH).

0.204 g of the above sodium 3 - [2 - [2 - (2 - chloroacetamido - 4 - thiazolyl) - 2 - methoxy-iminoacetamido] - 2 - (2 - chloroacetamido - 4 - thiazolyl)acetamido] - 2 - oxoazetidine - 1 - sulphonate (*syn*-isomer) is dissolved in 3 ml of water and, while the solution is stirred under ice-cooling, 0.085 g of sodium N-methyldithiocarbamate is added. The mixture is stirred for an hour, after which the insoluble matter is filtered off. The filtrate is purified by Amberlite XAD—II chromatography to provide 0.1 g of sodium 3 - [2 - [2 - (2 - amino - 4 - thiazolyl) - 2 - methoxyiminoacetamido] - 2 - (2 - amino - 4 - thiazolyl) acetamido] - 2 - oxoazetidine - 1 - sulphonate (*syn*-isomer).

IR$\nu_{max}^{KBr}$cm$^{-1}$; 3410, 3310, 1760, 1655, 1620, 1540, 1260, 1240, 1195, 1050.
NMR($d_6$-DMSO, ppm); 3.30 (m, $C_4$—H), 3.64 (m, $C_4$—H), 3.85 (s, $CH_3$), 4.88 (m, $C_3$—H),

5.38 (d, J=7Hz, —CH—), 6.49 ( s, 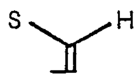 ), 6.95 ( s, 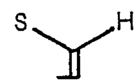 ),

7.10 (broad, s, $NH_2$), 8.73 (d, J=8Hz, NH), 8.90 (d, J=7Hz, NH).

Example 32

0.283 g of 3 - [D - 2 - (4 - n - octyl - 2,3 - dioxo - 1 - piperazinocarboxamido) - 2 - phenylacetamido] - 2 - oxoazetidine is dissolved in 3 ml of DMF, followed by addition of 0.191 g of pyridine-sulphur trioxide complex. The mixture is stirred for one day and worked up in the manner as described in Example 5. The above procedure provides 0.234 g of sodium 3 - [D - 2 - (4 - n - octyl - 2,3 - dioxo - 1 - piperazinocarboxamido) - 2 - phenylacetamido] - 2 - oxoazetidine - 1 - sulphonate.

IR$\nu_{max}^{KBr}$cm$^{-1}$; 3470, 3290, 2925, 2850, 1760, 1710, 1670, 1510, 1260, 1190, 1050.
NMR($d_6$-DMSO, ppm); 0.86 (t, $CH_3$), 3.12 (dd, J=3, 6Hz, $C_4$—H), 3.4—4.1 (m, —$CH_2$—), 4.86 (ddd, J=3, 6, 8Hz, $C_3$—H),

5.45 (d, J=7Hz, —CH—),

7.40 (s, aromatic H), 9.25 (d, J=8Hz, NH), 9.81 (d, J=7Hz, NH).

Example 33

0.274 g of 3 - [D - 2 - (coumarin - 3 - carboxamido) - 2 - phenylacetamido] - 2 - oxoazetidine is suspended in 3 ml of DMF, followed by addition of 0.223 g of pyridine-sulphur trioxide complex. The mixture is stirred for 2 days and worked up in the manner as described in Example 5. The above procedure provides 0.094 g of sodium 3 - [D - 2 - (coumarin - 3 - carboxamido) - 2 - phenylacetamido] - 2 - oxoazetidine - 1 - sulphonate.

IR$\nu_{max}^{KBr}$cm$^{-1}$; 3440, 3300, 1755, 1700, 1640, 1600, 1560, 1515, 1240, 1190, 1045.
NMR($d_6$-DMSO, ppm); 3.18 (dd, J=3, 6Hz, $C_4$—H), 3.54 (t, J=6Hz, $C_4$—H), 4.90 (ddd, J=3, 6, 8Hz, $C_3$—H),

5.67 (d, J=7Hz, —CH—),

7.3—8.1 (m, aromatic H), 8.89 (s, aromatic H), 9.32 (d, J=8Hz, NH), 9.65 (d, J=7Hz, NH).

## Example 34

0.454 g of 3 - [2 - (4 - n - octyl - 2,3 - dioxo - 1 - piperazinocarboxamido) - 2 - (2 - chloroacetamido - 4 - thiazolyl)acetamido] - 2 - oxoazetidine is dissolved in 4 ml of DMF, followed by addition of 0.255 g of pyridine-sulphur trioxide complex. The mixture is stirred for one day, and worked up in the manner of Example 5. The above procedure provides 0.182 g of sodium 3 - [2 - (4 - n - octyl - 2,3 - dioxo - 1 - piperazinocarboxamido) - 2 - (2 - chloroacetamido - 4 - thiazolyl)acetamido] - 2 - oxoazetidine - 1 - sulphonate.

IR$\nu_{max}^{KBr}$cm$^{-1}$; 3440, 2925, 1760, 1710, 1670, 1260, 1190, 1050.
NMR($d_6$-DMSO, ppm); 0.86 (t, —$CH_3$), 4.36 (s, $ClCH_2$—), 4.82 (m, $C_3$—H),

5.53 (d, J=7Hz, —CH—), 7.20 ( s , 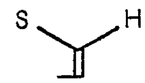 ),

8.98, 9.04 (d, J=8Hz, NH), 9.72 (d, J=7Hz, NH), 12.63 (broad s, NH).

0.115 g of the above sodium 3 - [2 - (4 - n - octyl - 2,3 - dioxo - 1 - piperazinocarboxamido) - 2 - (2 - chloroacetamido - 4 - thiazolyl)acetamido] - 2 - oxoazetidine - 1 - sulphonate is dissolved in 2 ml of water and, under ice-cooling and stirring, 0.025 g of sodium N-methyldithiocarbamate is added. The mixture is stirred for 90 minutes, after which the insoluble matter is filtered off. The filtrate is purified by Amberlite XAD—II chromatography to provide 0.046 g of sodium 3 - [2 - (4 - n - octyl - 2,3 - dioxo - 1 - piperazinocarboxamido) - 2 - (2 - amino - 4 - thiazolyl)acetamido] - 2 - oxoazetidine - 1 - sulphonate.

IR$\nu_{max}^{KBr}$cm$^{-1}$; 3400, 3300, 2920, 1755, 1710, 1670, 1510, 1250, 1195, 1050.
NMR($d_6$-DMSO, ppm); 0.86 (t, $CH_3$), 4.80 (m, $C_3$—H),

5.24 (d, J=7Hz, —CH—) 6.49 ( s , 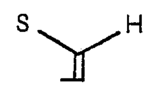 ),

7.0 (broad s, $NH_2$), 8.88, 8.92 (d, J=8Hz, NH), 9.58 (d, J=7Hz, NH).

## Example 35

0.161 g of 3 - [D - 2 - (4 - hydroxy - 7 - trifluoromethylquinoline - 3 - carboxamido) - 2 - phenylacetamido] - 2 - oxoazetidine is dissolved in 2 ml of DMF, followed by addition of 0.112 g of pyridine-sulphur trioxide complex. The mixture is stirred for one day and, then, worked up in the manner of Example 5. The above procedure provides 0.095 g of sodium 3 - [D - 2 - (4 - hydroxy - 7 - trifluoromethylquinoline - 3 - carboxamido) - 2 - phenylacetamido] - 2 - oxoazetidine - 1 - sulphonate.

IR$\nu_{max}^{KBr}$cm$^{-1}$; 3450, 3270, 1775, 1650, 1520, 1315, 1260, 1240, 1195, 1175, 1130, 1045.
NMR($d_6$-DMSO, ppm); 3.30 (dd, J=3, 6Hz, $C_4$—H), 3.66 (t, J=6Hz, $C_4$—H), 4.88 (ddd, J=3, 6, 8Hz, $C_3$—H),

5.70 (d, J=8Hz, —CH—),

7.3—8.9 (m, aromatic H), 9.21 (d, J=8Hz, NH), 10.80 (d, J=8Hz, NH).

## Example 36

0.34 g of 3 - [D - 2 - [(2 - oxo - 3 - furfurylideneaminoimidazolidin - 1 - yl)carboxamido] - 2 - phenylacetamido] - 2 - oxoazetidine is dissolved in 5 ml of DMF, followed by addition of 0.255 g of pyridine-sulphur trioxide complex. The mixture is stirred for one day and, then, worked up in the manner of Example 5. The above procedure provides 0.071 g of sodium 3 - (D - 2 - [(2 - oxo - 3 -

furfurylideneaminoimidazolidin - 1 - yl)carboxamido] - 2 - phenylacetamido] - 2 - oxoazetidine - 1 - sulphonate.

IR$\nu_{max}^{KBr}$cm$^{-1}$; 3450, 3300 (sh), 1755, 1720, 1665, 1520, 1470, 1410, 1270, 1230, 1050.
NMR($d_6$-DMSO, ppm); 3.12 (dd, J=3, 6Hz, $C_4$—H), 3.58 (t, J=6Hz, $C_4$—H), 3.80 (s, —$CH_2$—), 4.86 (ddd, J=3, 6, 8Hz, $C_3$—H),

5.45 (d, J=8Hz, —CH—),

6.5—7.9 (m, aromatic H), 7.75 (s, —CH=N—), 9.04 (d, J=8Hz, NH), 9.24 (d, J=8Hz, NH).

Example 37

0.287 g of 3 - [2 - (4 - n - octyl - 2,3 - dioxo - 1 - piperazino-carboxamido] - 2 - thienylacetamido] - 2 - oxoazetidine is dissolved in 2 ml of DMF, followed by addition of 0.191 g of pyridine-sulphur trioxide complex. The mixture is stirred for one day and, then, worked up in the manner of Example 5. The above procedure provides 0.299 g of sodium 3 - [2 - (4 - n - octyl - 2,3 - dioxo - 1 - piperazinocarboxamido) - 2 - thienylacetamido] - 2 - oxoazetidine - 1 - sulphonate.

IR$\nu_{max}^{KBr}$cm$^{-1}$; 3280, 2920, 1760, 1710, 1670, 1250, 1190, 1050.
NMR($d_6$-DMSO, ppm); 0.86 (t, $CH_3$), 3.4—4.1 (m, —$CH_2$—), 4.86 (m, $C_3$—H),

5.72 (d, J=7Hz, —CH—),

6.9—7.6 (m, thienyl-H), 9.26 and 9.30 (d, J=8Hz, NH), 9.73 (d, J=7Hz, NH).

Example 38

0.439 g of 3 - [D - 2 - (4 - n - octyl - 2,3 - dioxo - 1 - piperazinocarboxyamido) - 2 - (4 - hydroxyphenyl)acetamido] - 2 - oxoazetidine is dissolved in 2 ml of DMF, followed by additon of 0.172 g of pyridine-sulphur trioxide complex. The mixture is stirred for 2 days and, then, worked up in the manner of Example 5. The above procedure provides the following two products:

Sodium 3 - [D - 2 - (4 - n - octyl - 2,3 - dioxo - 1 - piperazinocarboxamido) - 2 - (4 - hydroxylphenyl)acetamido] - 2 - oxoazetidine - 1 - sulphonate (0.213 g).

IR$\nu_{max}^{KBr}$cm$^{-1}$; 3290, 2920, 1745, 1710, 1670, 1260, 1050.
NMR($d_6$-DMSO, ppm); 0.86 (t, $CH_3$), 2.96 (dd, J=3, 6Hz, $C_4$—H), 4.86 (m, $C_3$—H),

5.40 (d, J=7Hz, —CH),

7.13 (ABq, J=8, 18Hz, aromatic H), 9.10 (d, J=8Hz, NH), 9.78 (d, J=7Hz, NH).

Disodium 3 - [D - 2 -(4 - n - octyl - 2,3 - dioxo - 1 - piperazinocarboxamido) - 2 - (4 - sulphonatoxyphenyl)acetamido] - 2 - oxoazetidine - 1 - sulphonate (0.12 g).

IR$\nu_{max}^{KBr}$cm$^{-1}$; 2920, 1755, 1710, 1670, 1255, 1050.
NMR($d_6$-DMSO, ppm); 0.86 (t, $CH_3$), 3.16 (dd, J=3, 6Hz, $C_4$—H), 4.86 (m, $C_3$—H),

5.38 (d, J=7Hz, —CH—),

7.23 (ABq, J=8, 15Hz, aromatic H), 9.19 (d, J=8Hz, NH), 9.76 (d, J=Hz, NH).

Example 39

0.441 g of 3 - [D - 2 - [(2 - oxo - 3 - furfurylideneaminoimidazolidin - 1 - yl)carboxamido] - 2 - (4 - hydroxyphenyl) acetamido] - 2 - oxoazetidine is dissolved in 2 ml of DMF, followed by addition of 0.191 g of pyridine-sulphur trioxide complex. The mixture is stirred for 3 days and, then, worked up in the manner of Example 5. The above procedure provides the following two products:

Sodium 3 - [D - 2 - [(2 - oxo - 3 - furfurylideneaminoimidazolidin - 1 - yl)carboxamido] - 2 - (4 - hydroxyphenyl)acetamido] - 2 - oxoazetidine - 1 - sulphonate (0.026 g).

IR$\nu_{max}^{KBr}$cm$^{-1}$; 1755, 1720, 1660, 1420, 1270, 1235, 1050.

Disodium 3 - [D - 2 - (2 - oxo - 3 - furfurylideneaminoimidazolidin - 1 - yl)carboxamido] - 2 - (4 - sulphonatoxyphenyl)acetamido] - 2 - oxoazetidine - 1 - sulphonate (0.025 g).

IR$\nu_{max}^{KBr}$cm$^{-1}$; 1760, 1725, 1670, 1420, 1270, 1240, 1050.

Example 40

0.25 g of 3 - [2 - [(2 - oxo - 3 - furfurylideneaminoimidazolidin - 1 - yl)carboxamido] - 2 - thienylacetamido] - 2 - oxoazetidine is suspended in 5 ml of DMF, followed by addition of 0.185 g of pyridine-sulphur trixoide complex. The mixture is stirred for 3 days and, then, worked up in the manner of Example 5. The above procedure provides 0.121 g of sodium 3 - [2 - [(2 - oxo - 3 - furfurylideneaminoimidazolidin - 1 - yl)carboxamido] - 2 - thienylacetamido] - 2 - oxoazetidine - 1 - sulphonate.

IR$\nu_{max}^{KBr}$cm$^{-1}$; 1760, 1720, 1660, 1410, 1270, 1230, 1050.

NMR($d_6$-DMSO, ppm); 3.19 (3.25 (dd, J=3, 6Hz, $C_4$—H), 3.63, 3.65 (t, J=6Hz, $C_4$—H), 3.81 (broad s, ring $CH_2$), 4.88 (m, $C_3$—H),

5.72 (d, J=7Hz, —CH—),
|

6.5—7.9 (m, aromatic H), 7.75 (s, —CH=N—), 8.97, 8.98 (d, J=7Hz, NH), 9.26, 9.29 (d, J=8Hz, NH).

Example 41

0.441 g of 3 - [D - 2 - [[2 - oxo - 3 - (thiophene - 2 - aldoimino)imidazolidin - 1 - yl]carboxamido] - 2 - phenylacetamido] - 2 - oxoazetidine is suspended in 6 ml of DMF, followed by addition of 0.319 g of pyridine-sulphur trioxide complex. The mixture is stirred for 3 days, and then worked up in the manner of Example 5. The above procedure provides 0.208 g of sodium 3 - [D - 2 - [[2 - oxo - 3 - (thiophene - 2 - aldoimino)imidazolidin - 1 - yl) carboxamido] - 2 - phenylacetamido] - 2 - oxoazetidine - 1 - sulphonate.

IR$\nu_{max}^{KBr}$cm$^{-1}$; 1760, 1725, 1665, 1410, 1270, 1235, 1050.

NMR($d_6$-DMSO, ppm); 3.14 (dd, J=3, 6Hz, $C_4$—H), 3.59 (t, J=6Hz, $C_4$—H), 3.82 (broad s, —$CH_2$—), 4.87 (ddd, J=3, 6, 8Hz, $C_3$—H),

5.45 (d, J=8Hz, —CH—),
|

7.0—7.7 (m, aromatic H), 8.10 (s, —CH=N—), 9.06 (d, J=8Hz, NH), 9.24 (d, J=8Hz, NH).

Example 42

0.49 g of 3 - [2 - (4 - ethyl - 2,3 - dioxo - 1 - piperazinocarboxamido) - 2 - (2 - pyrrolyl)acetamido]- 2 - oxoazetidine is dissolved in 5 ml of DMF, followed by addition of 0.312 g of pyridine-sulphur trioxide complex. The mixture is stirred for 4 days and, then, worked up in the manner of Example 5. The above procedure provides 0.228 g of sodium 3 - [2 - (4 - ethyl - 2,3 - dioxo - 1 - piperazinocarboxamido) - 2 - (2 - pyrrolyl)acetamido] - 2 - oxoazetidine - 1 - sulphonate.

IR$\nu_{max}^{KBr}$cm$^{-1}$; 1755, 1705, 1670, 1505, 1250, 1190, 1050.

NMR($d_6$-DMSO, ppm); 1.09 (t, J=7Hz, —$CH_3$), 3.21 (dd, J=3, 6Hz, $C_4$—H), 3.41 (q, J=7Hz, —$CH_2$—), 3.4—4.1 (m, —$CH_2$—), 4.86 (m, $C_3$—H),

5.44 (d, J=7Hz, —CH—),
|

5.9—6.8 (m, pyrrolyl-H), 9.01 (d, J=8Hz, NH), 9.55 (d, J=8Hz, NH), 10.7 (broad s, NH).

Example 43

(A) 0.203 g of an equimolar mixture of 3 - [D - 2 - (4 - n - octyl - 2,3 - dioxo - 1 - piperazinocarboxamido) - 2 - thienylacetamido] - 3(S) - methoxy - 2 - oxoazetidine and 3 - [L - 2 - (4 - n - octyl - 2,3 - dioxo - 1 - piperazinocarboxamido) - 2 - thienylacetamido] - 3(R) - methoxy - 2 - oxoazetidine is dissolved in 2 ml of DMF, followed by addition of 0.128 g of pyridine-sulphur trioxide complex. The mixture is stirred for 2 days and, then, worked up in the manner of Example 5. The above procedure provides 0.136 g of an equimolar mixture of sodium 3 - [D - 2 - (4 - n - octyl - 2,3 - dioxo - 1 - piperazinocarboxamido) - 2 - thienylacetamido] - 3(S) - methoxy - 2 - oxoazetidine - 1 - sulphonate and sodium 3 - [L - 2 - (4 - n - octyl - 2,3 - dioxo - 1 - piperazinocarboxamido) - 2 - thienylacetamido] - 3(R) - methoxy - 2 - oxoazetidine - 1 - sulphonate.

IR$\nu_{max}^{KBr}$cm$^{-1}$; 3280, 2920, 1765, 1705, 1680, 1250, 1190, 1050.

NMR($d_6$-DMSO, ppm); 0.86 (t, $CH_3$), 3.16 (s, $OCH_3$), 3.56 and 3.72 (ABq, J=6, 16Hz), $C_4$—H),

5.92 (d, J=7Hz, —CH—),
|

6.9—7.6 (m, thienyl-H), 9.72 (d, J=7Hz, NH), 9.80 (s, NH).

(B) 0.095 g of an equimolar mixture of 3 - [D - 2 - (4 - n - octyl - 2,3 - dioxo - 1 - piperazinocarboxamido) - 2 - thienylacetamido] - 3(R) - methoxy - 2 - oxoazetidine and 3 - [L - 2 - (4 - n - octyl - 2,3 - dioxo - 1 - piperazinocarboxamido) - 2 - thienylacetamido] - 3(S) - methoxy - 2 - oxoazetidine is dissolved in 1 ml of DMF, followed by addition of 0.09 g of pyridine-sulphur trioxide complex. The mixture is stirred for 2 days and, then, worked up in the manner of Example 5. The above procedure provides 0.06 g of an equimolar mixture of sodium 3 - [D - 2 - (4 - n - octyl - 2,3 - dioxo - 1 - piperazinocarboxamido] - 2 - thienylacetamido] - 3(R) - methoxy - 2 - oxoazetidine - 1 - sulphonate and sodium 3 - [L - 2 - (4 - n - octyl - 2,3 - dioxo - 1 - piperazinocarboxamido) - 2 - thienylacetamido] - 3(S) - methoxy - 2 - oxoazetidine - 1 - sulphonate.

IR$\nu_{max}^{KBr}$cm$^{-1}$; 3280, 2920, 1770, 1710, 1680, 1250, 1190, 1050.
NMR($d_6$-DMSO, ppm); 0.86 (t, —$CH_3$), 3.35 (s, $OCH_3$),

5.88 (d, J=7Hz, —CH—),
|

6.9—7.6 (m, thienyl-H), 9.68 (d, J=7Hz, NH), 9.78 (s, NH).

Example 44

0.202 g of a mixture of 3 - [D - $\alpha$ - sulphophenylacetamido) - 3(R) - methoxy - 2 - oxoazetidine sodium salt and 3-(D-$\alpha$-sulphophenylacetamido-3(S)-methoxy-2-oxoazetidine sodium salt is dissolved in 3 ml of DMF, followed by addition of 0.191 g of pyridine-sulphur trioxide complex. The mixture is stirred for 3 days, after which it is treated as described in Example 5. The above procedure provides 0.039 g of a mixture of disodium 3 - (D - $\alpha$ - sulphophenylacetamido) - 3(R) - methoxy - 2 - oxoazetidine - 1 - sulphonate and disodium 3 - (D - $\alpha$ - sulphophenylacetamido) - 3(S) - methoxy - 2 - oxoazetidine - 1 - sulphonate.

IR$\nu_{max}^{KBr}$cm$^{-1}$; 1765, 1640, 1250—1200, 1040.

Example 45

0.500 g of 3-(N-benzyloxycarbonyl-D-alanyl-D-phenylglycylamino)-2-oxoazetidine and 0.375 g of pyridine-sulphur trioxide complex are dissolved in 2 ml of DMF. The mixture is stirred for 3 days, after which diethyl ether is added. The resultant oily precipitate is passed through Dowex 50W (Na-form) resin and the eluate is purified by Amberlite XAD—II chromatography to provide 0.453 g of sodium 3-(N-benzyloxycarbonyl-D-alanyl-D-phenylglycylamino)-2-oxoazetidine-1-sulphonate. (Dowex is a Registered Trade Mark).

IR$\nu_{max}^{KBr}$cm$^{-1}$; 1758, 1680, 1639, 1520.
NMR($d_6$-DMSO, ppm); 1.20 (d, J=6Hz, $CH_3$), 3.10 (dd, J=3, 6Hz, $C_4$—H), 3.54 (t, J=6Hz, $C_4$—H),

4.17 (t, $C_4$J=6Hz, —CH—),
|

4.82 (ddd, J=3, 6, 8Hz, $C_3$—H), 4.99 (s, —$CH_2$—),

5.40 (d, J=9Hz, —CH—),
|

8.30 (d, J, J=9Hz, NH), 9.08 (d, J=9Hz, NH).

0.115 g of the above sodium 3-(benzyloxycarbonyl-D-alanyl-D-phenylglycylamino)-2-oxoazetidine-1-sulphonate is dissolved in 10 ml of water and, after the addition of 0.065 g of palladium black, the mixture is stirred in a hydrogen gas stream at room temperature for 45 minutes. The catalyst is filtered off and the filtrate is freeze-dried, whereby 0.085 g of sodium 3-(D-alanyl-D-phenylglycylamino)-2-oxoazetidine-1-sulphonate is produced.

IR$\nu_{max}^{KBr}$cm$^{-1}$; 1759, 1661, 1520, 1240, 1048.
NMR($d_6$-DMSO, ppm); 1.16 (d, J=6Hz, $CH_3$), 3.13 (dd, J=3, 6Hz, $C_4$—H), 3.55 (t, J=6Hz, $C_4$—H),

4.17 (t, J=6Hz, —CH—),
|

4.82 (ddd, J=3, 6, 8Hz, $C_3$—H), 5.41 (d, J=9Hz, NH), 7.31 (s, aromatic H), 8.31 (d, J=8Hz, —NH), 9.16 (d, J=8Hz, NH).

Example 46

0.401 g of 3-[D-2-(2-ureido-2-thienylacetamido)-2-phenylacetamido]-2-oxoazetidine and 0.239 g of pyridine-sulphur trioxide complex are dissolved in 4 ml of DMF. The mixture is allowed to stand at room temperature for 3 days, after which it is treated as described in Example 1. The above procedure provides 0.180 g of pyridinium 3-[D-2-(2-ureido-2-thienylacetamido)-2-phenylacetamido]-2-oxoazetidine-1-sulphonate.

IR$\nu_{max}^{KBr}$cm$^{-1}$; 1750, 1645, 1510, 1225, 1040.

Example 47

0.200 g of 3-cyanomethyl-thioacetamido-2-oxoazetidine and 0.318 g of pyridine-sulphur trioxide complex are dissolved in 1 ml of DMF. The mixture is stirred for 3 days, after which diethyl ether is added. The resultant oily precipitate is passed through Dowex 50W (Na-form) resin to obtain 0.390 g of sodium 3-cyanomethylthioacetamido-2-oxoazetidine-1-sulphonate.

IR$\nu_{max}^{KBr}$cm$^{-1}$; 2240, 1758, 1660, 1530, 1240.
NMR($d_6$-DMSO, ppm); 3.27 (dd, J=3, 6Hz, $C_4$—H), 3.35 (s, —$CH_2$—), 3.66 (t, J=6Hz, $C_4$—H), 3.70 (s, —$CH_2$—), 4.80 (ddd, J=3, 6, 8Hz, $C_3$—H), 8.93 (d, J=8Hz, NH).

Example 48

0.330 g of 3-[D-2-[2-(2-chloroacetamido-4-thiazolyl)acetamido]-2-phenylacetamido]-2-oxoazetidine and 0.240 g of pyridine-sulphur trioxide complex are dissolved in 3 ml of DMF. The mixture is stirred for 3 days, after which it is treated as described in Example 5. The above procedure provides 0.110 g of sodium 3 - [D - 2 - [2 - (2 - chloroacetamido - 4 - thiazolyl)acetamido] - 2 - phenylacetamido] - 2 - oxoazetidine - 1 - sulphonate.

IR$\nu_{max}^{KBr}$cm$^{-1}$; 1758, 1660, 1530, 1260, 1230, 1043.
NMR($d_6$-DMSO, ppm); 3.17 (dd, J=3, 6Hz, $C_4$—H), 3.60 (t, J=6Hz, $C_4$—H), 3.66 (s, —$CH_2$—), 4.39 (s, $ClCH_2$—), 4.86 (ddd, J=3, 6, 8Hz, $C_3$—H),

5.50 (d, J=8Hz, —CH—),
|

6.99 (s, thiazole-H), 7.2—7.6 (s, aromatic H), 8.60, 9.13 (each d, J=8Hz, NH).

0.164 g of the above product is dissolved in 4 ml of water and, while the solution is stirred under ice-cooling, 0.043 g of $CH_3NHCS_2Na$ is added. Then, the mixture is stirred at room temperature for 20 minutes, and the precipitate is removed by filtration. The filtrate is purified by Amberlite XAD—II chromatography to yield 0.075 g of sodium 3 - [D - 2 - (2 - amino - 4 - thiazolyl)acetamido] - 2 - phenylacetamido] - 2 - oxoazetidine - 1 - sulphonate.

IR$\nu_{max}^{KBr}$cm$^{-1}$; 1759, 1650, 1510, 1240, 1050.
NMR($d_6$-DMSO, ppm); 3.16 (dd, J=3, 6Hz, $C_4$—H), 3.48 (s, —$CH_2$—), 3.63 (t, J=6Hz, $C_4$—H), 3.7 (br.s, $NH_2$), 4.85 (ddd, J=3, 6, 8Hz, $C_3$—H),

5.47 (d, J=7Hz, —CH—),
|

6.36 (s, thiazole-H), 7.37 (br.s), 8.57 (d, J=8Hz, NH), 9.10 (d, J=7Hz, NH).

Example 49

(A) 0.650 g of 3 - [DL - 2 - (4 - ethyl - 2,3 - dioxo - 1 - piperazinocarboxamido) - 2 - thienylacetamido] - 2 - oxoazetidine and 0.579 g of pyridine-sulphur trioxide complex are dissolved in 2 ml of DMF. The mixture is stirred for 30 hours, after which it is treated as described in Example 5. The above procedure provides 0.444 g of sodium 3 [DL - 2 - (4 - ethyl - 2,3 - dioxo - 1 - piperazinocarboxamido) - 2 - thienylacetamido] - 2 - oxoazetidine - 1 - sulphonate.

$[\alpha]_D^{25°}$ + 3.5° (c=0.627, $H_2O$).

IR$\nu_{max}^{KBr}$cm$^{-1}$; 1760, 1710, 1675, 1510, 1240, 1190, 1040.
NMR($d_6$-DMSO, ppm); 1.12 (t, J=7Hz, —$CH_3$), 3.13 (dd, J=3, 6Hz, $C_4$—H), 3.38 (q, J=7Hz, —$CH_2$—), 3.78 (t, J=6Hz, $C_4$—H), 4.83 (ddd, J=3, 6, 8Hz, $C_3$—H),

5.68 (d, J=7Hz, —CH—),

9.27, 9.30 (1:1, d, J=8Hz, NH), 9.72 (d, J=Hz, NH).

(B) 0.600 g of 3 - [D - 2 - (4 - ethyl - 2,3 - dioxo - 1 - piperazinocarboxamido) - 2 - thienylacetamido] - 2 - oxoazetidine, and 0.40 g of pyridine-sulphur trioxide complex are dissolved in 2 ml of DMF. The mixture is treated as described in (A) to obtain 0.402 g of sodium 3 - [D - 2 - (4 - ethyl - 2,3 - dioxo - 1 - piperazinocarboxamido) - 2 - thienylacetamido] - 2 - oxoazetidine - 1 - sulphonate.

$[\alpha]_D^{25°} - 37.8°$ (c=0.761, $H_2O$).
NMR($d_6$-DMSO, ppm); 9.30 (d, J=8Hz, $C_3$-CONH—), other signals agreeing with those of the product obtained in (A).

(C) 0.473 g of 3 - [L - 2 - (4 - ethyl - 2,3 - dioxo - 1 - piperazinocarboxamido) - 2 - thienylacetamido] -2 - oxoazetidine and 0.315 g of pyridine-sulphur trioxide complex are dissolved in 2 ml of DMF. The mixture is treated as described in (A) to obtain 0.206 g of sodium 3 - [L - 2 - (4 - ethyl - 2,3 - dioxo - 1 - piperazinocarboxamido) - 2 - thienyl - acetamido] - 2 - oxoazetidine - 1 - sulphonate.

$[\alpha]_D^{25°} + 42.4°$ (c=0.321, $H_2O$).
NMR($d_6$-DMSO, ppm); 9.27 (d, J=8Hz, NH), other signals agreeing with those of the product obtained in (A).

Example 50

0.290 g of 3 - (2 - thienyl - 2 - methoxyiminoacetamido) - 2 - oxoazetidine and 0.437 g of pyridine-sulphur trioxide complex are dissolved in 2 ml of DMF. The mixture is stirred for 19 hours, and diethyl ether is added. The oily precipitate is washed with methanol to obtain 0.310 g of pyridinium 3 - (2 - thienyl - 2 - methoxyiminoacetamido) - 2 - oxoazetidine - 1 - sulphonate.

IR$\nu_{max}^{KBr}$cm$^{-1}$; 1758, 1643, 1523, 1280, 1223, 1045.
NMR($d_6$-DMSO, ppm); 3.48 (dd, J=3, 6Hz, $C_4$—H), 3.65 (t, J=6Hz, $C_4$—H), 4.07 (s, $CH_3$), 4.95 (ddd, J=3, 6, 8Hz, $C_3$—H).

Example 51

0.300 g of 3-[2-thienyl-2-(3-morpholinopropoxyimino)acetamido]-2-oxoazetidine and 0.217 g of pyridine-sulphur trioxide complex are dissolved in 1.5 ml of DMF. The mixture is stirred for 2 days, after which it is treated as described in Example 5. The above procedure provides 0.313 g of sodium 3 - [2-thienyl - 2 -(3 - morpholinopropoxyimino)acetamido] - 2 - oxoazetidine - 1 - sulphonate.

IR$\nu_{max}^{KBr}$cm$^{-1}$; 1760, 1665, 1540, 1260, 1040.
NMR($d_6$-DMSO, ppm); 3.0—4.0 (m), 4.96 (ddd, J=3, 6, 8Hz, $C_3$—H), 7.05—7.45, 7.6—7.9 (m, aromatic H), 9.48 (d, J=8Hz, NH).

Example 52

0.223 g of 3 - [D - 2 -[DL - 2 - (4 - ethyl - 2,3 - dioxo - 1 - piperazinocarboxamido) - 2 - thienylacetamido] - 2 - phenylacetamido] - 2 - oxoazetidine and 0.141 g of pyridine-sulphur trioxide complex are dissolved in 0.5 ml of DMF. The mixture is stirred for 30 hours, after which it is treated as described in Example 5. The above procedure provides 0.290 g of sodium (3 - [D - 2 - [DL - 2 - (4 - ethyl - 2,3 - dioxo - 1 - piperazinocarboxamido) - 2 - thienylacetamido] - 2 - phenylacetamido] - 2 - oxoazetidine - 1 - sulphonate.

IR$\nu_{max}^{KBr}$cm$^{-1}$; 1760, 1708, 1670, 1508, 1230, 1180.

Example 53

0.167 g of 3-[2-(2,5-dioxo-1,2,4-triazino-6-carboxamido)-2-thienylacetamido]-2-oxoazetidine and 0.117 g of pyridine-sulphur trioxide complex are dissolved in 1 ml of DMF. The mixture is stirred for 3 days, after which it is treated as described in Example 5. The above procedure provides 0.052 g of sodium 3 - [2 - (2,5 - dioxo - 1,2,4 - triazino - 6 - 6 - carboxamido) - 2 - thienylacetamido] - 2 - oxoazetidine - 1 - sulphonate.

IR$\nu_{max}^{KBr}$cm$^{-1}$; 1750, 1700, 1502, 1260, 1173, 1040.

Example 54

0.530 g of 3 - [2 - (4 - ethyl - 2,3 - dioxo - 1 - piperazinocarboxamido) - 2 - (2 - methyl - 4 - thiazolyl) - acetamido] - 2 - oxoazetidine and 0.413 g of pyridine-sulphur trioxide complex are dissolved in 2 ml of DMF. The mixture is stirred for 3 days, after which it is treated as described in Example 5. The above procedure provides 0.270 g of 3 - [2 - (4 - ethyl - 2,3 - dioxo - 1 - piperazinocarboxamido) - 2 - (2 - methyl - 4 - thiazolyl)acetamido] - 2 - oxoazetidine - 1 - sulphonate.

IR$\nu_{max}^{KBr}$cm$^{-1}$; 1760, 1708, 1670, 1510, 1240—1280, 1185, 1048.
NMR($d_6$-DMSO, ppm); 1.09 (t, J=Hz, $CH_3$), 2.64 (s, $CH_3$), 3.4—4.1 (m, —$CH_2$—), 3.4—3.8 (m, $C_4$—H), 3.83 (q, J=7Hz, —$CH_2$—), 4.81 (ddd, J=3, 6, 8Hz, $C_3$—H),

5.52 (d, J=7Hz, —CH—),

7.40 (s, thiazol-H), 9.05 (br. d, J=8Hz, NH), 9.75 (d, J=7Hz, NH).

Example 55

0.170 g of 3-[2-(4-chlorobenzoylureido)-2-thienylacetamido]-2-oxoazetidine and 0.133 g pyridine-sulphur trioxide complex are dissolved in 1 ml of DMF. The mixture is allowed to stand at room temperature for 18 hours, and, then, worked up in the manner as described in Example 7 to obtain 0.085 g of pyridinium 3-[2-(4-chlorobenzoylureido)-2-thienylacetamido]-2-oxoazetidine-1-sulphonate.

IR$\nu_{max}^{KBr}$cm$^{-1}$; 1760, 1682, 1640, 1530, 1260, 1230, 1040.
NMR($d_6$-DMSO, ppm); 3.26 (dd, J=3, 6Hz, $C_4$—H), 3.63 (t, J=6Hz, $C_4$—H), 4.85 (ddd, J=3, 6, 8Hz, $C_3$—H),

5.72 (d, J=7Hz, —CH—).

Example 56

0.500 g of 3-cyanomethylthioacetamido-3-methoxy-2-oxoazetidine is added to 3 ml of DMF, followed by addition of 0.694 g of pyridine-sulphur trioxide complex. The mixture is stirred for 21 hours and, then, worked up in the manner as described in Example 3. The above procedure provides 0.216 g of sodium 3-cyanomethylthioacetamido-3-methoxy-2-oxoazetidine-1-sulphonate.

IR$\nu_{max}^{KBr}$cm$^{-1}$; 2250, 1760, 1650, 1600, 1250, 1050.

Example 57

0.450 g of 3-(2-benzyloxycarbonyl-2-phenylacetamido)-3-methoxy-2-oxoazetidine and 0.292 g of pyridine-sulphur trioxide complex are dissolved in 1 ml of DMF. The mixture is treated as described in Example 5 to obtain 0.286 g of sodium 3-(2-benzyloxy-carbonyl-2-phenylacetamido)-3-methoxy-2-oxoazetidine-1-sulphonate.

IR$\nu_{max}^{KBr}$cm$^{-1}$; 1760, 1680, 1250, 1055.
NMR($d_6$-DMSO, ppm); 3.54 (s, $CH_3$), 3.55, 3.75 (dd, J=6Hz, $C_4$—H),

4.96 (s, —CH—),

5.13 (s, —$CH_2$—), 7.30 (s, aromatic H), 9.61, 9.65 (each s, NH).

0.140 g of the above product is dissolved in 10 ml of water and, after the addition of 0.150 g of palladium black, the mixture is stirred in a hydrogen gas stream for 25 minutes. The catalyst is filtered off and the filtrate is freeze-dried to obtain 0.105 g of sodium 3-(2-carboxy-2-phenylacetamido)-3-methoxy-2-oxoazetidine-1-sulphonate.

IR$\nu_{max}^{KBr}$cm$^{-1}$; 1760, 1720, 1680, 1523, 1240, 1050.
NMR($d_6$-DMSO, ppm); 3.50 (s, $CH_3$), 3.50, 3.71 (dd, J=6Hz, $C_4$—H),

4.97 (s, —CH—),

7.31 (s, aromatic H), 9.50 (s, NH).

Example 58

0.450 g of 3-[2-(5,6-dihydro-1,4-oxathiin-2-yl)acetamido]-2-oxoazetidine and 0.636 g of pyridine-sulphur trioxide complex are dissolved in 3 ml of DMF. The mixture is allowed to stand at room temperature for 2 days, and treated as described in Example 5 to obtain 0.252 g of sodium 3-[2-(5,6-dihydro-1,4-oxathiin-2-yl)acetamido]-2-oxoazetidine-1-sulphonate.

$IR\nu^{KBr}_{max}cm^{-1}$; 1760, 1660, 1538, 1240, 1050.
NMR($d_6$-DMSO, ppm); 3.28 (dd, J=3, 6Hz, $C_4$—H), 3.62 (t, J=6Hz, $C_4$—H), 4.84 (ddd, J=3, 6, 8Hz, $C_3$—H), 5.11 (s, —$CH_2$—), 8.58 (d, J=8Hz, NH).

Example 59

0.450 g of 3-(D-N-carbamoyltryptophyl-D-phenylglycylamino)-2-oxoazetidine and 0.240 g of pyridine-sulphur trioxide complex are dissolved in 1 ml of DMF. The mixture is stirred for 20 hours, after which it is treated as described in Example 5. The above procedure provides 0.095 g of sodium 3-(D-N-carbamoyltryptophyl-D-phenylglycylamino)-2-oxoazetidine-1-sulphonate.

$IR\nu^{KBr}_{max}cm^{-1}$; 1755, 1660, 1520, 1230, 1042.

Example 60

0.450 g of 3 - [D - N - (4 - ethyl - 2,3 - dioxo - 1 - piperazinocarbonyl)phenylalanylamino] - 2 - oxoazetidine and 0.357 g of pyridine-sulphur trioxide complex are dissolved in 2 ml of DMF. The mixture is allowed to stand at room temperature for 2 days, and treated as described in Example 5 to obtain 0.222 g of sodium 3 - [D - N - (4 - ethyl - 2,3 - dioxo - 1 - piperazinocarbonyl) - phenylalanylamino] - 2 - oxoazetidine - 1 - sulphonate.

$IR\nu^{KBr}_{max}cm^{-1}$; 1760, 1710, 1670, 1520, 1280, 1185, 1042.
NMR($d_6$-DMSO, ppm); 1.08 (t, J=7Hz, $CH_3$), 2.93 (dd, J=3, 6Hz, $C_4$—H), 3.38 (q, J=7Hz, —$CH_2$—),

4.53 (dd, J=6, 8Hz, —CH—),
|

4.84 (ddd, J=3, 6, 8Hz, $C_3$—H), 7.22 (s, aromatic H), 8.90 (d, J=8Hz, NH), 9.16 (d, J=8Hz, NH).

Example 61

0.167 g of 3 - [2 - (2,4 - dioxopyrimidino - 5 - carboxamido) - 2 - thienylacetamido] - 2 - oxoazetidine and 0.117 g of pyridine-sulphur trioxide complex are dissolved in 1 ml of DMF. The mixture is stirred for 3 days, after which it is treated as described in Example 5. The above procedure provides 0.052 g of sodium 3 - [2 - (2,4 - dioxopyrimidino - 5 - carboxamido) - 2 - thienyl - acetamido] - 2 - oxoazetidine - 1 - sulphonate.

$IR\nu^{KBr}_{max}cm^{-1}$; 1750, 1700, 1640, 1502, 1260, 1173, 1040.

Example 62

0.510 g of 3 - [D - 2 - (2 - ureido - 2 - thienylacetamido) - 2 - (4 - hydroxyphenyl)-acetamido] - 2 - oxoazetidine and 0.285 g of pyridine-sulphur trioxide complex are dissolved in 2 ml of DMF. The mixture is stirred for 20 hours, after which it is treated as described in Example 5. The above procedure provides 0.036 g of disodium 3 - [D - 2 - (2 - ureido - 2 - thienylacetamido) - 2 - (4 - sulphonatoxyphenyl)acetamido] - 2 - oxoazetidine - 1 - sulphonate and 0.085 g of sodium 3 - [D - 2 - (2 - ureido - 2 - thienylacetamido) - 2 - (4 - hydroxyphenyl)acetamido] - 2 - oxoazetidine - 1 - sulphonate.

Disodium derivative; $IR\nu^{KBr}_{max}cm^{-1}$; 1755, 1660, 1510, 1240, 1050.
Monosodium derivative; $IR\nu^{KBr}_{max}cm^{-1}$; 1745, 1660, 1500, 1220, 1040.

Example 63

0.230 g of 3 - [$\alpha$ - D - N - (4 - ethyl - 2,3 - dioxo - 1 - piperazinocarbonyl)glutaminylamino] - 2 - oxoazetidine and 0.199 g of pyridine-sulphur trioxide complex are dissolved in 2 ml of DMF. The mixture is stirred for 4 days, after which it is treated as described in Example 5. The above procedure provides 0.015 g of sodium 3 - [$\alpha$ - D - N - (4 - ethyl - 2,3 - dioxo - 1 - piperazinocarbonyl)glutaminylamino] - 2 - oxoazetidine - 1 - sulphonate.

$IR\nu^{KBr}_{max}cm^{-1}$; 1755, 1640, 1240, 1190, 1042.

Example 64

0.391 g of 3 - [2 - (4 - ethyl - 2,3 - dioxo - 1 - piperazinocarboxamido) - 2 - (1 - cyclohexen - 1 - yl) - acetamido] - 2 - oxoazetidine and 0.32 g of pyridine-sulphur trioxide complex are dissolved in 2 ml

of DMF. The mixture is stirred at room temperature for 4 days, after which it is treated as described in Example 5. The above procedure provides 0.345 g of sodium 3 - [2 - (4 - ethyl - 2,3 - dioxo - 1 - piperazinocarboxamido) - 2 - (1 - cyclohexen - 1 - yl)acetamido] - 2 - oxoazetidine - 1 - sulphonate.

$IR\nu_{max}^{KBr}cm^{-1}$; 2920, 1760, 1710, 1670, 1510.

NMR($d_6$-DMSO, ppm); 1.10 (t, J=8Hz, $CH_3$), 1.30—1.70 (m, —$CH_2$—), 1.70—2.20 (m, —$CH_2$—), 3.40 (q, $CH_2$, J=4Hz),

4.73 (d, —CH—, J=8Hz), 6.70 ( br . s, 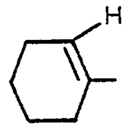 ),

8.90 (dd, J=,8,Hz, NH), 9.40 (d, J=8Hz, NH).

Example 65

0.179 g of 3 - [2 - (4 - ethyl - 2,3 - dioxo - 1 - piperazinocarboxamido) - 2 - (4 - chlorophenyl)acetamido] - 2 - oxoazetidine and 0.14 g of pyridine-sulphur trioxide complex are dissolved in 1 ml of DMF. The mixture is stirred at room temperature for 3 days, after which it is treated as described in Example 5. The above procedure provides 0.127 g of sodium 3 - [2 - (4 - ethyl - 2,3 - dioxo - 1 - piperazinocarboxamido) - 2 - (4 - chlorophenyl)acetamido] - 2 - oxoazetidine - 1 - sulphonate.

$IR\nu_{max}^{KBr}cm^{-1}$; 1760, 1710, 1670, 1510, 1250, 1192, 1043.

NMR($d_6$-DMSO, ppm); 1.06 (t, $J^s$7Hz, $CH_3$),

3.88 (m, 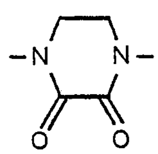 ), 4.80 (m, $C_3$—H), 5.38 (d, J=8Hz, —CH—),

7.35 (s, aromatic H), 9.20 (dd, J=8,8Hz, NH), 9.78 (d, J=8Hz, NH).

Example 66

0.3 g of 3 - [DL - 2 - (4 - ethyl - 2,3 - dioxo - 1 - piperazinocarboxamido) - 2 - (4 - trimethylsilylphenyl)acetamido - 2 - oxoazetidine and 0.21 g of pyridine-sulphur trioxide complex are dissolved in 1 ml of DMF. The mixture is stirred at room temperature for 3 days, after which it is treated as described in Example 5. The above procedure provides 0.142 g of sodium 3 - [DL - 2 - (4 - ethyl - 2,3 - dioxo - 1 - piperazinocarboxamido) - 2 - (4 - trimethylsilylphenyl)acetamido] - 2 - oxoazetidine - 1 - sulphonate.

$IR\nu_{max}^{KBr}cm^{-1}$; 1755, 1705, 1670, 1510, 1240, 1193, 1045.

NMR($d_6$-DMSO, ppm); 0.23 (s, $SiMe_3$), 1.06 (t, J=8Hz, $CH_3$), 3.85 (m, —$CH_2$—), 4.80 (m, $C_3$—H),

5.36 (d, J=8Hz, —CH—),

7.22 (d, J=7Hz, aromatic H), 7.38 (d, J=8Hz, aromatic H), 9.15, 9.20 (each d, J=8Hz, NH), 9.76 (d, J=8Hz, NH).

Example 67

0.36 g of 3 - [D - N - (4 - ethyl - 2,3 - dioxo - 1 - piperazinocarbonyl)methionyl - D - phenylglycylamino] - 2 - oxoazetidine and 0.22 g of pyridine-sulphur trioxide complex are dissolved in 3 ml of DMF. The mixture is stirred at room temperature for 5 days, after which it is treated as described in Example 5. The above procedure provides 0.3 g of sodium 3 - [D - N - (4 - ethyl - 2,3 - dioxo - 1 - piperazinocarbonyl)methionyl - D - phenylglycylamino] - 2 - oxoazetidine - 1 - sulphonate.

$IR\nu_{max}^{KBr}cm^{-1}$; 1760, 1710, 1675, 1520, 1250, 1195, 1050.

NMR($d_6$-DMSO, ppm); 1.06 (t, J=7Hz, $CH_3$), 2.04 (s, $SCH_3$), 3.12 (dd, J=3,6Hz, $C_4$—β—H), 3.54 (m, —$CH_2$—), 3.90 (m, —$CH_2$—),

4.55 (m, —CH—), 4.82 (m, $C_3$—H), 5.43 (d, J=8Hz, —CH—),

7.31 (aromatic H), 8.78 (d, J=8Hz, NH), 9.02 (d, J=8Hz, NH), 9.22 (d, J=8Hz, NH).

Example 68

0.34 g of 3 - [2 - (4 - ethyl - 2,3 - dioxo - 1 - piperazinocarboxamido) - 2 - (1 - cyclohexen - 1 - yl)acetamido] - 3 - methoxy - 2 - oxoazetidine and 0.26 g of pyridine-sulphur trioxide complex are dissolved in 3 ml of DMF. The mixture is stirred for 3 days, after which it is treated as described in Example 5. The above procedure provides 0.17 g of sodium 3 - [2 - (4 - ethyl - 2,3 - dioxo - 1 - piperazinocarboxamido) - 2 - (1 - cyclohexen - 1 - yl)acetamido] - 3 - methoxy - 2 - oxoazetidine - 1 - sulphonate.

IR$\nu_{max}^{KBr}$cm$^{-1}$; 1765, 1810, 1675, 1510, 1250, 1190, 1055.
NMR(d$_6$-DMSO, ppm); 1.07 (t, J=7Hz, $CH_3$),

1.50 (m, 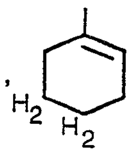 ), 1.95 (m, 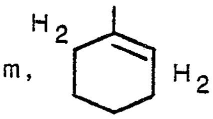 ),

3.33 (s, —$CH_3$), 3.90 (m, —$CH_2$—), 4.89 (d, J=8Hz, —CH—),

5.75 (m, 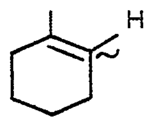 9.32 (d, J=8Hz, NH), 9.38 (s, NH).

Example 69

0.200 g of 3 - [D - 2 - (3 - methylcarbamoyl - 3 - methyl - 1 - ureido) - 2 - phenylacetamido] - 2 - oxoazetidine is dissolved in 3 ml of DMF, followed by addition of 0.144 g of pyridine-sulphur trioxide complex. The mixture is stirred for 3 days, and treated as described in Example 5. The above procedure provides 0.080 g of sodium 3 - [D - 2 - (3 - methylcarbamoyl - 3 - methyl - 1 - ureido) - 2 - phenylacetamido] - 2 - oxoazetidine - 1 - sulphonate.

IR$\nu_{max}^{KBr}$cm$^{-1}$; 3370, 1750, 1685, 1650, 1633, 1273, 1230, 1202, 1050.
NMR(d$_6$-DMSO, ppm); 2.65 (d, J=4Hz, —$CH_3$), 3.06 (s, $CH_3$), 3.10 (dd, J=3,5Hz, $C_4$—H), 3.53 (t, J=5Hz, $C_4$—H), 4.79 (m, $C_3$—H),

5.30 (d, J=7Hz, —CH—),

7.33 (s, aromatic H, NH), 9.13 (d, J=7Hz, NH), 9.90 (d, J=7Hz, NH).

Example 70

0.3 g of a diastereomeric mixture of 3 - [DL - 2(3 - methylcarbamoyl - 3 - methyl - 1 - ureido)-2 - thienylacetamido] - 2 - oxoazetidine is dissolved in 3 ml of DMF, followed by addition of 0.2 g of pyridine-sulphur trioxide complex. The mixture is stirred for 3 days and, then, worked up in the manner as described in Example 5. The above procedure provides 0.134 g of a diastereomeric mixture of sodium 3 - [DL - 2 - (3 - methylcarbamoyl - 3 - methyl - 1 - ureido) - 2 - thienylacetamido] - 2 - oxoazetidine - 1 - sulphonate.

IR$\nu_{max}^{KBr}$cm$^{-1}$; 3375, 1752, 1685, 1650, 1632, 1275, 1228, 1200, 1048.
NMR(d$_6$-DMSO, ppm); 2.64 (d, J=5Hz, $CH_3$), 3.07 (s, $CH_3$), 3.18, 3.25 (dd, J=3,6Hz, $C_4$—H), 3.57, 3.58 (t, J=6Hz, $C_4$—H), 4.80 (m, $C_3$—H),

5.58 (d, J=7Hz, —CH—),

6.87—7.50 (m, aromatic H, NH), 9.13, 9.18 (d, J=8Hz, NH), 9.83, 9.88 (d, J=7Hz, NH).

Example 71

0.19 g of 3 - [D - 2 - (3 - methylcarbamoyl - 3 - methyl - 1 - ureido) - 2 - (4 - benzyloxyphenyl) - acetamido] - 2 - oxoazetidine is dissolved in 4 ml of DMF, followed by addition of 0.11 g of pyridine-sulphur trioxide complex. The mixture is stirred for 3 days, then 30 ml of diethyl ether is added and the resultant crystals are collected and washed with ethanol (pyridinium salt). This salt is treated with Dowex 50W (Na-form) resin to obtain 0.138 g of sodium 3 - [D - 2 - (3 - methylcarbamoyl - 3 - methyl - 1 - ureido) - 2 - (4 - benzyloxyphenyl)acetamido] - 2 - oxoazetidine - 1 - sulphonate.

IR$\nu_{max}^{KBr}$cm$^{-1}$; 3380, 1755, 1685, 1640, 1245, 1051.
NMR($d_6$-DMSO, ppm); 2.63 (d, J=5Hz, $CH_3$), 3.03 (s, $CH_3$), 3.10 (dd, J=3,5Hz, $C_4$—H), 3.50 (t, J=6Hz, $C_4$—H), 4.78 (m, $C_3$—H), 5.03 (s, $CH_2$),

5.21 (d, J=7Hz, —CH—),
|

7.08 (ABq, J=9.30Hz, aromatic H), 7.37 (s, aromatic H), 9.03 (d, J=8Hz, NH), 9.78 (d, J=7Hz, NH).

43 g of the above sodium 3 - [D - 2 - (3 - methylcarbamoyl - 3 - methyl - 1 - ureido) - 2 - (4 - benzyloxyphenyl)acetamido] - 2 - oxoazetidine - 1 - sulphonate are added to 3 ml of water, followed by addition of 40 mg of palladium black. The mixture is stirred in a hydrogen gas stream for 40 minutes, and the catalyst is filtered off. The filtrate is freeze-dried to provide 35 mg of sodium 3 - [D - 2 - (3 - methylcarbamoyl - 3 - methyl - 1 - ureido) - 2 - (4 - hydroxyphenyl)acetamido] - 2 - oxoazetidine - 1 - sulphonate.

IR$\nu_{max}^{KBr}$cm$^{-1}$; 3350, 1760, 1680, 1638, 1270—1230, 1050.
NMR($d_6$-DMSO, ppm); 2.63 (d, J=5Hz, $CH_3$), 3.05 (s, $CH_3$), 3.12 (dd, J=3,5Hz, $C_4$—H), 3.51 (t, J=5Hz, $C_4$—H), 4.77 (m, $C_3$—H),

5.16 (d, J=7Hz, —CH—),
|

6.88 (ABq, J=9.40Hz, aromatic H), 7.32 (q, J=5Hz, NH), 8.98 (d, J=9Hz, NH), 9.33 (s, OH), 9.72 (d, 7Hz, NH).

Example 72

0.472 mg of 3 - [D - 2 - [3 - (2 - benzyloxybenzoyl) - 1 - ureido] - 2 - phenylacetamido] - 2 - oxoazetidine is dissolved in 4 ml of DMF, followed by addition of 0.318 g of pyridine-sulphur trioxide complex. The mixture is stirred for 3 days, after which it is treated as described in Example 5. The above procedure provides 0.13 g of sodium 3 - [D - 2 - [3 - (2 - benzyloxybenzoyl) - 1 - ureido] - 2 - phenylacetamido] - 2 - oxazetidine - 1 - sulphonate.

IR$\nu_{max}^{KBr}$cm$^{-1}$; 3310, 1760, 1705, 1668, 1290, 1240, 1050.
NMR($d_6$-DMSO, ppm); 3.11 (dd, J=3,6Hz, $C_4$—H), 3.62 (t, J=6Hz, $C_4$—H), 4.86 (m, $C_3$—H), 5.30 (s, —$CH_2$—),

5.40 (d, J=8Hz, —CH—),
|

7.04—7.88 (m, aromatic H), 9.28 (d, J=7Hz, NH), 9.45 (d, J=8Hz, NH), 10.32 (s, NH).

60 mg of the above sodium 3 - [D - 2 - [3 - (2 - benzyloxybenzoyl) - 1 - ureido] - 2 - phenylacetamido] - 2 - oxoazetidine - 1 - sulphonate are added to 4 ml of water, followed by addition of 60 mg of palladium black. The mixture is stirred in a hydrogen gas stream for 30 minutes, and the catalyst is filtered off. The filtrate is freeze-dried to provide 48.5 mg of sodium 3 - [D - 2 - [3 - (2 - hydroxybenzoyl) - 1 - ureido] - 2 - phenylacetamido] - 2 - oxoazetidine - 1 - sulphonate.

IR$\nu_{max}^{KBr}$cm$^{-1}$; 3450, 3270, 1760, 1660, 1240—1190, 1038.
NMR($d_6$-DMSO, ppm); 3.13 (dd, J=3,6Hz, $C_4$—H), 3.58 (t, J=6Hz, $C_4$—H), 4.87 (m, $C_3$—H),

5.48 (d, JJ=8Hz, —CH—),
|

6.90—8.03 (m, aromatic H), 9.28 (d, J=9Hz, NH), 9.52 (d, J=8Hz, NH), 10.52 (s, NH).

Example 73

(A) 0.3 g of 3 - [D - 2 - [3 - (2 - benzyloxybenzoyl) - 1 - ureido] - 2 - (4 - hydroxyphenyl)-acetamido] - 2 - oxoazetidine is dissolved in 4 ml of DMF, followed by addition of 0.116 g of pyridine-sulphur trioxide complex. The mixture is stirred for 3 days, after which it is treated as described in Example 5. The above procedure provides 0.021 g of disodium 3 - [D - 2 - [3 - (2 - benzyloxybenzoyl) - 1 - ureido] - 2 - (4 - sulphonatoxyphenyl)acetamido] - 2 - oxoazetidine - 1 - sulphonate.

IR$\nu_{max}^{KBr}$cm$^{-1}$; 3450—3310, 1758, 1670, 1240, 1050.
NMR($d_6$-DMSO, ppm); 3.24 dd, J=3,6Hz, $C_4$—H), 3.58 (t, J=6Hz, $C_4$—H), 4.88 (m, $C_3$—H), 5.28 (s, —$CH_2$—),

5.38 (d, 7=7Hz, —CH—),
|

7.00—7.86 (m, aromatic H), 9.21 (d, J=9Hz, NH), 9.37 (d, J=7Hz, NH), 10.28 (s, NH).

In adition, 0.059 g of sodium 3 - [D - 2 - [3 - (2 - benzyloxybenzoyl) - 1 - ureido] - 2 - (4 - hydroxyphenyl)acetamido] - 2 - oxoazetidine - 1 - sulphonate is also obtained.

IR$\nu_{max}^{KBr}$cm$^{-1}$; 3425, 3325, 1758, 1670, 1240, 1050.
NMR($d_6$-DMSO, ppm); 3.10 (dd, J=3,6Hz, $C_4$—H), 3.49 (t, J=6Hz, $C_4$—H), 4.86 (m, $C_3$—H), 5.28 (s, —$CH_2$—),

5.57 (d, J=7Hz, —CH—),
|

6.70—7.88 (m, aromatic H), 9.21 (d, J=9Hz, NH), 9.33 (s, OH), 9.37 (d, J=7Hz, NH), 10.28 (s, NH).

(B) 40 mg of the above sodium 3 - [D - 2 - [3 - (2 - benzyloxybenzoyl) - 1 - ureido] - 2 - (4 - hydroxyphenyl)acetamido] - 2 - oxoazetidine - 1 - sulphonate are added to 3 ml of water followed by addition of 40 mg of palladium black. The mixture is stirred in a hydrogen gas stream for 40 minutes, after which the catalyst is filtered off. The filtrate is freeze-dried to provide 31 mg of sodium 3 - [D - 2 - [3 - (2 - hydroxybenzoyl) - 1 - ureido] - 2 - (4 - hydroxyphenyl)acetamido] - 2 - oxoazetidine - 1 - sulphonate.

IR$\nu_{max}^{KBr}$cm$^{-1}$; 3500—3275, 1748, 1675—1640, 1260—1220, 1045.
NMR($d_6$-DMSO, ppm); 3.15 (dd, J=2,6Hz, $C_4$—H), 3.52 (t, J=6Hz, $C_4$—H), 4.88 (m, $C_3$—H),

5.48 (d, J=7Hz, —CH—),
|

6.70—8.00 (aromatic H), 9.09 (d, J=9Hz, NH), 9.48 (s, OH), 9.50 (d, J=7Hz, NH).

## Example 74

0.4 g of 3 - [2 - (3 - chloro - 4 - hydroxyphenyl) - 2 - (4 - ethyl - 2,3 - dioxo - 1 - piperazinocarboxamido) - acetamido] - 2 - oxoazetidine is dissolved in 4 ml of DMF, followed by addition of 0.175 g of pyridine-sulphur trioxide complex. The mixture is stirred for 4 days, after which it is treated as described in Example 5. The above procedure provides 0.02 g of disodium 3 - [2 - (3 - chloro - 4 - sulphonatoxyphenyl) - 2 - (4 - ethyl - 2,3 - dioxo - 1 - piperazinocarboxamido)acetamido]- 2 - oxoazetidine - 1 - sulphonate.

IR$\nu_{max}^{KBr}$cm$^{-1}$; 3290, 1750, 1708, 1670, 1230, 1040.

In addition, 0.065 g of sodium 3 - [2 - (3 - chloro - 4 - hydroxyphenyl) - 2 - (4 - ethyl - 2,3 - dioxo - 1 - piperazinocarboxamido) - 2 - oxoazetidine - 1 - sulphonate is obtained.

IR$\nu_{max}^{KBr}$cm$^{-1}$; 3420, 3280, 1755, 1703, 1688, 1280—1225, 1045.
NMR($d_6$-DMSO, ppm); 1.09 (t, J=7Hz, —$CH_3$), 3.10 (dd, J=2,6Hz, $C_4$—H), 3.40 (q, J=7Hz, —$CH_2$—), 3.44—3.68 (m, —$CH_2$—, $C_4$—H), 3.82—4.04 (m, —$CH_2$—), 4.84 (m, $C_3$—H),

5.32 (d, J=7Hz, —CH—),
|

6.91—7.40 (m, aromatic H), 9.17 (d, J=9Hz, NH),9.74 (d, J=7Hz, NH), 10.22 (s, OH).

## Example 75

0.3 g of 3 - [D - 2 - (3 - chloro - 4 - methoxyphenyl) - 2 - (4 - ethyl - 2,3 - dioxo - 1 - piperazinocarboxamido) - acetamido] - 2 - oxoazetidine is dissolved in 3 ml of DMF, followed by addition of 0.2 g of pyridine-sulphur trioxide complex. The mixture is stirred for 4 days, after which it is treated as described in Example 5. The above procedure provides 0.196 g of sodium 3 - [D - 2 - (3 - chloro - 4 - methoxyphenyl) - 2 - (4 - ethyl - 2,3 - dioxo - 1 - piperazinocarboxamido) - acetamido] - 2 - oxoazetidine - 1 - sulphonate.

IR$\nu_{max}^{KBr}$cm$^{-1}$; 3300, 1759, 1708, 1675, 1255, 1050.

NMR($d_6$-DMSO, ppm); 1.09 (t, J=7Hz, —$CH_3$), 3.09 (dd, J=3,5Hz, $C_4$—H), 3.40 (q, J=7Hz, —$CH_3$), 3.44—3.70 (m, —$CH_2$—, $C_4$—H), 3.76—4.04 (m, —$CH_2$—), 3.86 (s, —$CH_3$), 4.83 (m, $C_3$—H),

5.36 (d, J=7Hz, —CH—),

7.10—7.50 (m, aromatic H), 9.21 (d, J=9Hz, NH), 9.79 (d, J=7Hz, NH).

## Example 76

0.35 g of a diastereoisomeric mixture of 3 - [D - 2 - (2 - benzyloxycarboxamido - 3 - N - methylcarbamoylpropionamido) - 2 - phenylacetamido] - 2 - oxoazetidine is dissolved in 4 ml of DMF, followed by addition of 0.231 g of pyridine-sulphur trioxide complex. The reaction mixture is stirred for 3 days, after which diethyl ether is added, whereupon the pyridinium salt is obtained as crystals. These crystals are treated in the manner as described in Example 71. The above procedure provides 0.37 g of a diastereoisomeric mixture of sodium 3 - [D - 2 - (2 - benzyloxycarboxamido - 3 - N - methylcarbamoylpropionamido) - 2 - phenylacetamido] - 2 - oxoazetidine - 1 - sulphonate.

IR$\nu_{max}^{KBr}$cm$^{-1}$; 3280, 1755, 1692, 1641, 1260—1230, 1048.

NMR($d_6$-DMSO, ppm); 2.40—2.70 (m, —$CH_2$—, —$CH_3$), 3.30 (dd, J=2,6Hz, $C_4$—H), 3.60 (t, 6Hz, $C_4$—H),

4.46 (m, —CH—),

4.86 (m, $C_3$—H), 5.03, 5.06 (s, —$CH_2$—),

5.38 (d, J=8Hz, —CH—),

7.34 (s, aroamtic H), 7.35 (s, aromatic H), 7.75 (m, NH), 8.30 (m, NH), 9.06 (m, NH).

NMR($d_6$-DMSO+$D_2O$, ppm); 2.40—2.70 (m, —$CH_2$—, $CH_3$), 3.28 (dd, J=2,6Hz, $C_4$—H), 3.62 (t, J=6Hz, $C_4$—H),

4.46 (m, —CH—),

4.86 (m, $C_3$—H), 5.04, 5.08 (s, —$CH_2$—), 5.37,

5.39 (s, —CH—),

7.35, 7.36 (s, aromatic H).

102 mg of a diastereoisomeric mixture of the above sodium 3 - [D - 2 - (2 - benzyloxycarbamido - 3 - N - methylcarbamoylpropionamido) - 2 - phenylacetamido] - 2 - oxoazetidine - 1 - sulphonate are added to 5 ml of water, followed by addition of 60 mg of palladium black. The mixture is stirred in a hydrogen gas stream for 35 minutes, after which the catalyst is filtered off. The filtrate is freeze-dried whereupon 69 mg of sodium 3 - [D - 2 - (2 - amino - 3 - N - methylcarbamoylpropionamido) - 2 - phenylacetamido] - 2 - oxoazetidine - 1 - sulphonate are obtained.

IR$\nu_{max}^{KBr}$cm$^{-1}$; 3500—3300, 3290, 1765, 1650, 1270, 1235, 1050.

NMR($d_6$-DMSO+$D_2O$, ppm); 2.40—2.70 (m, —$CH_2$—), 2.59 (s, —$CH_3$), 3.26 (dd, J=3,6Hz, $C_4$—H), 4.84 (m, $C_3$—H),

5.38 (s, —CH—),

7.38 (s, aromatic H).

## Example 77

0.24 g of a diastereoisomeric mixture of 3 - [D - 2 - (3 - benzyloxycarboxamido - 3 - N - methylcarbamoyl - propionamido) - 2 - phenylacetamido] - 2 - oxazetidine is dissolved in 3 ml of DMF, followed by addition of 0.16 g of pyridine-sulphur trioxide complex. The mixture is stirred for 4 days, after which it is treated as described in Example 5.

The above procedure provides 0.08 g of a diastereomeric mixture of sodium 3 - [D - 2 - benzyloxycarboxamido - 3 - N - methylcarbamoylpropionamido) - phenylacetamido] - 2 - oxoazetidine - 1 - sulphonate.

$IR\nu_{max}^{KBr}cm^{-1}$; 3275, 1753, 1682, 1650, 1633, 1235, 1043.

NMR($d_6$-DMSO, ppm): 2.44—2.66 (m, $-CH_2-$, $-CH_3$), 3.24 (m, $C_4$—H), 3.54 (m, $C_4$—H),

4.30 (m, —CH—),

4.83 (m, $C_3$—H), 5.00 and 5.02 (s, $-CH_2$),

5.43 (d, J=7Hz, —CH—),

7.36 (s, aromatic H), 7.76 (m, NH), 8.46 (m, NH), 9.06 (m, NH).

NMR($d_6$-DMSO+$D_2O$, ppm); 2.47—2.70 (m, $-CH_2-$, $-CH_3$), 3.28 (m, $C_4$—H), 3.58 (m, $C_4$—H),

4.30 (m, —CH—),

4.82 (m, $C_3$—H), 5.01, 5.04 (s, $-CH_2-$),

5.40 (s, —CH—),

7.36 (s, aromatic H).

60 mg of a diastereoisomeric mixture of the above sodium 3 - [D - 2 - (3 - benzyloxycarboxamido - 3 - N - methylcarbamoylpropionamido) - 2 - phenylacetamido] - 2 - oxoazetidine - 1 - sulphonate are dissolved in 3 ml of water, followed by addition of 40 mg of palladium black. The mixture is stirred in a hydrogen gas stream for 40 minutes, after which the catalyst is filtered off. The filtrate is freeze-dried, whereupon 41 mg of a diastereoisomeric mixture of sodium 3 - [D - 2 - (3 - amino - 3 - N - methylcarbamoylpropionamido) - 2 - phenylacetamido] - 2 - oxoazetidine - 1 - sulphonate is obtained.

$IR\nu_{max}^{KBr}cm^{-1}$; 3500—3300, 1762, 1655, 1270, 1240, 1050.

NMR($d_6$-DMSO+$D_2O$, ppm); 2.42—2.60 (m, $-CH_2-$), 2.61 and 2.62 (s, $-CH_3$), 3.25 (dd, J=2,5Hz, $C_4$—H), 3.62 (t, J=5Hz, $C_4$—H), 4.83 (m, $C_3$—H), 5.37,

5.41 (s, —CH—),

7.38 (s, aromatic H).

Example 78

1.3 g of 3-[2-(2,5-dioxopyrrolidin-3-yl)acetamido]-2-oxoazetidine is dissolved in 13 ml of DMF, followed by addition of 2.4 g of pyridine-sulphur trioxide complex. The mixture is stirred at room temperature for 3 days, after which it is treated as described in Example 5. The above procedure provides 0.81 g of sodium 3-[2-(2,5-dioxopyrrolidin-3-yl)acetamido]-2-oxoazetidine-1-sulphonate.

$IR\nu_{max}^{KBr}cm^{-1}$; 1760, 1710, 1670, 1540, 1250, 1065.

NMR($D_2O$ ppm); 2.5—3.55 (m, $-CH_2-$), 3.81 (dd, J=3,6Hz, $C_4$—H), 4.05 (J=6Hz, $C_4$—H), 5.04 (dd, J=3,6Hz, $C_3$—H).

Example 79

0.57 g of 3-(2-succinimidoacetamido)-2-oxoazetidine is dissolved in 5 ml of DMF, followed by addition of 1.1 g of pyridine-sulphur trioxide complex. The mixture is stirred for 4 days, after which it is treated as described in Example 5. The above procedure provides 0.259 g of sodium 3-(2-succinimidoacetamido)-2-oxoazetidine-1-sulphonate.

$IR\nu_{max}^{KBr}cm^{-1}$; 1765, 1710, 1260, 1055.

NMR($D_2O$, ppm); 3.01 (s, $-CH_2CH_2-$), 3.83 (dd, J=4,6Hz, $C_4$—H), 4.06 (t, J=6Hz, $C_4$—H), 4.43 (s, $-CH_2-$), 5.08 (dd, J=4,6Hz, $C_3$—H).

Example 80

2.5 g of pyridine-sulphur trioxide complex are added to a solution of 1.63 g of 3 - [2 - (2 - carbobenzoxyaminomethylphenyl)acetamido] - 2 - oxoazetidine in 10 ml of DMF. The reaction mixture is

stirred for 5 days, and treated as described in Example 5 to obtain 0.815 g of sodium 3 - [2 - (2 - carbobenzoxyaminomethylphenyl)acetamido] - 2 - oxoazetidine - 1 - sulphonate.

IR$\nu_{max}^{KBr}$cm$^{-1}$; 1765, 1695, 1685, 1530, 1260, 1055.

NMR($D_2O$, ppm); 3.70 (dd, J=3.5,6Hz, $C_4$—H), 3.78 (s, —$CH_2$—), 3.88 (t, J=3.5,6Hz, $C_4$—H), 4.37 (s, —$CH_2$—), 4.87 (dd, J=3.5, 6Hz, $C_4$—H), 5.19 (s, —$CH_2$—), 7.42 (s, aromatic H), 7.50 (s, aromatic H).

0.30 g of the above sodium 3 - [2 - (2 - carbobenzoxyaminomethylphenyl)acetamido] - 2 - oxoazetidine - 1 - sulphonate is dissolved in 35 ml of 30 ml of 30% methanol, followed by addition of 0.68 ml of 6% acetic acid and 76 mg of palladium black. The mixture is stirred in a hydrogen gas stream at room temperature for 90 minutes. The catalyst is filtered off and the filtrate is purified on an Amberlite XAD—II column. The above procedure provides 0.167 g of sodium 3 - [2 - (2 - aminomethylphenyl)acetamido] - 2 - oxoazetidine - 1 - sulphonate acetate.

IR$\nu_{max}^{KBr}$cm$^{-1}$; 1760, 1645, 1530, 1240, 1045.

NMR($D_2O$, ppm); 2.01 (s, $CH_3$), 3.69 (dd, J=3.5, 6Hz, $C_4$—H), 3.74 (s, —$CH_2$—), 3.97 (t, J=6Hz, $C_4$—H), 4.41 (s, —$CH_2$—), 4.98 (dd, J=3.5, 6Hz, $C_3$—H), 7.8 (s, aromatic H).

Example 81

1.01 g of pyridine-sulphur trioxide complex is added to a solution of 0.50 g of 3 - (2 - methoxyimino - 2 - furylacetamido) - 2 - oxoazetidine in 2 ml of DMF. The reaction mixture is stirred for 2 days and treated as described in Example 5 to obtain 0.108 g of sodium 3 -(2 - methoxyimino - 2 - furylacetamido) - 2 - oxoazetidine - 1 - sulphonate.

IR$\nu_{max}^{KBr}$cm$^{-1}$; 1770, 1700, 1685, 1250, 1055.

NMR($D_2O$, ppm); 3.91 (dd, J=3.5, 6Hz, $C_4$—H), 4.13 (t, J=6Hz, $C_4$—H), 4.10 (s, $CH_3$—), 5.22 (dd, J=3.5, 6Hz, $C_3$—H), 6.72,

6.94 (m, 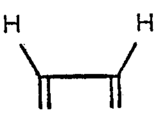 ), 7.79 (m, 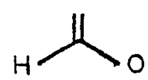 ).

Example 82

0.35 g of 3 - [2 - (2 - N - trichloroacetylureidomethylphenyl)acetamido] - 2 - oxazetidine is dissolved in 4 ml of DMF, followed by addition of 0.70 g of pyridine-sulphur trioxide complex. The reaction mixture is stirred at room temperature for 4 days and, then, worked up in the manner of Example 5. The resultant sodium 3 - [2 - (2 - N - trichloroacetylureidomethylphenyl)acetamido] - 2 - oxoazetidine - 1 - sulphonate is dissolved in 20 ml of water, and the solution is adjusted to pH 7.5 with sodium hydrogen carbonate and stirred at room temperature for 2 hours. The solution is adjusted to pH 6 and, then, purified on an Amberlite XAD—II column. The above procedure provides 87 mg of sodium 3 - [2 - (2 - ureidomethylphenyl)acetamido] - 2 - oxoazetidine - 1 - sulphonate.

IR$\nu_{max}^{KBr}$cm$^{-1}$; 1760, 1640, 1245, 1150.

Example 83

0.50 g of 3 - [2 - (3,5 - dichloro - 4 - pyridon - 1 - yl)acetamido] - 2 - oxoazetidine is dissolved in 4 ml of DMF, followed by addition of 0.85 g of pyridine- sulphur trioxide complex. The mixture is stirred at room temperature for 3 days and, then, worked up in the manner as described in Example 5. The above procedure provides 0.265 g of sodium 3 - [2 - (3,5 - dichloro - 4 - pyridon - 1 - yl)acetamido] - 2 - oxoazetidine - 1 - sulphonate.

IR$\nu_{max}^{KBr}$cm$^{-1}$; 1760, 1680, 1635, 1580, 1250, 1220, 1055.

NMR($D_2O$, ppm); 3.85 (dd, J=4, 6Hz, $C_4$—H), 4.07 (t, J=6Hz, $C_4$—H), 5.05 (dd, J=4, 6Hz, $C_3$—H), 5.07 (s, —$CH_2$—),

8.28 (s, 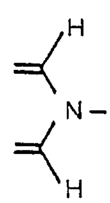 ).

Example 84

0.676 g of 3-(2-phenyl-2-benzyloxycarbonylacetamido)-2-oxozetidine is dissolved in 3 ml of

DMF, followed by addition of 0.955 g of pyridine-sulphur trioxide complex. Themixture is stirred at room temperature for 4 days and, then, worked up in the manner as described in Example 5. The above procedure provides 0.117 g of sodium 3-(2-phenyl-2-benzyloxycarbonylacetamido)-2-oxoazetidine-1-sulphonate.

$IR\nu_{max}^{KBr}cm^{-1}$; 1765, 1250, 1200, 1060.

NMR($D_2O$, ppm); 3.52 (dd, J=3.5, 5.5Hz, $C_4$—H), 3.75 (t, J=5.5Hz, $C_4$—H), 4.75 (dd, J=3.5, 5.5Hz, $C_3$—H),

4.77 (s, —CH—),
|

5.03 (s, —$CH_2$—), 7.16 (s, aromatic H), 7.26 (s, aromatic H).

Example 85

0.180 g of 3 - [2 - (N - carbobenzoxyprolylamino) - 2 - furylacetamido] - 2 - oxoazetidine is dissolved in 3 ml of DMF, followed by addition of 0.19 g of pyridine-sulphur trioxide complex. The mixture is stirred for 2 days and, then, worked up in the manner as described in Example 5. The above procedure provides 46 mg of sodium 3 - [2 - (N - carbobenzoxyprolylamino) - 2 - furylacetamido] - 2 - oxoazetidine - 1 - sulphonate.

$IR\nu_{max}^{KBr}cm^{-1}$; 1775, 1700, 1685, 1250, 1055.

Example 86

0.45 g of 3 - [2 - (1 - acetyl - 2,4 - dioxoimidazolidin - 3 - yl)acetamido] - 2 - oxoazetidine is dissolved in 3 ml of DMF, followed by addition of 1.0 g of pyridine-sulphur trioxide complex. The mixture is stirred for 2 days and, then, worked up in the same manner as Example 5. The above procedure provides 0.38 g of sodium 3 - [2 - (1 - acetyl - 2,4 - dioxoimidazolidin - 3 - yl) - acetamido] - 2 - oxoazetidine - 1 - sulphonate.

$IR\nu_{max}^{KBr}cm^{-1}$; 1735, 1260, 1155.

NMR($D_2O$, ppm); 2.66 (s, $CH_3$—), 3.82 (dd, J=3.5, 6Hz, $C_4$—H), 4.04 (t, J=6Hz, $C_4$—H), 4.50 (s, —$CH_2$—), 4.59 (s, —$CH_2$—), 5.08 (dd, J=3.5, 6Hz, $C_3$—H).

Example 87

0.30 g of 3 - [2 - (2 - oxoimidazolidin - 1 - yl)acetamido] - 2 - oxoazetidine is dissolved in 2 ml of DMF, followed by addition of 0.45 g of pyridine-sulphur trioxide complex. The mixture is reacted for 2 days and, then worked up in the manner as described in Example 5. The above procedure provides 65 mg of sodium 3 - [2 - (2 - oxoimidazolidin - 1 - yl)acetamido] - 2 - oxoazetidine - 1 - sulphonate.

$IR\nu_{max}^{KBr}cm^{-1}$; 1740, 1695, 1285, 1260, 1165.

NMR($D_2O$, ppm); 3.45—4.3 (m, —$CH_2$—, $C_4$—H), 5.02 (dd, J=3.5, 6Hz, $C_3$—H).

Example 88

0.30 g of the sodium 3 - [2 - (2 - carbobenzoxyaminomethylphenyl)acetamido] - 2 - oxoazetidine - 1 - sulphonate obtained in Example 80 is dissolved in 70 ml of 30% methanol, followed by addition of 0.68 ml of 6% acetic acid and 75 mg of palladium black. The mixture is stirred in a hydrogen gas stream at room temperature for 90 minutes, after which the catalyst is filtered off. The filtrate is concentrated under reduced pressure, followed by addition of 30 ml of tetrahydrofuran. Then, under ice-cooling, 0.15 g of 1-chlorocarbonylimidazolid-2-one is added to the above tetrahydrofuran solution. The mixture is stirred for 90 minutes (its pH being maintained at 8.5 with a 1% aqueous solution of sodium hydrogen carbonate). The reaction mixture is adjusted to pH 6.5 with phosphoric acid, and the tetrahydrofuran is distilled off under reduced pressure. The residue is purified on an Amberlite XAD—II column to provide 0.24 g of sodium 3 - [2 - [2 - (2 - oxoimidazolidin - 1 - yl) - carbonylaminomethylphenyl]acetamido] - 2 - oxoazetidine - 1 - sulphonate.

$IR\nu_{max}^{KBr}cm^{-1}$; 1770, 1740, 1675, 1280, 1060.

Example 89

The sodium 3 - [2 - (2 - carbobenzoxyaminomethylphenyl)acetamido] - 2 - oxoazetidine - 1 - sulphonate (0.30 g) obtained in Example 80 is treated in the manner as described in Example 88 except that 0.15 g of 1-chlorocarbonyl-3-benzylideneaminoimidazolid-2-one is used instead of 1-chlorocarbonylimidazolid-2-one. The procedure provides 0.21 g of sodium 3 - [2 - [2 - (2 - oxo - 3 - benzylideneaminoimidazolidin - 1 - yl)carboxyaminomethylphenylacetamido] - 2 - oxoazetidine - 1 - sulphonate.

$IR\nu_{max}^{KBr}cm^{-1}$; 1765, 1735, 1680, 1280, 1250, 1055.

Example 90

0.80 g of 3 - [2 - (4 - ethyl - 2,3 - dioxo - 1 - piperazinocarboxamido) - 2 - furylacetamido] - 2 - oxoazetidine is dissolved in 4 ml DMF, followed by addition of 1.35 g of pyridine-sulphur trioxide complex. The mixture is stirred for 2 days and, then, worked up in the manner as described in Example 5. The above procedure provides 0.20 g of sodium 3 - [2 - (4 - ethyl - 2,3 - dioxo - 1. - piperazinocarboxamido) - 2 - furylacetamido] - 2 - oxoazetidine - 1 - sulphonate.

NMR($D_2O$, ppm); 1.30 (t, J=7Hz, —$CH_3$), 3.61 (q, J=7Hz, —$CH_2$—), 3.65—4.3 (m, $C_4$—H, —$CH_2$—), 5.07 (dd, J=3.5, 5.5Hz, $C_3$—H),

5.74 (s, —CH—),

6.5—6.7 (m, 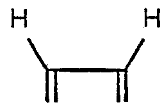 ), 7.67 (m, 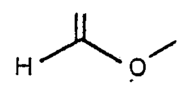 ).

Example 91

1.0 g of 3-[D-N-(thienylmethylcarbonyl)alanylamino-2-oxoazetidine is dissolved in 5 ml of DMF, followed by addition of 1.7 g of pyridine-sulphur trioxide complex. The mixture is stirred for 2 days and, then, worked up in the manner as described in Example 5. The above procedure provides 0.325 g of sodium 3 - [D - N - (thienylmethylcarbonyl) - alanylamino] - 2 - oxoazetidine - 1 - sulphonate.

$IR\nu_{max}^{KBr}cm^{-1}$; 1763, 1665, 1247, 1055.

NMR($D_2O$, ppm); 1.48 (d, J=7.5Hz, —$CH_3$), 3,74 (dd, 3.5, 6Hz, $C_4$—H), 3.96 (s, —$CH_2$—), 4.00 (t, J=6Hz, $C_4$—H),

4.47 (q, J=7.5Hz, —CH—),

5.02 (dd, J=3.5, 6Hz, $C_3$—H), 7.13 (m, 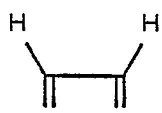 ), 7.48 (m, 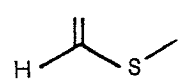 ).

Example 92

0.33 g of 3-(N-carbobenzoxy-D-alaninamido)-3-methoxy-2-oxoazetidine is dissolved in 2 ml of DMF, followed by addition of 0.32 g of pyridine-sulphur trioxide complex. The mixture is stirred at room temperature for 2 days and, then, worked up in the manner as described in Example 5. The above procedure provides 52 mg of sodium 3 - (N - carbobenzoxy - D - alaninamido) - 3 - methoxy - 2 - oxoazetidine - 1 - sulphonate.

$IR\nu_{max}^{KBr}cm^{-1}$; 1763, 1685, 1515, 1245, 1052.

NMR(DMSO-$d_6$, ppm); 1.22 (d, J=7Hz, $CH_3$), 3.30 (s, $CH_3$), 3.54, 3.70 (dd, J=7Hz, —$CH_2$—),

4.26 (m, J=7Hz, —CH—),

5.03 (s, —$CH_2$—), 7.36 (s, aromatic H), 9.12 (d, J=7Hz).

Example 93

0.393 g of 3 - [N - (4 - ethyl - 2,3 - dioxo - 1 - piperazinocarbonyl) - D - alaninamido] - 3 - methoxy - 2 - oxoazetidine is dissolved in 2 ml of DMF, followed by addition of 0.350 g of pyridine-sulphur trioxide complex. The mixture is stirred at room temperature for 4 days and, then, worked up in the manner as described in Example 5. The above procedure provides 45.5 mg of sodium 3 - [N - (4 - ethyl - 2,3 - dioxo - 1 - piperazinocarbonyl) - D -alaninamido] - 3 - methoxy - 2 - oxoazetidine - 1 - sulphonate.

$IR\nu_{max}^{KBr}cm^{-1}$; 1765, 1705, 1675, 1510, 1250, 1200, 1050.

NMR(DMSO-$d_6$, ppm); 1.10 (t, J=7Hz, $CH_3$), 1.32 (d, J=7Hz, $CH_3$), 3.55 (s, $CH_3$), 3.43 (q, J=7Hz, —$CH_2$—), 3.60 (m, —$CH_2$—), 3.90 (m, —$CH_2$—),

4.47 (m, —CH—),

9.23 (d, J=7Hz, NH), 9.40, 9.44 (each s, NH).

Example 94

516.5 mg of 3 - [N - (N - carbobenzoxy - D - phenylglycyl) - D - phenylglycinamido] - 3 - methoxy - 2 - oxoazetidine is dissolved in 2 ml of DMF, followed by addition of 320 mg of pyridine-sulphur trioxide complex. The mixture is stirred at room temperature for 4 days and, then, worked up in the manner as described in Example 5. The above procedure provides 145 mg of sodium 3 - [(N - carbobenzoxy - D - phenylglycyl) - D - phenylglycinamido] - 3 - methoxy - 2 - oxoazetidine - 1 - sulphonate.

$IR\nu_{max}^{KBr}cm^{-1}$; 1760, 1705, 1680, 1505, 1240, 1045.

NMR($DMSO-d_6+D_2O$, ppm); 3.08, 3.26 (each s, $CH_3$), 3.40 (m, —$CH_2$—), 5.06 (s, —$CH_2$—),

5.45 (s, —CH—), 5.59 (s, —CH—),

7.2—7.55 (m, aromatic H).

Example 95

0.300 g of 3 - [N - (4 - ethyl - 2,3 - dioxo - 1 - piperazinocarbonyl) - 3 - methioninamido] - 2 - oxoazetidine is dissolved in 1.5 ml of DMF, followed by addition of 0.250 g of pyridine-sulphur trioxide complex. The mixture is stirred at room temperature for 4 days and, then, worked up in the manner as described in Example 5. The above procedure provides 0.168 g of sodium 3 - [N - (4 - ethyl - 2,3 - dioxo - 1 - piperazinocarbonyl) - D - methioninamido] - 2 - oxoazetidine - 1 - sulphonate.

$IR\nu_{max}^{KBr}cm^{-1}$; 1760, 1705, 1670, 1520, 1245, 1190, 1050.

NMR($DMSO-d_6$, ppm); 1.10 (t, J=7Hz, $CH_3$), 1.96 (m, —$CH_2$—), 2.06 (s, $CH_3$), 2.44 (t, J=7Hz, —$CH_2$), 3.42 (q, J=7Hz, —$CH_2$—), 3.58 (m, —$CH_2$—), 3.92 (m, —$CH_2$—),

4.42(m, —CH—),

4,84 (m, $C_3$—H), 8.92 (d, J=7Hz, NH), 9.22 (d, J=7Hz, NH).

Example 96

0.186 g of 3 - [2 - D - [4 - (2 - phenethyl) - 2,3 - dioxo - 1 - piperazinocarboxamido] - 2 - phenylacetamido] - 2 - oxoazetidine is dissolved in 2 ml of DMF, followed by addition of 0.128 g of pyridine-sulphur trioxide complex. The mixture is stirred for 1 day and, then, worked up in the manner as described in Example 5. The above procedure provides 0.180 g of sodium 3 - [2 - D - [4 - (2 - phenethyl) - 2,3 - dioxo - 1 - piperazinocarboxamido] - 2 - phenylacetamido] - 2 - oxoazetidine - 1 - sulphonate.

$IR\nu_{max}^{KBr}cm^{-1}$; 3280, 1755, 1705, 1670, 1505, 1270—1230, 1190, 1050.

NMR($d_6$-DMSO, ppm); 2.84 (t, J=7Hz, —$CH_2$—), 3.13 (dd, J=3, 6Hz, $C_4$—H), 4.85 (ddd, J=3, 6, 8Hz, $C_4$—H),

5.44 (d, J=7Hz, —CH—),

7.2—7.5 (m, aromatic H), 9.24 (d, J=8Hz, NH), 9.78 (d, J=7Hz, NH).

Example 97

0.178 g of 3 - [2 - D - (4 - ethyl - 2,3 - dioxo - 1 - piperazinocarboxamido) - 2 - (4 - benzoyloxyphenyl)acetamido] - 2 - oxoazetidine is dissolved in 2 ml of DMF, followed by addition of 0.112 g of pyridine-sulphur trioxide complex. The mixture is stirred for 1 day and, then, worked up in the manner as described in Example 5. The above procedure provides 0.125 g of sodium 3 - [2 - D - (4 - ethyl - 2,3 - dioxo - 1 - piperazinocarboxamido) - 2 - (4 - benzoyloxyphenyl)acetamido] - 2 - oxoazetidine - 1 - sulphonate.

$IR\nu_{max}^{KBr}cm^{-1}$; 3300, 1760, 1730, 1705, 1675, 1505, 1270, 1210—1170, 1055.

NMR($d_6$-DMSO, ppm); 1.10 (t, —$CH_3$), 3.18 (dd, J=3, 6Hz, $C_4$—H), 3.62 (t, J=6Hz, $C_4$—H), 3.4—4.1 (m, —$CH_2$—), 4.90 (ddd, J=3, 6, 8Hz, $C_3$—H),

5.52 (d, J=7Hz, —CH—),

7.2—8.2 (m, aromatic H), 9.32 (d, J=8Hz, NH), 9.87 (d, J=7Hz, NH).

Example 98

0.278 g of 3 - [2 - benzyloxycarboxamido - 3 - (N-methylcarbamoyl)propionamido] - 3 - methoxy - 2 - oxoazetidine is dissolved in 3 ml of DMF, followed by addition of 0.175 g of pyridine-sulphur trioxide complex. The mixture is stirred for 2 days and, then, worked up in the manner as described in Example 5. The above procedure provides 32 mg of sodium 3 - [2 - benzyloxycarboxamido - 3 - (N - methylcarbamoyl)propionamido] - 3 - methoxy - 2 - oxoazetidine - 1 - sulphonate.

IR$\nu_{max}^{KBr}$cm$^{-1}$; 3395, 1768, 1670, 1655, 1250, 1052.
NMR(DMSO-$d_6$, ppm); 2.33—2.67 (m, $CH_2$, $CH_3$), 3.40 (s, $CH_3$), 3.50 (m, $C_4$—H),

4.37 (m, —CH—),

4.99 (s, —$CH_2$—), 7.31 (s, aromatic H), 7.70 (m, NH), 9.07 (s, NH).

Example 99

0.785 g of 3 - [2 - [(3 - furfurylideneamino - 2 - oxoimidazolidin - 1 - yl) - carboxamido] - 2 - (2 - chloroacetamido - 4 - thiazolyl)acetamido] - 2 - oxoazetidine is suspended in 10 ml of DMF, followed by addition of 0.478 g of pyridine-sulphur trioxide complex. The mixture is reacted for 1 day and, then, worked up in the manner as described in Example 5. The above procedure provides 0.465 g of sodium 3 - [2 - [(3 - furfurylideneamino - 2 - oxoimidazolidin - 1 - yl)carboxamido] - 2 - (2 - chloroacetamido - 4 - thiazolyl)acetamido] - 2 - oxoazetidine - 1 - sulphonate.

IR$\nu_{max}^{KBr}$cm$^{-1}$; 3300, 1755, 1720, 1665, 1525, 1410, 1270, 1230, 1050.
NMR($d_6$-DMSO, ppm); 3.20, 3.28 (dd, J=3, 6Hz, $C_4$—H), 3.82 (s, —$CH_2$—), 4.36 (s, —$CH_2$Cl), 4.85 (m, $C_3$—H),

5.54 (d, J=8Hz, —CH—),

6.5—7.9 (m, furyl—H), 7.18 ( s, 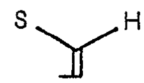 ),

7.75 (s, —CH=N—), 8.96 (d, J=8Hz, NH), 9.01, 9.05 (d, J=8Hz, NH), 12.66 (broad s, NH).

0.25 g of the above sodium 3 - [2 - [(3 - furfurylideneamino - 2 - oxoimidazolidin - 1 - yl)-carboxamido] - 2 - (2 - chloroacetamido - 4 - thiazolyl)acetamido] - 2 - oxoazetidine - 1 - sulphonate is dissolved in 5 ml of water, followed by addition of 0.057 g of sodium N-methyldithiocarbamate under stirring and ice-cooling. The solution is stirred for 75 minutes. The insoluble matter is filtered off and the filtrate is purified by an Amberlite XAD—II column. The above procedure provides 0.089 g of sodium 3 - [2 - [(3 - furfurylideneamino - 2 - oxoimidazolidin - 1 - yl) - carboxamido] - 2 - (2 - amino - 4 - thiazolyl)acetamido] - 2 - oxoazetidine - 1 - sulphonate.

IR$\nu_{max}^{KBr}$cm$^{-1}$; 3310, 3200, 1760, 1725, 1655, 1515, 1415, 1270, 1230, 1050.
NMR($d_6$-DMSO, ppm); 3.61 (t, J=6Hz, $C_4$—H), 3.82 (s, —$CH_2$—), 4.84 (m, $C_3$—H),

5.29 (d, J=8Hz, —CH—), 6.48 ( s, 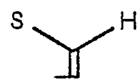 ),

6.5—7.9 (m, furyl —H), 7.74 (s, —CH=N—), 8.80 (d, J=8Hz, NH), 8.89, 8.93 (d, J=8Hz, NH).

Example 100

0.20 g of 3-[D-2-(2-phenylacetamido)propionamido]-3-methoxy-2-oxoazetidine is dissolved in 2 ml of DMF, followed by addition of 0.208 g of pyridine-sulphur trioxide complex. The mixture is stirred at room temperature for 2 days and, then, worked up in the manner as described in Example 5. The above procedure provides 20 mg of sodium 3 - [D - 2 - (2 - phenylacetamido)propionamido] - 3 - methoxy - 2 - oxoazetidine - 1 - sulphonate.

$IR\nu_{max}^{KBr}cm^{-1}$; 1765, 1645, 1520, 1240.

NMR(DMSO-$d_6$, ppm); 1.23, 12.4 (each d, J=7Hz, $CH_3$), 2.80, 3.00 (each s, —$CH_2$—), 3.47 (s, $CH_3$),

4.45 (m, —CH—),

7.29 (s, aromatic H).

Example 101

0.33 g of 3-(N-carbobenzoxy-D-alaninamido)-3-methoxy-2-oxoazetidine, which is obtained in Reference Example 15 is dissolved in 2 ml of DMF, followed by addition of 0.32 g of pyridine-sulphur trioxide complex. The mixture is stirred at room temperature for 3 days and, then, worked up in the manner as described in Example 5. The above procedure provides 16.5 mg of sodium 3 - (N - carbobenzoxy - D - alaninamido) - 3 - methoxy - 2 - oxoazetidine - 1 - sulphonate.

$IR\nu_{max}^{KBr}cm^{-1}$; 1765, 1695, 1520, 1245, 1050.

NMR(DMSO-$d_6$, ppm); 1.22 (d, J=7Hz, $CH_3$), 3.29 (s,—$CH_3$), 3.54, 3.64 (each d, J=7Hz, —$CH_2$—),

4.10 (m, —CH—),

5.03 (s, —$CH_2$—, 7.36 (s, aromatic H).

Example 102

0.447 g of 3 - [2 - [[2 - oxo - 3 - (thiophene - 2 - aldoimino) - imidazolidin - 1 - yl]carboxamido] - 2 - thienylacetamido] - 2 - oxoazetidine is dissolved in 8 ml of DMF, followed by addition of 0.319 g of pyridine-sulphur trioxide complex. The mixture is stirred for 1 day and, then, worked up in the manner as described in Example 5. The above procedure provides 0.234 g of sodium 3 - [2 - [[2 - oxo - 3 - (thiophene - 2 - aldoimino)imidazolidin - 1 - yl]carboxamido] - 2 - thienylacetamido] - 2 - oxoazetidine - 1 - sulphonate.

$IR\nu_{max}^{KBr}cm^{-1}$; 3290, 1760, 1720, 1665, 1410, 1275, 1230, 1050.

NMR($d_6$-DMSO, ppm); 3.20, 3.25 (dd, J=3, 6Hz, $C_4$—H), 3.63, 3.65 (t, J=6Hz, $C_4$—H), 3.83 (s, ring $CH_2$), 4.89 (m, $C_3$—H),

5.72 (d, J=7Hz, —CH—),

6.9—7.7 (m, thienyl—H), 8.10 (s, —CH=), 8.99 (d, J=7Hz, NH), 9.26, 9.30 (d, J=8Hz, NH).

Example 103

0.307 g of 3 - [D - 2 - [(3 - mesyl - 2 - oxoimidazolidin - 1 - yl)carboxamido] - 2 - phenylacetamido] - 2 - oxoazetidine is dissolved in 4 ml of DMF, followed by addition of 0.239 g of pyridine-sulphur trioxide complex. The mixture is stirred for 1 day and, then, worked up in the manner as described in Example 5. The above procedure provides 0.244 g of sodium 3 - [D - 2 - [(3 - mesyl - 2 - oxoimidazolidin - 1 - yl)carboxamido] - 2 - phenyl - acetamido] - 2 - oxoazetidine - 1 - sulphonate.

$IR\nu_{max}^{KBr}cm^{-1}$; 3320, 1755, 1730, 1665, 1520, 1395, 1350, 1255, 1160, 1050.

NMR($d_6$-DMSO, ppm); 3.14 (dd, J=3, 6Hz, $C_4$—H), 3.36 (s, $CH_3$—), 3.58 (t, J=6Hz, $C_4$—H), 3.79 (s, —$CH_2$—), 4.85 (ddd, J=3, 6, 8Hz, $C_3$—H),

5.43 (d, J=7Hz, —CH—),

7.39 (s, aromatic H), 8.77 (d, J=7Hz, NH), 9.25 (d, J=8Hz, NH).

Example 104

0.313 g of 3 - [2 - [(3 - mesyl - 2 - oxoimidazolidin - 1 - yl)carboxamido] - 2 - thienylacetamido] - 2 - oxoazetidine is dissolved in 4 ml of DMF, followed by addition of 0.239 g of pyridine-sulphur trioxide complex. The mixture is stirred for 3 days and, then, worked up in the manner as described in Example 5. The above procedure provides 0.19 g of sodium 3 - [2 - [(3 - mesyl - 2 - oxoimidazolidin - 1 - yl)carboxamido] - 2 - thienylacetamido] - 2 - oxoazetidine - 1 - sulphonate.

$IR\nu_{max}^{KBr}cm^{-1}$; 3320, 1760, 1725, 1670, 1520, 1395, 1355, 1250, 1165, 1050.

NMR($d_6$-DMSO, ppm); 3.18 (dd, J=3, 6Hz, $C_4$—H), 3.35 (s, $CH_3$), 3.61, 3.63 (t, J=6Hz, $C_4$—H), 4.85 (m, $C_3$—H),

5.79 (d, J=7Hz, —CH—),

6.7—7.6 (m, thienyl—H), 8.65 (d, J=7Hz, NH), 9.26, 9.30 (d, J=8Hz, NH).

Example 105

0.658 g of 3 - [D - 2 - (2,6 - dichlorophenylthioglycolamido) - 2 - phenylacetamido] - 2 - oxoazetidine is dissolved in 5 ml of DMF, followed by addition of 0.480 g of pyridine-sulphur trioxide complex. The mixture is stirred for 2 days and, then, worked up in the manner as described in Example 7. The above procedure provides 0.774 g of pyridinium 3 - [D - 2 - (2,6 - dichlorophenylthioglycolamido) - 2 - phenylacetamido] - 2 - oxoazetidine - 1 - sulphonate.

$IR\nu_{max}^{KBr}cm^{-1}$; 1760, 1659 (shoulder), 1631, 1228, 1040.

NMR(DMSO-$d_6$, ppm); 3.20 (dd, J=3, 6Hz, $C_4$—H), 3.60 (t, J=6Hz, $C_4$—H), 3.09, 3.90 (ABq, J=15Hz, —$CH_2$—), 4.86 (dd, J=3, 6Hz, $C_3$—H),

5.49 (d, J=8Hz, —CH—),

8.95 (d, J=7Hz, NH), 9.18 (d, J=8Hz, NH).

Example 106

0.172 g of 3-[(hexahydro-1H-azepin-1-yl)methylenamino]-2-oxoazetidine is dissolved in 1 ml of DMF, followed by addition of 0.183 g of pyridine-sulphur trioxide complex. The mixture is stirred at room temperature for 2 days and, then, worked up in the manner as described in Example 5. The above procedure provides 0.056 g of sodium 3 - [(hexahydro - 1H - azepin - 1 - yl)methylenamino)] - 2 - oxoazetidine - 1 - sulphonate.

$IR\nu_{max}^{KBr}cm^{-1}$; 1762, 1684, 1270, 1242, 1042.

NMR(DMSO-$d_6$, ppm); 1.4—1.9 (m, —$CH_2$—), 3.72 (t, J=6Hz, $C_4$—H), 4.90 (dd, J=3, 6Hz, $C_3$—H), 8.19 (s, —CH=).

Example 107

0.440 g of 3 - [D - 2 - (4 - ethyl - 2,3 - dioxo - 1 - piperazinocarboxamido) - 3 - phenylpropionamido] - 3 - methoxy - 2 - oxoazetidine is dissolved in 1.8 ml of DMF, followed by addition of 0.320 g of pyridine-sulphur trioxide complex. The mixture is stirred for 42 hours and, then, worked up in the manner as described in Example 5. The above procedure provides 0.152 g of sodium 3 - [D - 2 - (4 - ethyl - 2,3 - dioxo - 1 - piperazinocarboxamido) - 3 - phenylpropionamido] - 3 - methoxy - 2 - oxoazetidine - 1 - sulphonate.

$IR\nu_{max}^{KBr}cm^{-1}$; 1760, 1705, 1670, 1510, 1250, 1190, 1042.

NMR(DMSO-$d_6$, ppm); 1.09 (t, J=7Hz, —$CH_3$), 3.22, 3.30 (each s, $CH_3$),

4.70 (m, —CH—),

8.90, 9.17 (each d, J=7Hz, NH).

Example 108

0.35 g of 3-(2-dichloroacetoxyimino-2-thienylacetamido)-2-oxoazetidine is dissolved in 2 ml of DMF, followed by addition of 0.289 g of pyridine-sulphur trioxide complex. The mixture is stirred for 24 hours and, then, worked up in the manner as descrbed in Example 5. The procedure provides 0.28 g of sodium 3 - (2 - dichloroacetoxyimino - 2 - thienylacetamido) - 2 - oxoazetidine - 1 - sulphonate.

$IR\nu_{max}^{KBr}cm^{-1}$; 1760, 1660, 1613, 1530, 1240, 1190, 1150.

NMR(DMSO-$d_6$, ppm); 3.37 (dd, J=3, 6Hz, $C_4$—H), 3.68 (t, J=6Hz, $C_4$—H), 4.93 (ddd, J=3, 6, 8Hz, $C_3$—H), 7.10 (s, —$CHCl_2$).

0.25 g of the above sodium 3-(2-dichloroacetoxyimino-2-thienylacetamido)-2-oxoazetidine-1-sulphonate is dissolved in 10 ml of water and the solution is stirred at room temperature for 3 hours, the pH of the solution being maintained at 7 to 8 with sodium hydrogen carbonate. The reaction mixture is then purified on an Amberlite XAD—II column to obtain 0.1 g of sodium 3-(2-oxyimino-2-thienyl-acetamido)-2-oxoazetidine-1-sulphonate.

$IR\nu_{max}^{KBr}cm^{-1}$; 1760, 1670, 1615, 1515, 1250, 1200, 1050.
NMR(DMSO-$d_6$, ppm); 3.35 (dd, J=3, 6Hz, $C_4$—H), 3.65 (t, J=6Hz, $C_4$—H), 4.85 (ddd, J=2, 6, 8Hz, $C_3$—H).

Example 109

0.320 g of 3-(2-phenyl-2-sulphamoylacetamido)-2-oxoazetidine is dissolved in 1 ml of DMF, followed by addition of 0.336 g of pyridine-sulphur trioxide complex. The mixture is stirred at room temperature for 2 days and, then, worked up in the manner as described in Example 5. The above procedure provides 0.085 g of sodium 3-(2-phenyl-2-sulphamoylacetamido)-2-oxoazetidine-1-sulphonate.

$IR\nu_{max}^{KBr}cm^{-1}$; 1760, 1670, 1230, 1153, 1042.
NMR(DMSO-$d_6$, ppm); 3.22 (dd, J=3, 6Hz, $C_4$—H), 3.65 (t, J=6Hz, $C_4$—H), 4.85 (m, $C_3$—H),

5.04 (s, —CH—),

7.3—7.7 (m, aromatic H).

Example 110

0.197 g of 3-(2-N,N-dimethylsulphamoyl-2-phenylacetamido)-2-oxoazetidine is dissolved in 1 ml of DMF, followed by addition of 0.202 g of pyridine-sulphur trioxide complex. The mixture is stirred at room temperature overnight and, then, worked up in the manner as described in Example 5. The above procedure provides 0.125 g of sodium 3 - (2 - N,N - dimethylsulphamoyl - 2 - phenylacetamido) - 2 - oxoazetidine - 1 - sulphonate.

$IR\nu_{max}^{KBr}cm^{-1}$; 3350, 1760, 1678, 1518, 1280, 1240, 1142, 1052
NMR(DMSO-$d_6$, ppm); 2.66, 2.70 (each s, $CH_3$), 3.22 (dd, J=3, 6Hz, $C_4$—H), 3.69 T, J=6Hz, $C_4$—H), 4.87 (ddd, J=3, 6, 8Hz, $C_3$—H),

5.30 (s, —CH—),

9.19, 9.22 (each d, J=8Hz, NH), 7.3—7.8 (m, aromatic H).

Example 111

0.465 g of 3 - [2,5 - bis(4 - ethyl - 2,3 - dioxo - 1 - piperazinocarboxamido)pentanamido] - 2 - oxoazetidine is dissolved in 1.5 ml of DMF, followed by addition of 0.284 g of pyridine-sulphur trioxide complex. The mixture is stirred at room temperature for 2 days and, then, worked up in the manner as described in Example 5. The above procedure provides 0.145 g of sodium 3 - [2,5 - bis(4 - ethyl - 2,3 - dioxo - 1 - piperazinocarboxamido)pentanamido] - 2 - oxoazetidine - 1 - sulphonate.

$IR\nu_{max}^{KBr}cm^{-1}$; 1760, 1710, 1670, 1520, 1250, 1190, 1050.
NMR(DMSO-$d_6$, ppm); 1.12 (t, J=7Hz, $CH_3$), 3.27 (dd, J=3, 6Hz, $C_4$—H),

4.38 (m, —CH—),

4.86 (ddd, J=3, 6, 8Hz, $C_3$—H), 8.7—9.1 (m, NH), 9.21 (d, J=8Hz, NH).

Example 112

0.405 g of 3 - [2,5 - bis(4 - ethyl - 2,3 - dioxo - 1 - piperazinocarboxamido)pentanamido] - 3 - methoxy - 2 - oxoazetidine is dissolved in 1.5 ml of DMF, followed by addition of 0.284 g of pyridine-sulphur complex. The mixture is stirred at room temperature for 3 days and, then, worked up in the manner as described in Example 5. The above procedure provides 0.105 g of sodium 3 - [2,5 - bis(4 - ethyl - 2,3 - dioxo - 1 - piperazinocarboxamido)pentanamido] - 3 - methoxy - 2 - oxoazetidine - 1 - sulphonate.

$IR\nu_{max}^{KBr}cm^{-1}$;3300, 1760, 1710, 1670, 1520, 1250, 1190, 1050.
NMR(DMSO-$d_6$, ppm); 1.09 (t, J=7Hz, $CH_3$), 3.27 (dd, J=3, 6Hz, $C_4$—H), 3.52 (t, J=7Hz, $C_4$—H), 8.79 (m, NH), 9.20 (d, J=8Hz, NH).

Example 113

0.421 g of 3 - [D - 2 - [4 - (2 - chloroethyl) - 2,3 - dioxo - 1 - piperazinocarboxamido] - 2 - phenylacetamido] - 2 - oxoazetidine is dissolved in 5 ml of DMF, followed by addition of 0.30 g of

pyridine-sulphur trioxide complex. The mixture is stirred at room temperature for 2 days and, then, worked up in the manner as described in Example 5. The above procedure provides 0.32 g of sodium 3 - [D - 2 - [4 - (2 - chloroethyl) - 2,3 - dioxo - 1 - piperazinocarboxamido] - 2 - phenylacetamido] - 2 - oxoazetidine - 1 - sulphonate.

IR$\nu_{max}^{KBr}$cm$^{-1}$; 3450, 3280, 1760, 1705, 1665, 1510, 1255, 1190, 1050.
NMR(DMSO-$d_6$, ppm); 3.12 (dd, J=3, 6Hz, $C_4$—H), 3.40 (t, J=7Hz, —$CH_2$—), 3.80—4.00 (m, —$CH_2$—),

4.72 (m, —CH—),
|

4.92 (m, $C_3$—H), 7.2—7.5 (m, aromatic H), 9.20 (d, J=8Hz, NH), 9.80 (d, J=8Hz, NH).

Example 114

0.36 g of 3 - [D - 3 - chloro - 2 - (4 - ethyl - 2,3 - dioxo - 1 - piperazinocarboxamido)-propionamido] - 2 - oxoazetidine is dissolved in 2 ml of DMF, followed by addition of 0.32 g of pyridine-sulphur trioxide complex. The mixture is stirred at room temperature for 4 days and, then, worked up in the manner as described in Example 5. The above procedure provides 0.28 g of sodium 3 - [D - 2 - chloro - 2 - (4 - ethyl - 2,3 - dioxo - 1 - piperazinocarboxamido)propionamido] - 2 - oxoazetidine - 1 - sulphonate.

IR$\nu_{max}^{KBr}$cm$^{-1}$; 1760, 1710, 1675, 1720, 1260, 1195.
NMR(DMSO-$d_6$, ppm); 1.10 (t, J=7Hz, $CH_3$), 3.42 (q, J=7Hz, —$CH_2$—), 3.62 (m, —$CH_2$—), 3.94 (m, —$CH_2$—),

4.71 (m, —CH—),
|

4.90 (m, $C_3$—H), 9.05 (d, J=7Hz, NH), 9.42 (d, J=7Hz, NH).

Example 115

0.47 g of 3 - (2 - benzyloxycarboxamido - 2 - benzyloxycarbonylethanesulphonamido] - 2 - oxoazetidine is dissolved in 2 ml of DMF, followed by addition of 0.32 g of pyridine-sulphur trioxide complex. The mixture is stirred at room temperature for 2 days and, then, worked up in the manner as described in Example 5. The above procedure provides 0.39 g of sodium 3 - (2 - benzyloxycarboxamido - 2 - benzyloxycarbonylethanesulphonamido) - 2 - oxoazetidine - 1 - sulphonate.

IR$\nu_{max}^{KBr}$cm$^{-1}$; 1750, 1720, 1520, 1260.
NMR(DMSO-$d_6$, ppm); 3.64 (m, —$CH_2$—),

4.58 (m, —CH—, $C_3$—H),
|

5.06 (s, —$CH_2$—), 5.15 (s, —$CH_2$—), 7.34, 7.36 (each s, aromatic H) 7.86 (d, J=7Hz, NH), 8.38 (broad s, NH).

In 15 ml of water 0.14 g of the above sodium 3 - (2 - benzyloxycarboxamido - 2 - benzyloxycarbonylethanesulphonamido) - 2 - oxoazetidine - 1 - sulphonate is dissolved, followed by addition of 0.10 g of palladium black. The mixture is stirred in a hydrogen gas stream for 1 hour, and the catalyst is filtered off. The filtrate is freeze-dried to provide 90 mg of sodium 3 - (2 - amino - 2 - carboxyethanesulphonamido) - 2 - oxoazetidine - 1 - sulphonate.

IR$\nu_{max}^{KBr}$cm$^{-1}$; 1760, 1640, 1240.
NMR(DMSO-$d_6$, ppm); 3.34 (dd, J=2, 6Hz, $C_4$—$\beta$H), 3.68 (m, —$CH_2$—),

4.60 (m, —CH—, $C_3$—H).
|

Example 116

0.27 g of 3 - [D - 2 - (4 - ethyl - 2,3 - dioxo - 1 - piperazinocarboxamido) - 3 - [(1 - methyl - 5H - tetrazol - 5 - yl)thio]propionamido] - 2 - oxoazetidine is dissolved in 2 ml of DMF, followed by addition of 0.195 g of pyridine-sulphur trioxide complex. The mixture is stirred at room temperature for 3 days and, then, worked up in the manner as described in Example 5. The above procedure provides 0.144 g of sodium 3 - [D - 2 - (4 - ethyl - 2,3 - dioxo - 1 - piperazinocarboxamido) - 3 - [(1 - methyl - 5H - tetrazol - 5 - yl)thio]propionamido] - 2 - oxoazetidine - 1 - sulphonate.

$IR\nu_{max}^{KBr}cm^{-1}$; 1760, 1710, 1670, 1520, 1260, 1190.
NMR($DMSO-d_6$, ppm); 1.10 (t, J=7Hz, $CH_3$), 3.90 (s, $CH_3$),

4.73 (m, —CH—, $C_3$—H),

9.07 (d, J=7Hz, NH), 9.36 (d, J=Hz, NH).

Example 117

0.40 g of 3 - [D - 2 - (2 - benzyloxycarboxamido - 2 - benzyloxycarbonylethanesulphonamido) - 2 - phenylacetamido] - 2 - oxoazetidine is dissolved in 2 ml of DMF, followed by addition of 0.214 g of pyridine-sulphur trioxide complex. The mixture is stirred at room temperature for 3 days and, then, worked up in the manner as described in Example 5. The above procedure provides 0.24 g of sodium 3 - [D - 2 - (2 - benzyloxycarboxamido - 2 - benzyloxycarbonylethanesulphonamido) - 2 - phenylacetamido] - 2 - oxoazetidine - 1 - sulphonate.

$IR\nu_{max}^{KBr}cm^{-1}$; 1750, 1720, 1680, 1520, 1260.
NMR($DMSO-d_6$, ppm); 3.12 (dd, J=2, 6Hz, $C_4$—βh),

4.56 (m, —CH—), 4.74 (m, —CH—),

5.04, 5.12 (each s, —$CH_2$—), 7.36 (s, aromatic H), 7.80 (d, J=7Hz, NH), 8.21 (broad s, NH), 9.12 (d, J=7Hz, NH).

0.18 g of the above sodium 3 - [D - 2 - (2 - benzyloxycarboxamido - 2 - benzyloxycarbonylethanesulphonamido) - 2 - phenylacetamido] - 2 - oxoazetidine - 1 - sulphonate is dissolved in 15 ml of water, followed by addition of 0.10 g of palladium black. The mixture is stirred in a hydrogen gas stream for 1 hour, and the catalyst is filtered off. The filtrate is freeze-dried to provide 0.13 g of sodium 3 - [D - 2 - (2 - amino - 2 - carboxyethanesulphonamido) - 2 - phenylacetamido] - 2 - oxoazetidine - 1 - sulphonate.

$IR\nu_{max}^{KBr}cm^{-1}$; 1760, 1645, 1520, 1265, 1235.
NMR($DMSO-d_6$, ppm); 3.42 (dd, J=2, 6Hz, $C_4$—βH), 3.62 (m, —$CH_2$—),

4.78 (m, —CH—),

7.40 (broad s, aromatic H), 9.22 (d, J=7Hz, NH).

Example 118

0.523 g of 3 - [D - 2 - (2 - benzyloxycarboxamido - 3 - sulphamoylpropionamido) - 2 - phenylacetamido] - 2 - oxoazetidine is dissolved in 7 ml of DMF, followed by addition of 0.30 g of pyridine-sulphur trioxide complex. The mixture is stirred at room temperature for 2 days and, then, worked up in the manner as described in Example 5. The above procedure provides 0.360 g of sodium 3 - [D - 2 - (2 - benzyloxycarboxamido - 3 - sulphamoylpropionamido) - 2 - phenylacetamido] - 2 - oxoazetidine - 1 - sulphonate.

$IR\nu_{max}^{KBr}cm^{-1}$; 1765, 1705, 1670, 1260—1230.

Example 119

0.50 g of 3 - [D - 2 - [2 - benzyloxycarboxamido - 3 - (p - methoxybenzyloxycarboxamido)-propionamido] - phenylacetamido] - 2 - oxoazetidine is dissolved in 6 ml of DMF, followed by addition of 0.264 g of pyridine-sulphur trioxide complex. The mixture is stirred for 2 days and, then, worked up in the manner as described in Example 5. The above procedure provides 0.45 g of sodium 3 - [D - 2 - (2 - benzyloxycarboxamido) - 3 - (p - methoxybenzyloxycarboxamido)propionamido] - 2 - phenylacetamido) - 2 - oxoazetidine - 1 - sulphonate.

$IR\nu_{max}^{KBr}cm^{-1}$; 3285, 1758, 1688, 1645, 1243, 1048.
NMR($DMSO-d_6$, ppm); 3.10—3.30 (m, —$CH_2$—, $C_2$—H), 3.56 (t, J=6Hz, $C_4$—H), 3.74 (s, $CH_3$),

4.22 (m, —CH—),

4.84 (m, $C_3$—H), 4.96 (s, —$CH_2$—), 5.04 (s, —$CH_2$—),

5.42 (d, J=8Hz, —CH—),

6.90, 7.31 (each d, J=8Hz, aromatic H), 7.34 (s, aromatic H), 8.40 (d, J=7Hz, NH), 9.06 (d, J=8Hz, NH).

0.160 g of the above sodium 3 - [D - 2 - [2 - benzyloxycarboxamido - 3 - (p - methoxybenzyloxycarboxamido)propionamido] - 2 - phenylacetamido] - 2 - oxoazetidine - 1 - sulphonate is dissolved in a solution of 10 ml of water and 2 ml of ethyl alcohol, followed by addition of 0.115 g of palladium black. The mixture is stirred in a hydrogen gas stream for 80 minutes, and the catalyst is filtered off. The filtrate is freeze-dried to provide 90 mg of sodium 3 - [D - 2 - (2,3 - diaminopropionamido) - 2 - phenylacetamido] - 2 - oxoazetidine - 1 - sulphonate.

IR$\nu_{max}^{KBr}$cm$^{-1}$; 3450—3270, 1757, 1652, 1235, 1046.
NMR($DMSO-d_6+D_2O$, ppm); 3.26 (dd, J=3, 6Hz, $C_4$—H), 3.63 (t, J=6Hz, $C_4$—H), 4.82 (dd, J=3, 6Hz, $C_3$—H),

5.39 (s, —CH—),

7.38 (s, aromatic H).

Example 120

0.30 g of 3 - [D - 2 - [2 - benzyloxycarboxamido - 3 - (4 - ethyl - 2,3 - dioxo - 1 - piperazinocarboxamido) - propionamido] - 2 - phenylacetamido] - 2 - oxoazetidine is dissolved in 4 ml of DMF, followed by addition of 0.159 g of pyridine-sulphur trioxide complex. The mixture is stirred for 3 days and, then, worked up in the manner as described in Example 5. The above procedure provides 0.147 g of sodium 3 - [D - 2 - [2 - benzyloxycarboxamido - 3 - (4 - ethyl - 2,3 - dioxo - 1 - piperazinocarboxamido)propionamido] - 2 - phenylacetamido] - 2 - oxoazetidine - 1 - sulphonate.

IR$\nu_{max}^{KBr}$cm$^{-1}$; 3300, 1768, 1705, 1670, 1260—1230, 1048.
NMR($DMSO-d_6$, ppm); 1.07 (t, J=7Hz, —$CH_3$), 3.07 (dd, J=3,5Hz, $C_4$—H), 3.30—3.70 (m, —$CH_2$—), 3.38 (q, J=7Hz, —$CH_2$—), 3.87 (m, —$CH_2$—),

4.30 (m, —CH—),

4.80 (m, $C_3$—H), 5.00 (s, —$CH_2$—),

5.40 (d, J=8Hz, —CH—),

7.31 (s, aromatic H), 7.60 (d, J=8Hz, NH), 8.45 (d, J=8Hz, NH), 8.89 (t, J=6Hz, NH), 9.08 (d, J=8Hz, NH).

88.4 mg of the above sodium 3 - [D - 2 - [2 - benzyloxycarboxamido - 3 - (4 - ethyl - 2,3 - dioxo - 1 - piperazinocarboxamido)propionamido] - 2 - phenylacetamido] - 2 - oxoazetidine - 1 - sulphonate is dissolved in 10 ml of water, followed by addition of 90 mg of palladium black. The mixture is stirred in a hydrogen gas stream for 1 hour, and the catalyst is filtered off. The filtrate is freeze-dried to provide 70 mg of sodium 3 - [D - 2 - [2 - amino - 3 - (4 - ethyl - 2,3 - dioxo - 1 - piperazinocarboxamido) - propionamido] - 2 - phenylacetamido] - 2 - oxoazetidine - 1 - sulphonate.

IR$\nu_{max}^{KBr}$cm$^{-1}$; 3500—3300, 1760, 1708, 1670, 1265—1230, 1048.
NMR($DMSO-d_6+D_2O$, ppm); 1.11 (t, J=7Hz, —$CH_3$), 3.20 (dd, J=3, 6Hz, $C_4$—H), 3.82—4.0 (m, —$CH_2$—), 4.81 (dd, J=3, 6Hz, $C_3$—H),

5.43 (s, —CH—),

7.36 (s, aromatic H).

Example 121

0.34 g of 3 - [2 - (2 - benzyloxycarboxamido - 3 - N - methylcarbamoylpropionamido)-acetamido] - 3 - methoxy - 2 - oxoazetidine is dissolved in 5 ml of DMF, followed by addition of 0.211 g of pyridine-sulphur trioxide complex. The mixture is stirred for 3 days and, then, worked up in the manner as described in Example 5. The above procedure provides 0.015 g of sodium 3 - [2 - (2 - benzyloxycarboxamido - 3 - N - methylcarbamoylpropionamido) - acetamido] - 3 - methoxy - 2 - oxoazetidine - 1 - sulphonate.

$IR\nu_{max}^{KBr}cm^{-1}$; 3350, 1770, 1690, 1660, 1248, 1053.

Example 122

0.343 g of 3 - [2 - (4 - ethyl - 2,3 - dioxo - 1 - piperazinocarboxamido)acetamido] - 3 - methoxy - 2 - oxoazetidine is dissolved in 5 ml of DMF, followed by addition of 0.287 g of pyridine-sulphur trioxide complex. The mixture is stirred for 2 days and, then, worked up in the manner as described in Example 5. The above procedure provides 0.076 g of sodium 3 - [2 - (4 - ethyl - 2,3 - dioxo - 1 - piperazinocarboxamido)acetamido] - 3 - methoxy - 2 - oxoazetidine - 1 - sulphonate.

$IR\nu_{max}^{KBr}cm^{-1}$; 3320, 1765, 1708, 1670, 1250, 1051.

NMR(DMSO-$d_6$, ppm); 1.10 (t, J=7Hz, $CH_3$), 3.30 (s, $CH_3$), 3.42 (q, J=7Hz, $—CH_2—$), 3.60 (m, $—CH_2—$), 3.61 (ABq, J=4, 6Hz, $C_4$—H), 3.90 (m, $—CH_2—$), 3.97 (d, J=5Hz, $—CH_2—$), 9.09 (t, J=5Hz, NH), 9.27 (s, NH).

Example 123

0.30 g of 3 - [D - 2 - [2 - (4 - ethyl - 2,3 - dioxo - 1 - piperazinocarboxamido) - 3 - (N - methylcarbamoyl) - propionamido] - 2 - phenylacetamido] - 2 - oxoazetidine is dissolved in 4 ml of DMF, followed by addition of 0.184 g of pyridine-sulphur trioxide complex. The mixture is stirred for 3 days and, then, worked up in the manner as described in Example 45. The above procedure provides 0.141 g of sodium 3-[D - 2 - [2 - (4 - ethyl - 2,3 - dioxo - 1 - piperazinocarboxamido) - 3 - (N - methylcarbamoyl)propionamido] - 2 - phenylacetamido] - 2 - oxoazetidine - 1 - sulphonate.

$IR\nu_{max}^{KBr}cm^{-1}$; 3350—3290, 1768, 1707, 1668, 1265—1230, 1048.

NMR(DMSO-$d_6$, ppm); 1.08 (t, J=7Hz, $—CH_3$), 2.40—2.75 (m, $—CH_2—$, $CH_3$), 3.25 (dd, J=2, 5Hz, $C_4$—H), 3.38 (q, J=7Hz, $—CH_2—$), 3.53 (m, $—CH_2—$), 3.90 (m, $—CH_2—$),

4.60 (m, $—\overset{|}{CH}—$),

4.83 (m, $C_3$—H),

5.38 (d, J=9Hz, $—\overset{|}{CH}—$),

7.33 (s, aromatic H), 7.86 (m, NH), 8.55 (d, J=9Hz, NH), 8.93, 8.95 (each d, J=9Hz, NH), 9.31, 9.42 (each d, J=7Hz, NH).

Example 124

0.35 g of 3 - [2 - (4 - ethyl - 2,3 - dioxo - 1 - piperazinocarboxamido) - 3 - (N - methylcarbamoyl) - propionamido] - 2 - oxoazetidine is dissolved in 5 ml of DMF, followed by addition of 0.234 g of pyridine-sulphur trioxide complex. The mixture is stirred for 3 days and, then, worked up in the manner as described in Example 5. The above procedure provides 0.178 g of sodium 3 - [2 - (4 - ethyl - 2,3 - dioxo - 1 - piperazinocarboxamido) - 3 - (N - methylcarbamoyl)propionamido] - 2 - oxoazetidine - 1 - sulphonate.

$IR\nu_{max}^{KBr}cm^{-1}$; 3440—3270, 1755, 1705, 1655, 1260—1240, 1190, 1045.

NMR(DMSO-$d_6$, ppm); 1.10 (t, J=7Hz, $CH_3$), 2.58 (d, J=5Hz, $CH_3$), 2.40—2.67 (m, $—CH_2—$), 3.42 (q, J=7Hz, $—CH_2—$), 3.57 (m, $—CH_2—$), 3.93 (m, $—CH_2—$),

4.55 (m, $—\overset{|}{CH}—$),

4.80 (m, $C_3$—H), 7.83 (q, J=5Hz, NH), 8.72 (d, J=8Hz, NH), 9.31, 9.35 (d, J=8Hz, NH).

Example 125

0.40 g of 3 - [2 - (D - 2 - benzyloxycarboxamido - 2 - phenylacetamido) - 3 - (N - methylcarbamoyl) - propionamido] - 2 - oxoazetidine is dissolved in 8 ml of DMF, followed by addition of

0.264 g of pyridine-sulphur trioxide complex. The mixture is stirred for 4 days and, then, worked up in the manner as described in Example 45. The above procedure provides 0.212 g of sodium 3 - [2 - (D - 2 - benzyloxycarboxamido - 2 - phenylacetamido) - 3 - (N - methylcarbamoyl)propionamido] - 2 - oxoazetidine - 1 - sulphonate.

IR$\nu_{max}^{KBr}$cm$^{-1}$; 3290, 1760, 1690, 1645, 1245, 1050.
NMR(DMSO-$d_6$, ppm); 2.27—2.63 (m, —$CH_2$—, $CH_3$), 3.27 (m, $C_4$—H), 3.52, 3.57 (t, J=6Hz, $C_4$—H),

4.53 (m, —CH—),

4.80 (m, $C_3$—H), 5.03 (s, —$CH_2$—), 5.22,

5.23 (d, J=8Hz, —CH—),

7.33 (s, aromatic H), 7.50—7.93 (m, NH), 8.27—8.63 (m, NH).

0.10 g of the above sodium 3 - [2 - (D - 2 - benzyloxycarboxamido - 2 - phenylacetamido) - 3 - (N - methylcarbamoyl)propionamido] - 2 - oxoazetidine - 1 - sulphonate is dissolved in 8 ml of water, followed by addition of 50 mg of palladium black. The mixture is stirred in a hydrogen gas stream for 1 hour, and the catalyst is filtered off. The filtrate is freeze-dried to provide 68 mg of sodium 3 - [2 - (D - 2 - amino - 2 - phenylacetamido) - 3 - (N - methylcarbamoyl)propionamido] - 2 - oxoazetidine - 1 - sulphonate.

IR$\nu_{max}^{KBr}$cm$^{-1}$; 1758, 1670—1640, 1270—1238, 1048.
NMR(DMSO-$d_6$+$D_2O$, ppm); 2.47—2.83 (m, —$CH_2$—, $CH_3$), 3.48 (m, $C_4$—H),

4.53 (s, —CH—), 4.63 (m, —CH—),

4.87 (m, $C_3$—H), 7.42 (s, aromatic H).

Example 126—(A)

0.228 g of 3 - [2 - D - [(2 - oxo - 3 - furfurylideneaminoimidazolidin - 1 - yl)carboxamido] - 2 - phenylacetamido] - 3(S) - methoxy - 2 - oxoazetidine is dissolved in 2 ml of DMF, followed by addition of 0.199 g of pyridine-sulphur trioxide complex. The mixture is stirred for 1 day and, then, worked up in the manner as described in Example 5. The above procedure provides 0.11 g of sodium 3 - [2 - D - [(2 - oxo - 3 - furfurylideneaminoimadazolidin - 1 - yl)carboxamido] - 2 - phenylacetamido] - 3(S) - methoxy - 2 - oxoazetidine - 1 - sulphonate.

IR$\nu_{max}^{KBr}$cm$^{-1}$; 3300, 1770, 1720, 1670, 1475, 1420, 1270, 1235, 1050.
NMR($d_6$-DMSO, ppm); 3.03 (s, $CH_3$), 3.54, 3.72 (d, J=6Hz, $C_4$—H), 3.80 (s, —$CH_2$—),

5.63 (d, J=7Hz, —CH—),

6.5—7.9 (m, aromatic H(, 7.74 (s, —CH=N—), 9.02 (d, J=7Hz, NH), 9.71 (s, NH).

Example 126—(B)

0.114 g of 3 - [2 - D - [(2 - oxo - 3 - furfurylideneaminoimidazolidin - 1 - yl)carboxamido] - 2 - phenylacetamido] - 3(R) - methoxy - 2 - oxoazetidine is dissolved in 1 ml of DMF, followed by addition of 0.1 g of pyridine-sulphur trioxide complex. The mixture is stirred for 1 day and, then, worked up in the manner as described in Example 5. The above procedure provides 0.044 g of sodium 3 - [2 - D - [(2 - oxo - 3 - furfurylideneaminoimidazolidin - 1 - yl)carboxamido] - 2 - phenylacetamido] - 3(R) - methoxy - 2 - oxoazetidine - 1 - sulphonate.

IR$\nu_{max}^{KBr}$cm$^{-1}$; 3300, 1770, 1720, 1670, 1475, 1420, 1270, 1235, 1050.
NMR($d_6$-DMSO, ppm); 3.32 (s, $CH_3$), 3.46, 3.55 (d, J=6Hz, $C_4$—H), 3.80 (s, —$CH_2$—),

5.59 (d, J=7Hz, —CH—),

6.5—7.9 (m, aromatic H), 7.74 (s, —CH=N—), 8.96 (d, J=7Hz, NH), 9.65 (s, NH).

Example 127

0.096 g of 3 - [2 - (4 - ethyl - 2,3 - dioxo - 1 - piperazinocarboxamido) - 2 - (4 - n - octanoyloxyphenyl) - acetamido] - 2 - oxoazetidine is dissolved in 1.5 ml of DMF, followed by addition of 0.058 g of pyridine-sulphur trioxide complex. The mixture is stirred for 1 day and, then, worked up in the manner as described in Example 5. The above procedure provides 0.096 g of sodium 3 - [2 - (4 - ethyl - 2,3 - dioxo - 1 - piperazinocarboxamido) - 2 - (4 - n - octanoyloxyphenyl)-acetamido] - 2 - oxoazetidine - 1 - sulphonate.

IR$\nu_{max}^{KBr}$cm$^{-1}$; 3280, 2920, 2850, 1750, 1710, 1670, 1500, 1270—1230, 1190, 1050.

NMR($d_6$-DMSO, ppm); 0.87 (t, $CH_3$), 1.09 (t, $CH_3$), 1.2—2.6 (m, —$CH_2$—), 3.13 (dd, J=3, 6Hz, $C_4$—H), 3.41 (q, —$CH_2$—), 3.4—4.1 (m, —$CH_2$—), 4.86 (ddd, J=3, 6, 8Hz, $C_3$—H),

5.46 (d, J=7Hz, —CH—),
|

7.10, 7.44 (d, J=8Hz, aromatic H), 9.28 (d, J=8Hz, NH), 9.82 (d, J=7Hz, NH).

Example 128

0.35 g 3 - [2 - (4 - ethyl - 2,3 - dioxo - 1 - piperazinocarboxamido) - 3 - (N - ethoxycarbonylmethylcarbamoyl)propionamido] - 2 - oxoazetidine is dissolved in 5 ml of DMF, followed by addition of 0.23 g of pyridine-sulphur trioxide complex. The mixture is stirred for 2 days and, then, worked up in the manner as described in Example 5. The above procedure provides 0.165 g of sodium 3 - [2 - (4 - ethyl - 2,3 - dioxo - 1 - piperazinocarboxamido) - 3 - (N - ethoxycarbonylmethylcarbamoyl)propionamido] - 2 - oxoazetidine - 1 - sulphonate.

IR$\nu_{max}^{KBr}$cm$^{-1}$; 3480—3300, 1758, 1708, 1670, 1260—1230, 1192, 1048.

NMR(DMSO-$d_6$, ppm); 1.10 (t, J=7Hz, $CH_3$), 1.19 (t, J=7Hz, $CH_3$), 2.66 (m, —$CH_2$—), 3.35 (dd, J=3, 6Hz, $C_4$—H), 3.43 (q, J=7Hz, —$CH_2$—), 3.50—3.73 (m, —$CH_2$—), 3.74—4.00 (m, —$CH_2$—), 3.81 (d, J=6Hz, —$CH_2$—), 4.09 (q, J=7Hz, —$CH_2$—),

4.58 (m, —CH—),
|

4.82 (m, $C_3$—H), 8.36 (t, J=6Hz, NH), 8.73 (d, J=8Hz, NH), 9.34 (d, J=8Hz, NH).

Example 129

0.464 g of 3 - [D - 2 - (4 - ethyl - 2,3 - dioxo - 1 - piperazinocarboxamido) - 3 - (2 - thienylacetamido) - propionamido] - 2 - oxoazetidine is dissolved in 5 ml of DMF, followed by addition of 0.30 g of pyridine-sulphur trioxide complex. The mixture is stirred at room temperature for 2 days and, then, worked up in the manner as described in Example 5. The above procedure provides 0.31 g of sodium 3 - [D - 2 - (4 - ethyl - 2,3 - dioxo - 1 - piperazinocarboxamido) - 3 - (2 - thienylacetamido)propionamido] - 2 - oxoazetidine - 1 - sulphonate.

IR$\nu_{max}^{KBr}$cm$^{-1}$; 1760, 1710, 1670, 1250—1210.

NMR(DMSO-$d_6$, ppm); 1.10 (t, J=7Hz, $CH_3$), 3.20 (dd, J=2.5, 6Hz, $C_4$—H), 3.40 (q , J=7Hz, —$CH_2$—), 3.60—3.90 (m, —$CH_2$—), 3.70 (s, —$CH_2$—),

4.60 (m, —CH—),
|

4.82 (m, $C_3$—H), 6.7—7.4 (m, thienyl—H), 8.68 (d, J=8Hz, NH), 9.30 (d, J=8Hz, NH).

Example 130

0.28 g of 3-(N-mesyl-D-phenylglycinamido)-2-oxoazetidine is dissolved in 4 ml of DMF, followed by addition of 0.30 g of pyridine-sulphur trioxide complex. The mixture is stirred for 2 days and, then, worked up in the manner as described in Example 5. The above procedure provides 0.12 g of sodium 3-(N-mesyl-D-phenylglycinamido)-2-oxoazetidine-1-sulphonate.

IR$\nu_{max}^{KBr}$cm$^{-1}$; 1750, 1715, 1670, 1520, 1250.

NMR(DMSO-$d_6$, ppm); 3.10 (dd, J=3, 6Hz, $C_4$—H), 3.32 (s, $CH_3$),

4.52 (m, —CH—), 4.75 (m, —CH—),
| |

7.35 (s, aromatic H), 8.30 (broad s, NH), 9.20 (d, J=7Hz, NH).

Example 131

0.30 g of 3 - [D - 2 - [2 - (4 - ethyl - 2,3 - dioxo - 1 - piperazinocarboxamido)acetamido] - 2 - phenylacetamido] - 2 - oxoazetidine is dissolved in 4 ml of DMF, followed by addition of 0.215 of pyridine-sulphur trioxide complex.The mixture is stirred for 4 days and, then, worked up in the manner as described in Example 5. The above procedure provides 0.239 g of sodium 3 - [D - 2 - [2 - (4 - ethyl - 2,3 - dioxo - 1 - piperazinocarboxamido)acetamido] - 2 - phenylacetamido] - 2 - oxoazetidine - 1 - sulphonate.

IR$\nu_{max}^{KBr}$cm$^{-1}$; 3300, 1756, 1708, 1670, 1240, 1048.

NMR(DMSO-$d_6$, ppm); 1.10 (t, J=7Hz, $CH_3$), 3.15 (dd, J=3,5Hz, $C_4$—H), 3.28 (q, J=7Hz, —$CH_2$—), 3.30—3.70 (m, —$CH_2$—), 3.80—4.10 (m, —$CH_2$—), 3.98 (d, J=5Hz, —$CH_2$—), 4.83 (m, $C_3$—H),

5.47 (d, J=8Hz, —CH—),
|

7.37 (s, aromatic H), 8.68 (d, J=8Hz, NH), 9.05 (d, J=8Hz, NH), 9.16 (d, J=5Hz, NH).

Example 132

0.287 g of 3 - [2 - D - (4 - n - octyl - 2,3 - dioxo - 1 - piperazinocarboxamido) - 2 - thienylacetamido] - 2 - oxoazetidine is dissolved in 2 ml of DMF, followed by addition of 0.191 g of pyridine-sulphur trioxide complex. The mixture is stirred for one day and, then, worked up in the manner as described in Example 5. The above procedure provides 0.314 g of sodium 3 - [2 - D - (4 - n - octyl - 2,3 - dioxo - 1 - piperazinocarboxamido) - 2 - thienylacetamido] - 2 - oxoazetidine - 1 - sulphonate.

IR$\nu_{max}^{KBr}$cm$^{-1}$; 3280, 2920, 1760, 1710, 1670, 1250, 1190, 1050.

NMR($d_6$-DMSO, ppm); 0.86 (t, $CH_3$), 3.17 (dd, J=3, 6Hz, $C_4$—H), 3.4—4.1 (m, —$CH_2$—), 3.62 (t, J=6Hz, $C_4$—H), 4.86 (ddd, J=3, 6, 8Hz, $C_3$—H),

5.72 (d, J=7Hz, —CH—),
|

6.9—7.6 (m, thienyl—H), 9.30 (d, J=8Hz, NH), 9.73 (d, J=7Hz, NH).

**Claims for the contracting States: BE CH DE FR GB IT LI LU NL SE**

1. A compound of the formula:

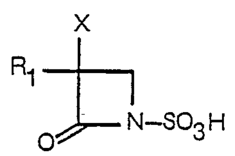

(I)

wherein $R_1$ is amino, acylated amino or protected amino and X is hydrogen or methoxy, or a salt thereof, but excluding the compound wherein X is methoxy and $R_1$ is amino substituted with an acyl group $R_7$—NH—CH($R_9$)—CO— in which $R_7$ is gammaglutamyl and $R_9$ is methyl.

2. A compound as claimed in claim 1, wherein $R_1$ is amino with an acyl group selected from: (a) a group of the formula

$R_7$—NH—CH—CO—
|
$R_9$

wherein $R_7$ is hydrogen; an optionally substituted amino acid residue; an amino-protecting group; a group of the formula $R_8$—$(CH_2)_n$—CO— in which $R_8$ is an optionally substituted heterocyclic group, an optionally substituted phenyl, an optionally substituted $C_{1-12}$ alkyl, an optionally substituted phenylthio or $C_{1-3}$ alkylthio, n is 0 or an integer of 1 to 4 and the group —$(CH_2)_n$— may optionally be substituted; a group of the formula

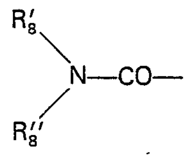

in which $R_8'$ and $R_8''$ may be the same or different, and are hydrogen; $C_{1-3}$ alkyl, $C_{1-3}$ alkyl carbamoyl, optionally substituted phenylcarbonyl or sulpho; or a group of the formula $R_8'''$ —$SO_2$— in which $R_8'''$ is optionally substituted $C_{1-6}$ alkyl; and $R_9$ is hydrogen, optionally substituted $C_{1-3}$ alkyl, optionally substituted phenyl, optionally substituted heterocyclic group, cycloalkenylene or optionally substituted heterocycle-carbonylamino in which an alkylene chain may stand between the heterocycle and carbonylamino moieties, and

(b) a group of the formula $R_{10}$—$R_{11}$—CO— wherein $R_{10}$ is a group of the formula

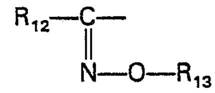

in which $R_{12}$ is an optionally substituted heterocyclic group or optionally substituted phenyl, $R_{13}$ is hydrogen, an optionally substituted $C_{2-4}$ acyl, $C_{1-3}$ alkyl or a group of the formula —$R_{14}$—$R_{15}$ where $R_{14}$ is $C_{1-3}$ alkylene or $C_{2-3}$ alkenylene and $R_{15}$ is carboxyl, ester thereof or heterocyclic group; and $R_{11}$ is a mere bond or a group of the formula

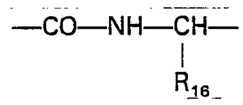

in which $R_{16}$ is a $C_{1-3}$ alkyl, optionally substituted phenyl or optionally substituted heterocyclic group.

3. A compound as claimed in claim 1, wherein $R_1$ is amino with a protective group selected from the protective groups used in the field of peptide synthesis.

4. A compound as claimed in claim 1, selected from:
3-amino-3-methoxy-2-oxoazetidine-1-sulphonic acid;
3-benzyloxycarboxamido-3-methoxy-2-oxoazetidine-1-sulphonic acid; and
3-phenylacetamido-3-methoxy-2-oxoazetidine-1-sulphonic acid.

5. A compound as claimed in claim 1, which is:
3-[D-(—)-N-(4-ethyl-2,3-dioxo-1-piperazinocarbonyl)-phenylglycinamido]-3-methoxy-2-oxoazetidine-1-sulphonic acid.

6. A compound as claimed in claim 1, selected from:
3-amino-2-oxoazetidine-1-sulphonic acid;
3-benzyloxycarboxamido-2-oxoazetidine-1-sulphonic acid;
3-phenylacetamido-2-oxoazetidine-1-sulphonic acid;
3-[2-(2-amino-4-thiazolyl)-2-methoxyiminoacetamido[-2-oxoazetidine-1-sulphonic acid;
3-[D-N-(4-ethyl-2,3-dioxo-1-piperazinocarbonyl)-phenylglycinamido]-2-oxoazetidine-1-sulphonic acid;
3-[2-(4-ethyl-2,3-dioxo-1-piperazinocarboxamido)-2-(2-amino-4-thiazolyl)acetamido]-2-oxoazetidine-1-sulphonic acid;
3-[DL-2-(4-ethyl-2,3-dioxo-1-piperazinocarboxamido)-2-thienylacetamido]-2-oxoazetidine-1-sulphonic acid;
3-[D-2-(4-ethyl-2,3-dioxo-1-piperazinocarboxamido)-2-thienylacetamido]-2-oxoazetidine-1-sulphonic acid;
3-[D-2-(4-n-octyl-2,3-dioxo-1-piperazinocarboxamido)-2-phenylacetamido]-2-oxoazetidine-1-sulphonic acid;
3-[2-(4-n-octyl-2,3-dioxo-1-piperazinocarboxyamido)-2-(2-amino-4-thiazolyl)acetamido]-2-oxoazetidine-1-sulphonic acid;
3-[D-2[(2-oxo-3-furfurylideneaminoimidazolidin-1-yl)-carboxamido]-2-phenylacetamido]-2-oxoazetidine-1-sulphonic acid;
3-[DL-2-(4-n-octyl-2,3-dioxo-1-piperazinocarboxamido)-2-thienylacetamido]-2-oxoazetidine-1-sulphonic acid;
3-[D-2-(4-n-octyl-2,3-dioxo-1-piperazinocarboxamido)-2-thienylacetamidoi]-2-oxoazetidine-1-sulphonic acid;
3-[D-2-[[2-oxo-3-(thiophen-2-aldoimino)-imidazolidin-1-yl]-carboxamido]-2-phenylacetamido]-2-oxoazetidine-1-sulphonic acid; and
3-[(2-oxo-3-furfurylideneaminoimidazolidin-1-yl)-carboxyamido]-3-thienylacetamido]-2-oxoazetidine-1-sulphonic acid.

7. A method for the production of a compound according to claim 1 of the formula:

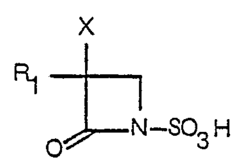

wherein $R_1$ is amino, acylated amino or protected amino and x is hydrogen or methoxy, or a salt thereof, which comprises sulphonating a compound of the formula:

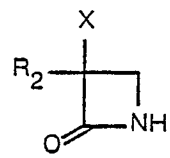

wherein $R_2$ is acylated amino or protected amino and X is as defined above, and optionally removing the amino-protecting group, or acylating the compound thus obtained of the formula:

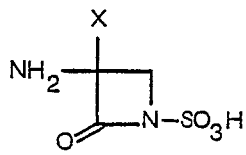

wherein X is as defined above, and optionally converting the product into a salt.

8. A pharmaceutical composition containing a compound as claimed in any one of claims 1 to 6.

**Claims for the contracting State: AT**

1. A method for the production of a compound of the formula:

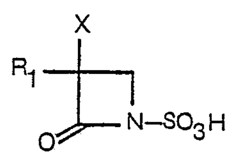

wherein $R_1$ is amino, acylated amino or protected amino and x is hydrogen or methoxy, or a salt thereof, which comprises sulphonating a compound of the formula:

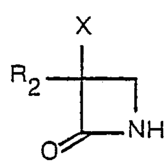

wherein$R_2$ is acylated amino or protected amino and X is as defined above, and optionally removing the amino-protecting group, or acylating the compound thus obtained of the formula:

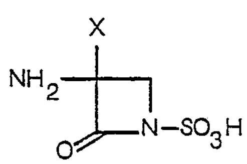

wherein X is as defined above, and optionally converting the product into a salt.

2. A method as claimed in claim 1, wherein $R_1$ or $R_2$ is amino with an acyl group selected from:

(a) a group of the formula

$$R_7\text{—NH—}\underset{\displaystyle R_9}{\underset{|}{\text{CH}}}\text{—CO—}$$

wherein $R_7$ is hydrogen; an optionally substituted amino acid residue; an amino-protecting group; a group of the formula $R_8$—$(CH_2)_n$ —CO— in which $R_8$ is an optionally substituted heterocyclic group, an optionally substituted phenyl, an optionally substituted $C_{1-12}$ alkyl, an optionally substituted phenylthio or $C_{1-3}$ alkylthio, n is 0 or an integer of 1 to 4 and the group —$(CH_2)_n$— may optionally be substituted; a group of the formula

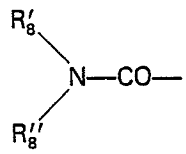

in which $R_8'$ and $R_8''$ may be the same or different, and are hydrogen; $C_{1-3}$ alkyl, $C_{1-3}$ alkyl carbamoyl, optionally substituted phenylcarbonyl or sulpho; or a group of the formula $R_8'''$ —$SO_2$— in which $R_8'''$ is optionally substituted $C_{1-6}$ alkyl; and $R_9$ is hydrogen, optionally substituted $C_{1-3}$ alkyl, optionally substituted phenyl, optionally substituted heterocyclic group, cycloalkenylene or optionally substituted heterocycle-carbonylamino in which an alkylene chain may stand between the heterocycle and carbonylamino moieties, and

(b) a group of the formula $R_{10}$—$R_{11}$—CO— wherein $R_{10}$ is a group of the formula

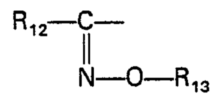

in which $R_{12}$ is an optionally substituted heterocyclic group or optionally substituted phenyl, $R_{13}$ is hydrogen, an optionally substituted $C_{2-4}$ acyl, $C_{1-3}$ alkyl or a group of the formula —$R_{14}$—$R_{15}$ where $R_{14}$ is $C_{1-3}$ alkylene or $C_{2-3}$ alkenylene and $R_{15}$ is carboxyl, ester thereof or heterocyclic group; and $R_{11}$ is a mere bond or a group of the formula

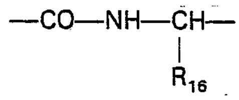

in which $R_{16}$ is a $C_{1-3}$ alkyl, optionally substituted phenyl or optionally substituted heterocyclic group.

3. A method as claimed in claim 1, wherein $R_1$ is amino with a protective group selected from the protective groups used in the field of peptide synthesis.

4. A method as claimed in claim 1, wherein the compound produced is::
3-amino-3-methoxy-2-oxoazetidine-1-sulphonic acid;
3-benzyloxycarboxamido-3-methoxy-2-oxoazetidine-1-sulphonic acid; or
3-phenylacetamido-3-methoxy-2-oxoazetidine-1-sulphonic acid.

5. A method as claimed in claim 1, wherein the compound produced is:
3-[D-(—)-N-(4-ethyl-2,3-dioxo-1-piperazinocarbonyl)-phenylglycinamido]-3-methoxy-2-oxoazetidine-1-sulphonic acid.

6. A method as claimed in claim 1, wherein the compound produced is:
3-amino-2-oxoazetidine-1-sulphonic acid;
3-benzyloxycarboxamido-2-oxoazetidine-1-sulphonic acid;
3-phenylacetamido-2-oxoazetidine-1-sulphonic acid;
3-[2-(2-amino-4-thiazolyl)-2-methoxyiminoacetamido[-2-oxoazetidine-1-sulphonic acid;
3-[D-N-(4-ethyl-2,3-dioxo-1-piperazinocarbonyl)-phenylglycinamido]-2-oxoazetidine-1-sulphonic acid;
3-[2-(4-ethyl-2,3-dioxo-1-piperazinocarboxamido)-2-(2-amino-4-thiazolyl)acetamido]-2-oxoazetidine-1-sulphonic acid;
3-[DL-2-(4-ethyl-2,3-dioxo-1-piperazinocarboxamido)-2-thienylacetamido]-2-oxoazetidine-1-sulphonic acid;
3-[D-2-(4-ethyl-2,3-dioxo-1-piperazinocarboxamido)-2-thienylacetamido]-2-oxoazetidine-1-sulphonic acid;
3-[D-2-(4-n-octyl-2,3-dioxo-1-piperazinocarboxamido)-2-phenylacetamido]-2-oxoazetidine-1-sulphonic acid;
3-[2-(4-n-octyl-2,3-dioxo-1-piperazinocarboxyamido)-2-(2-amino-4-thiazolyl)acetamido]-2-oxoazetidine-1-sulphonic acid;
3-[D-2[(2-oxo-3-furfurylideneaminoimidazolidin-1-yl)-carboxamido]-2-phenylacetamido]-2-oxoazetidine-1-sulphonic acid;
3-[DL-2-(4-n-octyl-2,3-dioxo-1-piperazinoicarboxamido)-2-thienylacetamido]-2-oxoazetidine-1-sulphonic acid;
3-[D-2-(4-n-octyl-2,3-dioxo-1-piperazinocarboxamido)-2-thienylacetamidoi]-2-oxoazetidine-1-sulphonic acid;
3-[D-2-[[2-oxo-3-(thiophen-2-aldoimino)-imidazolidin-1-yl]-carboxamido]-2-phenylacetamido]-2-oxoazetidine-1-sulphonic acid; or
3-[(2-oxo-3-furfurylideneaminoimidazolidin-1-yl)-carboxyamido]-2-thienylacetamido]-2-oxoazetidine-1-sulphonic acid.

**Patentansprüche für die Vertragsstaaten: BE CH DE FR GB IT LI LU NL SE**

1. Verbindung der Formel

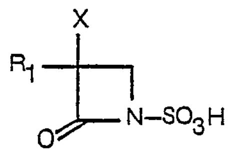

in der
$R_1$ Amino, acyliertes Amino oder geschütztes Amino ist und
X Wasserstoff oder Methoxy ist, oder ein Salz derselben, mit Ausnahme derjenigen Verbindung, in der X Methoxy ist und $R_1$ Amino ist, das mit einer Acyl-Gruppe $R_7$—NH—CH($R_9$)-CO—, worin $R_7$ gamma-Glutaryl ist und $R_9$ Methyl ist, substituiert ist.

2. Verbindung nach Anspruch 1, dadurch gekennzeichnet, daß $R_1$ Amino mit einer Acyl-Gruppe ist, die ausgewählt ist aus

(a) einer Gruppe der Formel

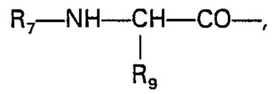

in der $R_7$ Wasserstoff, ein gegebenenfalls substituierter Aminosäure-Rest, eine Schutzgruppe für Amino, eine Gruppe der Formel $R_8$—$(CH_2)_n$—CO, in der $R_8$ eine gegebenenfalls substituierte heterocyclische Gruppe, ein gegebenenfalls substituiertes Phenyl, ein gegebenenfalls substituiertes $C_{1-12}$-Alkyl, ein gegebenenfalls substituiertes Phenylthio oder $C_{1-3}$-Alkylthio ist, n 0 oder eine ganze Zahl von 1 bis 4 ist und die Gruppe —$(CH_2)_n$— gegebenenfalls substituiert sein kann, eine Gruppe der Formel

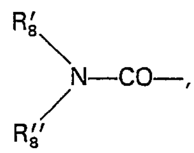

in der $R_8'$ und $R_8''$ gleich oder verschieden sein können und Wasserstoff, $C_{1-3}$-Alkyl, $C_{1-3}$-Alkylcarbamoyl, gegebenenfalls substituiertes Phenylcarbonyl oder Sulfo sind, oder eine Gruppe der Formel $R_8'''$—$SO_2$—, in der $R_8'''$ ein gegebenenfalls substituiertes $C_{1-6}$-Alkyl ist, ist und $R_9$ Wasserstoff, ein gegebenenfalls substituiertes $C_{1-3}$-Alkyl, ein gegebenenfalls substituiertes Phenyl, eine gegebenenfalls substituierte heterocyclische Gruppe, Cycloalkenylen oder ein gegebenenfalls substituiertes Heterocycluscarbonylamino ist, worin eine Alkylen-Kette zwischen den Heterocyclus- und Carbonylamino-Struktureinheiten stehen kann, und

(b) einer Gruppe der Formel $R_{10}$—$R_{11}$—CO—, in der $R_{10}$ eine Gruppe der Formel

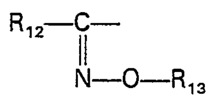

ist, worin $R_{12}$ eine gegebenenfalls substituierte heterocyclische Gruppe oder ein gegebenenfalls substituiertes Phenyl ist, $R_{13}$ Wasserstoff, ein gegebenenfalls substituiertes $C_{2-4}$-Acyl, $C_{1-3}$-Alkyl oder eine Gruppe der Formel —$R_{14}$—$R_{15}$ ist, worin $R_{14}$ $C_{1-3}$-Alkylen oder $C_{2-3}$-Alkenylen ist und $R_{15}$ Carboxyl, ein davon abgeleiteter Ester oder eine heterocyclische Gruppe ist, und $R_{11}$ eine einfache Bindung oder eine Gruppe der Formel

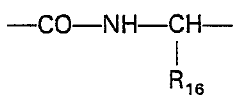

ist, in der $R_{16}$ $C_{1-3}$-Alkyl, ein gegebenenfalls substituiertes Phenyl oder ein gegebenenfalls substituierte heterocyclische Gruppe ist.

3. Verbindung nach Anspruch 1, dadurch gekennzeichnet, daß $R_1$ Amino mit einer Schutzgruppe ausgewählt aus den auf dem Gebiet der Peptid-Synthese verwendeten Schutzgruppen ist.

4. Verbindung nach Anspruch 1, ausgewählt aus:
3-Amino-3-methoxy-2-oxoazetidin-1-sulfonsäure;
3-Benzyloxycarboxamido-3-methoxy-2-oxoazetidin-1-sulfonsäure und
3-Phenylacetamido-3-methoxy-2-oxoazetidin-1-sulfonsäure.

5. Verbindung nach Anspruch 1, die 3-[D-(—)-N-(4-Ethyl-2,3-dioxo-1-piperazinocarbonyl)-phenylglycinamido]-3-methoxy-2-oxoazetidin-1-sulfonsäure ist.

6. Verbindung nach Anspruch 1, ausgewählt aus:
3-Amino-2-oxoazetidin-1-sulfonsäure;
3-Benzyloxycarboxamido-2-oxoazetidin-1-sulfonsäure;
3-Phenylacetamido-2-oxoazetidin-1-sulfonsäure;
3-[2-(2-Amino-4-thiazolyl)-2-methoxyiminoacetamido]-2-oxoazetidin-1-sulfonsäure;
3-[D-N-(4-Ethyl-2,3-dioxo-1-piperazinocarbonyl)-phenylglycinamido]-2-oxoazetidin-1-sulfonsäure;
3-[2-(4-Ethyl-2,3-dioxo-1-piperazinocarboxamido)-2-(2-amino-4-thiazolyl)acetamido]-2-oxoazetidin-1-sulfonsäure;
3-[DL-2-(4-Ethyl-2,3-dioxo-1-piperazinocarboxamido)-2-thienylacetamido]-2-oxoazetidin-1-sulfonsäure;
3-[D-2-(4-Ethyl-2,3-dioxo-1-piperazinocarboxamido)-2-thenylacetamido]-2-oxoazetidin-1-sulfonsäure;
3-[D-2-(4-n-Octyl-2,3-dioxo-1-piperazinocarboxamido)-2-phenylacetamido]-2-oxoazetidin-1-sulfonsäure;
3-[2-(4-n-Octyl-2,3-dioxo-1-piperazinocarboxyamido)-2-(2-amino-4-thiazolyl)acetamido]-2-oxoazetidin-1-sulfonsäure;
3-[D-2-{(2-Oxo-3-furfurylidenaminoimidazolidin-1-yl)-carboxamido}-2-phenylacetamido]-2-oxoazetidin-1-sulfonsäure;
3-[DL-2-(4-n-Octyl-2,3-dioxo-1-piperazinocarboxamido)-2-thienylacetamido]-2-oxoazetidin-1-sulfonsäure;
3-[D-2-(4-n-Octyl-2,3-dioxo-1-piperazinocarboxamido)-2-thienylacetamido]-2-oxoazetidin-1-sulfonsäure;
3-[D-2-{{2-Oxo-3-(thiophen-2-aldoimino)-imidazolidin-1-yl}-carboxamido}-2-phenylacetamido]-2-oxoazetidin-1-sulfonsäure und
3-[(2-Oxo-3-furfurylidenaminoimidazolidin-1-yl)carboxamido)-2-thienylacetamido]-2-oxoazetidin-1-sulfonsäure.

7. Verfahren zur Herstellung einer Verbindung nach Anspruch 1 der Formel

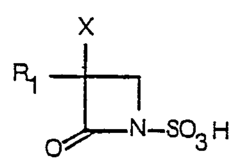

in der
$R_1$ Amino, acyliertes Amino oder geschütztes Amino ist und
X Wasserstoff oder Methoxy ist, oder eines Salzes derselben, dadurch gekennzeichnet, daß eine Verbindung der Formel

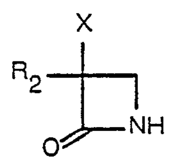

in der
$R_2$ acyliertes Amino oder geschütztes Amino ist und
X die im Vorstehenden angegebene Bedeutung hat, sulfoniert wird und gegebenenfalls die Schutzgruppe für die Amino-Gruppe entfernt wird oder die auf dieses Weise erhaltene Verbindung der Formel

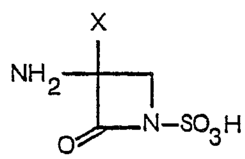

in der
X die im Vorstehenden angegebene Bedeutung hat, acyliert wird und das Produkt gegebenenfalls in ein Salz umgewandelt wird.

8. Pharmazeutische Zusammensetzung, enthaltend eine Verbindung nach irgendienem der Ansprüche 1 bis 6.

**Patentansprüche für den Vertragsstaat: AT**

1. Verfahren zur Herstellung einer Verbindung der Formel

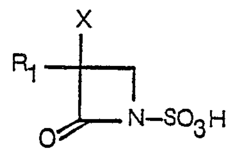

in der
$R_1$ Amino, acyliertes Amino oder geschütztes Amino ist und
X Wasserstoff oder Methoxy ist, oder eines Salzes derselben, dadurch gekennzeichnet, daß eine Verbindung der Formel

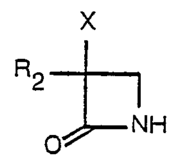

in der
$R_2$ acyliertes Amino oder geschütztes Amino ist und
X die im Vorstehenden angegebene Bedeutung hat, sulfoniert wird und gegebenenfalls die Schutzgruppe für die Amino-Gruppe entfernt wird oder die auf dieses Weise erhaltene Verbindung der Formel

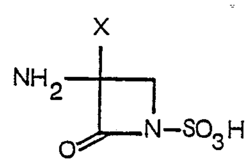

in der
X die im Vorstehenden angegebene Bedeutung hat, acyliert wird und das Produkt gegebenenfalls in ein Salz umgewandelt wird.

2. Verfahren nach Anspruch 1, dadurch gekennzeichnet, daß $R_1$ oder $R_2$ Amino mit einer Acyl-Gruppe ist, die ausgewählt ist aus

(a) einer Gruppe der Formel

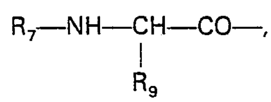

in der $R_7$ Wasserstoff, ein gegebenenfalls substituierter Aminosäure-Rest, eine Schutzgruppe für Amino, eine Gruppe der Formel $R_8$—$(CH_2)_n$—CO, in der $R_8$ eine gegebenenfalls substituierte heterocyclische Gruppe, ein gegebenenfalls substituiertes Phenyl, ein gegebenenfalls substituiertes $C_{1-12}$-Alkyl, ein gegebenenfalls substituiertes Phenylthio oder $C_{1-3}$-Alkylthio ist, n 0 oder eine ganze Zahl von 1 bis 4 ist und die Gruppe —$(CH_2)_n$— gegebenenfalls substituiert sein kann, eine Gruppe der Formel

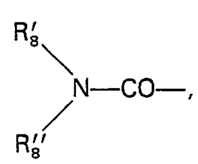

in der $R_8'$ und $R_8''$ gleich oder verschieden sein können und Wasserstoff, $C_{1-3}$-Alkyl, $C_{1-3}$-Alkylcarbamoyl, gegebenenfalls substituiertes Phenylcarbonyl oder Sulfo sind, oder eine Gruppe der Formel $R_8'''$—$SO_2$—, in der $R_8'''$ ein gegebenenfalls substituiertes $C_{1-6}$-Alkyl ist, ist und $R_9$ Wasserstoff, ein gegebenenfalls substituiertes $C_{1-3}$-Alkyl, ein gegebenenfalls substituiertes Phenyl, eine gegebenenfalls substituierte heterocyclische Gruppe, Cycloalkenylen oder ein gegebenenfalls substituiertes Heterocycluscarbonylamino ist, worin eine Alkylen-Kette zwischen den Heterocyclus- und Carbonylamino-Struktureinheiten stehen kann, und

(b) einer Gruppe der Formel $R_{10}$—$R_{11}$—CO—, in der $R_{10}$ eine Gruppe der Formel

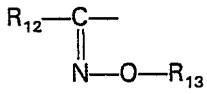

ist, worin $R_{12}$ eine gegebenenfalls substituierte heterocyclische Gruppe oder ein gegebenenfalls substituiertes Phenyl ist, $R_{13}$ Wasserstoff, ein gegebenenfalls substituiertes $C_{2-4}$-Acyl, $C_{1-3}$-Alkyl oder eine Gruppe der Formel $—R_{14}—R_{15}$ ist, worin $R_{14}$ $C_{1-3}$-Alkylen oder $C_{2-3}$-Alkenylen ist und $R_{15}$ Carboxyl, ein davon abgeleiteter Ester oder eine heterocyclische Gruppe ist, und $R_{11}$ eine einfache Bindung oder eine Gruppe der Formel

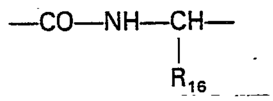

ist, in der $R_{16}$ $C_{1-3}$-Alkyl, ein gegebenenfalls substituiertes Phenyl oder ein gegebenenfalls substituierte heterocyclische Gruppe ist.

3. Verfahren nach Anspruch 1, dadurch gekennzeichnet, daß $R_1$ oder $R_2$ Amino mit einer Schutzgruppe ausgewählt aus den auf dem Gebiet der Peptid-Synthese verwendeten Schutzgruppen ist.

4. Verfahren nach Anspruch 1, dadurch gekennzeichnet, daß die hergestellte Verbindung:
3-Amino-3-methoxy-2-oxoazetidin-1-sulfonsäure;
3-Benzyloxycarboxamido-3-methoxy-2-oxoazetidin-1-sulfonsäure oder
3-Phenylacetamido-3-methoxy-2-oxoazetidin-1-sulfonsäure ist.

5. Verfahren nach Anspruch 1, dadurch gekennzeichnet, daß die hergestellte Verbindung 3-[D-(—)-N-(4-Ethyl-2,3-dioxo-1-piperazinocarbonyl)-phenylglycinamido]-3-methoxy-2-oxoazetidin-1-sulfonsäure ist.

6. Verfahren nach Anspruch 1, dadurch gekennzeichnet, daß die hergestellte Verbindung
3-Amino-2-oxoazetidin-1-sulfonsäure;
3-Benzyloxycarboxamido-2-oxoazetidin-1-sulfonsäure;
3-Phenylacetamido-2-oxoazetidin-1-sulfonsäure;
3-[2-(2-Amino-4-thiazolyl)-2-methoxyiminoacetamido]-2-oxoazetidin-1-sulfonsäure;
3-[D-N-(4-Ethyl-2,3-dioxo-1-piperazinocarbonyl)-phenylglycinamido]-2-oxoazetidin-1-sulfonsäure;
3-[2-(4-Ethyl-2,3-dioxo-1-piperazinocarboxamido)-2-(2-amino-4-thiazolyl)acetamido]-2-oxoazetidin-1-sulfonsäure;
3-[DL-2-(4-Ethyl-2,3-dioxo-1-piperazinocarboxamido)-2-thienylacetamido]-2-oxoazetidin-1-sulfonsäure;
3-[D-2-(4-Ethyl-2,3-dioxo-1-piperazinocarboxamido)-2-thenylacetamido]-2-oxoazetidin-1-sulfonsäure;
3-[D-2-(4-n-Octyl-2,3-dioxo-1-piperazinocarboxamido)-2-phenylacetamido]-2-oxoazetidin-1-sulfonsäure;
3-[2-(4-n-Octyl-2,3-dioxo-1-piperazinocarboxyamido)-2-(2-amino-4-thiazolyl)acetamido]-2-oxoazetidin-1-sulfonsäure;
3-[D-2-{(2-Oxo-3-furfurylidenaminoimidazolidin-1-yl)-carboxamido}-2-phenylacetamido]-2-oxoazetidin-1-sulfonsäure;
3-[DL-2-(4-n-Octyl-2,3-dioxo-1-piperazinocarboxamido)-2-thienylacetamido]-2-oxoazetidin-1-sulfonsäure;
3-[D-2-(4-n-Octyl-2,3-dioxo-1-piperazinocarboxamido)-2-thienylacetamido]-2-oxoazetidin-1-sulfonsäure;
3-[D-2-{[2-Oxo-3-(thiophen-2-aldoimino)-imidazolidin-1-yl]-carboxamido}-2-phenylacetamido]-2-oxoazetidin-1-sulfonsäure und
3-[(2-Oxo-3-furfurylidenaminoimidazolidin-1-yl)carboxamido)-2-thienylacetamido]-2-oxoazetidin-1-sulfonsäure ist.

**Revendications pour les Etats contractants: BE CH DE FR GB IT LI LU NL SE**

1. Un composé répondant à la formule:

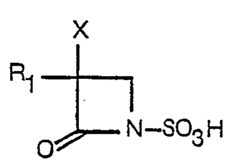

dans laquelle $R_1$ représente un groupe amino, un groupe amino acylé ou un groupe amino protégé et X représente un atome d'hydrogène ou un groupe méthoxy, ou un sel de ce composé, mais à l'exception

du composé dans lequel x représente un groupe méthoxy et $R_1$ représente un groupe amino substitué par un groupe acyle $R_7$—NH—CH($R_9$)—CO—, dans lequel $R_7$ représente un groupe gammaglutamyle et $R_9$ un groupe méthyle.

2. Un composé selon la revendication 1, dans lequel $R_1$ représente un groupe amino acylé par un groupe choisi parmi:

(a) un groupe de formule

$$\begin{array}{l} R_7\text{—NH—}\underset{\displaystyle R_9}{\underset{|}{\text{CH}}}\text{—CO—} \end{array}$$

dans laquelle $R_7$ représente un atome d'hydrogène; le radical d'un amino-acide éventuellement substitué; un groupe protecteur du groupe amino; un groupe de formule $R_8$—$(CH_2)_n$ —CO—, dans laquelle $R_8$ représente un groupe hétérocyclique éventuellement substitué, un groupe phényl éventuellement substitué, un groupe alcoyle en $C_{1-12}$ éventuellement substitué, un groupe phénylthio éventuellement substitué ou un groupe alcoylthio en $C_{1-3}$, n a la valeur 0 ou est un nombre entier de 1 à 4 et le groupe —$(CH_2)_n$— peut être éventuellement substitué; un groupe de formule

$$\begin{array}{l} R_8' \\ \quad \diagdown \\ \qquad \text{N—CO—} \\ \quad \diagup \\ R_8'' \end{array}$$

dans laquelle $R_8'$ et $R_8''$, qui peuvent être identiques ou différents, représentent de l'hydrogène; un groupe alcoyle en $C_{1-3}$, un groupe $(C_{1-3})$alcoyl carbamoyle, un groupe phénylcarbonyle éventuellement substitué ou un groupe sulfo; ou un groupe de formule $R_8'''$—$SO_2$—, dans laquelle $R_8'''$ représente un groupe alcoyle en $C_{1-6}$ éventuellement substitué; et $R_9$ représente de l'hydrogène, un groupe alcoyle en $C_{1-3}$ éventuellement substitué, un groupe phényle éventuellement substitué, un groupe hétérocyclique éventuellement substitué, un groupe cycloalkénylène ou un groupe hétérocyclique éventuellement substitué porté par un groupe carbonylamino et dans lequel une chaîne alkylène peut être interposée entre l'élément hétérocycle et l'élément carbonylamino, et

(b) un groupe de formule $R_{10}$—$R_{11}$—CO—, dans laquelle $R_{10}$ représente un groupe de formule

$$\begin{array}{l} R_{12}\text{—C—} \\ \qquad \| \\ \qquad \text{N—O—}R_{13} \end{array}$$

dans laquelle $R_{12}$ représente un groupe hétérocyclique éventuellement substitué ou un groupe phényle éventuellement substitué, $R_{13}$ représente de l'hydrogène ou un groupe acyle en $C_{2-4}$ éventuellement substitué, un groupe alcoyle en $C_{1-3}$ ou un groupe de formule —$R_{14}$—$R_{15}$—, dans laquelle $R_{14}$ représente un groupe alkylène en $C_{1-3}$ ou un groupe alkénylène en $C_{2-3}$ et $R_{15}$ représente un groupe carboxylique ou un ester de ce groupe ou un groupe hétérocyclique; et $R_{11}$ représente une simple liaison ou un groupe de formule

$$\text{—CO—NH—}\underset{\displaystyle R_{16}}{\underset{|}{\text{CH}}}\text{—}$$

dans laquelle $R_{16}$ représente un groupe alcoyle en $C_{1-3}$, un groupe phényle éventuellement substitué ou un groupe hétérocyclique éventuellement substitué.

3. Un composé selon la revendication 1, dans lequel $R_1$ représente un groupe amino protégé par un groupe choisi parmi les groupes protecteurs mis en jeu dans la synthèse des peptides.

4. Un composé selon la revendication 1, choisi parmi:
l'acide 3-amino-3-méthoxy-2-oxoazétidine-1-sulfonique;
l'acide 3-benzyloxycarboxamido-3-méthoxy-2-oxoazétidine-1-sulfonique et
l'acide 3-phénylacétamido-3-méthoxy-2-oxoazétidine-1-sulfonique.

5. Un composé selon la revendication 1, qui est l'acide 3-[D-(—)-N-(4-éthyl-2,3-dioxo-1-pipérazinocarbonyl)-phénylglycinamido]-3-méthoxy-2-oxoazétidine-1-sulfonique.

6. Un composé selon la revendication 1, choisi parmi:
l'acide 3-amino-2-oxoazétidine-1-sulfonique;
l'acide 3-benzyloxycarboxamido-2-oxoazétidine-1-sulfonique;
l'acide 3-phénylacétamido-2-oxoazétidine-1-sulfonique;
l'acide 3-[2-(2-amino-4-thiazolyl)-2-méthoxyiminoacétamido]-2-oxoazétidine-1-sulfonique;

l'acide 3-[D-N-(4-éthyl-2,3-dioxo-1-pipérazinocarbonyl)-phénylglycinamido]-2-oxoazétidine-1-sulfonique;

l'acide 3-[2-(4-éthyl-2,3-dioxo-1-pipérazinocarboxamido)-2-(2-amino-4-thiazolyl)acétamido]-2-oxoazétidine-1-sulfonique;

l'acide 3-[DL-2-(4-éthyl-2,3-dioxo-1-pipérazinocarboxamido)-2-thiénylacétamido]-2-oxoazétidine-1-sulfonique;

l'acide 3-[D-2-(4-éthyl-2,3-dioxo-pipérazinocarboxamido)-2-thiénylacétamido]-2-oxoazétidine-1-sulfonique;

l'acide 3-[D-2-(4-n-octyl-2,3-dioxo-1-pipérazinocarboxamido)-2-phénylacétamido]-2-oxoazétidine-1-sulfonique;

l'acide 3-[2-(4-n-octyl-2,3-dioxo-pipérazinocarboxamido)-2-(2-amino-4-thiazolyl)acétamido]-2-oxoazétidine-1-sulfonique;

l'acide 3-[D-2-[(2-oxo-3-furfurylidèneaminoimidazolidine-1-yl)-carboxamido]-2-phénylacétamido]-2-oxoazétidine-1-sulfonique;

l'acide 3-[DL-2-(4-n-octyl-2,3-dioxo-1-pipérazinocarboxamido)-2-thiénylacétamido]-2-oxoazétidine-1-sulfonique;

l'acide [D-2-(4-n-octyl-2,3-dioxo-1-pipérazinocarboxamido)-2-thiénylacétamido]-2-oxoazétidine-1-sulfonique;

l'acide [D-2-[[2-oxo-3-(thiophène-2-aldoimino)-imidazolidine-1-yl]-carboxamido]-2-phénylacétamido]-2-oxoazétidine-1-sulfonique; et

l'acide 3-[(2-oxo-3-furfurylidèneaminoimidazolidine-1-yl)carboxamido]-2-thiénylacétamido]-2-oxoazétidine-1-sulfonique;

7. Un procédé de préparation d'un composé selon la revendication 1, répondant à la formule:

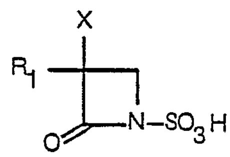

dans laquelle $R_1$ représente un groupe amino, un groupe amino acylé ou un groupe amino protégé et X représente un atome d'hydrogène ou un groupe méthoxy, ou d'un sel de ce composé, dans lequel on effectue la sulfonation d'un composé de formule:

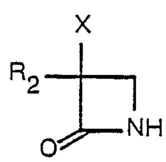

dans laquelle $R_2$ représente un groupe amino acylé ou un groupe amino protégé et X est tel que défini ci-dessus, et, si désiré, on élimine le groupe protecteur du groupe amino, ou on soumet à une acylation le composé ainsi obtenu, répondant à la formule:

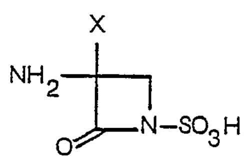

dans laquelle X est tel que défino ci-dessus, et, si désiré, on transforme le composé obtenu en un sel.

8. Une composition pharmaceutique comprenant un composé tel que revendiqué dans l'une quelconque des revendications 1 à 6.

**Revendications pour l'Etat contractant: AT**

1. Un procédé de préparation d'un composé répondant à la formule:

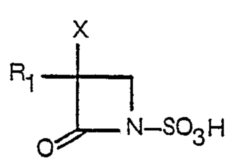

dans laquelle $R_1$ représente un groupe amino, un groupe amino acylé ou un groupe amino protégé et X représente un atome d'hydrogène ou un groupe méthoxy, ou d'un sel de ce composé, dans lequel on effectue la sulfonation d'un composé de formule:

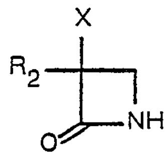

dans laquelle $R_2$ représente un groupe amino acylé ou un groupe amino protégé et X est tel que défini ci-dessus, et, si désiré, on élimine le groupe protecteur du groupe amino, ou on soumet à une acylation le composé ainsi obtenu, répondant à la formule:

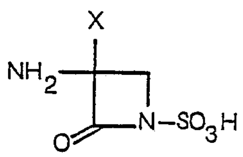

dans laquelle X est tel que défino ci-dessus, et, si désiré, on transforme le composé obtenu en un sel.

2. Un procédé selon la revendication 1, dans lequel $R_1$ ou $R_2$ représente un groupe amino acylé par un groupe choisi parmi:

(a) un groupe de formule

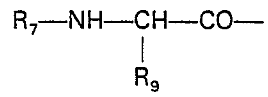

dans laquelle $R_7$ représente un atome d'hydrogène; le radical d'un amino-acide éventuellement substitué; un groupe protecteur du groupe amino; un groupe de formule $R_8$—$(CH_2)_n$ —CO—, dans laquelle $R_8$ représente un groupe hétérocyclique éventuellement substitué, un groupe phényl éventuellement substitué, un groupe alcoyle en $C_{1-12}$ éventuellement substitué, un groupe phénylthio éventuellement substitué ou un groupe alcoylthio en $C_{1-3}$, n a la valeur 0 ou est un nombre entier de 1 à 4 et le groupe —$(CH_2)_n$— peut être éventuellement substitué; un groupe de formule

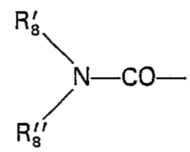

dans laquelle $R_8'$ et $R_8''$, qui peuvent être identiques ou différents, représentent de l'hydrogène; un groupe alcoyle en $C_{1-3}$, un groupe $(C_{1-3})$alcoyl carbamoyle, un groupe phénylcarbonyle éventuellement substitué ou un groupe sulfo; ou un groupe de formule $R_8'''$—$SO_2$—, dans laquelle $R_8'''$ représente un groupe alcoyle en $C_{1-6}$ éventuellement substitué; et $R_9$ représente de l'hydrogène, un groupe alcoyle en $C_{1-3}$ éventuellement substitué, un groupe phényle éventuellement substitué, un groupe hétérocyclique éventuellement substitute, un groupe cycloalkénylène ou un groupe hétérocyclique éventuellement substitué porté par un groupe carbonylamino et dans lequel une chaîne alkylène peut être interposée entre l'élément hétérocycle et l'élément carbonylamino, et

(b) un groupe de formule $R_{10}$—$R_{11}$—CO—, dans laquelle $R_{10}$ représente un groupe de formule

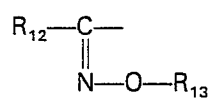

dans laquelle $R_{12}$ représente un groupe hétérocyclique éventuellement substitué ou un groupe phényle éventuellement substitué, $R_{13}$ représente de l'hydrogène ou un groupe acyle en $C_{2-4}$ éventuellement substitué, un groupe alcoyle en $C_{1-3}$ ou un groupe de formule —$R_{14}$—$R_{15}$—, dans laquelle $R_{14}$ représente un groupe alkylène en $C_{1-3}$ ou un groupe alkénylène en $C_{2-3}$ et $R_{15}$ représente un groupe carboxylique ou un ester de ce groupe ou un groupe hétérocyclique; et $R_{11}$ représente une simple liaison ou un groupe de formule

$$-CO-NH-\underset{\displaystyle R_{16}}{\underset{|}{CH}}-$$

dans laquelle $R_{16}$ représente un groupe alcoyle en $C_{1-3}$, un groupe phényle éventuellement substitué ou un groupe hétérocyclique éventuellement substitué.

3. Un procédé selon la revendication 1, dans lequel $R_1$ ou $R_2$ représente un groupe amino protégé par un groupe choisi parmi les groupes protecteurs mis en jeu dans la synthèse des peptides.

4. Un procédé selon la revendication 1, dans lequel le composé obtenu est:
l'acide 3-amino-3-méthoxy-2-oxoazétidine-1-sulfonique;
l'acide 3-benzyloxycarboxamido-3-méthoxy-2-oxoazétidine-1-sulfonique ou
l'acide 3-phénylacétamido-3-méthoxy-2-oxoazétidine-1-sulfonique.

5. Un procédé selon la revendication 1, dans lequel le composé obtenu est
l'acide 3-[D-(—)-N-(4-éthyl-2,3-dioxo-1-pipérazinocarbonyl)-phénylglycinamido]-3-méthoxy-2-oxoazétidine-1-sulfonique.

6. Un procédé selon la revendication 1, dans lequel composé obtenu est:
l'acide 3-amino-2-oxoazétidine-1-sulfonique;
l'acide 3-benzyloxycarboxamido-2-oxoazétidine-1-sulfonique;
l'acide 3-phénylacétamido-2-oxoazétidine-1-sulfonique;
l'acide 3-[2-(2-amino-4-thiazolyl)-2-méthoxyiminoacétamido]-2-oxoazétidine-1-sulfonique;
l'acide 3-[D-N-(4-éthyl-2,3-dioxo-1-pipérazinocarbonyl)-phénylglycinamido]-2-oxoazétidine-1-sulfonique;
l'acide 3-[2-(4-éthyl-2,3-dioxo-1-pipérazinocarboxamido)-2-(2-amino-4-thiazolyl)acétamido]-2-oxoazétidine-1-sulfonique;
l'acide 3-[DL-2-(4-éthyl-2,3-dioxo-1-pipérazinocarboxamido)-2-thiénylacétamido]-2-oxoazétidine-1-sulfonique;
l'acide 3-[D-2-(4-éthyl-2,3-dioxo-pipérazinocarboxamido)-2-thiénylacétamido]-2-oxoazétidine-1-sulfonique;
l'acide 3-[D-2-(4-n-octyl-2,3-dioxo-1-pipérazinocarboxamido)-2-phénylacétamido]-2-oxoazétidine-1-sulfonique;
l'acide 3-[2-(4-n-octyl-2,3-dioxo-pipérazinocarboxamido)-2-(2-amino-4-thiazolyl)acétamido]-2-oxoazétidine-1-sulfonique;
l'acide 3-[D-2-[(2-oxo-3-furfurylidèneaminoimidazolidine-1-yl)-carboxamido]-2-phénylacétamido]-2-oxoazétidine-1-sulfonique;
l'acide 3-[DL-2-(4-n-octyl-2,3-dioxo-1-pipérazinocarboxamido)-2-thiénylacétamido]-2-oxoazétidine-1-sulfonique;
l'acide [D-2-(4-n-octyl-2,3-dioxo-1-pipérazinocarboxamido)-2-thiénylacétamido]-2-oxoazétidine-1-sulfonique;
l'acide [D-2-[[2-oxo-3-(thiophène-2-aldoimino)-imidazolidine-1-yl]-carboxamido]-2-phénylacétamido]-2-oxoazétidine-1-sulfonique; ou
l'acide 3-[(2-oxo-3-furfurylidèneaminoimidazolidine-1-yl)carboxamido]-2-thiénylacétamido]-2-oxoazétidine-1-sulfonique;